# EUROPEAN PATENT SPECIFICATION

(11) **EP 1 528 103 B1**
(45) Date of publication and mention of the grant of the patent: **02.04.2008**
(21) Application number: 04076464.9
(22) Date of filing: 20.02.1996
(51) Int. Cl.: C12N 15/12, C12N 5/10, C07K 14/705, C07K 16/28, G01N 33/577

(54) **'Osteoclast-inhibitory factor' (OCIF) derived proteins and methods for producing the proteins**
'Osteoclast-Inhibitor Faktor' (OCIF) abgeleitete Proteinen und Methoden zu dessen Gewinnung
Protéines dérivées du 'facteur de l'inhibition des octéoclastes' (OCIF) et ses pocédés de production

(30) Priority: 20.02.1995 JP 5497795; 21.07.1995 JP 20750895
(43) Date of publication of application: 04.05.2005
(62) Divisional of application: 96902484.3
(73) Proprietor: Daiichi Sankyo Company, Limited, Chuo-ku Tokyo 103-8426 (JP)
(72) Inventor: Goto, Masaaki, Shimotsuga-gun, Tochigi 329-05 (JP); Tsuda, Eisuke, Shimotsuga-gun, Tochigi 329-05 (JP); Mochizuki, Shin'ichi, Kawachi-gun, Tochigi 329-04 (JP); Yano, Kazuki, Shimotsuga-gun, Tochigi 329-05 (JP); Kobayashi, Fumie, Kawachi-gun, Tochigi 329-11 (JP); Shima, Nobuyuki, Kawachi-gun, Tochigi 329-04 (JP); Yasuda, Hisataka, Kawachi-gun, Tochigi 329-04 (JP); Nakagawa, Nobuaki, Shimotsuga-gun, Tochigi 329-05 (JP); Morinaga, Tomonori, Shimotsuga-gun, Tochigi 321-02 (JP); Ueda, Masatsugu, Kawagoe-shi, Saitama 350-11 (JP); Higashio, Kanji, Kawagoe-shi, Saitama 350 (JP)
(74) Representative: Wakerley, Helen Rachael

(56) References cited:
- WO-A-96/28546
- WO-A-97/23614
- DATABASE EMROD EMBL DATABASES; Accession Number: M59378 28 June 1991 (1991-06-28), GOODWIN R ET AL: "Molecular cloning and expression of the type 1 and type 2 receptors for tumor necrosis factor" XP002077049
- CHAMBERS T ET AL: "Generation of osteoclast-inductive and osteoclastogenic cell lines from the H-2btsA58 transgenic mouse" PROCEEDINGS OF THE NATIONAL ACADEMY OF SCIENCES OF USA., vol. 90, June 1993 (1993-06), pages 5578-5582, XP002077046
- SMITH C A ET AL: "THE TNF RECEPTOR SUPERFAMILY OF CELLULAR AND VIRAL PROTEINS: ACTIVATION, COSTIMULATION, AND DEATH" CELL, vol. 76, 25 March 1994 (1994-03-25), pages 959-962, XP002029050
- TSUDA E ET AL: "Isolation of a novel cytokine from human fibroblasts that specifically inhibits osteoclastogenesis" BIOCHEMICAL AND BIOPHYSICAL RESEARCH COMMUNICATIONS., vol. 234, 8 May 1997 (1997-05-08), pages 137-142, XP002076438
- REDDI A: "Bone morphogenesis and modelling: soluble signals sculpt osteosomes in the solid state" CELL, vol. 89, 18 April 1997 (1997-04-18), pages 159-161, XP002076439
- SIMONET W ET AL: "Osteoprotegerin: a novel secreted protein involved in the regulation of bone density" CELL, vol. 89, 18 April 1997 (1997-04-18), pages 309-319, XP002077048

## Description

### Field of the invention

This invention relates to a protein, osteoclastogenesis inhibitory factor (OCIF), and methods for producing the protein.

### Background of the invention

Human bones are always remodelling by the repeated process of resorption and reconstitution. In the process, osteoblasts and osteoclasts are considered to be the cells mainly responsible for bone formation and bone resorption, respectively. A typical example of disease caused by the progression of abnormal bone metabolism is osteoporosis. The disease is known to be provoked by the condition in which bone resorption by osteoclasts exceeds bone formation by osteoblasts, but the mechanism of osteoporosis has not yet been completely elucidated. Osteoporosis causes pain in the bone and makes the bone fragile, leading to fracture. Since osteoporosis increases the number of bedridden old people, it has become a social issue with the increasing number of old people. Therefore, efficacious drugs for the treatment of the disease are expected to be developed. Bone mass reduction caused by the abnormal bone metabolism is thought to be prevented by inhibiting bone resorption, improving bone formation, or improving the balanced metabolism.

Bone formation is expected to be promoted by stimulating growth, differentiation, or activation of osteoblasts. Many cytokines are reported to stimulate growth or differentation of osteoblasts , i.e. fibroblast growth factor (FGF) (Rodan S.B. et al., Endocrinology vol. 121, p1917, 1987), insulin-like growth factor-I (IGF-I) (Hock J.M. et al., Endocrinology vol. 122, p254, 1988), insulin-like growth factor-II (IGF-II) (McCarthy T. et al., Endocrinology vol. 124, p301, 1989), Activin A (Centrella M. et al., Mol, Cell, Biol. vol. 11, p250, 1991), Vasculotropin (Varonique M et al., Biochem. Biophys. Res. Commun. vol. 199, p380, 1994), and bone morphogenetic protein (BMP) (Yamaguchi, A et al., J. Cell Biol. vol. 113, p682, 1991, Sampath T.K. et al., J. Biol Chem. vol. 267, p20532, 1992, and Knutsen R. et al., Biochem. Biophys. Res. Commun. vol. 194, p1352, 1993.

On the other hand, cytokines which inhibits differentiation and/or maturation of osteoclasts have been paid attention and have been intensively studied. Transforming growth factor-*β* (Chenu C. et al., Proc. Natl. Acad. Sci. USA, vol. 85, p5683, 1988) and interleukin-4 (Kasano K. et al., Bone-Miner., vol. 21, p179, 1993) are found to inhibit the differentiation of osteoclasts. Calcitonin (Bone-Miner., vol.17, p347, 1992), Macrophage colony-stimulating factor (Hattersley G. et al. J. Cell. Physiol. vol. 137, p199, 1988), interleukin-4 (Watanabe, K. et al., Biochem. Biophys. Res. Commun. vol. 172, p1035, 1990), and interferon-γ (Gowen M. et al., J. Bone Miner. Res., vol.1, p469, 1986) are found to inhibit bone resorption by osteoclasts.

These cytokines are expected to be efficacious drugs for improving bone mass reduction by stimulating bone formation and/or by inhibiting bone resorption. The cytokines such as insulin like growth factor-I and bone morphogenetic proteins are now investigated in clinical trials for their effects in treatment of patients with bone diseases. Calcitonin is already used as a drug to care osteoporosis and to diminish pain in osteoporosis.

Examples of drugs now clinically utilized for the treatment of bone diseases and for shortening the treatment period are dihydroxyvitamine D₃, vitamin K₂, calcitonin and its derivatives, hormones such as estradiol, ipriflavon, and calcium preparations . However, these drugs do not provide satisfactory therapeutic effects, and novel drug substances have been expected to be developed. As mentioned, bone metabolism is controlled in the balance between bone resorption and bone formation. Therefore, cytokines which inhibit osteoclast differentiation and/or maturation are expected to be developed as drugs for the treatment of bone diseases such as osteoporosis.

### Disclosure of Invention

This invention was initiated from the view point described above. The purpose of this invention is to offer both a factor termed osteoclastogenesis inhibitory factor (OCIF) and a procedure to produce the factor efficiently.

The inventors have intensively searched for osteoclastogenesis inhibitory factors in human embryonic fibloblast IMR-90 (ATCC CCL186) conditioned medium and have found a novel osteoclastogenesis inhibitory factor (OCIF) which inhibits differentiation and/or maturation of osteoclasts.

The inventors have established a method for accumulating the protein to a high concentration by culturing IMR-90 cells using alumina ceramic pieces as the cell adherence matrices.

The inventors have also established an efficient method for isolating the protein, OCIF, from the IMR-90 conditioned medium using the following sequential column chromatography, ion-exchange, heparin affinity, cibacron-blue affinity, and reverse phase.

The inventors, based on the amino acid sequence of the purified natural OCIF, successfully cloned a cDNA encoding this protein. The inventors established also a procedure to produce this protein which inhibits differentiation of osteoclasts. This invention concerns a protein which is produced by human lung fibroblast cells, has molecular weights in SDS-PAGE of 60 KD in the reducing conditions and 120 KD under the non-reducing conditions, has affinity for both cation-exchange resins and heparin, reduces its activity to inhibit differentiation and maturation of osteoclasts if treated for 10 minutes at 70 °C or for 30 minutes at 56 °C, and lose its activity to inhibit differentiation and maturation of osteoclasts by the treatment for 10 minutes at 90 °C. The amino acid sequence of the protein OCIF which is described in the present invention is clearly different from any of know factors inhibiting formation of osteoclasts.

Herein is described a method to purify OCIF protein, comprising ; (1) culturing human fibroblasts, (2) applying the conditioned medium to a heparin column to obtain the adsorbed fraction, (3) purifying the OCIF protein using a cation-exchange column, (4) purifying the OCIF protein using a heparin affinity column, (5) purifying the OCIF protein using a cibacron blue affinity column, (6) isolating the OCIF protein using reverse-phase column chromatography. Cibacron blue F3GA coupled to a carrier made of synthetic hydrophilic polymers is an example of materials used to prepare Cibacron blue columns. These columns are conventionally called "blue colomns".

Described is a method for accumulating the OCIF protein to a high concentration by culturing human fibroblasts using alumina ceramic pieces as the cell-adherence matrices.

Moreover, the inventors determined the amino acid sequences of the peptides derived from OCIF, designed the primers based on these amino acid sequences, and obtained cDNA fragments encoding OCIF from a cDNA library of IMR-90 cells.

### Detailed description of the invention

The OCIF protein of the present invention can be isolated from human fibroblast conditioned medium with high yield. The procedure to isolate OCIF is based on ordinary techniques for purifying proteins from biomaterials, in accordance with the physical and chemical properties of OCIF protein. For example, concentrating procedure includes ordinary biochemical techniques such as ultrafiltration, lyophylization, and dialysis. Purifying procedure includes combinations of several chromatographic techniques for purifying proteins such as ion-exchange column chromatography, affinity column chromatography, gel filtration column chromatography, hydrophobic column chromatography, reverse phase column chromatography, and preparative gel electrophoresis. The human fibroblast used for production of the OCIF protein is preferably IMR-90. A method for producing the IMR-90 conditioned medium is preferably a process comprising, adhering human embryonic fibroblast IMR-90 cells to alumina ceramic pieces in roller-bottles, using DMEM medium supplemented with 5 % new born calf serum for the cell culture, and cultivating the cells in roller-bottles for 7 to 10 days by stand cultivation. CHAPS (3-[(3-cholamid opropyl)-dimethylammonio]-1-propanesulfonate) is prefarably added to the buffer as a detergent in the purification steps of OCIF protein.

OCIF protein of the instant invention can be initially obtained as a heparin binding basic OCIF fraction by applying the culture medium to a heparin column (Heparin-Sepharose CL-6B, Pharmacia), eluting with 10 mM Tris-HCl buffer, pH 7.5, containing 2 M NaCl, and then by applying the OCIF fraction to a Q · anion-exchange column (HiLoad-Q/FF, Pharmacia), and collecting non-adsorbed fraction. OCIF protein can be purified by subjecting the obtained OCIF fraction to purification on a S · cation-exchange column (HiLoad-S/FF, Pharmacia). a heparin column (Heparin-5PW, TOSOH), Cibacrone Blue column (Blue-5PW, TOSOH), and a reverse-phase column (BU-300 C4, Perkin Elmer) and the material is defined by the previously described properties.

The present invention relates to a method of cloning cDNA encoding the OCIF protein based on the amino acid sequence of natural OCIF and a method of obtaining recombinant OCIF protein that inhibits differentiation and/or maturation of osteoclasts. The OCIF protein is purified according to the method described in the present invention and is treated with endopeptidase (for example, lysylendopeptidase). The amino acid sequences of the peptides produced by the digestion are determined and the mixture of oligonucleotides that can encode each internal amino acid sequence was systhesized. The OCIF cDNA fragment is obtained by PCR (preferably RT-PCR, reverse transcriptase PCR) using the oligonucleotide mixtures described above as primers. The full length OCIF cDNA encoding the OCIF protein is cloned from a cDNA library using the obtained OCIF DNA fragment as a probe. The OCIF cDNA containing the entire coding region is inserted into an expression vector. The recombinant OCIF can be produced by expressing the OCIF cDNA containing the entire coding region in mammalian cells or bacteria.

The present invention relates to the novel proteins OCIF2, OCIF3, OCIF4, and OCIF5 that are variants of OCIF and have the activity described above. These OCIF variants are obtained from the cDNA library constructed with IMR-90 poly(A) + RNA by hybridization using the OCIF cDNA fragment as a probe. Each of the OCIF variant cDNAs containing the entire coding region is inserted into an expression vector. Each recombinant OCIF variant can be produced by expressing each of the OCIF variant cDNAs containing the entire coding region in the conventional hosts. Each recombinant OCIF variant can be purified according to the method described in this invention. Each recombinant OCIF variant has an ability to inhibit osteoclastogenesis.

The present invention further includes OCIF mutants. They are substitution mutants comprising replacement of one cysteine.residue possibly involved in dimer formation with serine residue, and various deletion mutants of OCIF. Substitutions or deletions are introduced into the OCIF cDNA using polymerase chain reaction (PCR) or by restriction enzyme digestion. Each of these mutated OCIF cDNAs is inserted into a vector containing an appropriate promoter for gene expression. The resultant expression vector for each of the OCIF mutants is transfected into eukaryotic cells such as mammalian cells. Each of OCIF mutants can be obtained and purified from the conditioned media of the transfected cells.

In the present invention are described polyclonal antibodies and a method to quantitatively determine OCIF concentration using these polyclonal antibodies.

As antigens (immunogens), natural OCIF obtained from IMR-90 conditioned medium, recombinant OCIF produced by such hosts as microorganisms and eukaryotes using OCIF cDNA, synthetic peptides designed based on the amino acid sequence of OCIF, or peptides obtained from OCIF by partial digestion can be used. Anti-OCIF polyclonal antibodies are obtained by immunizing appropriate mammals with the antigens in combination with adjuvants for immunization if necessary, purifying from the serum by the ordinary purification methods. The anti-OCIF polyclonal antibodies which are labelled with rasioisotopes or enzymes can be used in radio-immunoassay (RIA) system or immunoassay (EIA) system. By using these assay systems, the concentrations of OCIF in biological materials such as blood and ascites and cells-culture medium can be easily determined.

The antibodies in the present invention can be used in radio immunoassay (RIA) or enzyme immunoassay (EIA). By using these assay systems, the concentration of OCIF in biological materials such as blood and ascites can be easily determined.

Also monoclonal antibodies and a method to quantitatively determine OCIF concentration using these monoclonal antibodies are described.

Anti-OCIF monoclonal antibodies can be produced by the conventional method using OCIF as an antigen. Native OCIF obtained from the culture medium of IMR-90 cells and recombinant OCIF produced by such hosts as microorganisms and eukaryotes using OCIF cDNA can be used as antigens. Alternatively, synthesized peptides designed based on the amino acid sequence of OCIF and peptides obtained from OCIF by partial digestion can be also used as antigens. Immunized lymphocytes obtained by immunization of mammals with the antigen or by an in vitro immunization method were fused with myeloma of mammals to obtain hybridoma. The hybridoma clones secreting antibody which recognizes OCIF were selected from the hybridomas obtained by the cell fusion. The desired antibodies can be obtained by cell culture of the selected hybridoma clones. In preparation of hybridoma, small animals such as mice or rats are generally used for immunization. To immunize, OCIF is suitably diluted with a saline solution (0.15 M NaCl), and is intravenously or intraperitoneally administered with an adjuvant to animals for 2 -5 times every 2 -20 days. The immunized animal was killed three days after final immunization, the spleen was taken out and the splenocytes were used as immunized B lymphocytes.

Mouse myeloma cell lines for cell fusion with the immunized B lymphocytes include, for example, p3/x63-Ag8, p3-U1, NS-1, MPC-11, SP-2/0, F0, p3x63 Ag8.653, and S194. Rat R-210 cell line may also be used. Human B lymphocytes are immunized by an in vitro immunization method and are fused with human myeloma cell line or EB virus transformed human B lymphocytes which are used as a parent cell line for cell fusion, to produce human type antibody.

Cell fusion of the immunized B lymphocytes and myeloma cell line is carried out principally by the conventional methods. For example, the method of Koehler G. et al. (Nature 256, 495-497, 1975) is generally used, and also an electric pulse method can be applied to cell fusion. The immunized B lymphocytes and transformed B cells are mixed at conventional ratios and a cell culture medium without FBS containing polyethylene glycol is generally used for cell fusion. The B lymphocytes fused with myeloma cell lines are cultured in HAT selection medium containing FBS to select hybridoma.

For screening of hybridoma producing anti-OCIF antibody, EIA, plaque assay, Ouchterlony, or agglutination assay can be principally used. Among them, EIA is simple and easy to operate with sufficient accuracy and is generally used. By EIA using purified OCIF, the desired antibody can be selected easily and accurately. Thus obtained hybridoma can be cultured by the conventional method of cell culture and frozen for stock if necessary. The antibody can be produced by culturing hybridoma using the ordinary cell culture method or by transplanting hybridoma intraperitoneally to animals. The antibody can be purified by the ordinary purification methods such as salt precipitation, gel filtration, and affinity chromatography. The obtained antibody specifically reacts with OCIF and can be used for determination of OCIF concentration and for purification of OCIF. The antibodies of the present invention recognize epitopes of OCIF and have high affinity to OCIF. Therefore, they can be used for the construction of EIA. By (using) this assay system, the concentration of OCIF in biological materials such as blood and ascites can be easily determined.

The agents used for treating bone diseases that contain OCIF as an effective ingredient are provided by the present invention. Rats were subjected to denervation of left forelimb. Test compounds were administered daily after surgery for 14 days. After 2 weeks treatment, the animals were sacrificed and their forelimbs were dissected. Thereafter bones were tested for mechanical strength by three point bending method. OCIF improved mechanical strength of bone in a dose dependent manner.

The OCIF protein of the invention is useful as a pharmaceutical ingredients for treating or improving decreased bone mass in such as osteoporosis, bone diseases such as rheumatism, osteoarthritis, and abnormal bone metabolism in multiple myeloma. The OCIF protein is also useful as an antigen to establish immunological diagnosis of the diseases. Pharmaceutical preparations containing the OCIF protein as an active ingredients are formulated and can be orally or parenterally administered. The preparation contains the OCIF protein of the present invention as an efficacious ingredient and is safely administered to human and animals. Examples of the pharmaceutical preparations include compositions for injection or intravenous drip, suppositories, nasal preparations, sublingual preparations, and tapes for percutaneous absorption. The pharmaceutical preparation for injection can be prepared by mixing the pharmacologically efficacious amount of OCIF protein and pharmaceutically acceptable carriers. The carriers are vehicles and/or activators, e.g. amino acids, saccharides, cellulose derivatives, and other organic and inorganic compounds which are generally added to active ingredients. When the OCIF protein is mixed with the vehicles and/or activators to prepare injections, pH adjuster, buffer, stabilizer, solubilizing agent, etc. can be added, if necessary.

### Brief description of the figures

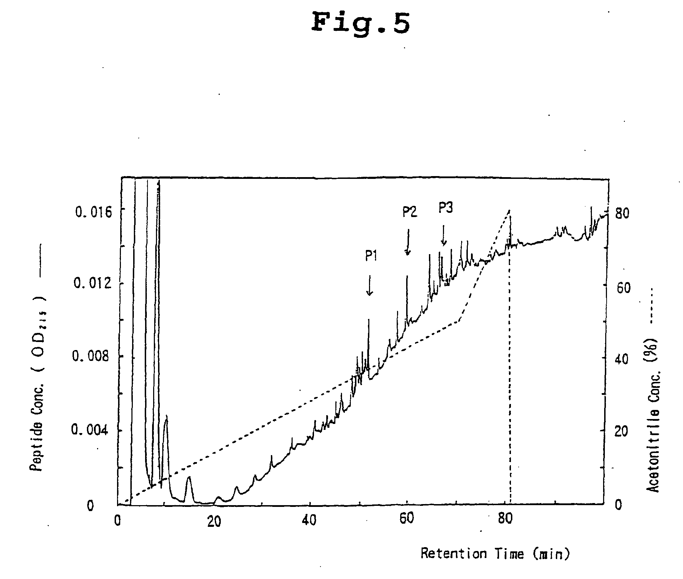

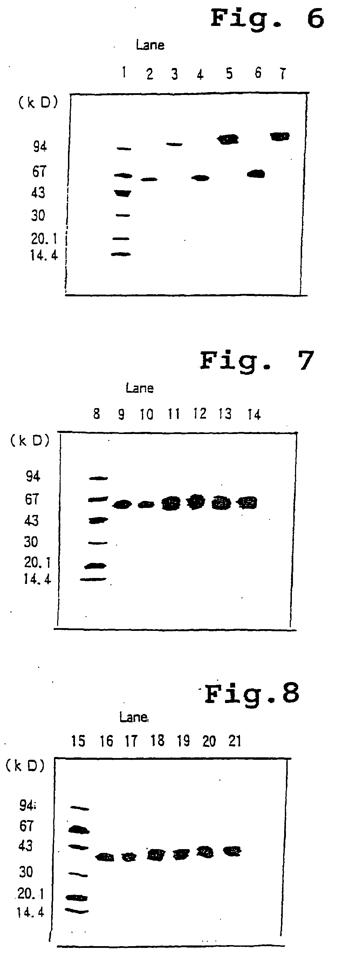

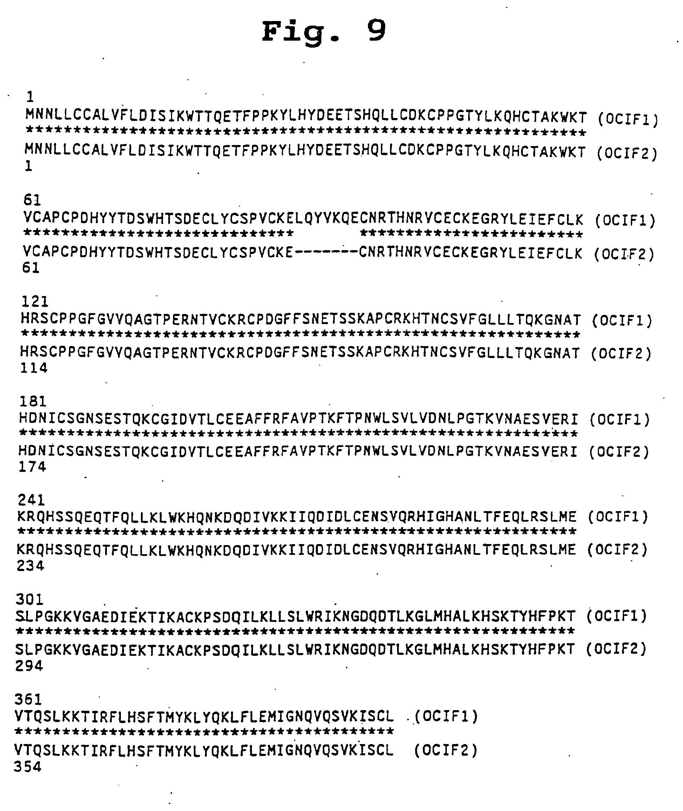

Figure 1 shows the elution pattern of crude OCIF protein (Hiload-Q/FF pass-through fraction ; sample 3) from a Hiload-S/HP column.
Figure 2 shows the elution pattern of crude OCIF protein (heparin-5PW fraction ; sample 5) from a blue-5PW column.
Figure 3 shows the elution pattern of OCIF protein (blue-5PW fraction 49 to 50) from a reverse-phase column.
Figure 4 shows the SDS-PAGE of isolated OCIF proteins under reducing conditions or non-reducing conditions.
   Description of the lanes,
   lane 1,4 ; molecular weight marker proteins
   lane 2,5 ; OCIF protein of peak 6 in figure 3
   lane 3,6 ; OCIF protein of peak 7 in figure 3
Figure 5 shows the elution pattern of peptides obtained by the digestion of pyridyl ethylated OCIF protein digested with lysylendopeptidase, on a reverse-phase column.
Figure 6 shows the SDS-PAGE of isolated natural(n) OCIF protein and recombinant(r) OCIF proteins under non-reducing conditions. rOCIF(E) and rOCIF(C) were produced in 293/EBNA cells and in CHO cells, respectively.
   Description of the lanes,
   lane 1 ; molecular weight marker proteins
   lane 2 ; a monomer type nOCIF protein
   lane 3 ; a dimer type nOCIF protein
   lane 4 ; a monomer type rOCIF(E) protein
   lane 5 ; a dimer type rOCIF(E) protein
   lane 6 ; a monomer type rOCIF(C) protein
   lane 7 ; a dimer type rOCIF(C) protein
Figure 7 shows the SDS-PAGE of isolated natural(n) OCIF proteins and recombinant (r) OCIF proteins under reducing conditions. rOCIF(E) and rOCIF(C) were produced in 293/EBNA cells and in CHO cells, respectively.
   Description of the lanes,
   lane 8 ; molecular weight marker proteins
   lane 9 ; a monomer type nOCIF protein
   lane 10 ; a dimer type nOCIF protein
   lane 11 ; a monomer type rOCIF(E) protein
   lane 12 ; a dimer type rOCIF(E) protein
   lane 13 : a monomer type rOCIF(C) protein
   lane 14 ; a dimer type rOCIF(C) protein
Figure 8 shows the SDS-PAGE of isolated natural(n) OCIF proteins and recombinant(r) OCIF proteins from which N-linked sugar chains were removed under reducing conditions. r0CIF(E) and r0CIF(C) are rOCIF protein produced in 293/EBNA cells and in CHO cells, respectively.
   Description of the lanes,
   lane 15 ; molecular weight marker proteins
   lane 16 ; a monomer type nOCIF protein
   lane 17 ; a dimer type nOCIF protein
   lane 18 ; a monomer type rOCIF(E) protein
   lane 19 ; a dimer type rOCIF(E) protein
   lane 20 ; a monomer type rOCIF(C) protein
   lane 21 ; a dimer type rOCIF(C) protein
Figure 9 shows comparison of amino acid sequences between OCIF and OCIF2.

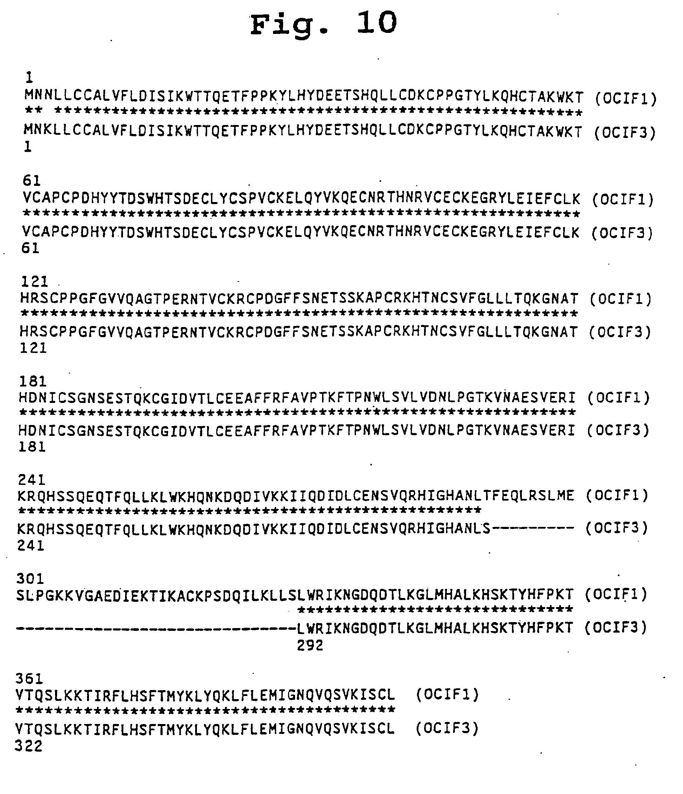

Figure 10 shows comparison of amino acid sequences between OCIF and OCIF3.

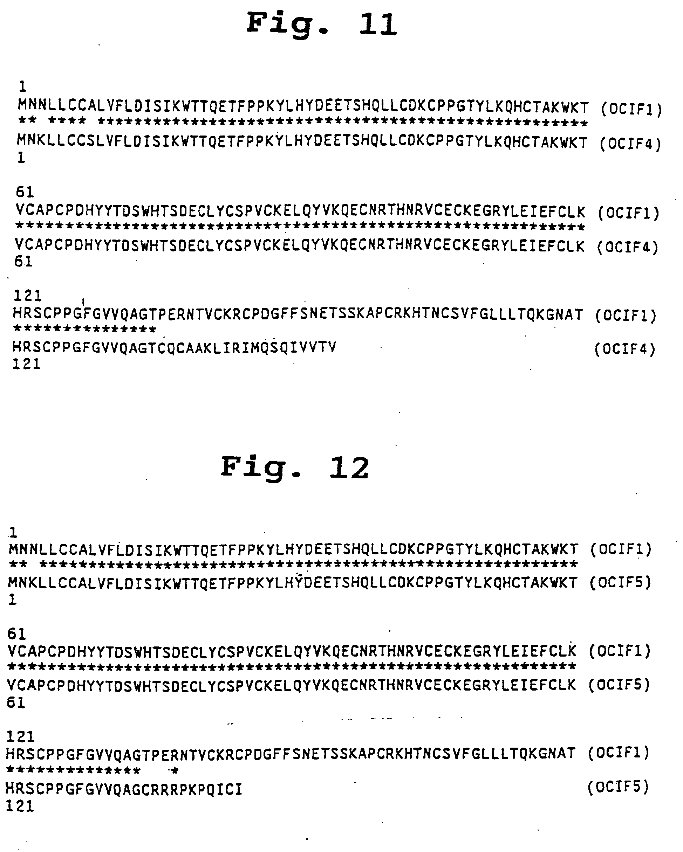

Figure 11 shows comparison of amino acid sequences between OCIF and OCIF4.
Figure 12 shows comparison of amino acid sequences between OCIF and OCIF5.

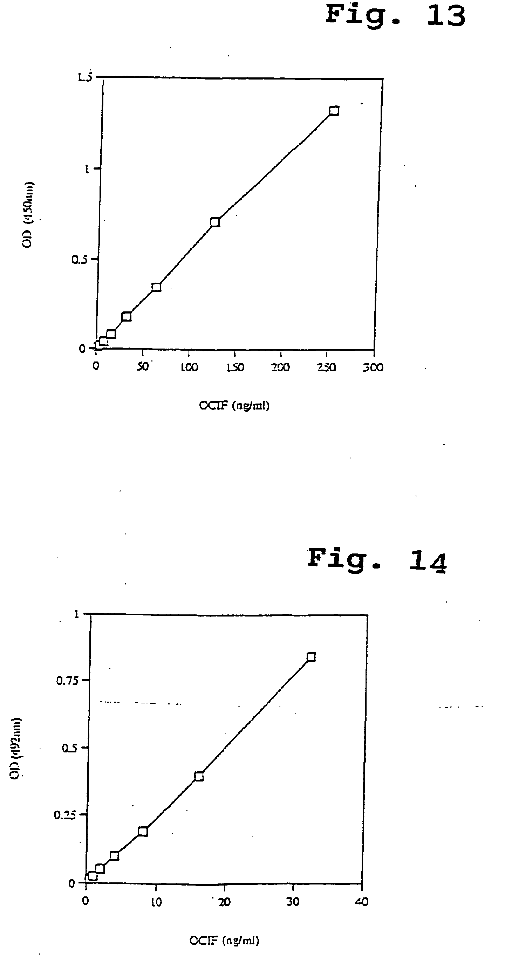

Figure 13 shows standard curve for determination of OCIF protein concentration by an EIA employing anti-OCIF polyclonal antibodies.
Figure 14 shows standard curve for determination of OCIF protein concentration by an EIA employing anti-OCIF monoclonal antibodies.

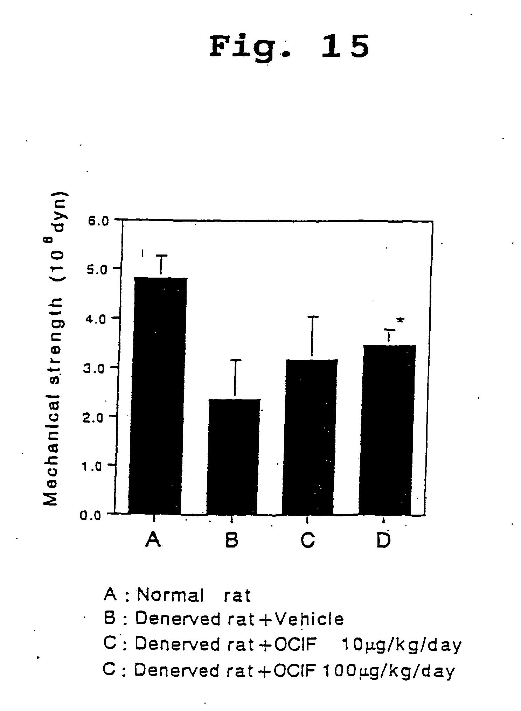

Figure 15 shows the effect of rOCIF protein on osteoporosis.

### Best Mode for Carrying Out the Invention

The present invention will be further explained by the following examples,

### EXAMPLE 1

### Preparation of a conditioned medium of human fibroblast IMR-90

Human fetal lung fibroblast IMR-90 (ATCC-CCL186) cells were cultured on alumina ceramic pieces (80 g) (alumina: 99.5%, manufactured by Toshiba Ceramic K.K.) in DMEM medium (manufactured by Gibco BRL Co.) supplemented with 5% CS and 10mM HEPES buffer (500 ml/roller bottle) at 37°C under the presence of 5% CO₂ for 7 to 10 days using 60 roller bottles (490 cm², 110 x 171mm, manufactured by Coning Co.)in static culture. The conditioned medium was harvested, and a fresh medium was added to the roller bottles. About 30L of IMR-90 conditioned medium per batch culture was obtained. The conditioned medium was designated as sample 1.

### EXAMPLE 2

### Assay method for osteoclast development inhibitory activity

Osteoclast development inhibitory activity was assayed by measuring tartrate-resistant acid phosphatase(TRAP) activity according to the methods of M. Kumegawa et. al (Protein · Nucleic Acid · Enzyme, vol. 34 p999, 1989) and N. Takahashi et. al (Endocrynology, vol. 122, p1373, 1988) with modifications. Briefly, bone marrow cells obtained from 17 day-old mouse were suspended in α-MEM (manufactured by GIBCO BRL Co.) containing 10% FBS, 2x10⁻⁸M of activated vitamin D₃, and each test sample, and were inoculated to each well of 96-well plate at a cell density of 3x10⁵ cells/0.2 ml/well. The plates were incubated for 7 days at 37°C in humidified 5%CO₂. Cultures were further continued by replacing 0. 16 ml of old medium with the same volume of fresh medium on day 3 and day 5 after starting cultivation. On day 7, after washing the plates with phosphate buffered saline, cells were fixed with ethanol/acetone (1:1) for 1 min. at room temperature, and then osteoclast development was tested by determining for phosphatase activity using a kit (Acid Phosphatase, Leucocyte, Catalog No.387-A, manufactured by Sigma Co.). The decrease of TRAP positive cells was taken as an indication of OCIF activity.

### EXAMPLE 3

### Purification of OCIF

### i) Heparin Sepharose CL-6B column chromatography

The 90L of IMR-90 conditioned medium (sample 1) was filtrated with 0.22 µ membrane filter (hydrophilic Milidisk, 2000 cm², Milipore Co.), and was divided into three portions. Each portion (30 1) was applied to a heparin Sepharose CL-6B column (5 x 4.1 cm, Pharmacia Co.) equilibrated with 10mM Tris-HCl containing 0.3M NaCl, pH 7.5. After washing the column with 10mM Tris-HCl, pH 7.5 at a flow rate of 500 ml/hr., heparin Sepharose CL-6B adsorbent protein fraction was eluted with 10mM Tris-HCl, pH 7.5, containing 2M NaCl. The fraction was designated as sample 2.

### ii) HiLoad-Q/FF column chromatography

The heparin Sepharose-adsorbent fraction (sample 2) was dialyzed against 10mM Tris-HCl, pH 7.5, supplemented with CHAPS to a final concentration of 0.1%, incubated at 4 °C overnight, and divided into two portions. Each portion was then applied to an anion-exchange column (HiLoad-Q/FF, 2.6 x 10 cm, Pharmacia Co.) which was equilibrated with 50mM Tris-HCl, 0.1% CHAPS, pH 7.5 to obtain a non-adsorbent fraction (1000 ml). The fraction was designated as sample 3.

### iii) HiLoad-S/HP column chromatography

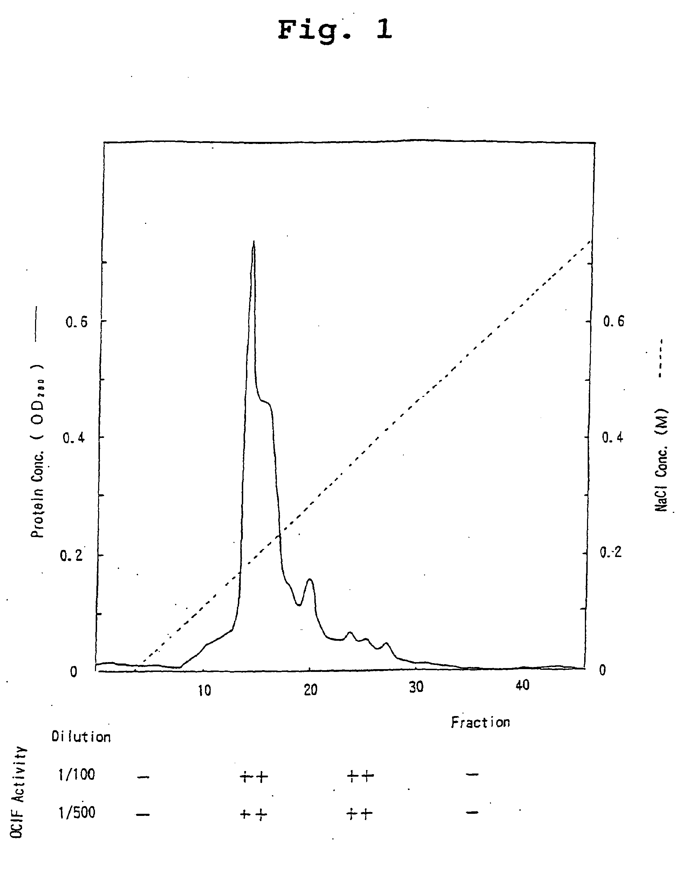

The HiLoad-Q non-adsorbent fraction (sample 3) was applied to a cation-exchange column (HiLoad-S/HP, 2.6 x 10 cm, Pharmacia Co.) which was equilibrated with 50 mM Tris-HCl, 0.1% CHAPS, pH 7.5. After washing the column with 50 mM Tris-HCl, 0.1% CHAPS, pH 7.5, the adsorbed protein was eluted with linear gradient from 0 to 1 M NaCl at a flow rate of 8 ml/min for 100 min. and fractions (12 ml) were collected. Each ten fractions from number 1 to 40 was pooled to form one portion. Each 100 µl of the four portions was tested for OCIF activity. OCIF activity was observed in fractions from 11 to 30 (as shown in Figure 1). The fractions from 21 to 30 which had higher specific activity were collected and was designated as sample 4.

### iv) Heparin-5PW affinity column chromatography

One hundred and twenty ml of HiLoad-S fraction from 21 to 30 (sample 4) was diluted with 240 ml of 50 mM Tris-HCl, 0.1% CHAPS, pH 7.5, and applied to heparin-5PW affinity column (0.8 x 7.5 cm, Tosoh Co.) which was equilibrated with 50mM Tris-HCl, 0.1% CHAPS, pH 7.5. After washing the column with 50mM Tris-HCl, 0.1% CHAPS, pH 7.5, the adsorbed protein was eluted with linear gradient from 0 to 2M NaCl at a flow rate of 0.5ml/min for 60 min. and fractions (0. 5 ml) were collected. Fifty µl was removed from each fraction to test for OCIF activity. The active fractions, eluted with 0.7 to 1.3M NaCl was pooled and was designated as sample 5.

### v) Blue 5PW affinity column chromatography

Ten ml of sample 5 was diluted with 190 ml of 50mM Tris-HC1, 0. 1% CHAPS, pH 7. 5 and applied to a blue-5PW affinity column, (0. 5x5 cm, Tosoh Co.) which was equilibrated with 50mM Tris-HCl, 0.1% CHAPS, pH 7.5. After washing the column with 50mM Tris-HCl, 0. 1% CHAPS, pH7.5, the adsorbed protein was eluted with a 30 ml linear gradient from 0 to 2M NaCl at a flow rate of 0.5 ml/min., and fractions (0.5 ml) were collected. Using 25 µl of each fraction, OCIF activity was evaluated. The fractions number 49 to 70, eluted with 1.0-1.6M NaCl had OCIF activity.

### vi) Reverse phase column chromatography

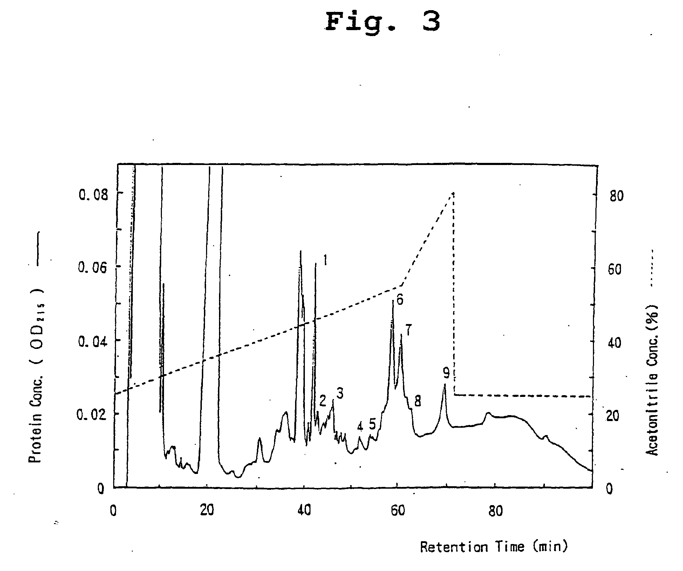

The blue 5PW fraction obtained by collecting fractions from 49 to 50 was acidified with 10µl of 25% TFA and applied to a reverse phase C4 column (BU-300, 2.1x220mm, manufactured by Perkin-Elmer) which was equilibrated with 0.1% of TFA and 25% of acetonitrile. The adsorbed protein was eluted with linear gradient from 25 to 55% acetonitrile at a flow rate of 0.2 ml/min. for 60 min., and each protein peak was collected (Fig. 3). One hundred µl of each peak fraction was tested for OCIF activity, and peak 6 and the peak 7 had OCIF activity. The result was shown in Table 1.

**Table 1**

| OCIF activity eluted from reverse phase C4 column | | | | |
|---|---|---|---|---|
| Sample | Dilution | | | |
| | 1/40 | 1/120 | 1/360 | 1/1080 |
| Peak 6 | ++ | ++ | + | - |
| Peak 7 | ++ | + | - | - |

| | | | | |
|---|---|---|---|---|
| [ ++ means OCIF activity inhibiting osteoclast development more than 80%, + means OCIF activity inhibiting osteoclast development between 30% and 80%, and - means no OCIF activity.] | | | | |

### EXAMPLE 4

### Molecular weight of OCIF protein

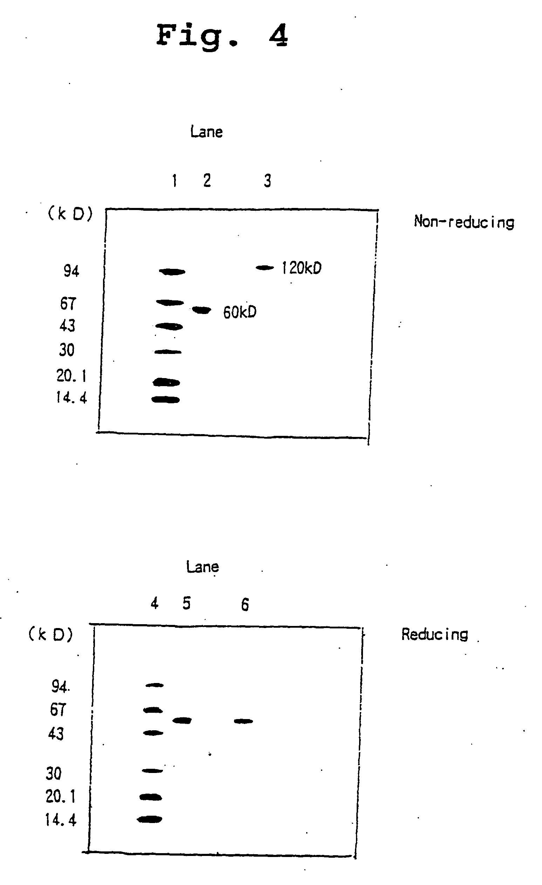

The two protein peaks (6 and 7) with OCIF activity were subjected to SDS-polyacrylamide gel electrophoresis under reducing and non-reducing conditions. Briefly, 20µl of each peak fraction was concentrated under vacuum and dissolved in 1.5µl of 10mM Tris-HCl, pH 8, 1mM EDTA, 2. 5% SDS, 0.01% bromophenol blue, and incubated at 37°C overnight under non-reducing conditions or under reducing conditions (with 5% of 2-mercaptoethanol). Each 1.0 µl of sample was then analyzed by SDS-polyacrylamide gel electrophoresis with a gradient gel of 10-15% acrylamide (Pharmacia Co.) and an electrophoresis-device (Fast System, Pharmacia Co.). The following molecular weight marker proteins were used to calculate molecular weight : phosphorylase b (94 kD), bovine serum albumin (67 kD), ovalbumin (43 kD), carbonic anhydrase (30 kD), trypsin inhibitor (20.0 kD), and lactalbumin (14.4 kD). After electrophoresis, protein bands were visualized by silver stain using Phast Silver Stain Kit. The results were shown in Fig. 4.

A protein band with an apparent 60 KD was detected in the peak 6 protein under both reducing and non-reducing conditions. A protein band with an apparent 60 KD was detected under reducing conditions and a protein band with an apparent 120 KD was detected under non-reducing conditions in the peak 7 protein. Therefore, the protein of peak 7 was considered to be a homodimer of the protein of peak 6.

### EXAMPLE 5

### Thermostability of OCIF

Twenty µl of sample from the blue-5PW fractions 51 and 52 was diluted to 30µl with 10 mM phosphate buffered saline, pH 7.2, and incubated for 10 min. at 70°C or 90 °C, or for 30 min. at 56°C. The heat-treated samples were tested for OCIF activity. The results were shown in Table 2.

**Table 2**

| Thermostability of OCIF | | | |
|---|---|---|---|
| Sample | Dilution | | |
| | 1/300 | 1/900 | 1/2700 |
| untreated | ++ | + | - |
| 70°C, 10 min | + | - | - |
| 56°C, 30 min | + | - | - |
| 90°C, 10 min | - | - | - |

| | | | |
|---|---|---|---|
| [ ++ means OCIF activity inhibiting osteoclast development more than 80%, +means OCIF activity inhibiting osteoclast development between 30% and 80%, and - means no OCIF activity.] | | | |

### EXAMPLE 6

### Internal amino acid sequence of OCIF protein

Each 2 fractions (1 ml) from No. 51-70 of blue-5PW fraction was acidified with 10 µl of 25% TFA, and was applied to a reverse phase C4 column (BU-300, 2.1x220mm, manufactured by Perkin-Elmer Co.) equilibrated with 25% of acetonitrile containing 0.1 % TFA. The adsorbed protein was eluted with a 12 ml linear gradient of 25 to 55% acetonitrile at a flow rate of 0.2 ml/min, and the protein fractions corresponding to peak 6 and peak 7 were collected, respectively. The protein of each peak was applied to a protein sequencer (PROCISE 494, Perkin-Elmer Co.). However, the N-terminal sequence of the protein of each peak could not be analyzed. Therefore, N-terminal of the protein of each peak was considered to be blocked. So, internal amino acid sequences of these proteins were analyzed.

The protein of peak 6 or peak 7 purified by C4-HPLC was concentrated by centrifugation and pyridilethylated under reducing conditions. Briefly, 50 µl of 0. 5 M Tris-HCl, pH 8.5, containing 100µg of dithiothreitol, 10mM EDTA, 7 M guanidine-HCl, and 1% CHAPS was added to each samples, and the mixture was incubated overnight in the dark at a room temperature. Each the mixture was acidified with 25% TFA (a final concentration 0.1%) and was applied to a reversed phase C4 column (BU-300, 2.1x30mm, Perkin-Elmer Co.) equilibrated with 20 % acetonitrile containing 0.1 % TFA. The pyridil-ethylated OCIF protein was eluted with a 9 ml linear gradient from 20 to 50% acetonitrile at a flow rate of 0.3 ml/min, and each protein peak was collected. The pyridil-ethyrated OCIF protein was concentrated under vacuum , and dissolved in 25µl of 0. 1 M Tris-HCl, pH 9, containing 8 M Urea, and 0. 1 % Tween 80. Seventy three µl of 0.1 M Tris-HCl, pH 9, and 0.02 µg of lysyl endopeptidase (Wako Pure Chemical, Japan) were added to the tube, and incubated at 37 °C for 15 hours. Each digest was acidified with 1 µl of 25% TFA and was applied to a reverse phase C8 column (RP-300, 2.1x220mm, Perkin-Elmer Co.) equilibrated with 0.1% TFA.
The peptide fragments were eluted from the column with linear gradient from 0 to 50 % acetonitrile at a flow rate of 0.2 ml/min for 70 min., and each peptide peak was collected. Each peptide fragment (P1 - P3) was applied to the protein sequencer. The sequences of the peptides were shown in Sequence Numbers 1 - 3, respectively.

### EXAMPLE 7

### Determination of nucleotide sequence of the OCIF cDNA

### i) Isolation of poly(A) + RNA from IMR-90 cells

About 10 ug of poly(A) + RNA was isolated from 1x10⁸ cells of IMR-90 by using Fast Track mRNA isolation kit (Invitrogen) according to the manufacturer's instructions.

### ii) Preparation of mixed primers

The following two mixed primers were synthesized based on the amino acid sequences of two peptides (peptide P2 and peptide P3, sequence numbers 2 and 3, respectively). All the oligonucleotides in the mixed primers No. 2F can code for the amino acid sequence from the sixth residue, glutamine (Gln) to the twelfth residue, leucine (Leu), in peptide P2. All the oligonucleotides in the mixed primers No. 3R can code for the amino acid sequence from the sixth residue, histidine (His), to the twelfth residue, lysine (Lys), in peptide P3. The sequences of the mixed primers No. 2F and No. 3R were shown in Table 3.

**Table 3**

| |
|---|
| No. 2F |
| |
| No. 3R |
| |

### iii) Amplification of OCIF cDNA fragment by PCR (Polymerase chain reaction)

First strand cDNA was generated using Superscript II cDNA synthesis kit (Gibco BRL) and 1 ug of poly(A) + RNA obtained in the example 7-i) according to the manufacturer's instructions. The DNA fragment encoding OCIF was obtained by PCR using the cDNA template and the primers shown in EXAMPLE 7-ii).
PCR was performed with the conditions as follows;

| | |
|---|---|
| 10X Ex Taq Buffer (Takara Shuzo) | 5 ul |
| 2.5 mM solution of dNTPs | 4 ul |
| cDNA solution | 1 ul |
| Ex Taq (Takara Shuzo) | 0.25 ul |
| sterile distilled water | 29.75 ul |
| 40 uM solution of primers No. 2F | 5 ul |
| 40 uM solution of primers No. 3R | 5 ul |

The components of the reaction were mixed in a microcentrifuge tube. An initial denaturation step at 95 °C for 3 min was followed by 30 cycles of denaturation at 95°C for 30 sec annealing at 50 °C for 30 sec and extention at 70 °C for 2min. After the amplification, final extention step was performed at 70 °C for 5min. The size of PCR products were determined on a 1. 5 % agarose gel electrophoresis. About 400 bp OCIF DNA fragment was obtained.

### EXAMPLE 8

### Cloning of the OCIF cDNA fragment amplified by PCR and determination of its DNA sequence

The OCIF cDNA fragment amplified by PCR in EXAMPLE 7-iii) was inserted in the plasmid, pBluescript II SK⁻ using DNA ligation kit ver. 2 (Takara Shuzo) according to the method by Marchuk, D. et al. (Nucleic Acids Res., vol 19, p1154, 1991). E. coli. DH5 α(Gibco BRL) was transformed with ligation mixture. The transformants were grown and a plasmid containing the OCIF cDNA (about 400 bp) was purified using the commonly used method. This plasmid was called pBSOCIF. The sequence of OCIF cDNA in pBSOCIF was determined using Taq Dye Deoxy Terminater Cycle Sequencing kit (Perkin Elmer). The size of the OCIF cDNA is 397 bp. The OCIF cDNA encodes an amino acid sequence containing 132 residues. The amino acid sequences of the internal peptides (peptide P2 and peptide P3, sequence number 2 and 3, respectively) that were used to design the primers were found at N- or C- terninal side in the amino acid sequence of the 132 amino acid polypeptide predicted by the 397 bp OCIF cDNA. In addition, the amino acid sequence of the internal peptide P1 (sequence number 1) was also found in the predicted amino acid sequence of the polypeptide. These data show that the 397 bp OCIF cDNA is a portion of the full length OCIF cDNA.

### EXAMPLE 9

### Preparation of the DNA probe

The 397 bp OCIF cDNA was prepared according to the conditions described in EXAMPLE 7-iii). The OCIF cDNA was subjected to a preparative agarose gel electrophoresis. The OCIF cDNA was purified from the gel using QIAEX gel extraction kit (QIAGEN), labeled with [α³²P]dCTP using Megaprime DNA labeling system (Amersham) and used to select a phage containing the full length OCIF cDNA.

### EXAMPLE 10

### Preparation of the cDNA library

cDNA was generated using Great Lengths cDNA synthesis kit (Clontech), oligo (dT) primer, [α³²P]dCTP and 2.5 ug of poly (A) + RNA obtained in the example 7-i) according to the manufacturer's instructions. EcoRI-SalI-NotI adaptor was ligated to the cDNA. The cDNA was separated from the free adaptor and unincorporated free [α³²P]dCTP. The purified cDNA was precipitated with ethanol and dissolved in 10 ul of TE buffer (10 mMTris-HCl (pH8.0), 1 mM EDTA). The cDNA with the adaptor was inserted in λZAP EXPRESS vector (Stratagene) at EcoRI site. The recombinant λZAP EXPRESS phage DNA containing the cDNA was in vitro packaged using Gigapack gold II packaging extract (Stratagene) and recombinant λZAP EXPRESS phage library was prepared.

### EXAMPLE 11

### Screening of recombinant phage

Recombinant phages obtained in EXAMPLE 10 were infected to E. Coli, XL1-Blue MRF' (Stratagene) at 37 °C for 15 min.. The infected E.coli cells were added to NZY medium containing 0.7 % agar at 50°C and plated on the NZY agar plates. After the plates were incubated at 37 °C overnight, Hybond N (Amersham) were placed on the surface of plates containing plaques. The membranes were denatured in the alkali solution, neutralized, and washed in 2xSSC according to the standard protocol. The phage DNA was immobilized on the membranes using UV Crosslink (Stratagene). The membranes were incubated in the hybridization buffer (Amersham) containing 100 µg/ml salmon sperm DNA at 65°C for 4 hours and then incubated at 65 °C overnight in the same buffer containing 2x10⁵ cpm/ml denatured OCIF DNA probe. The membranes were washed twice with 2xSSC and twice with a solution containing 0.1xSSC and 0.1% SDS at 65 °C for 10 min each time. The positive clones were purified by repeating the screening twice. The purified λZAP EXPRESS phage clone containing about 1.6 kb DNA insert was used in the experiments described below. This phage was called λ OCIF. The purified λ OCIF and the infected into E. Coli XL1-Blue MRF' (Stratagene) according to a protocol of λZAP EXPRESS cloning kit (Stratagene). The culture broth of infected XL1-Blue MRF' was prepared. Purified IOCIF and ExAssist helper phage (Stratagene) were co-infected into E. coli strain XL-1 blue MRF' according to the protocol supplied with the kit. The culture broth of the co-infected XL-1 blue MRF' was added to a culture of E. coli strain XLOR (Stratagene) to transform them. Thus we obtained a Kanamycin-resistant transformant harboring a plasmid designated pBKOCIF which is a pBKCMV (Stratagene) vector containing the 1.6 kb insert fragment. The transformant including the plasmid containing about 1.6 kb OCIF cDNA was obtained by picking up the kanamycin-resistant colonies. The plasmid was called pBKOCIF. The transformant has been deposited to National Institute of Bioscience and Human-Technology (NIBH), Agency of Industrial Science and Tecnology as "FERM BP-5267" as pBK/01F10. A national deposit (Accession number, FERM P-14998) was transfered to the international deposit, on October 25, 1995 according to the Budapest treaty. The transformant pBK/O1F10 was grown and the plasmid pBKOCIF was purified according to the standard protocol.

### EXAMPLE 12

### Determination of the nucleotide sequence of OCIF cDNA containing the full coding region.

The nucleotide sequence of OCIF cDNA obtained in EXAMPLE 11 was determined using Taq Dye Deoxy Terminater Cycle Sequencing kit (Perkin Elmer). The primers used were T3, T7 primers (Stratagene) and synthetic primers designed according to the OCIF cDNA sequence. The sequences of these primers are shown in sequence numbers 16 to 29. The nucleotide sequence of the OCIF cDNA is shown in sequence number 6 and the amino acid sequence predicted by the cDNA sequence is shown in sequence number 5.

### EXAMPLE 13

### Production of recombinant OCIF by 293/EBNA cells

### i) Construction of the plasmid for expressing OCIF cDNA

pBKOCIF containing about 1.6 kb OCIF cDNA was prepared as described in EXAMPLE 11, and digested with restriction enzymes, BamHI and XhoI. The OCIF cDNA insert was cut out, separated by an agarose gel electrophoresis, and purified using QIAEX gel extraction kit (QIAGEN). The purified OCIF cDNA insert was ligated using DNA ligation kit ver. 2 (Takara Shuzo) to the expression vector pCEP4 (Invitrogen) digested with restriction enzymes, BamHI and XhoI. E. coli. DH5α (Gibco BRL) was transformed with the ligation mixture. The transformants were grown and the plasmid containing the OCIF cDNA (about 1.6 kb) was purified using QIAGEN column (QIAGEN). The expression plasmid pCEPOCIF was precipitated with ethanol, and dissolved in sterile distilled water was used in the expreriments described below.

### ii) Transient expression of OCIF cDNA and analysis of the biological activity

Recombinant OCIF was produced using the expression plasmid, pCEPOCIF prepared in EXAMPLE 13-i) according to the method described below. 8x10⁵ cells of 293/EBNA (Invitrogen) were inoculated in each well of the 6-well plate using IMDM containing 10 % fetal calf serum (Gibco BRL). After the cells were incubated for 24 hours, the culture medium was removed and the cells were washed with serum free IMDM. The expression plasmid, pCEPOCIF and lipofectamine (Gibco BRL) were diluted with OPTI-MEM (Gibco BRL) and were mixed, and added to the cells in each well according to the manufacture's instructions. Three µg of pCEPOCIF and 12 µl of lipofectamine were used for each transfection. After the cells were incubated with pCEPOCIF and lipofectamine for 38 hours, the medium was replaced with 1 ml of OPTI-MEM. After the transfected cells were incubated for 30 hours, the conditioned medium was harvested and used for the biological assay. The biological activity of OCIF was analysed according to the method described below. Bone marrow cells obtained from mice, 17 days-old, were suspended in α-MEM (manufactured by GIBCO BRL Co.) containing 10% FBS, 2x10⁻⁸M activated vitamin D₃ and each test sample, and were inoculatd and cultured for 7 days at 37°C in humidified 5%CO₂ as described in EXAMPLE 2. During incubation, 160 µl of old medium in each well was replaced with the same volume of the fresh medium containing test sample diluted with 1x10⁻⁸M of activated vitamin D₃ and α-MEM containing FBS on day 3 and day 5. On day 7, after washing the wells with phosphate buffered saline, cells were fixed with ethanol/acetone (1:1) for 1 min. and then osteoclast development was tested using acid phosphatase activity mesuring kit (Acid Phosphatase, Leucocyte, Catalog No. 387-A, Sigma Co.). The decrease of the number of TRAP positive cells was taken as an OCIF activity. As result, the conditioned medium showed the same OCIF activity as natural OCIF protein from IMR-90 conditioned medium (Table 4).

**Table 4**

| OCIF activity of 293/EBNA conditioned medium. | | | | | | | |
|---|---|---|---|---|---|---|---|
| Cultured Cell | Dilution | | | | | | |
| | 1/20 | 1/40 | 1/80 | 1/160 | 1/320 | 1/640 | 1/1280 |
| OCIF expression vector transfected | ++ | ++ | ++ | ++ | ++ | + | - |
| vector transfected | - | - | - | - | - | - | - |
| untreated | - | - | - | - | - | - | - |

| | | | | | | | |
|---|---|---|---|---|---|---|---|
| [ ++ ; OCIF activity inhibiting osteoclast development more than 80%, + ; OCIF activity inhibiting osteoclast development between 30% and 80%, and - ; no OCIF activity. ] | | | | | | | |

### iii) Isolation of recombinant OCIF protein from 293/EBNA-conditioned medium

293/EBNA-conditioned medium (1.8 1) obtained by cultivating the cells described in example 13-ii) was supplemented with 0.1 % of CHAPS and filtrated with 0.22 µm membrane filter (Steribecs GS, Milipore Co.). The conditioned medium was applied to 50 ml of a heparin Sepharose CL-6B column (2.6 x 10 cm, Pharmacia Co.) equilibrated with 10mM Tris-HCl, pH 7.5. After washing the column with 10mM Tris-HCl, pH 7.5, the adsorbed protein was eluted from the column with linear gradient from 0 to 2 M NaCl at a flow rate of 4 ml/min for 100 min. and fractions (8 ml) were collected. Using 150 µl of each fraction, OCIF activity was assayed according to the method described in EXAMPLE 2. OCIF active fraction (112 ml) eluted with approximately 0.6 to 1.2 M NaCl was obtained.

One hundred twelve ml of the active fraction was diluted to 1000 ml with 10 mM Tris-HCl, 0.1% CHAPS, pH 7.5, and applied to a heparin affinity column (heparin-5PW, 0.8 x 7.5 cm, Tosoh Co.) equilibrated with 10mM Tris-HCl, 0. 1% CHAPS, pH 7.5. After washing the column with 10mM Tris-HCl, 0. 1% CHAPS, pH 7.5, the adsorbed protein was eluted from the column with linear gradient from 0 to 2 M NaCl at a flow rate of 0.5ml/min for 60 min., and fractions (0.5 ml) were collected. Four µl of each fraction was analyzed by SDS-polyacrylamide gel electrophoresis under reducing and non-reducing conditions as described in EXAMPLE 4. On SDS-PAGE under reducing conditions, a single band of rOCIF protein with an apparent 60 KD was detected in fractions from 30 to 32, under non-reducing conditions, bands of rOCIF protein with an apparent 60 KD and 120 KD were also detected in fractions from 30 to 32. The isolated rOCIF fraction from 30 to 32 was designated as recombinant OCIF derived from 293/EBNA (rOCIF(E)). 1.5 ml of the rOCIF(E) (535 µg/ml) was obtained when determined by the method of Lowry using bovine serum albumin as a standard protein.

### EXAMPLE 14

### Production of recombinant OCIF using CHO cells

### i) Construction of the plasmid for expressing OCIF

pBKOCIF containing about 1.6 kb OCIF cDNA was prepared as described in EXAMPLE 11, and digested with restriction enzymes, SalI and EcoRV. About 1.4 kb OCIF cDNA insert was separated by an agarose gel electrophoresis, and purified from the gel using QIAEX gel extraction kit (QIAGEN). The expression vector, pcDL-SR α296 (Molecular and Cellular Biology, vol 8, p466, 1988) was digested with restriction enzymes, PstI and KpnI. About 3.4 kb of the expression vector fragment was cut out, separated by agarose gel electrophoresis, and purified from the gel using QIAEX gel extraction kit (QIAGEN). The ends of the purified OCIF cDNA insert and the expression vector fragment were blunted using DNA blunting kit (Takara Shuzo). The purified OCIF cDNA insert and the expression vector fragment were ligated using DNA ligation kit ver. 2 (Takara Shuzo). E.coli. DH5a α (Gibco BRL) was transformed with the ligation mixture. The transformant containing the OCIF expression plasmid, pSRα0CIF was obtained.

### ii) Preparation of expression plasmid

The transformant containing the OCIF expression plasmid, pSR αOCIF preprared in the example 13-i) and the transformant containing the mouse DHFR expression plasmid, pBAdDSY shown in WO92/01053 were grown according to the standard method. Both plasmids were purified by alkali treatment, polyethylene glycol precipitation, and cesium chrolide density gradient ultra centrifugation according to method of Maniatis et al. (Molecular cloning, 2nd edition).

### iii) Adaptation of CHOdhFr- cells to the protein free medium

CHOdhFr- cells (ATCC, CRL 9096) were cultured in IMDM containing 10 % fetal calf serum. The cells were adapted to EX-CELL 301 (JRH Biosciecnce) and then adapted to EX-CELL PF CHO (JRH Biosciecnce) according to the manufacture's instructions.

### iv) Transfection of the OCIF expression plasmid, and the mouse DHFR expression plasmid, to CHOdhFr- cells.

CHOdhFr- cells prepared in EXAMPLE 14-iii) were transfected by electroporation with pSR a OCIF and pBAdDSV prepared in EXAMPLE 14-ii). 200 µg of pSRαOCIF and 20 µg of pBAdDSV were dissolved under sterile conditions in 0.8 ml of IMDM (Gibco BRL) containing 10 % fetal calf serum CG. 2x10⁷ cells of CH0dhFr- were suspended in 0.8 ml of this medium. The cell suspension was transfered to a cuvette (Bio Rad) and the cells were transfected by electroporation using gene pulser (Bio Rad) under condition of 360 V and 960 µF. The suspension of electroporated cells was transferred to T-flasks (Sumitomo Bakelite) containing 10 ml of EX-CELL PF-CHO, and incubated in the CO₂ incubator for 2 days. Then the transfected cells were inoculated in each well of a 96 well plate (Sumitomo Bakelite) at a density of 5000 cells/well and cultured for about 2 weeks. The transformants expressing DHFR are selected since EX-CELL PF-CHO does not contain nucleotides and the parental cell line CHO dhFr- can not grow in this medium. Most of the transformants expressing DHFR express OCIF since the OCIF expression plasmid was used ten times as much as the mouse DHFR expression plasmid. The transformants whose conditioned medium had high OCIF activity were selected among the transformants expressing DHFR according to the method described in EXAMPLE 2. The transformants that express large amounts of OCIF were cloned by limiting dilution. The clones whose conditioned medium had high OCIF activity were selected as described above and the transformant expressing large amount of OCIF, 5561, was obtained.

### v) Production of recombinant OCIF

To produce recombinant OCIF (rOCIF), EX-CELL 301 medium (3 1) in a 3 1-spiner flask was inoculated with the clone (5561) at a cell-density of 1x10⁵ cells/ml. The 5561 cells were cultured in a spiner flask at 37°C for 4 to 5 days. When the concentration of the 5561 cells reached to 1x10⁶ cells/ml, about 2.7 1 of the conditioned medium was harvested. Then about 2.7 1 of EX-CELL 301 was added to the spiner flask and the 5561 cells were cultured repeatedly. About 20 1 of the conditioned medium was harvested using the three spiner flasks.

### vi) Isolation of recombinant OCIF protein from CHO cells-conditioned medium

CHOcells-conditioned medium (1.0 1) described in EXAMPL 14-v) was supplemented with 1.0 g of CHAPS and filtrated with 0.22 µm membrane filter (Steribecks GS, Milipore Co.). The conditioned medium was applied to a heparin Sepharose-FF column (2.6 x 10 cm, Pharmacia Co.) equilibrated with 10 mM Tris-HCl, pH 7.5. After washing the column with 10 mM Tris-HCl, 0.1 % CHAPS, pH 7.5, the adsorbed protein was eluted from the column with linear gradient from 0 to 2 M NaCl at a flow rate of 4 ml/min for 100 min. and fractions (8 ml) were collected. Using 150µl of each fraction, OCIF activity was assayed according to the method described in EXAMPLE 2. Active fraction (112 ml) eluted with approximately 0.6 to 1.2 M NaCl was obtained.

The 112 ml of active fraction was diluted to 1200 ml with 10 mM Tris-HCl, 0.1% CHAPS, pH 7.5, and applied to a affinity column (blue-5PW, 0. 5 x 5.0 cm, Tosoh Co.) equilibrated with 10 mM Tris-HCl, 0.1% CHAPS, pH 7. 5. After washing the column with 10 mM Tris-HCl, 0. 1% CHAPS, pH 7. 5, the adsorbed protein was eluted from the column with linear gradient from 0 to 3 M NaCl at a flow rate of 0.5ml/min for 60 min., and fractions (0.5 ml) were collected. Four µl of each fraction was subjected to SDS-polyacrylamide gel electrophoresis under reducing and non-reducing conditions as described in EXAMPLE 4. On SDS-PAGE under reducing conditions, a single band of rOCIF protein with apparent 60 KD was detected in fractions 30 to 38, under non-reducing conditions, bands of rOCIF protein with apparent 60 KD and 120 KD were also detected in fractions 30 to 38. The isolated rOCIF fraction, 30 to 38, was designated as purified recombinant OCIF derived from CHO cells (rOCIF(C)). 4.5 ml of the rOCIF(C) (113 µg/ml) was obtained when determined by the method of Lowry using bovine serum albumin as a standard protein.

### EXAMPLE 15

### Determination of N-terminal amino acid sequence of rOCIFs

Each 3 µg of the isolated rOCIF(E) and rOCIF(C) was adsorbed to polyvinylidene difluoride (PVDF) membranes with Prospin (PERKIN ELMER Co.). The membranes were washed with 20 % ethanol and the N-terminal amino acid sequences of the adsorbed proteins were analyzed by protein sequencer (PROCISE 492, PERKIN ELMER Co.). The determined N-terminal amino acid sequence is shown in sequence No. 7.

The N-terminal amino acid of rOCIF(E) and rOCIF(C) was the 22th amino acid of glutamine from Met as translation starting point, as shown in sequence number 5. The 21 amino acids from Met to Gln were identified as a signal peptide. The N-terminal amino acid sequence of OCIF isolated from IMR-90 conditioned medium was undetectable. Accordingly, the N-terminal glutamine of OCIF may be blocked by converting from glutamine to pyroglutamine within culturing or purifing.

### EXAMPLE 16

### Biological activity of recombinant(r) OCIF and natural(n) OCIF

### i) Inhibition of vitamin D₃ induced osteoclast formation from murine bone marrow cells

Each the rOCIF(E) and nOCIF sample was diluted with α-MEM (GIBCO BRL Co.) containing 10% FBS and 2x10⁻⁸M of activated vitamin D₃ (a final concentration of 250 ng/ml). Each sample was serially diluted with the same medium, and 100 µl of each diluted sample was added to each well in 96-well plates. Bone marrow cells obtained from mice, 17 days-old, were inoculated at a cell density of 3x10⁵ cells/100µl/ well to each well in 96-well plates and cultured for 7 days at 37°C in humidified 5%CO₂. On day 7, the cells were fixed and stained with a acid phosphatase mesuring kit (Acid Phosphatase, Leucocyte, No387-A, Sigma) according to the method described in EXAMPLE 2. The decrease of acid phosphatase activity (TRAP) was taken as OCIF activity. The decrease of acid phosphatase-positive cells was evaluated by solubilizing the pigment of dye and measuring absorbance. In detail, 100 µl of a mixture of 0.1 N NaOH and dimethylsulfoxide (1:1) was added to each well and the well was vibrated to solubilize the dye. After solubilizing the dye completely, an absorbance of each well was measured at 590 nm subtracting the absorbance at 490 nm using microplate reader (Immunoreader NJ-2000, InterMed). The microplate reader was adjusted to 0 absorbance using a well with monolayered bone marrow cells which was cultured in the medium without activated vitamin D₃. The decrease of TRAP activity was expressed as a percentage of the control absorbance value (=100%) of the solubilized dye from wells with bone marrow cells which were cultured in the absence of OCIF. The results are shown in Table 5.

**Table 5**

| Inhibition of vitamin D3-induced osteoclast formation from murine bone marrow cells | | | | | | |
|---|---|---|---|---|---|---|
| OCIF concentration(ng/ml) | 250 | 125 | 63 | 31 | 16 | 0 |
| rOCIF (E) | 0 | 0 | 3 | 62 | 80 | 100 |
| nOCIF | 0 | 0 | 27 | 27 | 75 | 100 (%) |

Both nOCIF and rOCIF(E) inhibited osteoclast formation in a dose dependent manner in the concentration of 16 ng/ml or higher

### ii) Inhibition of vitamin D3-induced osteoclast formation in co-cultures of stromal cells and mouse spleen cells.

Effect of OCIF on osteoclast formation induced by Vitamin D₃ in co-cultures of stromal cells and mouse spleen cells was tested according to the method of N. Udagawa et al. (Endocrinology, vol. 125, p1805-1813, 1989). In detail, each of rOCIF(E), rOCIF(C), and nOCIF sample was serially diluted with α-MEM (GIBCO BRL Co.) containing 10% FBS, 2x10⁻⁸M of activated vitamin D₃, and 2x10⁻⁷M dexamethasone, and 100µl of each the diluted samples was added to each well in 96 well-microwell plates. Murine bone marrow-derived stromal ST2 cells (RIKEN Cell Bank RCB0224) ; 5x10³ cells per 100 µl of α-MEM containing 10% FBS, and spleen cells from ddy mice, 8 weeks-old, ; 1x10⁵ cells per 100 µl in the same medium, were inoculated to each well in 96-well plates and cultured for 5 days at 37°C in humidified 5%CO₂. On day 5, the cells were fixed and stained with a kit for acid phosphatase (Acid Phosphatase, Leucocyte, No387-A, Sigma). The decrease of acid phosphatase-positive cells was taken as OCIF activity. The decrease of acid phosphatase-positive cells was evaluated according to the method described in EXAMPLE 16-i). The results are shown in Table 6 ; rOCIF(E) and rOCIF(C), and Table 7 ; rOCIF(E) and nOCIF.

**Table 6**

| Inhibition of osteoclast formation in co-cultures of stromal cells and mouse spleen cells. | | | | | |
|---|---|---|---|---|---|
| OCIF concentration(ng/ml) | 50 | 25 | 13 | 6 | 0 |
| rOCIF(E) | 3 | 22 | 83 | 80 | 100 |
| rOCIF(C) | 13 | 19 | 70 | 96 | 100 (%) |

**Table 7**

| Inhibition of osteoclast formation in co-cultures of stromal cells and mouse spleen cells. | | | | |
|---|---|---|---|---|
| OCIF concentration(ng/ml) | 250 | 63 | 16 | 0 |
| rOCIF(E) | 7 | 27 | 37 | 100 |
| rOCIF(C) | 13 | 23 | 40 | 100 (%) |

nOCIF, rOCIF(E) and rOCIF(C) inhibited osteoclast formation in a dose dependent manner in the concentration of 6 - 16 ng/ml or higher

### iii) Inhibition of PTH-induced osteoclast formation from murine bone marrow cells.

Effect of OCIF on osteoclast formation induced by PTH was tested according to the method of N. Takahashi et al. (Endocrinology, vol. 122, p1373-1382, 1988). In detail, each the rOCIF(E) and nOCIF sample (125 ng/ml) was serially diluted with α-MEM (manufactured by GIBCO BRL Co.) containing 10% FBS and 2x10⁻⁸M PTH, and 100µl of each the diluted samples was added to 96 well-plates. Bone marrow cells from ddy mice, 17 days-old, at a cell density of 3x10⁵ cells per 100µl of α-MEM containing 10% FBS were inoculated to each well in 96-wells plates and cultured for 5 days at 37°C in humidified 5%CO₂. On day 5, the cells were fixed with ethanol/aceton (1:1) for 1 min. at room temperature and stained with a kit for acid phosphatase (Acid Phosphatase, Leucocyte, No387-A, Sigma) according to the method described in EXAMPLE 2. The decrease of acid phosphatase-positive cells was taken as OCIF activity. The decrease of acid phosphatase-positive cells was evaluated according to the method described in EXAMPLE 16-i). The results are shown in Table 8.

**Table 8**

| Inhibition of PTH-induced osteoclast formation from murine bone marrow cells. | | | | | | |
|---|---|---|---|---|---|---|
| OCIF concentration(ng/ml) | 125 | 63 | 31 | 16 | 8 | 0 |
| rOCIF(E) | 6 | 58 | 58 | 53 | 88 | 100 |
| nOCIF | 18 | 47 | 53 | 56 | 91 | 100 |

nOCIF and rOCIF(E) inhibited osteoclast formation in a dose dependent manner in the concentration of 16 ng/ml or higher

### iv) Inhibition of IL-11-induced osteoclast formation

Effect of OCIF on osteoclast formation induced by IL-11 was tested according to the method of T. Tamura et al. (Proc. Natl. Acad. Sci. USA, vol. 90, p11924-11928, 1993). In detail, each rOCIF(E) and nOCIF sample was serially diluted with α-MEM (GIBCO BRL Co.) containing 10% FBS and 20 ng/ml IL-11 and 100 µl of each the diluted sample was added to each well in 96-well plates. Newborn mouse calvaria-derived pre-adipocyte MC3T3-G2/PA6 cells (RIKEN Cell Bank RCB1127) ; 5x10³ cells per 100µl of α-MEM containing 10% FBS, and spleen cells from ddy mouse, 8 weeks-old, ; 1x10⁵ cells per 100 µl in the same medium, were inoculated to each well in 96-well plates and cultured for 5 days at 37 °C in humidified 5%CO₂. On day 5, the cells were fixed and stained with a kit for acid phosphatase (Acid Phosphatase, Leucocyte, No387-A, Sigma). Acid phosphatase positive cells were counted under microscope and a decrease of the cell numbers was taken as OCIF activity. The results are shown in Table 9.

**Table 9**

| OCIF concentration (ng/ml) | 500 | 125 | 31 | 7.8 | 2.0 | 0.5 | 0 |
|---|---|---|---|---|---|---|---|
| nOCIF | 0 | 0 | 1 | 4 | 13 | 49 | 31 |
| rOCIF (E) | 0 | 0 | 1 | 3 | 10 | 37 | 31 |

Both nOCIF and rOCIF(E) inhibited osteoclast formation in a dose dependent manner in the concentration of 2 ng/ml or higher

The results shown in Table 4-8 indicated that OCIF inhibits all the vitamin D₃, PTH, and IL-11-induced osteoclast formations at almost the same doses. Accordingly, OCIF would be able to be used for treatment of the different types of bone disorders with decreased bone mass, which are caused by different substances which induce bone resorption.

### EXAMPLE 17

### Isolation of monomer-type OCIF and dimer-type OCIF

Each rOCIF(E) and rOCIF(C) sample containing 100 µg of OCIF protein, was supplemented with 1/100 volume of 25 % trifluoro acetic acid and applied to a reverse phase column (PROTEIN-RP, 2.0x250 mm, YMC Co.) equilibrated with 30 % acetonitrile containing 0.1 % trifluoro acetic acid. OCIF protein was eluted from the column with linear gradient from 30 to 55 % acetonitrile at a flow rate of 0.2 ml/min for 50 min. and each OCIF peak was collected. Each the monomer-type OCIF peak fraction and dimer-type OCIF peak fraction was lyophilized, respectively.

### EXAMPLE 18

### Determination of molecular weight of recombinant OCIFs

Each 1 µg of the isolated monomer-type and dimer-type nOCIF purified using reverse phase column according to EXAMPLE 3-iv) and each 1 µg of monomer-type and dimer-type rOCIF described in EXAMPLE 17 was concentrated under vaccum, respectively. Each sample was incubated in the buffer for SDS-PAGE, subjected to SDS-polyacrylamide gel electrophoresis, and protein bands on the gel were stained with silver according to the method described in EXAMPLE 4. Results of electrophoresis under non-reducing conditions and reducing conditions are shown in Figure 6 and Figure 7.

A protein band with an apparent molecular weight of 60 KD was detected in each monomer-type OCIF sample, and a protein band with an apparent molecular weight of 120 KD was detected in each dimer-type OCIF sample in non-reducing conditions. A protein band with an apparent molecular weight of 60 KD was detected in each monomer-type OCIF sample under reducing conditions. Accordingly, molecular weights of monomer-type nOCIF from IMR-90 cells, rOCIF from 293/EBNA cells and rOCIF from CHO cells were almost the same. Molecular weights of dimer-type nOCIF from IMR-90 cells, rOCIF from 293/EBNA cells, and rOCIF from CHO cells were also the same.

### EXAMPLE 19

### Remove N-linked Oligosaccharide chain and Mesuring molecular weight of natural and recombinant OCIF

Each sample containing 5µg of the isolated monomer-type and dimer-type nOCIF purified using reverse phase column according to EXAMPLE 3-iv) and each sample containing 5 µg of monomer-type and dimer-type rOCIF described in EXAMPLE 17 were concentrated under vaccum. Each sample was dissolved in 9.5 µl of 50 mM sodium phosphate buffer, pH 8.6, containing 100 mM 2-mercaptoethanol, supplemented with 0.5 µl of 250 U/ml N-glycanase (Seikagaku kogyo Co.) and incubated for one day at 37 °C. Each sample was supplemented with 10 µl of 20 mM Tris-HCl, pH 8.0 containing 2 mM EDTA, 5 % SDS, and 0.02 % bromo-phenol blue and heated for 5 min at 100 °C. Each 1 µ1 of the samples was subjected to SDS-polyacrylamide gel electrophoresis, and protein bands on the gel were stained with silver as described in EXAMPLE 4. The patterns of electrophoresis are shown in Figure 8.

An apparent molecular weight of each the deglycosylated nOCIF from IMR-90 cells, rOCIF from CHO cells, and rOCIF from 293/EBNA cells was 40 KD under reducing conditions. An apparent molecular weight of each untreated nOCIF from IMR-90 cells, rOCIF from 293/EBNA cells, and rOCIF from CHO cells was 60 KD under reducing conditions. Accordingly, the results indicate that the OCIF proteins are glycoproteins with N-linked sugar chains.

### EXAMPLE 20

### Cloning of OCIF variant cDNAs and determination of their DNA squences

The plasmid pBKOCIF, which is inserted OCIF cDNA to pBKCMV (Stratagene), was obtained from one of some purified positive phage as in example 10 and 11. And more, during the screening of the cDNA library with the 397 bp OCIF cDNA probe, the transformants containing plasmids whose insert sizes were different from that of pBKOCIF were obtained. These transformants containing the plasmids were grown and the plasmids were purified according to the standard method. The sequence of the insert DNA in each plasmid was determined using Taq Dye Deoxy Terminater Cycle Sequencing kit (Perkin Elmer). The used primers were T3, T7 primers (Stratagene) and synthetic primers prepared based on the nucleotide sequence of OCIF cDNA. There are four OCIF variants (OCIF2, 3, 4, and 5) in addition to OCIF. The nucleotide sequence of OCIF2 is shown in the sequence number 8 and the amino acid sequence of OCIF 2 predicted by the nucleotide sequence is shown in the sequence number 9. The nucleotide sequence of OCIF3 is shown in the sequence number 10 and the amino acid sequence of OCIF3 predicted by the nucleotide sequence is shown in the sequence number 11. The nucleotide sequence of OCIF4 is shown in the sequence number 12 and the amino acid sequence of OCIF4 predicted by the nucleotide sequence is shown in the sequence number 13. The nucleotide sequence of OCIF5 is shown in the sequence number 14 and the amino acid sequence of OCIF5 predicted by the nucleotide sequence is shown in the sequence number 15. The structures of OCIF variants are shown in Figures 9 to 12 and are described in brief below.

### OCIF2

OCIF2 cDNA has a deletion of 21 bp from guanine at nucleotide number 265 to guanine at nucleotide number 285 in OCIF cDNA (sequence number 6). Accordingly OCIF2 has a deletion of 7 amino acids from glutamic acid (Glu) at amino acid number 68 to glutamine (Gln) at amino acid number 74 in OCIF (sequence number 5).

### OCIF3

OCIF3 cDNA has a point mutation at nucleotide number 9 in OCIF cDNA (sequence number 6) where cytidine is replaced with guanine. Accordingly OCIF3 has a mutation and asparagine (Asn) at amino acid number -19 in OCIF (sequence number 5) is replaced with lysine (Lys). The mutation seems to be located in the signal sequence and have no essential effect on the secreted OCIF3. OCIF3 cDNA has a deletion of 117 bp from guanine at nucleotide number 872 to cytidine at nucleotide number 988 in OCIF cDNA (sequence number 6).
Accordingly OCIF3 has a deletion of 39 amino acids from threonine (Thr) at amino acid number 270 to leucine (Leu) at amino acid number 308 in OCIF (sequence number 5).

### OCIF4

OCIF4 cDNA has two point mutations in OCIF cDNA (sequence number 6). Cytidine at nucleotide number 9 is replaced with guanine and guanine at nucleotide number 22 is replaced with thymidine in OCIF cDNA (sequence number 6).
Accordingly OCIF4 has two mutations. Asparagine (Asn) at amino acid number -19 in OCIF (sequence number 5) is replaced with lysine (Lys), and alanine (Ala) at amino acid number -14 is replaced with serine (Ser). These mutations seem to be located in the signal sequence and have no essential effect on the secreted OCIF4.
OCIF4 cDNA has about 4 kb DNA, which is the intron 2 of OCIF gene, inserted between nucleotide number 400 and nucleotide number 401 in OCIF cDNA (sequence number 6). The open reading frame stops in intron 2.
Accordingly OCIF4 has an additional novel amino acid sequence containing 21 amino acids after alanine (Ala) at amino acid number 112 in OCIF (sequence number 5).

### OCIF5

OCIF5 cDNA has a point mutation at nucleotide number 9 in OCIF cDNA (sequence number 6) where cytidine is replaced with guanine.
Accordingly OCIF5 has a mutation and asparagine (Asn) at amino acid number -19 in OCIF (sequence number 5) is replaced with lysine (Lys). The mutation seems to be located in the signal sequence and have no essential effect on the secreted OCIF5.
OCIF5 cDNA has the latter portion (about 1.8 kb) of intron 2 between nucleotide number 400 and nucleotide number 401 in OCIF cDNA (sequence number 6). The open reading frame stops in the latter portion of intron 2. Accordingly OCIF5 has an additional novel amino acid sequence containing 12 amino acids after alanine (Ala) at amino acid number 112 in OCIF (sequence number 5).

### EXAMPLE 21

### Production of OCIF variants

### i) Construction of the plasmid for expressing OCIF variants

The plasmid containing OCIF2 or OCIF3 cDNA was obtained as described in EXAMPLE 20 and called pBKOCIF2 and pBKOCIF3, respectively. pBKOCIF2 and pBKOCIF3 were digested with restriction enzymes, BamHI and XhoI. The OCIF2 and OCIF3 cDNA inserts were separated by agarose gel electrophoresis, and purified from the gel using QIAEX gel extraction kit (QIAGEN). The purified OCIF2 and OCIF3 cDNA inserts were individually ligated using DNA ligation kit ver. 2 (Takara Shuzo) to the expression vector pCEP4 (Invitrogen) that had been digested with restriction enzymes, BamHI and XhoI. E. coli. DH5α (Gibco BRL) was transformed with the ligation mixture.
The plasmid containing OCIF4 cDNA was obtained as described in EXAMPLE 20 and called pBKOCIF4. pBKOCIF4 was digested with restriction enzymes, SpeI and XhoI (Takara Shuzo). The OCIF4 cDNA insert was separated by an agarose gel electrophoresis, and purified from the gel using QIAEX gel extraction kit (QIAGEN). The purified OCIF4 cDNA insert was ligated using DNA ligation kit ver. 2 (Takara Shuzo) to the expression vector pCEP4 (Invitrogen) that had been digested with restriction enzymes,-NheI and XhoI (Takara Shuzo). E. coli. DH5 α (Gibco BRL) was transformed with the ligation mixture.
The plasmid containing OCIF5 cDNA was obtained as described in EXAMPLE 20 and was called pBKOCIF5. pBKOCIF5 was digested with restriction enzyme, HindIII (Takara Shuzo). The 5' portion of the coding region in the OCIF5 cDNA insert was separated by agarose gel electrophoresis, and purified from the gel using QIAEX gel extraction kit (QIAGEN). The OCIF expression plasmid, pCEPOCIF, obtained in EXAMPLE 13-i) was digested with restriction enzyme, HindIII (Takara Shuzo). The 5' portion of the coding region in the OCIF cDNA was removed. The rest of the plasmid that contains pCEP vector and the 3'portion of the coding region of OCIF cDNA was called pCEPOCIF-3'. pCEPOCIF-3' was separated by an agarose gel electrophoresis, and purified from the gel using QIAEX gel extraction kit (QIAGEN). The OCIF5 cDNA HindIII fragment and pCEPOCIF-3' were ligated using DNA ligation kit ver. 2 (Takara Shuzo). E.coli. DH5 α (Gibco BRL) was transformed with the ligation mixture.
The obtained transformants were grown at 37 °C overnight and the OCIF variants expression plasmids (pCEPOCIF2, pCEPOCIF3, pCEPOCIF4, and pCEPOCIF5) were purified using QIAGEN column (QIAGEN). These OCIF-variants-expression plasmids were precipitated with ethanol, dissolved in sterile distilled water, and used in the expreriments described below.

### ii) Transient expression of OCIF variant cDNAs and analysis of the biological activity of recombinant OCIF variants.

Recombinant OCIF variants were produced using the expression plasmid, pCEPOCIF2, pCEPOCIF3, pCEPOCIF4, and pCEPOCIF5 prepared as described in EXAMPLE 21-i) according to the method described in EXAMPLE 13-ii). The biological activities of recombinant OCIF variants were analysed. The results were that these OCIF variants (OCIF2, OCIF3, OCIF4, and OCIF5) had a weak activity.

### EXAMPLE 22

### Preparation of OCIF mutants

### i) Construction of a plasmid vector for subcloning cDNAs encoding OCIF mutants

The plasmid vector (5 µg) described in EXAMPLE 11 was digested with restriction enzymes Bam HI and Xho I ( Takara Shuzo). The digested DNA was subjected to a preparative agarose gel electrophoresis. DNA fragment with an approximate size of 1.6 kilobase pairs (kb) that contained the entire coding sequence for OCIF was purified from the gel using QIAEX gel extraction kit (QIAGEN). The purified DNA was dissolved in 20 µl of sterile distilled water. This solution was designated DNA solution 1. p Bluescript II SK + (3 µg) (Stratagene) was digested with restriction enzymes Bam HI and Xho I (Takara Shuzo). The digested DNA was subjected to preparative agarose gel electrophoresis. DNA fragment with an approximate size of 3.0 kb was purified from the gel using QIAEX DNA extraction kit (QIAGEN). The purified DNA was dissolved in 20 µl of sterile distilled water. The solution was designated DNA solution 2. One microliter of DNA solution 2, 4 µl of DNA solution 1 and 5 µl of ligation buffer I of DNA ligation kit ver. 2 (Takara Shuzo) were mixed and incubated at 16 °C for 30 min. (The ligation mixture was used for the transformation of E. coli in a manner described below). Conditions for transformation of E. coli were as follows. One hundred microliters of competent E. coli DH5 α cells (GIBCO BRL) and 5µl of the ligation mixture was mixed in a sterile 15-ml tube (IWAKI glass). The tube was kept on ice for 30 min. After incubation for 45 sec at 42°C, to the cells was added 250 µl of L broth (1% Tryptone, 0.5% yeast extract, 1% NaCl). The cell suspension was then incubated for 1hr. at 37°C with shaking. Fifty microliters of the cell suspension was plated onto an L-agar plate containing 50µg/ml of ampicillin. The plate was incubated overnight at 37°C.

Six colonies which grew on the plate were individually incubated in 2 ml each of L-broth containing 50µg/ml of ampicillin overnight at 37°C with shaking. The structure of the plasmids in the colonies was analyzed. A plasmid in which the 1.6-kb DNA fragment containing the entire OCIF cDNA is inserted between the digestion sites of Bam HI and Xho I of pBluescript II SK + was obtained and designated as pSK + -OCIF.

### ii) Preparation of mutants in which one of the Cys residues in OCIF is replaced with Ser residue

### 1) Introduction of mutations into OCIF cDNA

OCIF mutants were prepared in which one of the five Cys residues present in OCIF at positions 174, 181, 256, 298 and 379 (in SEQUENCE NO 4) was replaced with Ser residue and were designated OCIF-C19S(174Cys to Ser), OCIF-C20S (181Cys to Ser), OCIF-C21S (256Cys to Ser), OCIF-C22S (298Cys to Ser) and OCIF-C23S (379Cys to Ser), respectively.

To prepare the mutants, nucleotides encoding the corresponding Cys residues were replaced with those encoding Ser. Mutagenesis was carried out by a two-step polymerase chain reaction (PCR). The first step of the PCRs consisted_of two reactions, PCR 1 and PCR 2.

| | | |
|---|---|---|
| PCR 1 | 10X Ex Taq Buffer (Takara Shuzo) | 10 µl |
| | 2.5 mM solution of dNTPs | 8 µl |
| | the plasmid vector described in EXAMPLE 11 (8ng/ml) | 2 µl |
| | sterile distilled water | 73.5 µl |
| | 20 µM solution of primer 1 | 5 µl |
| | 100 µM solution of primer 2 (for mutagenesis) | 1 µl |
| | Ex Taq (Takara Shuzo) | 0.5 µl |
| | | |
| PCR 2 | 10X Ex Taq Buffer (Takara Shuzo) | 10 µl |
| | 2.5 mM solution of dNTPs | 8 µl |
| | the plasmid vector described in EXAMPLE 11 (8ng/ml) | 2 µl |
| | sterile distilled water | 73.5 µl |
| | 20 µM solution of primer 3 | 5 µl |
| | 100 µM solution of primer 4 (for mutagenesis) | 1 µl |
| | Ex Taq (Takara Shuzo) | 0.5 µl |

Specific sets of primers were used for each mutation and other components were unchanged. Primers used for the reactions are shown in Table '10. The nucleotide sequences of the primers are shown in SEQUENCE NO: 20,23,27 and 30-40. The PCRs were performed under the following conditions as follows. An initial denaturation step at 97°C for 3 min was followed by 25 cycles of denaturation at 95°C for 1 min annealing at 55°C for 1 min and extension at 72°C for 3 min. After these amplification cycles, final extension was performed at 70°C for 5 min. The size of the PCR prodcts was confirmed by agarose gel electrophoresis using reaction solution. After the first PCR, excess primers were removed using Amicon microcon (Amicon). The final volume of the solutions that contained the PCR products were made to 50 µl with sterile distilled water. These purified PCR products were used for the second PCR (PCR 3).

| | | |
|---|---|---|
| PCR 3 | 10X Ex Taq Buffer (Takara Shuzo) | 10 µl |
| | 2.5 mM solution of dNTPs | 8 µl |
| | solution containing DNA fragment obtained from PCR 1 | 5 µl |
| | solution containing DNA fragment obtained from PCR 2 | 5 µl |
| | sterile distilled water | 61. 5 µl |
| | 20 µM solution of primer 1 | 5 µl |
| | 20 µM solution of primer 3 | 5 µl |
| | Ex Taq (Takara Shuzo) | 0.5 µl |

**Table 10**

| mutants | primer-1 | primer-2 | primer-3 | primer-4 |
|---|---|---|---|---|
| OCIF-C19S | IF 10 | C19SR | IF 3 | C19SF |
| OCIF-C20S | IF 10 | C20SR | IF 3 | C20SF |
| OCIF-C21S | IF 10 | C21SR | IF 3 | C21SF |
| OCIF-C22S | IF 10 | C22SR | IF 14 | C22SF |
| OCIF-C23S | IF 6 | C23SR | IF 14 | C23SF |

The reaction conditions were exactly the same as those for PCR 1 or PCR 2. The size of the PCR prodcts was confirmed by 1.0 % or 1.5 % agarose gel electrophoresis. The DNA fragments were precipitated with ethanol, dried under vacuum and dissolved in 40 µl of sterile distilled water. The solutions containing DNA fragments with mutation C19S, C20S, C21S, C22S and C23S were designated as DNA solution A, DNA solution B, DNA solution C, DNA solution D and DNA solution E, respectively.
The DNA fragment which is contained in solution A (20µl) was digested with restriction enzymes Nde I and Sph I (Takara Shuzo). A DNA fragment with an approximate size of 400 base pairs (bp) was extracted from a preparative agarose gel and dissolved in 20 µl of sterile distilled water. This DNA solution was designated DNA solution 3. Two micrograms of pSK + -OCIF was digested with restriction enzymes Nde I and Sph I. A DNA fragment with an approximate size of 4.2 kb was purified from a preparative agarose gel with QIAEX gel extraction kit and dissolved in 20 µl of sterile distilled water. This DNA solution was designated as DNA solution 4. Two microliters of DNA solution 3, 3 µl of DNA solution 4 and 5 µl of ligation buffer I of DNA ligation kit ver. 2 were mixed and ligation reaction was carried out. Competent E. coli DH5 a cells were transformed with 5 µl of the ligation mixture. Ampicillin-resistant transformants were screened for a clone containing a plasmid DNA. DNA structure was analyzed by restriction enzyme mapping and by DNA sequencing. The plasmid thus obtained was named pSK-OCIF-C19S.

The DNA fragment which is contained in solution B (20 µl) was digested with restriction enzymes Nde I and Sph I. A DNA fragment with an approximate size of 400 bp was extracted from a preparative agarose gel with QIAEX gel extraction kit and dissolved in 20µl of sterile distilled water. This DNA solution was designated DNA solution 5. Two microliters of DNA solution 5, 3 µl of DNA solution 4 and 5 µl of ligation buffer I of DNA ligation kit ver. 2 were mixed and ligation reaction was carried out. Competent E. coli DH5 α cells were transformed with 5 µl of the ligation mixture. Ampicillin-resistant transformants were screened for a clone containing a plasmid DNA. DNA structure was analyzed by restriction enzyme mapping and by DNA sequencing. The plasmid thus obtained was named pSK-OCIF-C20S.
The DNA fragment which is contained in solution C (20 µl) was digested with restriction enzymes Nde I and Sph I. A DNA fragment with an approximate size of 400 bp was extracted from a preparative agarose gel with QIAEX gel extraction kit and dissolved in 20µl of sterile distilled water. This DNA solution was designated as DNA solution 6. Two microliters of DNA solution 6, 3 µl of DNA solution 4 and 5µl of ligation buffer I of DNA ligation kit ver. 2 were mixed and ligation reaction was carried out. Competent E. coli DH5 α cells were transformed with 5 µl of the ligation mixture. Ampicillin-resistant transformants were screened for a clone containing a plasmid DNA. DNA structure was analyzed by restriction enzyme mapping and by DNA sequencing. The plasmid thus obtained was named pSK-OCIF-C21S.
The DNA fragment which is contained in solution D (20 µl) was digested with restriction enzymes Nde I and Bst PI. A DNA fragment with an approximate size of 600 bp was extracted from a preparative agarose gel with QIAEX gel extraction kit and dissolved in 20 µl of sterile distilled water. This DNA solution was designated as DNA solution 7. Two micrograms of pSK + -OCIF was digested with restriction enzymes Nde I and Bst PI. A DNA fragment with an approximate size of 4.0 kb was extracted from a preparative agarose gel with QIAEX gel extraction kit and dissolved in 20µl of sterile distilled water. This DNA solution was designated as DNA solution 8. Two microliters of DNA solution 7, 3 µl of DNA solution 8 and 5µl of ligation buffer I of DNA ligation kit ver. 2 were mixed and ligation reaction was carried out. Competent E. coli DH5 α cells were transformed with 5 µl of the ligation mixture. Ampicillin-resistant transformants were screened for a clone containing a plasmid DNA in which the 600-bp Nde I-BstPI fragment with the mutation (the C22S mutation) is substituted for the 600-bp Nde I-Bst PI fragment of pSK+ -OCIF by analyzing the DNA structure. DNA structure was analyzed by restriction enzyme mapping and by DNA sequencing. The plasmid thus obtained was named pSK-OCIF-C22S.

The DNA fragment which is contained in solution E (20 µl) was digested with restriction enzymes Bst PI and Eco RV. A DNA fragment with an approximate size of 120 bp was extracted from a preparative agarose gel with QIAEX gel extraction kit and dissolved in 20µl of sterile distilled water. This DNA solution was designated as DNA solution 9. Two micrograms of pSK + -OCIF was digested with restriction enzymes Bst EII and Eco RV. A DNA fragment with an approximate size of 4.5 kb was extracted from a preparative agarose gel with QIAEX gel extraction kit and dissolved in 20µl of sterile distilled water. This DNA solution was designated as DNA solution 10. Two microliters of DNA solution 9, 3 µl of DNA solution 10 and 5 µl of ligation buffer I of DNA ligation kit ver. 2 were mixed and ligation was carried out. Competent E. coli DH5 α cells were transformed with 5µl of the ligation mixture. Ampicillin-resistant transformants were screened for a clone containing a plasmid DNA. DNA structure was analyzed by restriction enzyme mapping and by DNA sequencing. The plasmid thus obtained was named pSK-OCIF-C23S.

### 2) Construction of vectors for expressing the OCIF mutants

pSK-OCIF-C19S, pSK-OCIF-C20S, pSK-OCIF-C21S, pSK-OCIF-C22S and pSK-OCIF-C23S were digested with restriction enzymes Bam HI and Xho I. The 1.6 kb Bam HI-Xho I DNA fragment encoding each OCIF mutant was isolated and dissolved in 20µl of sterile distilled water. The DNA solutions that contain 1. 6 kb cDNA fragments derived from pSK-OCIF-C19S, pSK-OCIF-C20S, pSK-OCIF-C21S, pSK-OCIF-C22S and pSK-OCIF-C23S were designated C19S DNA solution, C20S DNA solution, C21S DNA solution, C22S DNA solution and C23S DNA solution, respectively. Five micrograms of a expression vector pCEP 4 (Invitrogen) was digested with restriction enzymes Bam HI and Xho I. A DNA fragment with an approximate size of 10 kb was purified and dissolved in 40µl of sterile distilled water. This DNA solution was designated as pCEP 4 DNA solution. One microliter of pCEP 4 DNA solution and 6 µl of either C19SDNA solution, C20S DNA solution, C21S DNA solution, C22S DNA solution or C23S DNA solution were independently mixed with 7 µl of ligation buffer I of DNA ligation kit ver. 2 and ligation reactions were carried out. Competent E. coli DH5α cells (100µl) were transformed with 7 µl of each ligation mixture. Ampicillin-resistant transformants were screened for clones containing plasmid in which a 1.6-kb cDNA fragment is inserted between the recognition sites of Bam HI and Xho I of pCEP 4 by analyzing the DNA structure. The plasmide which were obtained containing the cDNA encoding OCIF-C19S, OCIF-C20S, OCIF-C21S, OCIF-C22S and OCIF-C23S were designated pCEP4-OCIF-C19S, pCEP4-OCIF-C20S, pCEP4-OCIF-C21S, pCEP4-OCIF-C22S and pCEP4-OCIF-C23S, respectively.

### ii) Preparation of domain-deletion mutants of OCIF

### (1) deletion mutagenesis of OCIF cDNA

A series of OCIF mutants with deletions of from Thr 2 to Ala 42, from Pro 43 to Cys 84, from Glu 85 to Lys 122, from Arg 123 to Cys 164, from Asp 177 to Gln 251 and from Ile 252 to His 326 were prepared (positions of the amino acid residues are shown in SEQUENCE NO: 4). These mutants were designated as OCIF-DCR1, OCIF-DCR2, OCIF-DCR3, OCIF-DCR4, OCIF-DDD1 and OCIF-DDD2, respectively.
Mutagenesis was performed by two-step PCR as described in EXAMPLE 22-(ii). The primer sets for the reactions are shown in Table 11 and the nucleotide sequences of the primers are shown in SEQUENCE NO:19, 25, 40-53, and 54.

**Table 11**

| mutants | primer-1 | primer-2 | primer-3 | primer-4 |
|---|---|---|---|---|
| OCIF-DCR1 | XhoI F | DCR1R | IF 2 | DCR1F |
| OCIF-DCR2 | XhoI F | DCR2R | IF 2 | DCR2F |
| OCIF-DCR3 | XhoI F | DCR3R | IF 2 | DCR3F |
| OCIF-DCR4 | XhoI F | DCR4R | IF 16 | DCR4F |
| OCIF-DDD1 | IF 8 | DDD1R | IF 14 | DDD1F |
| OCIF-DDD2 | IF 8 | DDD2R | IF 14 | DDD2F |

The final PCR products were precipitated with ethanol, dried under vacuum and dissolved in 40µl of sterile distilled water. Solutions of DNA fragment coding for portions of OCIF-DCR1, OCIF-DCR2, OCIF-DCR3, OCIF-DCR4, OCIF-DDD1 and OCIF-DDD2 were designated as DNA solutions F, G, H, I, J and K, respectively.
The DNA fragment which is contained in solution F (20 µl) was digested with restriction enzymes Nde I and Xho I. A DNA fragment with an approximate size of 500 bp was extracted from a preparative agarose gel with QIAEX gel extraction kit and dissolved in 20 µl of sterile distilled water. This DNA solution was designated DNA solution 11. Two micrograms of pSK+ -OCIF was digested with restriction enzymes Nde I and Xho I. A DNA fragment with an approximate size of 4.0 kb was extracted from a preparative agarose gel with QIAEX gel extraction kit and dissolved in 20µl of sterile distilled water. This DNA solution was designated DNA solution 12. Two microliters of DNA solution 11, 3 µl of DNA solution 12 and 5 µl of ligation buffer I of DNA ligation kit ver. 2 were mixed and ligation was carried out. Competent E. coli DH5 α cells were transformed with 5 µl of the ligation mixture. Ampicillin-resistant transformants were screened for a clone containing a plasmid DNA. DNA structure was analyzed by restriction enzyme mapping and by DNA sequencing. The plasmid thus obtained was named pSK-OCIF-DCR1.

The DNA fragment which is contained in solution G (20 µl) was digested with restriction enzymes Nde I and Xho I. A DNA fragment with an approximate size of 500 bp was extracted from a preparative agarose gel with QIAEX gel extraction kit and dissolved in 20µl of sterile distilled water. This DNA solution was designated as DNA solution 13. Two microliters of DNA solution 13, 3 µl of DNA solution 12 and 5 µl of ligation buffer I of DNA ligation kit ver. 2 were mixed and ligation was carried out. Competent E. coli DH5a cells were transformed with 5 µl of the ligation mixture. Ampicillin-resistant transformants were screened for a clone containing plasmid DNA. DNA structure was analyzed by restriction enzyme mapping and by DNA sequencing. The plasmid thus obtained was named pSK-OCIF-DCR2. The DNA fragment which is contained in solution H (20 µl) was digested with restriction enzymes Nde I and Xho I. A DNA fragment with an approximate size of 500 bp was extracted from a preparative agarose gel with QIAEX gel extraction kit and dissolved in 20µl of sterile distilled water. This DNA solution was designated as DNA solution 14. Two microliters of DNA solution 14, 3 µl of DNA solution 12 and 5 µl of ligation buffer I of DNA ligation kit ver. 2 were mixed and ligation reaction was carried out. Competent E. coli DH5 a cells were transformed with 5 µl of the ligation mixture. Ampicillin-resistant transformants were screened for a clone containing a plasmid DNA. DNA structure was analyzed by restriction enzyme mapping and by DNA sequencing. The plasmid thus obtained was named pSK-OCIF-DCR3.
The DNA fragment which is contained in solution I (20 µl) was digested with restriction enzymes Xho I and Sph I. A DNA fragment with an approximate size of 900 bp was extracted from a preparative agarose gel with QIAEX gel extraction kit and dissolved in 20µl of sterile distilled water. This DNA solution was designated as DNA solution 15. Two micrograms of pSK+ -OCIF was digested with restriction enzymes Xho I and Sph I. A DNA fragment with an approximate size of 3.6 kb was extracted from a preparative agarose gel with QIAEX gel extraction kit and dissolved in 20µl of sterile distilled water. This DNA solution was designated as DNA solution 16. Two microliters of DNA solution 15, 3 µl of DNA solution 16 and 5 µl of ligation buffer I of DNA ligation kit ver. 2 were mixed and ligation reaction was carried out. Competent E. coli DH5 α cells were transformed with 5 µl of the ligation mixture. Ampicillin-resistant transformants were screened for a clone containing a plasmid DNA. DNA structure was analyzed by restriction enzyme mapping and by DNA sequencing. The plasmid thus obtained was named pSK-OCIF-DCR4.
The DNA fragment which is contained in solution J (20 µl) was digested with restriction enzymes BstP I and Nde I. A DNA fragment with an approximate size of 400 bp was extracted from a preparative agarose gel with QIAEX gel extraction kit and dissolved in 20 µl of sterile distilled water. This DNA solution was designated as DNA solution 17. Two microliters of DNA solution 17, 3 µl of DNA solution 8 and 5µl of ligation buffer I of DNA ligation kit ver. 2 were mixed and ligation reaction was carried out. Competent E. coli DH5 α cells were transformed with 5µl of the ligation mixture. Ampicillin-resistant transformants were screened for a clone containing a plasmid DNA. DNA structure was analyzed by restriction enzyme mapping and by DNA sequencing. The plasmid thus obtained was named pSK-OCIF-DDD1.
The DNA fragment which is contained in solution K (20 µl) was digested with restriction enzymes Nde I and BstP I. A DNA fragment with an approximate size of 400 bp was extracted from a preparative agarose gel with QIAEX gel extraction kit and dissolved in 20 µl of sterile distilled water. This DNA solution was designated as DNA solution 18. Two microliters of DNA solution 18, 3 µl of DNA solution 8 and 5µl of ligation buffer I of DNA ligation kit ver. 2 were mixed and ligation reaction was carried out. Competent E. coli DH5 α cells were transformed with 5 µl of the ligation mixture. Ampicillin-resistant transformants were screened for a clone containing a plasmid DNA. DNA structure was analyzed by restriction enzyme mapping and by DNA sequencing. The plasmid thus obtained was named pSK-OCIF-DDD2.

### 2) Construction of vectors for expressing the OCIF mutants

pSK-OCIF-DCR1, pSK-OCIF-DCR2, pSK-OCIF-DCR3, pSK-OCIF-DCR4, pSK-OCIF-DDD1 and pSK-OCIF-DDD2 were digested with restriction enzymes Bam HI and Xho I. The Bam HI-Xho I DNA fragment containing entire coding sequence for each OCIF mutant was isolated and dissolved in 20 µl of sterile distilled water. These DNA solutions that contain the Bam HI-Xho I fragment derived from pSK-OCIF-DCR1, pSK-OCIF-DCR2, pSK-OCIF-DCR3, pSK-OCIF-DCR4, pSK-OCIF-DDD1 and pSK-OCIF-DDD2 were designated DCR1 DNA solution, DCR2 DNA solution, DCR3 DNA solution, DCR4 DNA solution, DDD1 DNA solution and DDD2 DNA solution, respectively. One microliter of pCEP 4 DNA solution and 6µl of either DCR1 DNA solution, DCR2 DNA solution, DCR3 DNA solution, DCR4 DNA solution, DDD1 DNA solution or DDD2 DNA solution were independently mixed with 7µl of ligation buffer I of DNA ligation kit ver. 2 and ligation reactions were carried out. Competent E. coli DH5α cells (100 µl) were transformed with 7 µl of each ligation mixture. Ampicillin-resistant transformants were screened for a clone containing a plasmid DNA in which the DNA fragment with deletions is inserted between the recognition sites of Bam HI and Xho I of pCEP 4 by analyzing the DNA structure. The plasmids containing the cDNA encoding OCIF-DCR1, OCIF-DCR2, OCIF-DCR3, OCIF-DCR4, OCIF-DDD1 and OCIF-DDD2 were designated as pCEP4-OCIF-DCR1, pCEP4-OCIF-DCR2, pCEP4-OCIF-DCR3, pCEP4-OCIF-DCR4, pCEP4-OCIF-DDD1 and pCEP4-OCIF-DDD2, respectively.

### iii) Preparation of OCIF with C-terminal domain truncation

### (1) mutagenesis of OCIF cDNA

A series of OCIF mutants with deletions of from Cys at amino acid residue 379 to Leu 380, from Ser 331 to Leu 380, from Asp 252 to Leu 380, from Asp 177 to Leu 380, from Arg 123 to Leu 380 and from Cys 86 to Leu 380 was prepared. Positions of the amino acid residues are shown in SEQUENCE NO: 4. These mutants were designated as OCIF-CL, OCIF-CC, OCIF-CDD2, OCIF-CDD1, OCIF-CCR4 and OCIF-CCR3, respectively.

Mutagenesis for OCIF-CL was performed by the two-step PCR as described in EXAMPLE 22-(ii). The primer set for the reaction is shown in Table 12. The nucleotide sequences of the primers are shown in SEQUENCE NO:23, 40, 55, and 56. The final PCR products were precipitated with ethanol, dried under vacuum and dissolved in 40µl of sterile distilled water. This DNA solution was designated as solution L.

The DNA fragment which is contained in solution L (20 µl) was digested with restriction enzymes BstP I and EcoR V. A DNA fragment with an approximate size of 100 bp was extracted from a preparative agarose gel with QIAEX gel extraction kit and dissolved in 20µl of sterile distilled water. This DNA solution was designated as DNA solution 19. Two microliters of DNA solution 19, 3 µl of DNA solution 10 (described in EXAMPLE 22-(ii)) and 5µl of ligation buffer I of DNA ligation kit ver. 2 were mixed and ligation reaction was carried out. Competent E. coli DH5 α cells were transformed with 5µl of the ligation mixture. Ampicillin-resistant transformants were screened for a clone containing a plasmid DNA. DNA structure was analyzed by restriction enzyme mapping and by DNA sequencing. The plasmid thus obtained was named pSK-OCIF-CL Mutagenesis of OCIF cDNA to prepare OCIF-CC, OCIF-CDD2, OCIF-CDD1, OCIF-CCR4 and OCIF-CCR3 was performed by a one-step PCR. PCR reactions for mutagenesis to prepare OCIF-CC, OCIF-CDD2, OCIF-CDD1, OCIF-CCR4 and OCIF-CCR3

| | |
|---|---|
| 10X Ex Taq Buffer (Takara Shuzo) | 10 µl |
| 2.5 mM solution of dNTPs | 8 µl |
| the plasmid vector containing the entire OCIF cDNA described in EXAMPLE 11 (8ng/ml) | 2 µl |
| sterile distilled water | 73.5 µl |
| 20 µM solution of primer OCIF Xho F | 5 µl |
| 100 µM solution of primer (for mutagenesis) | 1 µl |
| Ex Taq (Takara Shuzo) | 0.5 µl |

**Table 12**

| mutants | primer-1 | primer-2 | primer-3 | primer-4 |
|---|---|---|---|---|
| OCIF-CL | IF 6 | CL R | IF 14 | CL F |

Specific primers were used for each mutagenesis and other components were unchanged.
Primers used for the mutagenesis are shown in Table 13. Their nucleotide sequences are shown in SEQUENCE NO:57-61. The components of each PCR were mixed in a microcentrifuge tube and PCR was performed as follows. The microcentrifuge tubes were treated for 3 minutes at 97 °C and then incubated sequentially, for 30 seconds at 95 °C, 30 seconds at 50 °C and 3 minutes at 70 °C. This three-step incubation procedure was repeated 25 times, and after that, the tubes were incubated for 5 minutes at 70 °C. An aliquot of the reaction mixture was removed from each tube and analyzed by an agarose gel electrophoresis to confirm the size of each product.
The size of the PCR products was confirmed on an agarose gel. Excess primers in the PCRs were removed using Amicon microcon (Amicon) after completion of the reaction. The DNA fragments were precipitated with ethanol, dried under vacuum and dissolved in 40 µl of sterile distilled water. The DNA fragment in each DNA solution was digested with restriction enzymes Xho I and Bam HI. After the reactions, DNA was precipitated with ethanol, dried under vacuum and dissolved in 20µl of sterile distilled water.

The solutions containing DNA fragment with the CC deletion, the CDD2 deletion, the CDD1 deletion, the CCR4 deletion and the CCR3 deletion were designated as CC DNA solution, CDD2 DNA solution, CDD1 DNA solution, CCR4 DNA solution and CC R3 DNA solution, respectively.

**Table 13**

| mutants | primers for the mutagenesis |
|---|---|
| OCIF-CC | CC R |
| OCIF-CDD2 | CDD2 R |
| OCIF-CDDI | CDD1 R |
| OCIF-CCR4 | CCR4 R |
| OCIF-CCR3 | CCR3 R |

### (2) Construction of vectors for expressing the OCIF mutants

pSK-OCIF-CL was digested with restriction enzymes Bam HI and Xho I. The Bam HI-Xho I DNA fragment containing the entire coding sequence for OCIF-CL was isolatedand dissolved in 20 µl of sterile distilled water. This DNA solution was designated as CL DNA solution. One microliter of pCEP 4 DNA solution and 6 µl of either of CL DNA solution, CC DNA solution, CDD2 DNA solution, CDD1 DNA solution, CCR4 DNA solution or CCR3 DNA solution were independently mixed with 7 µl of ligation buffer I of DNA ligation kit ver. 2 and ligation reactions were carried out. Competent E. coli DH5α cells (100 µl) were transformed with 7 µl of each ligation mixture. Ampicillin-resistant transformants were screened for clones containing plasmids which have the desirable mutations in OCIF cDNA by analyzing the DNA structure. In each plasmid, OCIF cDNA fragment having a deletion were inserted between the recognition sites of Xho I and Bam HI of pCEP 4. The plasmids containing the cDNA encoding OCIF-CL, OCIF-CC, OCIF-CDD1, OCIF-CDD2, OCIF-CCR4 and OCIF-CCR3 were designated pCEP4-OCIF-CL, pCEP4-OCIF-CC, pCEP4-OCIF-CDD2, pCEP4-OCIF-CDD1, pCEP4-OCIF-CCR4 and pCEP4-OCIF-CCR3, respectively.

### iv) Preparation of OCIF mutants with C-terminal truncation

### (1) Introduction of C-terminal truncation to OCIF

A series of OCIF mutants with C-terminal truncation was prepared. OCIF mutant in which 10 residues of from Gln at 371 to Leu at 380 are replaced with 2 residues of Leu-Val was designated OCIF-CBst. OCIF mutant in which 83 residues of from Cys 298 to Leu 380 are replaced with 3 residues of Ser-Leu-Asp was designated OCIF-CSph. OCIF mutant in which 214 residues of from Asn 167 to Leu 380 are removed was designated OCIF-CBsp. OCIF muatant in which 319 residues of from Asp 62 to Leu 380 are replaced with 2 residues of Leu-Val was designated OCIF-CPst. Positions of the amino acid residues are shown in SEQUENCE NO: 4.

Two micrograms each of pSK + -OCIF was digested with one of the restriction enzymes, Bst PI, Sph I, PstI (Takara Shuzo), and Bsp EI (New England Biolabs), and followed by phenol extraction and ethanol precipitation. The precipitated DNA was dissolved in 10 µl of sterile distilled water. Ends of the DNAs in 2 µl of each solution were blunted using a DNA blunting kit in final volumes of 5 µ1. To the reaction mixtures, 1 µg (1 µl) of an Amber codon-containing Xba I linker (5'-CTAGTCTAGACTAG-3') and 6 µl of ligation buffer I of DNA ligation kit ver. 2 were added.

After the ligation reactions, 6 µl each of the reaction mixtures was used to transform E. coli DH5α. Ampicillin-resistant transformants were screened for clones containing plasmids. DNA structure was analyzed by restriction enzyme mapping and by DNA sequencing. The plasmids thus obtained were named pSK-OCIF-CBst, pSK-OCIF-CSph, pSK-OCIF-CBsp and pSK-OCIF-CPst, respectively.

### (2) Construction of vectors for expressing the OCIF mutants

pSK-OCIF-CBst, pSK-OCIF- CSph, pSK-OCIF-CBsp and pSK-OCIF-CPst were digested with restriction enzymes Bam HI and Xho I. The 1.5 kb of DNA fragment containing entire coding sequence for each OCIF mutant was isolated and dissolved in 20 µl of sterile distilled water. These DNA solutions that contain the Bam HI-XhoI fragment derived from pSK-OCIF-CBst, pSK-OCIF- CSph, pSK-OCIF-CBsp and pSK-OCIF-CPst were designated as CBst DNA solution, CSph DNA solution, CBsp DNA solution and CPst DNA solution, respectively. One microliter of pCEP 4 DNA solution (described in EXAMPLE 22-ii)) and 6 µl of either CBst DNA solution, CSph DNA solution, CBsp DNA solution or CPst DNA solution were independently mixed with 7 µl of ligation buffer I of DNA ligation kit ver. 2 and ligation reactions were carried out. Competent E. coli DH5α cells (100 µl) were transformed with 7 µl of each ligation mixture. Ampicillin-resistant transformants were screened for clones containing plasmids in which cDNA fragment is inserted between the recognition sites of Bam HI and Xho I of pCEP 4 by analyzing the DNA structure. The plasmids containing the cDNA encoding OCIF-CBst, OCIF-CSph, OCIF-CBsp and OCIF-CPst were designated as pCEP4-OCIF-CBst, pCEP4-OCIF- CSph, pCEP4-OCIF-CBsp and pCEP4-OCIF-CPst, respectively.

### v) Preparation of vectors for expressing the OCIF mutants

E. coli clones harboring the expression vectors for OCIF mutants (total of 21 clones) were grown and the vectors were purified by QIAGEN column (QIAGEN). All the expression vectors were precipitated with ethanol and dissolved in appropriate volumes of sterile distilled water and used for further manipupations shown below.

### vi) Transient expression of the cDNAs for OCIF mutants and biological activities of the mutants

OCIF mutants were produced using the expression vectors prepared in EXAMPLE 22-v). The method was essentially the same as described in EXAMPLE 13. Only the modified points are described below. A 24-well plate was used for the DNA transfection. 2X10⁶ cells of 293/EBNA suspended in IMDM containing 10% fetal bovine serum were seeded into each well of the plate. One microgram of purified vector DNA and 4µl of lipofectamine were used for each transfection. Mixture of an expression vector and lipofectamine in OPTI-MEM (GIBCO BRL) in a final volume of 0.5 ml was added to the cells in a well. After the cells were incubated at 37°C for 24 hr in a CO₂ incubator, the medium was replaced with 0.5 ml of Ex-cell 301 medium (JSR). The cells were incubated at 37 °C for 48 more hours in the CO₂ incubator. The conditioned medium was collected and used for assay for in vitro biological activity. The nucleotide sequences of cDNAs for the OCIF mutants are shown in SEQUENCE NO:83-103. The deduced amino acid sequences for the OCIF mutants are shown in SEQUENCE NO: 62-82. The assay for in vitro biological activity was performed as described in EXAMPLE 13. Antigen concentration of each conditioned medium was determined by ELISA as described in EXAMPLE 24. Table 14 shows specific activity of the mutants relative to that of the unaltered OCIF.

**Table 14**

| mutants | activity |
|---|---|
| the unaltered OIF | ++ |
| OCIF-C19S | + |
| OCIF-C20S | ± |
| OCIF-C21S | ± |
| OCIF-C22S | + |
| OCIF-C23S | ++ |
| OCIF-DCR1 | ± |
| OCIF-DCR2 | ± |
| OCIF-DCR3 | ± |
| OCIF-DCR4 | ± |
| OCIF-DDD1 | + |
| OCIF-DDD2 | ± |
| OCIF-CL | ++ |
| OCIF-CC | ++ |
| OCIF-CDD2 | ++ |
| OCIF-CDD1 | + |
| OCIF-CCR4 | ± |
| OCIF-CCR3 | ± |
| OCIF-CBst | ++ |
| OCIF-CSph | ++ |
| OCIF-CBsp | ± |
| OCIF-CPst | ± |

| | |
|---|---|
| ++ indicates relative activity more than 50% of that of the unaltered OCIF + indicates relative activity between 10% and 50% ± indicates relative activity less than 10%, or production level too low to determine the accurate biological activity | |

### vii) western blot analysis

Ten microliters of the final conditioned medium was used for western blot analysis. Ten microliters of the sample were mixed with 10 µl of SDS-PAGE sample buffer (0.5 M Tris-HCl, 20% glycerol, 4% SDS, 20µg/ml bromo phenol blue, pH 6.8) boiled for 3 min. and subjected to a 10 % SDS polyacryl amide gel electrophoresis under non-reducing conditions. After the electrophoresis, the separated proteins were blotted to PVDF membrane (ProBlott^{R}, Perkin Elmer) using a semi-dry electroblotter (BIO-RAD). The membrane was incubated at 37°C with horseradish peroxidase labeled anti-OCIF antibodies for 2 hr. After the membrane was washed, protein bands which react with the labeled antibodies were detected using ECL system (Amersham). Two protein bands with approximate molecular masses of 60kD and 120kD were detected for the unaltered OCIF. On the other hand, almost exclusively 60kD protein band was detected for OCIF-C23S, OCIF-CL and OCIF CC. Protein bands with an approximate masses of 40kD-50kD and 30kD-40kD were the major ones for OCIF-CDD2 and OCIF-CDD1, respectively. These results indicate that Cys at 379 is responsible for the dimer formation, both the monomers and the dimers maintain the biological activity and a deletion of residues from Asp at 177 to Leu at 380 does not abolish the biological activity of OCIF (positions of the amino acid resare shown in SEQUENCE NO: 4).

### EXAMPLE 23

### Isolation of human genomic OCIF gene

### i) Screening of a human genomic library

An amplified human placenta genomic library in Lambda FIX II vector purchased from STRATAGENE was screened for the gene encoding human OCIF using the human OCIF cDNA as a probe. Essentially, screening was done according to the instruction manual supplied with the genomic library. The basic protocols described in Molecular Cloning: A Laboratory Manual also were employed to manipulate phage, E. coli, and DNA .

The library was titered, and 1x10⁶ pfu of phage was mixed with XL1-Blue MRA host E. coli cells and plated on 20 plates (9 cm x 13 cm) with 9 ml per plate of top agarose. The plates were incubated overnight at 37°C. Filter plaque lifts were prepared using Hybond-N nylon membranes (Amersham). The membranes were processed by denaturation in a solution containing 1.5 M NaCl and 0.5 M NaOH for 1 minute at room temperature. The membranes were then neutralized by placing successively for one minute each in 1 M Tris-HCl (pH7. 5) and a solution containing 1.5 M NaCl and 0.5 M Tris-HCl (pH 7.5). The membranes were then transferred onto a filter paper wet with 2xSSC. Phage DNA was fixed on the membranes with 1200 µ Joules of UV energy in STRATALINKER UV crosslinker 2400 (STRATAGENE) and the membranes were air dried. The membranes were immersed in Rapid Hybridization buffer (Amersham) and incubated for one hour at 65 °C before hybridization with ³²P-labeled cDNA probe in the same buffer overnight at 65°C. Screening probe was prepared by labeling the OCIF cDNA with ³²P using the Megaprime DNA labeling system (Amersham). Approximately, 5x10⁵cpm probe was used for each ml of hybridization buffer. After the hybridization, the membranes were rinsed in 2xSSC for five minutes at room temperature. The membranes were then washed four times, 20 minutes each time, in 0.5xSSC containing 0. 1 % SDS at 65 °C. After the final wash, the membranes were dried and subjected to autoradiography at -80 °C with SUPER HR-H X-ray film (FUJI PFOTO FILM Co., Ltd.) and an intensifying screen. Upon examination of the autoradiograms, six positive signals were detected. Agar plugs were picked from the regions corresponded to these signals for phage purification. Each agar plug was soaked overnight in 0.5 ml of SM buffer containing 1% chloroform to extract phage. Each extract containing phage was diluted 1000 fold with SM buffer and an aliquot of 1 ml or 20 ml was mixed with host E. coli described above. The mixture was plated on agar plates with top agarose as described above. The plates were incubated overnight at 37 °C, and filter lifts were prepared, prehybridized, hybridized, washed and autoradiographed as described above. This process of phage purification was applied to all six positive signals initially detected on the autoradiograms and was repeated until all phage plaques on agar plates hybridize with the cDNA probe. After purification, agar plugs of each phage isolate were soaked in SM buffer containing 1% chloroform and stored at 4 °C. Six individual phage isolates were designated λOIF3, λOIF8, λOIF9, λOIF11, λOIF12 and λOIF17, respectively.

### ii) Analysis of the genomic clones by restriction enzyme digestion and Southern blot hybridization

DNA was prepared from each phage isolate by the plate lysate method as described in Molecular Cloning: A Laboratory Manual. DNA prepared from each phage was digested with restriction enzymes and the fragments derived from the digestion were separated on agarose gels. The fragments were then transferred to nylon membranes and subjected to Southern blot hybridization using OCIF cDNA as a probe. The results of the analysis revealed that the six phage isolates are individual clones. Among these fragments derived from the restriction enzyme digestion, those fragments which hybridized with the OCIF cDNA probe were subcloned into plasmid vectors and subjected to the nucleotide sequence analysis as described below.

### iii) Subcloning restriction fragments derived from genomic clones into plasmid vectors and determination of the nucleotide sequence.

λOIF8 DNA was digested with restriction enzymes EcoRI and NotI, and the DNA fragments derived these from were separated on a 0.7% agarose gel. The 5.8 kilobase pairs (kb) EcoRI/NotI fragment was extracted from the gel using QIAEX II Gel Extraction Kit (QIAGEN) according to the procedure recommended by the manufacturer. The 5.8 kb EcoRI/NotI fragment was ligated with pBluescript II SK+ vector (STRATAGENE) which had been linearized with restriction enzymes EcoRI and NotI, using Ready-To-Go T4 DNA Ligase (Pharmacia) according to the procedure recommended by the manufacturer. Competent DH5 α E. coli cells (Amersham) were transformed with the recombinant plasmid and transformants were selected on L-plates containing 50 µg/ml of ampicillin. A clone harboring the recombinant plasmid containing the 5.8 kb EcoRI/NotI fragment was isolated and this plasmid was termed pBSG8-5.8. pBSG8-5.8 was digested with HindIII and 0.9 kb of DNA fragment derived from this digestion was isolated in the same manner as described above. This 0.9 kb fragment was then cloned in pBluescript II SK- at the HindIII site as described above. This recombinant plasmid containing 0.9 kb HindIII fragment was denoted pBS8H0.9.

λOIF11 DNA was digested with EcoRI and 6 kb, 3.6 kb, 2.6 kb EcoRI fragments were isolated in the same manner as described above and cloned in pBluescript II SK+ vector at the EcoRI site as described above. These recombinant plasmids were termed pBSG11-6, pBSG11-3.6, and pBSG11-2.6, respectively. pBSG11-6 was digested with HindIII and the digest was applied on a 0.7 % agarose gel. Three fragments, 2.2 kb, 1.1 kb, and 1.05 kb in length, were extracted from the gel and cloned independently in pBluescript II SK- vector at the HindIII site in the same manner as described above. These recombinant plasmids were termed pBS6H2.2, pBS6 H1.1 and pBS6H1.05, respectively.

The nucleotide sequence of the cloned genomic DNA was determined using ABI Dyedeoxy Terminator Cycle Sequencing Ready Reaction Kit (PERKIN ELMER) and 373A DNA Sequencing system (Applied Biosystems). Plasmids pBSG8-5.8, pBS8H0.9, pBSG11-6, pBSG11-3.6, pBSG11-2.6, pBS6H2.2, pBS6H1.1 and pBS6H1.05 were prepared according to the alkaline-SDS procedure as described in Molecular Cloning: A Laboratory Manual and used as templates for the DNA sequence analysis. Nucleotide sequence of the human OCIF gene was presented in Sequence No 104 and Sequence No 105. The nucleotide sequence of the DNA, between exon 1 and exon 2 was not entirely determined. There is a stretch of approximately 17 kb of nucleotides between the sequences given in sequence No. 104 and sequence No. 105.

### EXAMPLE 24

### Quantitation of OCIF by EIA

### i) Preparation of anti-OCIF antibody

Male JW rabbits (Kitayama LABES Co. , LTD) weighing 2.5-3.0 kg were used for immunization for preparing antisera. Three male JW rabbits (Kitayama LABES Co., LTD) weighing 2.5-3.0 kg were used for immunization. For immunization, emulsion was prepared by mixing an equal volume of rOCIF (200 µg/ml) and complete Freund's adjuvant (Difco, Cat. 0638-60-7). The rabbits were immunized subcutaneously six times at the interval of one week with 1 ml of emulsion per injection. The rabbits were injected six times at the interval of seven days subcutaneously. Whole blood was obtained ten days after the final immunization and serum was separated. Antibody was purified from serum as follows. Antiserum was diluted two-fold with PBS. After adding ammonium sulfate at a final concentration of 40 w/v %, antiserum was allowed to stand at 4 °C for 1 hr.. Precipitate obtained by centrifugation at 8000 x g for 20 min. was dissolved in a small volume of PBS and was dialyzed against PBS. The resulting solution was loaded onto a Protein G-Sepharose column (Pharmacia). After washing with PBS, absorbed immunoglobulin G was eluted with 0.1 M glycine-HCL buffer (pH 3.0). Elutes were neutralized with 1.5 M Tris-HCL buffer (pH 8.7) immediately and were dialyzed against PBS. Protein concentration was determined by absorbance at 280nm (E¹² 13.5).

Horseradish peroxidase labeled antibody was prepared using ImmunoPure Maleimide Activated Horseradish Peroxidase Kit (Pierce, Cat.31494). Briefly, one mg of IgG was incubated with 80 ug of N-succinimidyl-S-acetylthioacetate for 30 min. After deacetylation with 5 mg of hydroxylamine HCl, modified IgG was separeted by polyacrylamide desalting column. Protein pool mixed with one mg of maleimide activated horseradish peroxidase was incubated at room temperature for 1 hr.

### ii) Quantitation of OCIF by sandwich EIA

Microtiter plates (Nunc MaxiSorp Immunoplate) were coated with rabbit anti-OCIF IgG by incubating 0.2 ug in 100 ul of 50 mM sodium bicarbonate buffer pH 9.6 at 4C overnight. After blocking the plates by incubating for 1 hour at 37°C with 300 ul of 25% BlockAce/PBS (Snow Brand Milk Products), 100ul of samples were incubated for 2 hours at room temperature. After washing the plates three times with PBST (PBS containing 0.05% Tween20), 100 ul of 1:10000 diluted horseradish peroxidase labeled anti-OCIF IgG was added and incubated for 2 hours at room temperture. The amount of OCIF was determined by incubation with 100 ul of a substrate solution (TMB, ScyTek Lab.,Cat.TM4999) and measurement of the absorbance at 450 nm using an ImmunoReader (Nunc NJ2000). Purified recombinant OCIF was used as a standard protein and a typical standared curve was shown in Fig. 13.

### EXAMPLE 25

### Anti-OCIF monoclonal antibody

### i) Preparation of hybridoma producing anti-OCIF monoclonal antibody.

OCIF was purified to homogeneity from culture medium of human fibroblasts, IMR-90 by the purification method described in Eample 11. Purified OCIF was dissolved in PBS at a concentration of 10 µg/100 µl. BALB/c mice were immunized by administrating this solution intraperitoneally three times every two weeks. In the first and the second immunizations, the emulsion composed of an equal volume of OCIF and Freund's complete adjuvant was administered. Three days after the final administration, the spleen was taken out, lymphocytes were isolated and fused with mouse myeloma p3x63-Ag8.653 cells according to the conventinal method using polyethyleneglycol. Then the fused cells were cultured in HAT medium to select hybridoma. Subsequently, to check whether the selected hybridomas produce anti-OCIF antibody, anti-OCIF antibody in each culture medium of hybridomas was determined by solid phase ELISA which was prepared by coating each well in 96-well immunoplates (Nunc) with 100µl of purified OCIF (10µg/ml in 0.1 M NaHCO₃) and by blocking each well with 50% BlockAce (Snow Brand Milk Products Co. Ltd.). The hybridoma clones secreting anti-OCIF antibody were established by cloning 3 - 5 times by limit dilution and by screening using the above solid phase ELISA. Among thus obtained hybridoma clones, several hybridoma clones with high production of anti-OCIF antibody were selected.

### ii) Production of anti-OCIF monoclonal antibodies.

Each hybridoma clone secreting anti-OCIF antibody; which was obtained in EXAMPLE 25-i), was transplanted intraperitoneally to mice given Pristane (Aldrich) at a cell density of 1 x 10⁶ cells/mouse. The accumulated ascites was collected 10 - 14 days after the transplantation and the ascites containing anti-OCIF specific monoclonal antibody of the present invention was obtained. Purified antibodies were obtained by Affigel protein A Sepharose chromatography (BioRad) according to the maufacturer's manual. That is, the ascites was diluted with equal volume of a binding buffer (BioRad) and applied to protein A column. The column was washed with a sufficient volume of the binding buffer and eluted with an elution buffer (BioRad). After neutralizing, the obtained eluate was dialyzed in water and subsequently lyophilized. The purity of the obtained antibody was analyzed by SDS/PAGE and a homogenous band with a molecular weight of about 150,000 was detected.

### iii) Selection of monoclonal antibody having high affinity to OCIF

Each antibody obtained in EXAMPLE 25-ii) was dissolved in PBS and the concentration of protein in the solution was determined by the method of Lowry. Each antibody solution with the same concentration was prepared and then serially diluted with PBS. Monoclonal antibodies, which can recognize OCIF even at highly diluted solution, were selected by solid phase ELISA described in EXAMPLE 25-ii). Thus three monoclonal antibodies A1G5, E3H8 and D2F4 can be selected.

### iv) Determination of class and subclass of antibodies

The class and subclass of the antibodies of the present invention obtained in EXAMPLE 25-iii) were analyzed using an immunoglobulin class and subclass analysis kit (Amersham). The procedure was carried out according to the protocol disclosed in the directions. The results were shown in Table 15. The antibodies of the present invention, E3H8, A1G5 and D2F4 belong to IgG₁, IgG₂ₐ and IgG_{2b}, respectively.

**Table 15**

| Analysis of class and subclass of the antibodies in the present invention. | | | | | | | |
|---|---|---|---|---|---|---|---|
| Antibody | IgG₁ | IgG₂ₐ | IgG_{2b} | IgG₃ | IgA | IgM | κ |
| A1G5 | - | + | - | - | - | - | + |
| E3H8 | + | - | - | - | - | - | + |
| D2F4 | - | - | + | - | - | - | + |

### v) Determination of OCIF by ELISA

Three kinds of monoclonal antibodies, A1G5, E3H8 and D2F4, which were obtained in EXAMPLE 25-iv), were used as solid phase antibodies and enzyme-labeled antibodies, respectively. Sandwich ELISA was constructed by each combination of solid phase antibody and labeled antibody. The labeled antibody was prepared using Immuno Pure Maleimide Activated Horseradish Peroxidase Kit (Pierce, Cat. No. 31494). Each monoclonal antibody was dissolved in 0.1 M NaHCO₃ at a concentration of 10 µg/ml, and 100 µ1 of the solution was added to each well in 96-well immunoplates (Nunc, MaxiSorp Cat. No. 442404) followed by allowing to stand at room temperature overnight. Subsequently, each well in the plates was blocked with 50% Blockace (Snow Brand Milk Products, Co., Ltd.) at room temperature for 50 minutes, and then was washed three times with PBS containing 0.1% Tween 20 (washing buffer).

A series of concentrations of OCIF was prepared by diluting OCIF with 1st reaction buffer (0.2 M Tris-HCl bufer, pH 7.4, containing 40% Blockace and 0.1% Tween 20). Each well in 96-well immunoplates was filled with 100µl of the prepared OCIF solution with each concentration, allowed to stand at 37 °C for 3 hours, and subsequently washed three times with the washing buffer. For dilution of POD-labeled antibody, 2nd reaction buffer (0. 1 M Tris-HCl buffer, pH 7.4, containing 25% Blockace and 0.1% Tween 20) was used. POD-labeled antibody was diluted 400-fold with 2nd reaction buffer, and 100 µl of the diluted solution was added to each well in the immunoplates. Each imunoplate was allowed to stand at 37 °CC for 2 hours, and subsequently washed three times with the washing buffer. After washing, 100 µl of a substrate solution (0.1 M citrate-phosphate buffer, pH 4.5, containing 0.4 mg/ml of o-phenylenediamine HCl and 0.006% H₂O₂) was added to each well in the immunoplates and the immunoplates were incubated at 37°C for 15 min. The enzyme reaction was terminated by adding 50 µl of 6 N H₂SO₄ to each well. The optical density of each well was determined at 492 nm using an immunoreader (ImmunoReader NJ 2000, Nunc).

Using three kinds of monoclonal antibody in the present invention, each combination of solid phase and POD-labeled antibodies leads to a accurate determination of OCIF. Each monoclonal antibody in the present invention was confirmed to recognize a different epitope of OCIF. A typical standard curve of OCIF using a combination of solid phase antibody, A1G5 and POD-labeled antibody, E3H8 was shown in Fig. 14.

### vi) Determination of OCIF in human serum

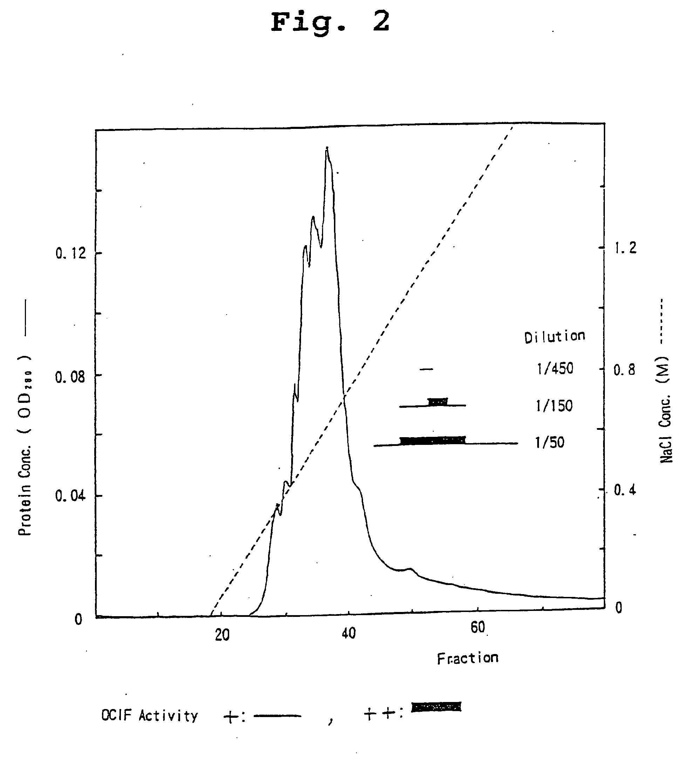

Concentration of OCIF in five samples of normal human serum was determined using an EIA system described in EXAMPLE 25-v). The immunoplates were coated with A1G5 as described in EXAMPLE 25-v), and 50 µl of 1st. reaction buffer was added to each well in the immunoplates. Subsequently, 50µl of each human serum was added to each well in the immunoplates. The immnuoplates were incubated at 37°C for 3 hours and then washed three times with the washing buffer. After washing, each well in the immunoplates was filled with 100µl of POD-E3H8 antibody diluted 400-fold with 2nd. reaction buffer and incubated at 37°C for 2 hours. After washing the immunoplates three times with the washing buffer, 100 µl of the substrate solution described in EXAMPLE 25-v) was added to each well and incubated at 37°C for 15 min. The enzyme reaction was terminated by adding 50 µl of 6 N H₂SO₄ to each well in the immunoplates. The optical density of each well was determined at 492 nm using an immunoreader (ImmunoReader NJ 2000, Nunc).
1st. reaction buffer containing the known amount of OCIF was treated in the same way and a standard curve of OCIF as shown in fig. 2 was obtained. Using the standard curve of OCIF, the amount of OCIF in human serum sample was determined. The results were shown in Table 14.

**Table 14**

| The amount of OCIF in normal human serum | |
|---|---|
| Serum Sample | OCIF Concentration (ng/ml) |
| 1 | 5. 0 |
| 2 | 2. 0 |
| 3 | 1. 0 |
| 4 | 3. 0 |
| 5 | 1. 5 |

### EXAMPLE 26

### Therapeutic effect on osteoporosis

### (1) Method

Male Fischer rats, 6 weeks-old, were subjected to denervation of left forelimb. These rats were assigned to four groups(10 rats/group) and treated as follows ; group A, sham operated rats without administration ; group B, denervated rats with intravenous administration of vehicle ; group C, denervated rats administered OCIF intravenously at a dose of 5 µg/kg twice a day ; group D, denervated rats administered OCIF intravenously at a dose of 50 µg/kg twice a day. After denervation, OCIF was administered daily for 14 days. After 2 weeks treatment, the animals were sacrificed and their forelimbs were dissected. Thereafter bones were tested for mechanical strength.

### (2) Results

Decrease of bone strength was observed in the animals of control groups as compared to those animals of the normal groups while bone strength was increase in the groups of animal received 50 mg of OCIF per kg body weight.

### Industrial availability

The present invention provides both a protein which inhibits formation of osteoclasts and a efficient procedure to produce the protein. The protein of the present invention has an activity to inhibit formation of osteoclasts. The protein will be useful for the treatment of many diseases accompanying bone loss, such as osteoporosis, and as an antigen to be used for the immunological diagnosis of such diseases.

**Referring to the deposited the microorgainsm**

| Name and Address of the Depositary Authority | |
|---|---|
| Name: | National Institute of Bioscience and Human-Technology Agency of Industrial Science and Technology Ministry of International Trade and Industry |
| Address: | 1-3, Higashi 1-chome, Tsukuba-shi, Ibaraki-ken 305, JAPAN |
| Deposited date: | June 21, 1995 |

(It was transferred from Bikkoken No. P-14998, which was deposited on June 21, 1995. Transferred date: October 25, 1995)

### Acession Number: FERM BP-5267

## Claims

1. A protein with amino acid sequence as provided in SEQ ID No. 5.

2. A protein selected from:
a protein which has an amino acid sequence of amino acid numbers -21 to 373 of SEQ ID No. 9;
a protein which has an amino acid sequence of amino acid numbers -21 to 341 of SEQ ID No. 11;
a protein which has an amino acid sequence of amino acid numbers -21 to 133 of SEQ ID No. 13;
a protein which has an amino acid sequence of amino acid numbers -21 to 124 of SEQ ID No. 15;
a protein which has an amino acid sequence of amino acid numbers -21 to 380 of SEQ ID No. 62;
a protein which has an amino acid sequence of amino acid numbers -21 to 380 of SEQ ID No. 63;
a protein which has an amino acid sequence of amino acid numbers -21 to 380 of SEQ ID No. 64:
a protein which has an amino acid sequence of amino acid numbers -21 to 380 of SEQ ID No. 65;
a protein which has an amino acid sequence of amino acid numbers -21 to 380 of SEQ ID No. 66;
a protein which has an amino acid sequence of amino acid numbers -21 to 339 of SEQ ID No. 67;
a protein which has an amino acid sequence of amino acid numbers -21 to 338 of SEQ ID No. 68;
a protein which has an amino acid sequence of amino acid numbers -21 to 342 of SEQ ID No. 69;
a protein which has an amino acid sequence of amino acid numbers -21 to 338 of SEQ ID No. 70;
a protein which has an amino acid sequence of amino acid numbers -21 to 305 of SEQ ID No. 71;
a protein which has an amino acid sequence of amino acid numbers -21 to 306 of SEQ ID No. 72;
a protein which has an amino acid sequence of amino acid numbers -21 to 378 of SEQ ID No. 73;
a protein which has an amino acid sequence of amino acid numbers -21 to 330 of SEQ ID No. 74;
a protein which has an amino acid sequence of amino acid numbers -21 to 251 of SEQ ID No. 75;
a protein which has an amino acid sequence of amino acid numbers -21 to 176 of SEQ ID No. 76;
a protein which has an amino acid sequence of amino acid numbers -21 to 122 of SEQ ID No. 77;
a protein which has an amino acid sequence of amino acid numbers -21 to 85 of SEQ ID No. 78;
a protein which has an amino acid sequence of amino acid numbers -21 to 372 of SEQ ID No. 79;
a protein which has an amino acid sequence of amino acid numbers -21 to 300 of SEQ ID No. 80;
a protein which has an amino acid sequence of amino acid numbers -21 to 166 of SEQ ID No. 81;
a protein which has an amino acid sequence of amino acid numbers -21 to 63 of SEQ ID No. 82.

3. A cDNA selected from:
a cDNA which has a nucleotide sequence of nucleotide numbers 64 to 1185 of SEQ ID No. 8;
a cDNA which has a' nucleotide sequence of nucleotide numbers 64 to 1089 of SEQ ID No. 10;
a cDNA which has a nucleotide sequence of nucleotide numbers 64 to 465 of SEQ ID No. 12;
a cDNA which has a nucleotide sequence of nucleotide numbers 64 to 438 of SEQ ID No. 14;
a cDNA which has a nucleotide sequence of nucleotide numbers 64 to 1206 of SEQ ID No. 83;
a cDNA which has a nucleotide sequence of nucleotide numbers 64 to 1206 of SEQ ID No. 84;
a cDNA which has a nucleotide sequence of nucleotide numbers 64 to 1206 of SEQ ID No. 85;
a cDNA which has a nucleotide sequence of nucleotide numbers 64 to 1206 of SEQ ID No. 86;
a cDNA which has a nucleotide sequence of nucleotide numbers 64 to 1206 of SEQ ID No. 87;
a cDNA which has a nucleotide sequence of nucleotide numbers 64 to 1083 of SEQ ID No. 88;
a cDNA which has a nucleotide sequence of nucleotide numbers 64 to 1080 of SEQ ID No. 89;
a cDNA which has a nucleotide sequence of nucleotide numbers 64 to 1092 of SEQ ID No. 90;
a cDNA which has a nucleotide sequence of nucleotide numbers 64 to 1080 of SEQ ID No. 91;
a cDNA which has a nucleotide sequence of nucleotide numbers 64 to 981 of SEQ ID No. 92;
a cDNA which has a nucleotide sequence of nucleotide numbers 64 to 984 of SEQ ID No. 93;
a cDNA which has a nucleotide sequence of nucleotide numbers 64 to 1200 of SEQ ID No. 94;
a cDNA which has a nucleotide sequence of nucleotide numbers 64 to 1056 of SEQ ID No. 95;
a cDNA which has a nucleotide sequence of nucleotide numbers 64 to 819 of SEQ ID No. 96;
a cDNA which has a nucleotide sequence of nucleotide numbers 64 to 594 of SEQ ID No. 97;
a cDNA which has a nucleotide sequence of nucleotide numbers 64 to 432 of SEQ ID No. 98;
a cDNA which has a nucleotide sequence of nucleotide numbers 64 to 321 of SEQ ID No. 99;
a cDNA which has a nucleotide sequence of nucleotide numbers 64 to 1182 of SEQ ID No. 100;
a cDNA which has a nucleotide sequence of nucleotide numbers 64 to 966 of SEQ ID No. 101;
a cDNA which has a nucleotide sequence of nucleotide numbers 64 to 564 of SEQ ID No. 102;
a cDNA which has a nucleotide sequence of nucleotide numbers 64 to 255 of SEQ ID No. 103.

4. A protein which has an amino acid sequence of SEQ ID No. 4 or SEQ ID No. 5 in which one or more amino acids, up to 204 amino acids, are deleted starting from the C-terminus amino acid "Leu" toward the N-terminus, wherein said protein has a biological activity to inhibit osteoclast differentiation and/or maturation.

5. A pharmaceutical composition comprising a protein according to any of claims 1, 2 and 4.

6. Use of a protein according to any of claims 1, 2 and 4 in the preparation of a medicament for the treatment or prevention of osteoporosis.

7. Use of a protein according to any of claims 1, 2 and 4 in the preparation of a medicament for the treatment or prevention of decreased bone mass.

8. Use of a protein according to any of claims 1, 2 and 4 in the preparation of a medicament for the treatment or prevention of an abnormal bone metabolism.

## Patentansprüche

1. Protein mit einer Aminosäuresequenz wie in SEQ ID NO: 5 bereitgestellt.

2. Protein, ausgewählt aus:
einem Protein, das eine Aminosäuresequenz mit den Aminosäurenummern -21 bis 373 von SEQ ID NO: 9 aufweist;
einem Protein, das eine Aminosäuresequenz mit den Aminosäurenummern -21 bis 341 von SEQ ID NO: 11 aufweist;
einem Protein, das eine Aminosäuresequenz mit den Aminosäurenummern -21 bis 133 von SEQ ID NO: 13 aufweist;
einem Protein, das eine Aminosäuresequenz mit den Aminosäurenummern -21 bis 124 von SEQ ID NO: 15 aufweist;
einem Protein, das eine Aminosäuresequenz mit den Aminosäurenummern -21 bis 380 von SEQ ID NO: 62 aufweist;
einem Protein, das eine Aminosäuresequenz mit den Aminosäurenummern -21 bis 380 von SEQ ID NO: 63 aufweist;
einem Protein, das eine Aminosäuresequenz mit den Aminosäurenummern -21 bis 380 von SEQ ID NO: 64 aufweist:
einem Protein, das eine Aminosäuresequenz mit den Aminosäurenummern -21 bis 380 von SEQ ID NO: 65 aufweist;
einem Protein, das eine Aminosäuresequenz mit den Aminosäurenummern -21 bis 380 von SEQ ID NO: 66 aufweist;
einem Protein, das eine Aminosäuresequenz mit den Aminosäurenummern -21 bis 339 von SEQ ID NO: 67 aufweist;
einem Protein, das eine Aminosäuresequenz mit den Aminosäurenummern -21 bis 338 von SEQ ID NO: 68 aufweist;
einem Protein, das eine Aminosäuresequenz mit den Aminosäurenummern -21 bis 342 von SEQ ID NO: 69 aufweist;
einem Protein, das eine Aminosäuresequenz mit den Aminosäurenummern -21 bis 338 von SEQ ID NO: 70 aufweist;
einem Protein, das eine Aminosäuresequenz mit den Aminosäurenummern -21 bis 305 von SEQ ID NO: 71 aufweist;
einem Protein, das eine Aminosäuresequenz mit den Aminosäurenummern -21 bis 306 von SEQ ID NO: 72 aufweist;
einem Protein, das eine Aminosäuresequenz mit den Aminosäurenummern -21 bis 378 von SEQ ID NO: 73 aufweist;
einem Protein, das eine Aminosäuresequenz mit den Aminosäurenummern -21 bis 330 von SEQ ID NO: 74 aufweist;
einem Protein, das eine Aminosäuresequenz mit den Aminosäurenummern -21 bis 251 von SEQ ID NO: 75 aufweist;
einem Protein, das eine Aminosäuresequenz mit den Aminosäurenummern -21 bis 176 von SEQ ID NO: 76 aufweist;
einem Protein, das eine Aminosäuresequenz mit den Aminosäurenummern -21 bis 122 von SEQ ID NO: 77 aufweist;
einem Protein, das eine Aminosäuresequenz mit den Aminosäurenummern -21 bis 85 von SEQ ID NO: 78 aufweist;
einem Protein, das eine Aminosäuresequenz mit den Aminosäurenummern -21 bis 372 von SEQ ID NO: 79 aufweist;
einem Protein, das eine Aminosäuresequenz mit den Aminosäurenummern -21 bis 300 von SEQ ID NO: 80 aufweist;
einem Protein, das eine Aminosäuresequenz mit den Aminosäurenummern -21 bis 166 von SEQ ID NO: 81 aufweist;
einem Protein, das eine Aminosäuresequenz mit den Aminosäurenummern -21 bis 63 von SEQ ID NO: 82 aufweist.

3. cDNA, ausgewählt aus:
einer cDNA, die eine Nukleotidsequenz mit den Nukleotidnummern 64 bis 1185 von SEQ ID NO: 8 aufweist;
einer cDNA, die eine Nukleotidsequenz mit den Nukleotidnummern 64 bis 1089 von SEQ ID NO: 10 aufweist;
einer cDNA, die eine Nukleotidsequenz mit den Nukleotidnummern 64 bis 465 von SEQ ID NO: 12 aufweist;
einer cDNA, die eine Nukleotidsequenz mit den Nukleotidnummern 64 bis 438 von SEQ ID NO: 14 aufweist;
einer cDNA, die eine Nukleotidsequenz mit den Nukleotidnummern 64 bis 1206 von SEQ ID NO: 83 aufweist;
einer cDNA, die eine Nukleotidsequenz mit den Nukleotidnummern 64 bis 1206 von SEQ ID NO: 84 aufweist;
einer cDNA, die eine Nukleotidsequenz mit den Nukleotidnummern 64 bis 1206 von SEQ ID NO: 85 aufweist;
einer cDNA, die eine Nukleotidsequenz mit den Nukleotidnummern 64 bis 1206 von SEQ ID NO: 86 aufweist;
einer cDNA, die eine Nukleotidsequenz mit den Nukleotidnummern 64 bis 1206 von SEQ ID NO: 87 aufweist;
einer cDNA, die eine Nukleotidsequenz mit den Nukleotidnummern 64 bis 1083 von SEQ ID NO: 88 aufweist;
einer cDNA, die eine Nukleotidsequenz mit den Nukleotidnummern 64 bis 1080 von SEQ ID NO: 89 aufweist;
einer cDNA, die eine Nukleotidsequenz mit den Nukleotidnummern 64 bis 1092 von SEQ ID NO: 90 aufweist;
einer cDNA, die eine Nukleotidsequenz mit den Nukleotidnummern 64 bis 1080 von SEQ ID NO: 91 aufweist;
einer cDNA, die eine Nukleotidsequenz mit den Nukleotidnummern 64 bis 981 von SEQ ID NO: 92 aufweist;
einer cDNA, die eine Nukleotidsequenz mit den Nukleotidnummern 64 bis 984 von SEQ ID NO: 93 aufweist;
einer cDNA, die eine Nukleotidsequenz mit den Nukleotidnummern 64 bis 1200 von SEQ ID NO: 94 aufweist,
einer cDNA, die eine Nukleotidsequenz mit den Nukleotidnummern 64 bis 1056 von SEQ ID NO: 95 aufweist;
einer cDNA, die eine Nukleotidsequenz mit den Nukleotidnummern 64 bis 819 von SEQ ID NO: 96 aufweist;
einer cDNA, die eine Nukleotidsequenz mit den Nukleotidnummern 64 bis 594 von SEQ ID NO: 97 aufweist;
einer cDNA, die eine Nukleotidsequenz mit den Nukleotidnummern 64 bis 432 von SEQ ID NO: 98 aufweist;
einer cDNA, die eine Nukleotidsequenz mit den Nukleotidnummern 64 bis 321 von SEQ ID NO: 99 aufweist;
einer cDNA, die eine Nukleotidsequenz mit den Nukleotidnummern 64 bis 1182 von SEQ ID NO: 100 aufweist;
einer cDNA, die eine Nukleotidsequenz mit den Nukleotidnummern 64 bis 966 von SEQ ID NO: 101 aufweist;
einer eDNA, die eine Nukleotidsequenz mit den Nukleotidnummern 64 bis 564 von SEQ ID NO: 102 aufweist;
einer cDNA, die eine Nukleotidsequenz mit den Nukleotidnummern 64 bis 255 von SEQ ID NO: 103 aufweist.

4. Protein, das eine Aminosäuresequenz von SEQ ID NO: 4 oder SEQ ID NO: 5 aufweist, worin eine oder mehr Aminosäure(n), bis zu 204 Aminosäuren, deletiert sind, beginnend mit der C-terminalen Aminosäure "Leu" in Richtung des N-Terminus, worin das Protein eine biologische Aktivität zum Hemmen der Osteoklastendifferenzierung und/oder -reifung aufweist.

5. Pharmazeutische Zusammensetzung, umfassend ein Protein nach einem der Ansprüche 1, 2 und 4.

6. Verwendung eines Proteins nach einem der Ansprüche 1, 2 und 4 bei der Herstellung eines Arzneimittels zur Behandlung oder Prävention von Osteoporose.

7. Verwendung eines Proteins nach einem der Ansprüche 1, 2 und 4 bei der Herstellung eines Arzneimittels zur Behandlung oder Prävention von verminderter Knochenmasse.

8. Verwendung eines Proteins nach einem der Ansprüche 1, 2 und 4 bei der Herstellung eines Arzneimittels zur Behandlung oder Prävention eines abnormen Knochenmetabolismus.

## Revendications

1. Protéine ayant la séquence d'acides aminés telle que fournie dans SEQ ID No. 5.

2. Protéine sélectionnée parmi :
une protéine qui a une séquence d'acides aminés comprenant les acides aminés numéros -21 à 373 de SEQ ID No. 9 ;
une protéine qui a une séquence d'acides aminés comprenant les acides aminés numéros -21 à 341 de SEQ ID No. 11 ;
une protéine qui a une séquence d'acides aminés comprenant les acides aminés numéros -21 à 133 de SEQ ID No. 13 ;
une protéine qui a une séquence d'acides aminés comprenant les acides aminés numéros -21 à 124 de SEQ ID No. 15 ;
une protéine qui a une séquence d'acides aminés comprenant les acides aminés numéros -21 à 380 de SEQ ID No. 62 ;
une protéine qui a une séquence d'acides aminés comprenant les acides aminés numéros -21 à 380 de SEQ ID No. 63 ;
une protéine qui a une séquence d'acides aminés comprenant les acides aminés numéros -21 à 380 de SEQ ID No. 64 ;
une protéine qui a une séquence d'acides aminés comprenant les acides aminés numéros -21 à 380 de SEQ ID No. 65 ;
une protéine qui a une séquence d'acides aminés comprenant les acides aminés numéros -21 à 380 de SEQ ID No. 66 ;
une protéine qui a une séquence d'acides aminés comprenant les acides aminés numéros -21 à 339 de SEQ ID No. 67 ;
une protéine qui a une séquence d'acides aminés comprenant les acides aminés numéros -21 à 338 de SEQ ID No. 68 ;
une protéine qui a une séquence d'acides aminés comprenant les acides aminés numéros -21 à 342 de SEQ ID No. 69 ;
une protéine qui a une séquence d'acides aminés comprenant les acides aminés numéros -21 à 338 de SEQ ID No. 70 ;
une protéine qui a une séquence d'acides aminés comprenant les acides aminés numéros -21 à 305 de SEQ ID No. 71 ;
une protéine qui a une séquence d'acides aminés comprenant les acides aminés numéros -21 à 306 de SEQ ID No. 72 ;
une protéine qui a une séquence d'acides aminés comprenant les acides aminés numéros -21 à 378 de SEQ ID No. 73 ;
une protéine qui a une séquence d'acides aminés comprenant les acides aminés numéros -21 à 330 de SEQ ID No. 74 ;
une protéine qui a une séquence d'acides aminés comprenant les acides aminés numéros -21 à 251 de SEQ ID No. 75 ;
une protéine qui a une séquence d'acides aminés comprenant les acides aminés numéros -21 à 176 de SEQ ID No. 76 ;
une protéine qui a une séquence d'acides aminés comprenant les acides aminés numéros -21 à 122 de SEQ ID No. 77 ;
une protéine qui a une séquence d'acides aminés comprenant les acides aminés numéros -21 à 85 de SEQ ID No. 78 ;
une protéine qui a une séquence d'acides aminés comprenant les acides aminés numéros -21 à 372 de SEQ ID No. 79 ;
une protéine qui a une séquence d'acides aminés comprenant les acides aminés numéros -21 à 300 de SEQ ID No. 80 ;
une protéine qui a une séquence d'acides aminés comprenant les acides aminés numéros -21 à 166 de SEQ ID No. 81 ;
une protéine qui a une séquence d'acides aminés comprenant les acides aminés numéros -21 à 63 de SEQ ID No. 82.

3. ADNc sélectionné parmi :
un ADNc qui a une séquence de nucléotides comprenant les nucléotides numéros 64 à 1185 de SEQ ID No. 8 ;
un ADNc qui a une séquence de nucléotides comprenant les nucléotides numéros 64 à 1089 de SEQ ID No. 10 ;
un ADNc qui a une séquence de nucléotides comprenant les nucléotides numéros 64 à 465 de SEQ ID No. 12 ;
un ADNc qui a une séquence de nucléotides comprenant les nucléotides numéros 64 à 438 de SEQ ID No. 14 ;
un ADNc qui a une séquence de nucléotides comprenant les nucléotides numéros 64 à 1206 de SEQ ID No. 83 ;
un ADNc qui a une séquence de nucléotides comprenant les nucléotides numéros 64 à 1206 de SEQ ID No. 84 ;
un ADNc qui a une séquence de nucléotides comprenant les nucléotides numéros 64 à 1206 de SEQ ID No. 85 ;
un ADNc qui a une séquence de nucléotides comprenant les nucléotides numéros 64 à 1206 de SEQ ID No. 86 ;
un ADNc qui a une séquence de nucléotides comprenant les nucléotides numéros 64 à 1206 de SEQ ID No. 87 ;
un ADNc qui a une séquence de nucléotides comprenant les nucléotides numéros 64 à 1083 de SEQ ID No. 88 ;
un ADNc qui a une séquence de nucléotides comprenant les nucléotides numéros 64 à 1080 de SEQ ID No. 89 ;
un ADNc qui a une séquence de nucléotides comprenant les nucléotides numéros 64 à 1092 de SEQ ID No. 90 ;
un ADNc qui a une séquence de nucléotides comprenant les nucléotides numéros 64 à 1080 de SEQ ID No. 91 ;
un ADNc qui a une séquence de nucléotides comprenant les nucléotides numéros 64 à 981 de SEQ ID No. 92 ;
un ADNc qui a une séquence de nucléotides comprenant les nucléotides numéros 64 à 984 de SEQ ID No. 93 ;
un ADNc qui a une séquence de nucléotides comprenant les nucléotides numéros 64 à 1200 de SEQ ID No. 94 ;
un ADNc qui a une séquence de nucléotides comprenant les nucléotides numéros 64 à 1056 de SEQ ID No. 95 ;
un ADNc qui a une séquence de nucléotides comprenant les nucléotides numéros 64 à 819 de SEQ ID No. 96 ;
un ADNc qui a une séquence de nucléotides comprenant les nucléotides numéros 64 à 594 de SEQ ID No. 97 ;
un ADNc qui a une séquence de nucléotides comprenant les nucléotides numéros 64 à 432 de SEQ ID No. 98 ;
un ADNc qui a une séquence de nucléotides comprenant les nucléotides numéros 64 à 321 de SEQ ID No. 99 ;
un ADNc qui a une séquence de nucléotides comprenant les nucléotides numéros 64 à 1182 de SEQ ID No. 100 ;
un ADNc qui a une séquence de nucléotides comprenant les nucléotides numéros 64 à 966 de SEQ ID No. 101 ;
un ADNc qui a une séquence de nucléotides comprenant les nucléotides numéros 64 à 564 de SEQ ID No. 102 ;
un ADNc qui a une séquence de nucléotides comprenant les nucléotides numéros 64 à 255 de SEQ ID No. 103.

4. Protéine qui a une séquence d'acides aminés de SEQ ID No. 4 ou SEQ ID No. 5 dans laquelle un ou plusieurs acides aminés, jusqu'à concurrence de 204 acides aminés, sont délétés en commençant par l'acide aminé « Leu » de l'extrémité C-terminale vers l'extrémité N-terminale, dans laquelle ladite protéine exerce une activité biologique pour inhiber la différenciation et/ou la maturation des ostéoclastes.

5. Composition pharmaceutique comprenant une protéine selon l'une quelconque des revendications 1, 2 et 4.

6. Utilisation d'une protéine selon l'une quelconque des revendications 1, 2 et 4 dans la préparation d'un médicament pour le traitement ou la prévention de l'ostéoporose.

7. Utilisation d'une protéine selon l'une quelconque des revendications 1, 2 et 4 dans la préparation d'un médicament pour le traitement ou la prévention de la réduction de la masse osseuse.

8. Utilisation d'une protéine selon l'une quelconque des revendications 1, 2 et 4 dans la préparation d'un médicament pour le traitement ou la prévention d'un métabolisme osseux anormal.


### SEQUENCE LISTING

(1) GENERAL INFORMATION:
   (i) APPLICANT:
      (A) NAME: SNOW BRANDS MILK PRODUCTS CO., LTD.
      (B) STREET:
      (C) CITY:
      (D) STATE:
      (E) COUNTRY:
      (F) POSTAL CODE (ZIP) :
      (G) TELEPHONE:
      (H) TELEFAX:
      (I) TELEX:
   (ii) TITLE OF INVENTION: Novel proteins and methods for producing the proteins
   (iii) NUMBER OF SEQUENCES: 105
   (iv) COMPUTER READABLE FORM:
      (A) MEDIUM TYPE: Floppy disk
      (B) COMPUTER:
      (C) OPERATING SYSTEM:
      (D) SOFTWARE: Wordperfect windows
   (V) CURRENT APPLICATION DATA:
      (A) APPLICATION NUMBER: JP
      (B) FILE REFERENCE:
      (C) FILING DATE:
(2) INFORMATION FOR SEQUENCE ID NO: 1:
   (i) SEQUENCE CHARACTERISTICS:
      (A) LENGTH : 6
      (B) TYPE : amino acid
      (D) TOPOLOGY : linear
   (ii) MOLECULE TYPE : peptide (an internal amino acid sequence of the protein)
   (xi) SEQUENCE DESCRIPTION :SEQ ID NO: 1:
   Xaa Tyr His Phe Pro Lys
   1 5
(2) INFORMATION FOR SEQUENCE ID NO: 2:
   (i) SEQUENCE CHARACTERISTICS:
      (A) LENGTH : 14
      (B) TYPE : amino acid
      (D) TOPOLOGY : linear
   (ii) MOLECULE TYPE : peptide (an internal amino acid sequence of the protein)
   (xi) SEQUENCE DESCRIPTION :SEQ ID NO:2:
   Xaa Gln His Ser Xaa Gln Glu Gln Thr Phe Gln Leu Xaa Lys
   1 5 10
(2) INFORMATION FOR SEQUENCE ID NO: 3:
   (i) SEQUENCE CNARACTERISTICS:
      (A) LENGTH : 12
      (B) TYPE : amino acid
      (D) TOPOLOGY : linear
   (ii) MOLECULE TYPE : peptide (an internal amino acid sequence of the protein)
   (xi) SEQUENCE DESCRIPTION :SEQ ID NO: 3:
   Xaa Ile Arg Phe Leu His Ser Phe Thr Met Tyr Lys
   1 5 10
(2) INFORMATION FOR SEQUENCE ID NO: 4:
   (i) SEQUENCE CHARACTERISTICS:
      (A) LENGTH : 380
      (B) TYPE : amino acid
      (D) TOPOLOGY : linear
   (ii) MOLECULE TYPE : protein (OCIF protein without signal peptide)
   (xi) SEQUENCE DESCRIPTION :SEQ ID NO:4:
   Glu Thr Phe Pro Pro Lys Tyr Leu His Tyr Asp Glu Glu Thr Ser
   1 5 10 15
   His Gln Leu Leu Cys Asp Lys Cys Pro Pro Gly Thr Tyr Leu Lys
   20 25 30
   Gln His Cys Thr Ala Lys Trp Lys Thr Val Cys Ala Pro Cys Pro
   35 40 45
   Asp His Tyr Tyr Thr Asp Ser Trp His Thr Ser Asp Glu Cys Leu
   50 55 60
   Tyr Cys Ser Pro Val Cys Lys Glu Leu Gln Tyr Val Lys Gln Glu
   65 70 75
   Cys Asn Arg Thr His Asn Arg Val Cys Glu Cys Lys Glu Gly Arg
   80 85 90
   Tyr Leu Glu Ile Glu Phe Cys Leu Lys His Arg Ser Cys Pro Pro
   95 100 105
   Gly Phe Gly Val Val Gln Ala Gly Thr Pro Glu Arg Asn Thr Val
   110 115 120
   Cys Lys Arg Cys Pro Asp Gly Phe Phe Ser Asn Glu Thr Ser Ser
   125 130 135
   Lys Ala Pro Cys Arg Lys His Thr Asn Cys Ser Val Phe Gly Leu
   140 145 150
   Leu Leu Thr Gln Lys Gly Asn Ala Thr His Asp Asn Ile Cys Ser
   155 160 165
   Gly Asn Ser Glu Ser Thr Gln Lys Cys Gly Ile Asp Val Thr Leu
   170 175 180
   Cys Glu Glu Ala Phe Phe Arg Phe Ala Val Pro Thr Lys Phe Thr
   185 190 195
   Pro Asn Trp Leu Ser Val Leu Val Asp Asn Leu Pro Gly Thr Lys
   200 205 210
   Val Asn Ala Glu Ser Val Glu Arg Ile Lys Arg Gln His Ser Ser
   215 220 225
   Gln Glu Gln Thr Phe Gln Leu Leu Lys Leu Trp Lys His Gln Asn
   230 235 240
   Lys Asp Gln Asp Ile Val Lys Lys Ile Ile Gln Asp Ile Asp Leu
                   245                 250                 255
   Cys Glu Asn Ser Val Gln Arg His Ile Gly His Ala Asn Leu Thr
                   260                 265                 270
   Phe Glu Gln Leu Arg Ser Leu Met Glu Ser Leu Pro Gly Lys Lys
                   275                 280                 285
   Val Gly Ala Glu Asp Ile Glu Lys Thr Ile Lys Ala Cys Lys Pro
                   290                 295                 300
   Ser Asp Gln Ile Leu Lys Leu Leu Ser Leu Trp Arg Ile Lys Asn
                   305                 310                 315
   Gly Asp Gln Asp Thr Leu Lys Gly Leu Met His Ala Leu Lys His
                   320                 325                 330
   Ser Lys Thr Tyr His Phe Pro Lys Thr Val Thr Gln Ser Leu Lys
                   335                 340                 345
   Lys Thr Ile Arg Phe Leu His Ser Phe Thr Met Tyr Lys Leu Tyr
                   350                 355                 360
   Gln Lys Leu Phe Leu Glu Met Ile Gly Asn Gln Val Gln Ser Val
                   365                 370                 375
   Lys Ile Ser Cys Leu
                   380
(2) INFORMATION FOR SEQUENCE ID NO: 5:
   (i) SEQUENCE CHARACTERISTICS:
      (A) LENGTH : 401
      (B) TYPE : amino acid
      (D) TOPOLOGY : linear
   (ii) MOLECULE TYPE : protein (OCIF protein with signal peptide)
   (xi) SEQUENCE DESCRIPTION : SEQ ID NO: 5:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5              -1   1              5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40 45 50
   Thr Ser Asp Glu Cys Leu Tyr Cys Ser Pro Val Cys Lys Glu Leu
   55 60 65
   Gln Tyr Val Lys Gln Glu Cys Asn Arg Thr His Asn Arg Val Cys
   70 75 80
   Glu Cys Lys Glu Gly Arg Tyr Leu Glu Ile Glu Phe Cys Leu Lys
   85 90 95
   His Arg Ser Cys Pro Pro Gly Phe Gly Val Val Gln Ala Gly Thr
   100 105 110
   Pro Glu Arg Asn Thr Val Cys Lys Arg Cys Pro Asp Gly Phe Phe
   115 120 125
   Ser Asn Glu Thr Ser Ser Lys Ala Pro Cys Arg Lys His Thr Asn
   130 135 140
   Cys Ser Val Phe Gly Leu Leu Leu Thr Gln Lys Gly Asn Ala Thr
   145 150 155
   His Asp Asn Ile Cys Ser Gly Asn Ser Glu Ser Thr Gln Lys Cys
   160 165 170
   Gly Ile Asp Val Thr Leu Cys Glu Glu Ala Phe Phe Arg Phe Ala
   175 180 185
   Val Pro Thr Lys Phe Thr Pro Asn Trp Leu Ser Val Leu Val Asp
   190 195 200
   Asn Leu Pro Gly Thr Lys Val Asn Ala Glu Ser Val Glu Arg Ile
   205 210 215
   Lys Arg Gln His Ser Ser Gln Glu Gln Thr Phe Gln Leu Leu Lys
   220 225 230
   Leu Trp Lys His Gln Asn Lys Asp Gln Asp Ile Val Lys Lys Ile
   235 240 245
   Ile Gln Asp Ile Asp Leu Cys Glu Asn Ser Val Gln Arg His Ile
   250 255 260
   Gly His Ala Asn Leu Thr Phe Glu Gln Leu Arg Ser Leu Met Glu
   265 270 275
   Ser Leu Pro Gly Lys Lys Val Gly Ala Glu Asp Ile Glu Lys Thr
   280 285 290
   Ile Lys Ala Cys Lys Pro Ser Asp Gln Ile Leu Lys Leu Leu Ser
   295 300 305
   Leu Trp Arg Ile Lys Asn Gly Asp Gln Asp Thr Leu Lys Gly Leu
   310 315 320
   Met His Ala Leu Lys His Ser Lys Thr Tyr His Phe Pro Lys Thr
   325 330 335
   Val Thr Gln Ser Leu Lys Lys Thr Ile Arg Phe Leu His Ser Phe
   340 345 350
   Thr Met Tyr Lys Leu Tyr Gln Lys Leu Phe Leu Glu Met Ile Gly
   355 360 365
   Asn Gln Val Gln Ser Val Lys Ile Ser Cys Leu
   370 375 380
(2) INFORMATION FOR SEQUENCE ID NO: 6:
   (i) SEQUENCE CHARACTERISTICS:
      (A) LENGTH : 1206
      (B) TYPE : nucleic acid
      (C) STRANDEDNESS : single
      (D) TOPOLOGY : linear
   (ii) MOLECULE TYPE : cDNA (OCIF)
   (xi) SEQUENCE DESCRIPTION :SEQ ID NO: 6:
   ATGAACAACT TGCTGTGCTG CGCGCTCGTG TTTCTGGACA TCTCCATTAA GTGGACCACC 60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG CAAGGAAGGG CGCTACCTTG AGATAGAGTT CTGCTTGAAA 360
   CATAGGAGCT GCCCTCCTGG ATTTGGAGTG GTGCAAGCTG GAACCCCAGA GCGAAATACA 420
   GTTTGCAAAA GATGTCCAGA TGGGTTCTTC TCAAATGAGA CGTCATCTAA AGCACCCTGT 480
   AGAAAACACA CAAATTGCAG TGTCTTTGGT CTCCTGCTAA CTCAGAAAGG AAATGCAACA 540
   CACGACAACA TATGTTCCGG AAACAGTGAA TCAACTCAAA AATGTGGAAT AGATGTTACC 600
   CTGTGTGAGG AGGCATTCTT CAGGTTTGCT GTTCCTACAA AGTTTACGCC TAACTGGCTT 660
   AGTGTCTTGG TAGACAATTT GCCTGGCACC AAAGTAAACG CAGAGAGTGT AGAGAGGATA 720
   AAACGGCAAC ACAGCTCACA AGAACAGACT TTCCAGCTGC TGAAGTTATG GAAACATCAA 780
   AACAAAGACC AAGATATAGT CAAGAAGATC ATCCAAGATA TTGACCTCTG TGAAAACAGC 840
   GTGCAGCGGC ACATTGGACA TGCTAACCTC ACCTTCGAGC AGCTTCGTAG CTTGATGGAA 900
   AGCTTACCGG GAAAGAAAGT GGGAGCAGAA GACATTGAAA AAACAATAAA GGCATGCAAA 960
   CCCAGTGACC AGATCCTGAA GCTGCTCAGT TTGTGGCGAA TAAAAAATGG CGACCAAGAC 1020
   ACCTTGAAGG GCCTAATGCA CGCACTAAAG CACTCAAAGA CGTACCACTT TCCCAAAACT 1080
   GTCACTCAGA GTCTAAAGAA GACCATCAGG TTCCTTCACA GCTTCACAAT GTACAAATTG 1140
   TATCAGAAGT TATTTTTAGA AATGATAGGT AACCAGGTCC AATCAGTAAA AATAAGCTGC 1200
   TTATAA 1206
(2) INFORMATION FOR SEQUENCE ID NO: 7:
   (i) SEQUENCE CHARACTERISTICS:
      (A) LENGTH : 15
      (B) TYPE : amino acid
      (D) TOPOLOGY : linear
   (ii) MOLECULE TYPE : peptide (a N-terminal amino acid sequence of the protein)
   (xi) SEQUENCE DESCRIPTION :SEQ ID NO:7:
   Glu Thr Phe Pro Pro Lys Tyr Leu His Tyr Asp Glu Glu Thr Ser
   1 5 10 15
(2) INFORMATION FOR SEQUENCE NO ID NO: 8:
   (i) SEQUENCE CHARACTERISTICS:
      (A) LENGTH : 1185
      (B) TYPE : nucleic acid
      (C) STRANDEDNESS : single
      (D) TOPOLOGY : linear
   (ii) MOLECULE TYPE : cDNA (OCIF2)
   (xi) SEQUENCE DESCRIPTION :SEQ ID NO:8
(2) INFORMATION FOR SEQUENCE ID NO: 9:
   (i) SEQUENCE CHARACTERISTICS:
      (A) LENGTH : 394
      (B) TYPE : amino acid
      (D) TOPOLOGY : linear
   (ii) MOLECULE TYPE : protein (OCIF2)
   (xi) SEQUENCE DESCRIPTION: SEQ ID NO: 9:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
        -5              -1   1               5
   Tyr Asp Glu Glu Thr Ser His Gln Leu Leu Cys Asp Lys Cys Pro
    10                  15                  20
   Pro Gly Thr Tyr Leu Lys Gln His Cys Thr Ala Lys Trp Lys Thr
    25                  30                  35
   Val Cys Ala Pro Cys Pro Asp His Tyr Tyr Thr Asp Ser Trp His
    40                  45                  50
   Thr Ser Asp Glu Cys Leu Tyr Cys Ser Pro Val Cys Lys Glu Cys
    55                  60                  65
   Asn Arg Thr His Asn Arg Val Cys Glu Cys Lys Glu Gly Arg Tyr
    70                  75                  80
   Leu Glu Ile Glu Phe Cys Leu Lys His Arg Ser Cys Pro Pro Gly
    85                  90                  95
   Phe Gly Val Val Gln Ala Gly Thr Pro Glu Arg Asn Thr Val Cys
   100                 105                 110
   Lys Arg Cys Pro Asp Gly Phe Phe Ser Asn Glu Thr Ser Ser Lys
   115                 120                 125
   Ala Pro Cys Arg Lys His Thr Asn Cys Ser Val Phe Gly Leu Leu
   130 135 140
   Leu Thr Gln Lys Gly Asn Ala Thr His Asp Asn Ile Cys Ser Gly
   145 150 155
   Asn Ser Glu Ser Thr Gln Lys Cys Gly Ile Asp Val Thr Leu Cys
   160 165 170
   Glu Glu Ala Phe Phe Arg Phe Ala Val Pro Thr Lys Phe Thr Pro
   175 180 185
   Asn Trp Leu Ser Val Leu Val Asp Asn Leu Pro Gly Thr Lys Val
   190 195 200
   Asn Ala Glu Ser Val Glu Arg Ile Lys Arg Gln His Ser Ser Gln
   205 210 215
   Glu Gln Thr Phe Gln Leu Leu Lys Leu Trp Lys His Gln Asn Lys
   220 225 230
   Asp Gln Asp Ile Val Lys Lys Ile Ile Gln Asp Ile Asp Leu Cys
   235 240 245
   Glu Asn Ser Val Gln Arg His Ile Gly His Ala Asn Leu Thr Phe
   250 255 260
   Glu Gln Leu Arg Ser Leu Met Glu Ser Leu Pro Gly Lys Lys Val
   265 270 275
   Gly Ala Glu Asp Ile Glu Lys Thr Ile Lys Ala Cys Lys Pro Ser
   280 285 290
   Asp Gln Ile Leu Lys Leu Leu Ser Leu Trp Arg Ile Lys Asn Gly
   295 300 305
   Asp Gln Asp Thr Leu Lys Gly Leu Met His Ala Leu Lys His Ser
   310 315 320
   Lys Thr Tyr His Phe Pro Lys Thr Val Thr Gln Ser Leu Lys Lys
   325 330 335
   Thr Ile Arg Phe Leu His Ser Phe Thr Met Tyr Lys Leu Tyr Gln
   340 345 350
   Lys Leu Phe Leu Glu Met Ile Gly Asn Gln Val Gln Ser Val Lys
   355 360 365
   Ile Ser Cys Leu
   370 373
(2) INFORMATION FOR SEQUENCE ID NO: 10:
   (i) SEQUENCE CHARACTERISTICS:
      (A) LENGTH : 1089
      (B) TYPE : nucleic acid
      (C) STRANDEDNESS : single
      (D) TOPOLOGY : linear
   (ii) MOLECULE TYPE : cDNA (OCIF3)
   (xi) SEQUENCE DESCRIPTION ID NO: 10:
   ATGAACAAGT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCC CTTGCCCTGA CCACTACTAC ACAGACAGCT GGCACACCAG TGACGAGTGT  240
   CTATACTGCA GCCCCGTGTG CAAGGAGCTG CAGTACGTCA AGCAGGAGTG CAATCGCACC  300
   CACAACCGCG TGTGCGAATG CAAGGAAGGG CGCTACCTTG AGATAGAGTT CTGCTTGAAA  360
   CATAGGAGCT GCCCTCCTGG ATTTGGAGTG GTGCAAGCTG GAACCCCAGA GCGAAATACA  420
   GTTTGCAAAA GATGTCCAGA TGGGTTCTTC TCAAATGAGA CGTCATCTAA AGCACCCTGT  480
   AGAAAACACA CAAATTGCAG TGTCTTTGGT CTCCTGCTAA CTCAGAAAGG AAATGCAACA  540
   CACGACAACA TATGTTCCGG AAACAGTGAA TCAACTCAAA AATGTGGAAT AGATGTTACC  600
   CTGTGTGAGG AGGCATTCTT CAGGTTTGCT GTTCCTACAA AGTTTACGCC TAACTGGCTT  660
   AGTGTCTTGG TAGACAATTT GCCTGGCACC AAAGTAAACG CAGAGAGTGT AGAGAGGATA  720
   AAACGGCAAC ACAGCTCACA AGAACAGACT TTCCAGCTGC TGAAGTTATG GAAACATCAA  780
   AACAAAGACC AAGATATAGT CAAGAAGATC ATCCAAGATA TTGACCTCTG TGAAAACAGC  840
   GTGCAGCGGC ACATTGGACA TGCTAACCTC AGTTTGTGGC GAATAAAAAA TGGCGACCAA  900
   GACACCTTGA AGGGCCTAAT GCACGCACTA AAGCACTCAA AGACGTACCA CTTTCCCAAA  960
   ACTGTCACTC AGAGTCTAAA GAAGACCATC AGGTTCCTTC ACAGCTTCAC AATGTACAAA 1020
   TTGTATCAGA AGTTATTTTT AGAAATGATA GGTAACCAGG TCCAATCAGT AAAAATAAGC 1080
   TGCTTATAA                                                         1089
(2) INFORMATION FOR SEQUENCE ID NO: 11:
   (i) SEQUENCE CHARACTERISTICS:
      (A) LENGTH : 362
      (B) TYPE : amino acid
      (C) STRANDEDNESS : single
      (D) TOPOLOGY : linear
   (ii) MOLECULE TYPE : protein (OCIF3)
   (xi) SEQUENCE DESCRIPTION: SEQ ID NO: 11:
   Met Asn Lys Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5              -1  1               5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys Arg Cys Pro Asp Gly Phe Phe
   115                 120                 125
   Ser Asn Glu Thr Ser Ser Lys Ala Pro Cys Arg Lys His Thr Asn
   130                 135                 140
   Cys Ser Val Phe Gly Leu Leu Leu Thr Gln Lys Gly Asn Ala Thr
   145                 150                 155
   His Asp Asn Ile Cys Ser Gly Asn Ser Glu Ser Thr Gln Lys Cys
   160                 165                 170
   Gly Ile Asp Val Thr Leu Cys Glu Glu Ala Phe Phe Arg Phe Ala
   175                 180                 185
   Val Pro Thr Lys Phe Thr Pro Asn Trp Leu Ser Val Leu Val Asp
   190                 195                 200
   Asn Leu Pro Gly Thr Lys Val Asn Ala Glu Ser Val Glu Arg Ile
   205                 210                 215
   Lys Arg Gln His Ser Ser Gln Glu Gln Thr Phe Gln Leu Leu Lys
   220                 225                 230
   Leu Trp Lys His Gln Asn Lys Asp Gln Asp Ile Val Lys Lys Ile
   235                 240                 245
   Ile Gln Asp Ile Asp Leu Cys Glu Asn Ser Val Gln Arg His Ile
   250 255 260
   Gly His Ala Asn Leu Ser Leu Trp Arg Ile Lys Asn Gly Asp Gln
   265 270 275
   Asp Thr Leu Lys Gly Leu Met His Ala Leu Lys His Ser Lys Thr
   280 285 290
   Tyr His Phe Pro Lys Thr Val Thr Gln Ser Leu Lys Lys Thr Ile
   295 300 305
   Arg Phe Leu His Ser Phe Thr Met Tyr Lys Leu Tyr Gln Lys Leu
   310 315 320
   Phe Leu Glu Met Ile Gly Asn Gln Val Gln Ser Val Lys Ile Ser
   325 330 335
   Cys Leu
   340 341
(2) INFORMATION FOR SEQUENCE ID NO: 12:
   (i) SEQUENCE CHARACTERISTICS:
      (A) LENGTH : 465
      (B) TYPE : nucleic acid
      (C) STRANDEDNESS : single
      (D) TOPOLOGY : linear
   (ii) MOLECULE TYPE : cDNA (OCIF4)
   (xi) SEQUENCE DESCRIPTION :SEQ ID NO: 12:
   ATGAACAAGT TGCTGTGCTG CTCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCC CTTGCCCTGA CCACTACTAC ACAGACAGCT GGCACACCAG TGACGAGTGT  240
   CTATACTGCA GCCCCGTGTG CAAGGAGCTG CAGTACGTCA AGCAGGAGTG CAATCGCACC  300
   CACAACCGCG TGTGCGAATG CAAGGAAGGG CGCTACCTTG AGATAGAGTT CTGCTTGAAA  360
   CATAGGAGCT GCCCTCCTGG ATTTGGAGTG GTGCAAGCTG GTACGTGTCA ATGTGCAGCA  420
   AAATTAATTA GGATCATGCA AAGTCAGATA GTTGTGACAG TTTAG                  465
(2) INFORMATION FOR SEQUENCE ID NO: 13:
   (i) SEQUENCE CHARACTERISTICS:
      (A) LENGTH :154
      (B) TYPE : amino acid
      (C) STRANDEDNESS : single
      (D) TOPOLOGY : linear
   (ii) MOLECULE TYPE : protein (OCIF4)
   (xi) SEQUENCE DESCRIPTION: SEQ ID NO: 13:
(2) INFORMATION FOR SEQUENCE ID NO: 14:
   (i) SEQUENCE CHARACTERISTICS:
      (A) LENGTH : 438
      (B) TYPE : nucleic acid
      (C) STRANDEDNESS : single
      (D) TOPOLOGY : linear
   (ii) MOLECULE TYPE : cDNA (OCIF5)
   (xi) SEQUENCE DESCRIPTION ID NO: 14:
   ATGAACAAGT TGCTGTGCTG CGCGCTCGTG TTTCTGGACA TCTCCATTAA GTGGACCACC    60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCC CTTGCCCTGA CCACTACTAC ACAGACAGCT GGCACACCAG TGACGAGTGT  240
   CTATACTGCA GCCCCGTGTG CAAGGAGCTG CAGTACGTCA AGCAGGAGTG CAATCGCACC  300
   CACAACCGCG TGTGCGAATG CAAGGAAGGG CGCTACCTTG AGATAGAGTT CTGCTTGAAA  360
   CATAGGAGCT GCCCTCCTGG ATTTGGAGTG GTGCAAGCTG GATGCAGGAG AAGACCCAAG  420
   CCACAGATAT GTATCTGA                                                 438
(2) INFORMATION FOR SEQUENCE ID NO: 15:
   (i) SEQUENCE CHARACTERISTICS:
      (A) LENGTH :140
      (B) TYPE : amino acid
      (C) STRANDEDNESS : single
      (D) TOPOLOGY : linear
   (ii) MOLECULE TYPE : protein (OCIF5)
   (xi) SEQUENCE DESCRIPTION: ID NO: 15:
(2) INFORMATION FOR SEQUENCE ID NO: 16:
   (i) SEQUENCE CHARACTERISTICS:
      (A) LENGTH : 20
      (B) TYPE : nucleic acid
      (C) STRANDEDNESS : single
      (D) TOPOLOGY : linear
   (ii) MOLECULE TYPE : synthetic DNA (primer T3)
   (xi) SEQUENCE DESCRIPTION: SEQ ID NO: 16:
      AATTAACCCT CACTAAAGGG 20
(2) INFORMATION FOR SEQUENCE ID NO: 17:
   (i) SEQUENCE CHARACTERISTICS:
      (A) LENGTH : 22
      (B) TYPE : nucleic acid
      (C) STRANDEDNESS : single
      (D) TOPOLOGY : linear
   (ii) MOLECULE TYPE : synthetic DNA (primer T7)
   (xi) SEQUENCE DESCRIPTION: SEQ ID NO: 17:
      GTAATACGAC TCACTATAGG GC 22
(2) INFORMATION FOR SEQUENCE ID NO: 18:
   (i) SEQUENCE CHARACTERISTICS:
      (A) LENGTH : 20
      (B) TYPE : nucleic acid
      (C) STRANDEDNESS : single
      (D) TOPOLOGY : linear
   (ii) MOLECULE TYPE : synthetic DNA (primer IF1)
   (xi) SEQUENCE DESCRIPTION :SEQ ID NO: 18:
      ACATCAAAAC AAAGACCAAG 20
(2) INFORMATION FOR SEQUENCE ID NO: 19:
   (i) SEQUENCE CHARACTERISTICS:
      (A) LENGTH : 20
      (B) TYPE : nucleic acid
      (C) STRANDEDNESS : single
      (D) TOPOLOGY : linear
   (ii) MOLECULE TYPE : synthetic DNA (primer IF2)
   (xi) SEQUENCE DESCRIPTION :SEQ ID NO: 19:
      TCTTGGTCTT TGTTTTGATG 20
(2) INFORMATION FOR SEQUENCE ID NO: 20:
   (i) SEQUENCE CHARACTERISTICS:
      (A) LENGTH : 20
      (B) TYPE : nucleic acid
      (C) STRANDEDNESS : single
      (D) TOPOLOGY : linear
   (ii) MOLECULE TYPE : synthetic DNA (primer IF3)
   (xi) SEQUENCE DESCRIPTION :SEQ ID NO: 20:
      TTATTCGCCA CAAACTGAGC 20
(2) INFORMATION FOR SEQUENCE ID NO: 21:
   (i) SEQUENCE CHARACTERISTICS:
      (A) LENGTH : 20
      (B) TYPE : nucleic acid
      (C) STRANDEDNESS : single
      (D) TOPOLOGY : linear
   (ii) MOLECULE TYPE : synthetic DNA (primer IF4)
   (xi) SEQUENCE DESCRIPTION :SEQ ID NO: 21:
      TTGTGAAGCT GTGAAGGAAC 20
(2) INFORMATION FOR SEQUENCE ID NO: 22:
   (i) SEQUENCE CHARACTERISTICS:
      (A) LENGTH : 20
      (B) TYPE : nucleic acid
      (C) STRANDEDNESS : single
      (D) TOPOLOGY : linear
   (ii) MOLECULE TYPE : synthetic DNA (primer IF5)
   (xi) SEQUENCE DESCRIPTION :SEQ ID NO: 22:
      GCTCAGTTTG TGGCGAATAA 20
(2) INFORMATION FOR SEQUENCE ID NO: 23:
   (i) SEQUENCE CHARACTERISTICS:
      (A) LENGTH : 20
      (B) TYPE : nucleic acid
      (C) STRANDEDNESS : single
      (D) TOPOLOGY : linear
   (ii) MOLECULE TYPE : synthetic DNA (primer IF6)
   (xi) SEQUENCE DESCRIPTION :SEQ ID NO: 23:
      GTGGGAGCAG AAGACATTGA 20
(2) INFORMATION FOR SEQUENCE ID NO: 24:
   (i) SEQUENCE CHARACTERISTICS:
      (A) LENGTH : 20
      (B) TYPE : nucleic acid
      (C) STRANDEDNESS : single
      (D) TOPOLOGY : linear
   (ii) MOLECULE TYPE : synthetic DNA (primer IF7)
   (xi) SEQUENCE DESCRIPTION :SEQ ID NO: 24:
      AATGAACAAC TTGCTGTGCT 20
(2) INFORMATION FOR SEQUENCE ID NO: 25:
   (i) SEQUENCE CHARACTERISTICS:
      (A) LENGTH : 20
      (B) TYPE : nucleic acid
      (C) STRANDEDNESS : single
      (D) TOPOLOGY : linear
   (ii) MOLECULE TYPE : synthetic DNA (primer IF8)
   (xi) SEQUENCE DESCRIPTION :SEQ ID NO: 25:
      TGACAAATGT CCTCCTGGTA 20
(2) INFORMATION FOR SEQUENCE ID NO: 26:
   (i) SEQUENCE CHARACTERISTICS:
      (A) LENGTH : 20
      (B) TYPE : nucleic acid
      (C) STRANDEDNESS : single
      (D) TOPOLOGY : linear
   (ii) MOLECULE TYPE : synthetic DNA (primer IF9)
   (xi) SEQUENCE DESCRIPTION :SEQ ID NO: 26:
      AGGTAGGTAC CAGGAGGACA 20
(2) INFORMATION FOR SEQUENCE ID NO: 27:
   (i) SEQUENCE CHARACTERISTICS:
      (A) LENGTH : 20
      (B) TYPE : nucleic acid
      (C) STRANDEDNESS : single
      (D) TOPOLOGY : linear
   (ii) MOLECULE TYPE : synthetic DNA (primer IF10)
   (xi) SEQUENCE DESCRIPTION :SEQ ID NO: 27:
      GAGCTGCCCT CCTGGATTTG 20
(2) INFORMATION FOR SEQUENCE ID NO: 28:
   (i) SEQUENCE CHARACTERISTICS:
      (A) LENGTH : 20
      (B) TYPE : nucleic acid
      (C) STRANDEDNESS : single
      (D) TOPOLOGY : linear
   (ii) MOLECULE TYPE : synthetic DNA (primer IF11)
   (xi) SEQUENCE DESCRIPTION :SEQ ID NO: 28:
      CAAACTGTAT TTCGCTCTGG 20
(2) INFORMATION FOR SEQUENCE ID NO: 29:
   (i) SEQUENCE CHARACTERISTICS:
      (A) LENGTH : 20
      (B) TYPE : nucleic acid
      (C) STRANDEDNESS : single
      (D) TOPOLOGY : linear
   (ii) MOLECULE TYPE : synthetic DNA (primer IF12)
   (xi) SEQUENCE DESCRIPTION :SEQ ID NO: 29:
      GTGTGAGGAG GCATTCTTCA 20
(2) INFORMATION FOR SEQUENCE ID NO: 30:
   (i) SEQUENCE CHARACTERISTICS:
      (A) LENGTH : 32
      (B) TYPE : nucleic acid
      (C) STRANDEDNESS : single
      (D) TOPOLOGY : linear
   (ii) MOLECULE TYPE : synthetic DNA (primer C19SF)
   (xi) SEQUENCE DESCRIPTION :SEQ ID NO: 30:
      GAATCAACTC AAAAAAGTGG AATAGATGTT AC 32
(2) INFORMATION FOR SEQUENCE ID NO: 31:
   (i) SEQUENCE CHARACTERISTICS:
      (A) LENGTH : 32
      (B) TYPE : nucleic acid
      (C) STRANDEDNESS : single
      (D) TOPOLOGY : linear
   (ii) MOLECULE TYPE : synthetic DNA (primer C19SR)
   (xi) SEQUENCE DESCRIPTION :SEQ ID NO: 31:
      GTAACATCTA TTCCACTTTT TTGAGTTGAT TC 32
(2) INFORMATION FOR SEQUENCE ID NO: 32:
   (i) SEQUENCE CHARACTERISTICS:
      (A) LENGTH : 30
      (B) TYPE : nucleic acid
      (C) STRANDEDNESS : single
      (D) TOPOLOGY : linear
   (ii) MOLECULE TYPE : synthetic DNA (primer C20SF)
   (xi) SEQUENCE DESCRIPTION :SEQ ID NO: 32:
      ATAGATGTTA CCCTGAGTGA GGAGGCATTC 30
(2) INFORMATION FOR SEQUENCE ID NO: 33:
   (i) SEQUENCE CHARACTERISTICS:
      (A) LENGTH : 30
      (B) TYPE : nucleic acid
      (C) STRANDEDNESS : single
      (D) TOPOLOGY : linear
   (ii) MOLECULE TYPE : synthetic DNA (primer C20SR)
   (xi) SEQUENCE DESCRIPTION :SEQ ID NO: 33:
      GAATGCCTCC TCACTCAGGG TAACATCTAT 30
(2) INFORMATION FOR SEQUENCE ID NO: 34:
   (i) SEQUENCE CHARACTERISTICS:
      (A) LENGTH : 31
      (B) TYPE : nucleic acid
      (C) STRANDEDNESS : single
      (D) TOPOLOGY : linear
   (ii) MOLECULE TYPE : synthetic DNA (primer C21SF)
   (xi) SEQUENCE DESCRIPTION :SEQ ID NO: 34:
      CAAGATATTG ACCTCAGTGA AAACAGCGTG C 31
(2) INFORMATION FOR SEQUENCE ID NO: 35:
   (i) SEQUENCE CHARACTERISTICS:
      (A) LENGTH : 31
      (B) TYPE : nucleic acid
      (C) STRANDEDNESS : single
      (D) TOPOLOGY : linear
   (ii) MOLECULE TYPE : synthetic DNA (primer C21SR)
   (xi) SEQUENCE DESCRIPTION :SEQ ID NO: 35:
      GCACGCTGTT TTCACTGAGG GCAATATCTT G 31
(2) INFORMATION FOR SEQUENCE ID NO: 36:
   (i) SEQUENCE CHARACTERISTICS:
      (A) LENGTH : 31
      (B) TYPE : nucleic acid
      (C) STRANDEDNESS : single
      (D) TOPOLOGY : linear
   (ii) MOLECULE TYPE : synthetic DNA (primer C22SF)
   (xi) SEQUENCE DESCRIPTION :SEQ ID NO: 36:
      AAAACAATAA AGGCAAGCAA ACCCAGTGAC C 31
(2) INFORMATION FOR SEQUENCE ID NO: 37:
   (i) SEQUENCE CHARACTERISTICS:
      (A) LENGTH : 31
      (B) TYPE : nucleic acid
      (C) STRANDEDNESS : single
      (D) TOPOLOGY : linear
   (ii) MOLECULE TYPE : synthetic DNA (primer C22SR)
   (xi) SEQUENCE DESCRIPTION :SEQ ID NO: 37:
      GGTCACTGGG TTTGCTTGCC TTTATTGTTT T 31
(2) INFORMATION FOR SEQUENCE ID NO: 38:
   (i) SEQUENCE CHARACTERISTICS:
      (A) LENGTH : 31
      (B) TYPE : nucleic acid
      (C) STRANDEDNESS : single
      (D) TOPOLOGY : linear
   (ii) MOLECULE TYPE : synthetic DNA (primer C23SF)
   (xi) SEQUENCE DESCRIPTION :SEQ ID NO: 38:
      TCAGTAAAAA TAAGCAGCTT ATAACTGGCC A 31
(2) INFORMATION FOR SEQUENCE ID NO: 39:
   (i) SEQUENCE CHARACTERISTICS:
      (A) LENGTH : 31
      (B) TYPE : nucleic acid
      (C) STRANDEDNESS : single
      (D) TOPOLOGY : linear
   (ii) MOLECULE TYPE : synthetic DNA (primer C23SR)
   (xi) SEQUENCE DESCRIPTION :SEQ ID NO: 39:
      TGGCCAGTTA TAAGCTGCTT ATTTTTACTG A 31
(2) INFORMATION FOR SEQUENCE ID NO: 40:
   (i) SEQUENCE CHARACTERISTICS:
      (A) LENGTH : 22
      (B) TYPE : nucleic acid
      (C) STRANDEDNESS : single
      (D) TOPOLOGY : linear
   (ii) MOLECULE TYPE : synthetic DNA (primer IF 14)
   (xi) SEQUENCE DESCRIPTION :SEQ ID NO: 40:
      TTGGGGTTTA TTGGAGGAGA TG 22
(2) INFORMATION FOR SEQUENCE ID NO: 41:
   (i) SEQUENCE CHARACTERISTICS:
      (A) LENGTH : 36
      (B) TYPE : nucleic acid
      (C) STRANDEDNESS : single
      (D) TOPOLOGY : linear
   (ii) MOLECULE TYPE : synthetic DNA (primer DCR1F)
   (xi) SEQUENCE DESCRIPTION :SEQ ID NO: 41:
      ACCACCCAGG AACCTTGCCC TGACCACTAC TACACA 36
(2) INFORMATION FOR SEQUENCE ID NO: 42:
   (i) SEQUENCE CHARACTERISTICS:
      (A) LENGTH : 36
      (B) TYPE : nucleic acid
      (C) STRANDEDNESS : single
      (D) TOPOLOGY : linear
   (ii) MOLECULE TYPE : synthetic DNA (primer DCR1R)
   (xi) SEQUENCE DESCRIPTION :SEQ ID NO: 42:
      GTCAGGGCAA GGTTCCTGGG TGGTCCACTT AATGGA 36
(2) INFORMATION FOR SEQUENCE ID NO: 43:
   (i) SEQUENCE CHARACTERISTICS:
      (A) LENGTH : 36
      (B) TYPE : nucleic acid
      (C) STRANDEDNESS : single
      (D) TOPOLOGY : linear
   (ii) MOLECULE TYPE : synthetic DNA (primer DCR2F)
   (xi) SEQUENCE DESCRIPTION :SEQ ID NO: 43:
      ACCGTGTGCG CCGAATGCAA GGAAGGGCGC TACCTT 36
(2) INFORMATION FOR SEQUENCE ID NO: 44:
   (i) SEQUENCE CHARACTERISTICS:
      (A) LENGTH : 36
      (B) TYPE : nucleic acid
      (C) STRANDEDNESS : single
      (D) TOPOLOGY : linear
   (ii) MOLECULE TYPE : synthetic DNA (primer DCR2R)
   (xi) SEQUENCE DESCRIPTION :SEQ ID NO: 44:
      TTCCTTGCAT TCGGCGCACA CGGTCTTCCA CTTTGC 36
(2) INFORMATION FOR SEQUENCE ID NO: 45:
   (i) SEQUENCE CHARACTERISTICS: -
      (A) LENGTH : 36
      (B) TYPE : nucleic acid
      (C) STRANDEDNESS : single
      (D) TOPOLOGY : linear
   (ii) MOLECULE TYPE : synthetic DNA (primer DCR3F)
   (xi) SEQUENCE DESCRIPTION : SEQ ID NO: 45:
      AACCGCGTGT GCAGATGTCC AGATGGGTTC TTCTCA 36
(2) INFORMATION FOR SEQUENCE ID NO: 46:
   (i) SEQUENCE CHARACTERISTICS:
      (A) LENGTH : 36
      (B) TYPE : nucleic acid
      (C) STRANDEDNESS : single
      (D) TOPOLOGY : linear
   (ii) MOLECULE TYPE : synthetic DNA (primer DCR3R)
   (xi) SEQUENCE DESCRIPTION :SEQ ID NO: 46:
      ATCTGGACAT CTGCACACGC GGTTGTGGGT GCGATT 36
(2) INFORMATION FOR SEQUENCE ID NO: 47:
   (i) SEQUENCE CHARACTERISTICS:
      (A) LENGTH : 36
      (B) TYPE : nucleic acid
      (C) STRANDEDNESS : single
      (D) TOPOLOGY : linear
   (ii) MOLECULE TYPE : synthetic DNA (primer DCR4F)
   (xi) SEQUENCE DESCRIPTION :SEQ ID NO: 47:
      ACAGTTTGCA AATCCGGAAA CAGTGAATCA ACTCAA 36
(2) INFORMATION FOR SEQUENCE ID NO: 48:
   (i) SEQUENCE CHARACTERISTICS:
      (A) LENGTH : 36
      (B) TYPE : nucleic acid
      (C) STRANDEDNESS : single
      (D) TOPOLOGY : linear
   (ii) MOLECULE TYPE : synthetic DNA (primer DCR4R)
   (xi) SEQUENCE DESCRIPTION :SEQ ID NO: 48:
      ACTGTTTCCG GATTTGCAAA CTGTATTTCG CTCTGG 36
(2) INFORMATION FOR SEQUENCE ID NO: 49:
   (i) SEQUENCE CHARACTERISTICS:
      (A) LENGTH : 36
      (B) TYPE : nucleic acid
      (C) STRANDEDNESS : single
      (D) TOPOLOGY : linear
   (ii) MOLECULE TYPE : synthetic DNA (primer DDD1F)
   (xi) SEQUENCE DESCRIPTION :SEQ ID NO: 49:
      AATGTGGAAT AGATATTGAC CTCTGTGAAA ACAGCG 36
(2) INFORMATION FOR SEQUENCE ID NO: 50:
   (i) SEQUENCE CHARACTERISTICS:
      (A) LENGTH : 36
      (B) TYPE : nucleic acid
      (C) STRANDEDNESS : single
      (D) TOPOLOGY : linear
   (ii) MOLECULE TYPE : synthetic DNA (primer DDD1R)
   (xi) SEQUENCE DESCRIPTION :SEQ ID NO: 50:
      AGAGGTCAAT ATCTATTCCA CATTTTTGAG TTGATT 36
(2) INFORMATION FOR SEQUENCE ID NO: 51:
   (i) SEQUENCE CHARACTERISTICS:
      (A) LENGTH : 36
      (B) TYPE : nucleic acid
      (C) STRANDEDNESS : single
      (D) TOPOLOGY : linear
   (ii) MOLECULE TYPE : synthetic DNA (primer DDD2F)
   (xi) SEQUENCE DESCRIPTION :SEQ ID NO: 51:
      AGATCATCCA AGACGCACTA AAGCACTCAA AGACGT 36
(2) INFORMATION FOR SEQUENCE ID NO: 52:
   (i) SEQUENCE CHARACTERISTICS:
      (A) LENGTH : 36
      (B) TYPE : nucleic acid
      (C) STRANDEDNESS : single
      (D) TOPOLOGY : linear
   (ii) MOLECULE TYPE : synthetic DNA (primer DDD2R)
   (xi) SEQUENCE DESCRIPTION :SEQ ID NO: 52:
      GCTTTAGTGC GTCTTGGATG ATCTTCTTGA CTATAT 36
(2) INFORMATION FOR SEQUENCE ID NO: 53:
   (i) SEQUENCE CHARACTERISTICS:
      (A) LENGTH : 29
      (B) TYPE : nucleic acid
      (C) STRANDEDNESS : single
      (D) TOPOLOGY : linear
   (ii) MOLECULE TYPE : synthetic DNA (primer XhoI F)
   (xi) SEQUENCE DESCRIPTION :SEQ ID NO: 53:
      GGCTCGAGCG CCCAGCCGCC GCCTCCAAG 29
(2) INFORMATION FOR SEQUENCE ID NO: 54:
   (i) SEQUENCE CHARACTERISTICS:
      (A) LENGTH : 20
      (B) TYPE : nucleic acid
      (C) STRANDEDNESS : single
      (D) TOPOLOGY : linear
   (ii) MOLECULE TYPE : synthetic DNA (primer IF 16)
   (xi) SEQUENCE DESCRIPTION :SEQ ID NO: 54:
      TTTGAGTGCT TTAGTGCGTG 20
(2) INFORMATION FOR SEQUENCE ID NO: 55:
   (i) SEQUENCE CHARACTERISTICS:
      (A) LENGTH : 30
      (B) TYPE : nucleic acid
      (C) STRANDEDNESS : single
      (D) TOPOLOGY : linear
   (ii) MOLECULE TYPE : synthetic DNA (primer CL F)
   (xi) SEQUENCE DESCRIPTION :SEQ ID NO: 55:
      TCAGTAAAAA TAAGCTAACT GGAAATGGCC 30
(2) INFORMATION FOR SEQUENCE ID NO: 56:
   (i) SEQUENCE CHARACTERISTICS:
      (A) LENGTH : 30
      (B) TYPE : nucleic acid
      (C) STRANDEDNESS : single
      (D) TOPOLOGY : linear
   (ii) MOLECULE TYPE : synthetic DNA (primer CL R)
   (xi) SEQUENCE DESCRIPTION :SEQ ID NO: 56:
      GGCCATTTCC AGTTAGCTTA TTTTTACTGA 30
(2) INFORMATION FOR SEQUENCE ID NO: 57:
   (i) SEQUENCE CHARACTERISTICS:
      (A) LENGTH : 29
      (B) TYPE : nucleic acid
      (C) STRANDEDNESS : single
      (D) TOPOLOGY : linear
   (ii) MOLECULE TYPE : synthetic DNA (primer CC R)
   (xi) SEQUENCE DESCRIPTION :SEQ ID NO: 57:
      CCGGATCCTC AGTGCTTTAG TGCGTGCAT 29
(2) INFORMATION FOR SEQUENCE ID NO: 58:
   (i) SEQUENCE CHARACTERISTICS:
      (A) LENGTH : 29
      (B) TYPE : nucleic acid
      (C) STRANDEDNESS : single
      (D) TOPOLOGY : linear
   (ii) MOLECULE TYPE : synthetic DNA (primer CCD2 R)
   (xi) SEQUENCE DESCRIPTION :SEQ ID NO: 58:
      CCGGATCCTC ATTGGATGAT CTTCTTGAC 29
(2) INFORMATION FOR SEQUENCE ID NO: 59:
   (i) SEQUENCE CHARACTERISTICS:
      (A) LENGTH : 29
      (B) TYPE : nucleic acid
      (C) STRANDEDNESS : single
      (D) TOPOLOGY : linear
   (ii) MOLECULE TYPE : synthetic DNA (primer CCD1 R)
   (xi) SEQUENCE DESCRIPTION :SEQ ID NO: 59:
      CCGGATCCTC ATATTCCACA TTTTTGAGT 29
(2) INFORMATION FOR SEQUENCE ID NO: 60:
   (i) SEQUENCE CHARACTERISTICS:
      (A) LENGTH : 29
      (B) TYPE : nucleic acid
      (C) STRANDEDNESS : single
      (D) TOPOLOGY : linear
   (ii) MOLECULE TYPE : synthetic DNA (primer CCR4 R)
   (xi) SEQUENCE DESCRIPTION :SEQ ID NO: 60:
      CCGGATCCTC ATTTGCAAAC TGTATTTCG 29
(2) INFORMATION FOR SEQUENCE ID NO: 61:
   (i) SEQUENCE CHARACTERISTICS:
      (A) LENGTH : 29
      (B) TYPE : nucleic acid
      (C) STRANDEDNESS : single
      (D) TOPOLOGY : linear
   (ii) MOLECULE TYPE : synthetic DNA (primer CCR3 R)
   (xi) SEQUENCE DESCRIPTION :SEQ ID NO: 61:
      CCGGATCCTC ATTCGCACAC GCGGTTGTG 29
(2) INFORMATION FOR SEQUENCE ID NO: 62:
   (i) SEQUENCE CHARACTERISTICS:
      (A) LENGTH : 401
      (B) TYPE : amino acid
      (C) STRANDEDNESS : single
      (D) TOPOLOGY : linear
   (ii) MOLECULE TYPE : Protein (DCIF-C19S)
   (xi) SEQUENCE DESCRIPTION :SEQ ID NO: 62:
   Met Asn Asn Leu Leu Cys Cys Ala Leu Val Phe Leu Asp Ile Ser
   -20 -15 -10
   Ile Lys Trp Thr Thr Gln Glu Thr Phe Pro Pro Lys Tyr Leu His
   -5 -1 1 5
   Tyr Asp Glu Glu Thr Ser His Gln Leu Leu Cys Asp Lys Cys Pro
   10 15 20
   Pro Gly Thr Tyr Leu Lys Gln His Cys Thr Ala Lys Trp Lys Thr
   25 30 35
   Val Cys Ala Pro Cys Pro Asp His Tyr Tyr Thr Asp Ser Trp His
   40 45 50
   Thr Ser Asp Glu Cys Leu Tyr Cys Ser Pro Val Cys Lys Glu Leu
   55 60 65
   Gln Tyr Val Lys Gln Glu Cys Asn Arg Thr His Asn Arg Val Cys
   70 75 80
   Glu Cys Lys Glu Gly Arg Tyr Leu Glu Ile Glu Phe Cys Leu Lys
   85 90 95
   His Arg Ser Cys Pro Pro Gly Phe Gly Val Val Gln Ala Gly Thr
   100 105 110
   Pro Glu Arg Asn Thr Val Cys Lys Arg Cys Pro Asp Gly Phe Phe
   115 120 125
   Ser Asn Glu Thr Ser Ser Lys Ala Pro Cys Arg Lys His Thr Asn
   130 135 140
   Cys Ser Val Phe Gly Leu Leu Leu Thr Gln Lys Gly Asn Ala Thr
   145 150 155
   His Asp Asn Ile Cys Ser Gly Asn Ser Glu Ser Thr Gln Lys Ser
   160 165 170
   Gly Ile Asp Val Thr Leu Cys Glu Glu Ala Phe Phe Arg Phe Ala
   175 180 185
   Val Pro Thr Lys Phe Thr Pro Asn Trp Leu Ser Val Leu Val Asp
   190 195 200
   Asn Leu Pro Gly Thr Lys Val Asn Ala Glu Ser Val Glu Arg Ile
   205 210 215
   Lys Arg Gln His Ser Ser Gln Glu Gln Thr Phe Gln Leu Leu Lys
   220 225 230
   Leu Trp Lys His Gln Asn Lys Asp Gln Asp Ile Val Lys Lys Ile
   235 240 245
   Ile Gln Asp Ile Asp Leu Cys Glu Asn Ser Val Gln Arg His Ile
   250 255 260
   Gly His Ala Asn Leu Thr Phe Glu Gln Leu Arg Ser Leu Met Glu
   265 270 275
   Ser Leu Pro Gly Lys Lys Val Gly Ala Glu Asp Ile Glu Lys Thr
   280 285 290
   Ile Lys Ala Cys Lys Pro Ser Asp Gln Ile Leu Lys Leu Leu Ser
   295 300 305
   Leu Trp Arg Ile Lys Asn Gly Asp Gln Asp Thr Leu Lys Gly Leu
   310 315 320
   Met His Ala Leu Lys His Ser Lys Thr Tyr His Phe Pro Lys Thr
   325 330 335
   Val Thr Gln Ser Leu Lys Lys Thr Ile Arg Phe Leu His Ser Phe
   340 345 350
   Thr Met Tyr Lys Leu Tyr Gln Lys Leu Phe Leu Glu Met Ile Gly
   355 360 365
   Asn Gln Val Gln Ser Val Lys Ile Ser Cys Leu
   370 375 380
(2) INFORMATION FOR SEQUENCE ID NO: 63:
   (i) SEQUENCE CHARACTERISTICS:
      (A) LENGTH : 401
      (B) TYPE : amino acid
      (C) STRANDEDNESS : single
      (D) TOPOLOGY : linear
   (ii) MOLECULE TYPE : Protein (OCIF-C20S)
   (xi) SEQUENCE DESCRIPTION :SEQ ID NO: 63:
   Met Asn Asn Leu Leu Cys Cys Ala Leu Val Phe Leu Asp Ile Ser
   -20 -15 -10
   Ile Lys Trp Thr Thr Gln Glu Thr Phe Pro Pro Lys Tyr Leu His
   -5 -1 1 5
   Tyr Asp Glu Glu Thr Ser His Gln Leu Leu Cys Asp Lys Cys Pro
   10 15 20
   Pro Gly Thr Tyr Leu Lys Gln His Cys Thr Ala Lys Trp Lys Thr
   25 30 35
   Val Cys Ala Pro Cys Pro Asp His Tyr Tyr Thr Asp Ser Trp His
   40 45 50
   Thr Ser Asp Glu Cys Leu Tyr Cys Ser Pro Val Cys Lys Glu Leu
   55 60 65
   Gln Tyr Val Lys Gln Glu Cys Asn Arg Thr His Asn Arg Val Cys
   70 75 80
   Glu Cys Lys Glu Gly Arg Tyr Leu Glu Ile Glu Phe Cys Leu Lys
   85 90 95
   His Arg Ser Cys Pro Pro Gly Phe Gly Val Val Gln Ala Gly Thr
   100 105 110
   Pro Glu Arg Asn Thr Val Cys Lys Arg Cys Pro Asp Gly Phe Phe
   115 120 125
   Ser Asn Glu Thr Ser Ser Lys Ala Pro Cys Arg Lys His Thr Asn
   130 135 140
   Cys Ser Val Phe Gly Leu Leu Leu Thr Gln Lys Gly Asn Ala Thr
   145 150 155
   His Asp Asn Ile Cys Ser Gly Asn Ser Glu Ser Thr Gln Lys Cys
   160 165 170
   Gly Ile Asp Val Thr Leu Ser Glu Glu Ala Phe Phe Arg Phe Ala
   175 180 185
   Val Pro Thr Lys Phe Thr Pro Asn Trp Leu Ser Val Leu Val Asp
   190 195 200
   Asn Leu Pro Gly Thr Lys Val Asn Ala Glu Ser Val Glu Arg Ile
   205 210 215
   Lys Arg Gln His Ser Ser Gln Glu Gln Thr Phe Gln Leu Leu Lys
   220 225 230
   Leu Trp Lys His Gln Asn Lys Asp Gln Asp Ile Val Lys Lys Ile
   235 240 245
   Ile Gln Asp Ile Asp Leu Cys Glu Asn Ser Val Gln Arg His Ile
   250 255 260
   Gly His Ala Asn Leu Thr Phe Glu Gln Leu Arg Ser Leu Met Glu
   265 270 275
   Ser Leu Pro Gly Lys Lys Val Gly Ala Glu Asp Ile Glu Lys Thr
   280 285 290
   Ile Lys Ala Cys Lys Pro Ser Asp Gln Ile Leu Lys Leu Leu Ser
   295 300 305
   Leu Trp Arg Ile Lys Asn Gly Asp Gln Asp Thr Leu Lys Gly Leu
   310 315 320
   Met His Ala Leu Lys His Ser Lys Thr Tyr His Phe Pro Lys Thr
   325 330 335
   Val Thr Gln Ser Leu Lys Lys Thr Ile Arg Phe Leu His Ser Phe
   340 345 350
   Thr Met Tyr Lys Leu Tyr Gln Lys Leu Phe Leu Glu Met Ile Gly
   355 360 365
   Asn Gln Val Gln Ser Val Lys Ile Ser Cys Leu
   370 375 380
(2) INFORMATION FOR SEQUENCE ID NO: 64:
   (i) SEQUENCE CHARACTERISTICS:
      (A) LENGTH : 401
      (B) TYPE : amino acid
      (C) STRANDEDNESS : single
      (D) TOPOLOGY : linear
   (ii) MOLECULE TYPE : Protein (OCIF-C21S)
   (xi) SEQUENCE DESCRIPTION :SEQ ID NO: 64:
   Met Asn Asn Leu Leu Cys Cys Ala Leu Val Phe Leu Asp Ile Ser
   -20 -15 -10
   Ile Lys Trp Thr Thr Gln Glu Thr Phe Pro Pro Lys Tyr Leu His
   -5 -1 1 5
   Tyr Asp Glu Glu Thr Ser His Gln Leu Leu Cys Asp Lys Cys Pro
   10 15 20
   Pro Gly Thr Tyr Leu Lys Gln His Cys Thr Ala Lys Trp Lys Thr
   25 30 35
   Val Cys Ala Pro Cys Pro Asp His Tyr Tyr Thr Asp Ser Trp His
   40 45 50
   Thr Ser Asp Glu Cys Leu Tyr Cys Ser Pro Val Cys Lys Glu Leu
   55 60 65
   Gln Tyr Val Lys Gln Glu Cys Asn Arg Thr His Asn Arg Val Cys
   70 75 80
   Glu Cys Lys Glu Gly Arg Tyr Leu Glu Ile Glu Phe Cys Leu Lys
   85 90 95
   His Arg Ser Cys Pro Pro Gly Phe Gly Val Val Gln Ala Gly Thr
   100 105 110
   Pro Glu Arg Asn Thr Val Cys Lys Arg Cys Pro Asp Gly Phe Phe
   115 120 125
   Ser Asn Glu Thr Ser Ser Lys Ala Pro Cys Arg Lys His Thr Asn
   130 135 140
   Cys Ser Val Phe Gly Leu Leu Leu Thr Gln Lys Gly Asn Ala Thr
   145 150 155
   His Asp Asn Ile Cys Ser Gly Asn Ser Glu Ser Thr Gln Lys Cys
   160 165 170
   Gly Ile Asp Val Thr Leu Cys Glu Glu Ala Phe Phe Arg Phe Ala
   175 180 185
   Val Pro Thr Lys Phe Thr Pro Asn Trp Leu Ser Val Leu Val Asp
   190 195 200
   Asn Leu Pro Gly Thr Lys Val Asn Ala Glu Ser Val Glu Arg Ile
   205 210 215
   Lys Arg Gln His Ser Ser Gln Glu Gln Thr Phe Gln Leu Leu Lys
   220 225 230
   Leu Trp Lys His Gln Asn Lys Asp Gln Asp Ile Val Lys Lys Ile
   235 240 245
   Ile Gln Asp Ile Asp Leu Ser Glu Asn Ser Val Gln Arg His Ile
   250 255 260
   Gly His Ala Asn Leu Thr Phe Glu Gln Leu Arg Ser Leu Met Glu
   265 270 275
   Ser Leu Pro Gly Lys Lys Val Gly Ala Glu Asp Ile Glu Lys Thr
   280 285 290
   Ile Lys Ala Cys Lys Pro Ser Asp Gln Ile Leu Lys Leu Leu Ser
   295 300 305
   Leu Trp Arg Ile Lys Asn Gly Asp Gln Asp Thr Leu Lys Gly Leu
   310 315 320
   Met His Ala Leu Lys His Ser Lys Thr Tyr His Phe Pro Lys Thr
   325 330 335
   Val Thr Gln Ser Leu Lys Lys Thr Ile Arg Phe Leu His Ser Phe
   340 345 350
   Thr Met Tyr Lys Leu Tyr Gln Lys Leu Phe Leu Glu Met Ile Gly
   355 360 365
   Asn Gln Val Gln Ser Val Lys Ile Ser Cys Leu
   370 375 380
(2) INFORMATION FOR SEQUENCE ID NO: 65:
   (i) SEQUENCE CHARACTERISTICS:
      (A)LENGTH : 401
      (B) TYPE : amino acid
      (C) STRANDEDNESS : single
      (D) TOPOLOGY : linear
   (ii) MOLECULE TYPE : Protein (OCIF-C22S)
   (xi) SEQUENCE DESCRIPTION :SEQ ID NO: 65:
   Met Asn Asn Leu Leu Cys Cys Ala Leu Val Phe Leu Asp Ile Ser
   -20 -15 -10
   Ile Lys Trp Thr Thr Gln Glu Thr Phe Pro Pro Lys Tyr Leu His
   -5 -1 1 5
   Tyr Asp Glu Glu Thr Ser His Gln Leu Leu Cys Asp Lys Cys Pro
   10 15 20
   Pro Gly Thr Tyr Leu Lys Gln His Cys Thr Ala Lys Trp Lys Thr
   25 30 35
   Val Cys Ala Pro Cys Pro Asp His Tyr Tyr Thr Asp Ser Trp His
   40 45 50
   Thr Ser Asp Glu Cys Leu Tyr Cys Ser Pro Val Cys Lys Glu Leu
   55 60 65
   Gln Tyr Val Lys Gln Glu Cys Asn Arg Thr His Asn Arg Val Cys
   70 75 80
   Glu Cys Lys Glu Gly Arg Tyr Leu Glu Ile Glu Phe Cys Leu Lys
   85 90 95
   His Arg Ser Cys Pro Pro Gly Phe Gly Val Val Gln Ala Gly Thr
   100 105 110
   Pro Glu Arg Asn Thr Val Cys Lys Arg Cys Pro Asp Gly Phe Phe
   115 120 125
   Ser Asn Glu Thr Ser Ser Lys Ala Pro Cys Arg Lys His Thr Asn
   130 135 140
   Cys Ser Val Phe Gly Leu Leu Leu Thr Gln Lys Gly Asn Ala Thr
   145 150 155
   His Asp Asn Ile Cys Ser Gly Asn Ser Glu Ser Thr Gln Lys Cys
   160 165 170
   Gly Ile Asp Val Thr Leu Cys Glu Glu Ala Phe Phe Arg Phe Ala
   175 180 185
   Val Pro Thr Lys Phe Thr Pro Asn Trp Leu Ser Val Leu Val Asp
   190 195 200
   Asn Leu Pro Gly Thr Lys Val Asn Ala Glu Ser Val Glu Arg Ile
   205 210 215
   Lys Arg Gln His Ser Ser Gln Glu Gln Thr Phe Gln Leu Leu Lys
   220 225 230
   Leu Trp Lys His Gln Asn Lys Asp Gln Asp Ile Val Lys Lys Ile
   235 240 245
   Ile Gln Asp Ile Asp Leu Cys Glu Asn Ser Val Gln Arg His Ile
   250 255 260
   Gly His Ala Asn Leu Thr Phe Glu Gln Leu Arg Ser Leu Met Glu
   265 270 275
   Ser Leu Pro Gly Lys Lys Val Gly Ala Glu Asp Ile Glu Lys Thr
   280 285 290
   Ile Lys Ala Ser Lys Pro Ser Asp Gln Ile Leu Lys Leu Leu Ser
   295 300 305
   Leu Trp Arg Ile Lys Asn Gly Asp Gln Asp Thr Leu Lys Gly Leu
   310 315 320
   Met His Ala Leu Lys His Ser Lys Thr Tyr His Phe Pro Lys Thr
   325 330 335
   Val Thr Gln Ser Leu Lys Lys Thr Ile Arg Phe Leu His Ser Phe
   340 345 350
   Thr Met Tyr Lys Leu Tyr Gln Lys Leu Phe Leu Glu Met Ile Gly
   355 360 365
   Asn Gln Val Gln Ser Val Lys Ile Ser Cys Leu
   370 375 380
(2) INFORMATION FOR SEQUENCE ID NO: 66:
   (i) SEQUENCE CHARACTERISTICS:
      (A) LENGTH : 401
      (B) TYPE : amino acid
      (C) STRANDEDNESS : single
      (D) TOPOLOGY : linear
   (ii) MOLECULE TYPE : Protein (OCIF-C23S)
   (xi) SEQUENCE DESCRIPTION :SEQ ID NO: 66:
   Met Asn Asn Leu Leu Cys Cys Ala Leu Val Phe Leu Asp Ile Ser
   -20 -15 -10
   Ile Lys Trp Thr Thr Gln Glu Thr Phe Pro Pro Lys Tyr Leu His
   -5 -1 1 5
   Tyr Asp Glu Glu Thr Ser His Gln Leu Leu Cys Asp Lys Cys Pro
   10 15 20
   Pro Gly Thr Tyr Leu Lys Gln His Cys Thr Ala Lys Trp Lys Thr
   25 30 35
   Val Cys Ala Pro Cys Pro Asp His Tyr Tyr Thr Asp Ser Trp His
   40 45 50
   Thr Ser Asp Glu Cys Leu Tyr Cys Ser Pro Val Cys Lys Glu Leu
   55 60 65
   Gln Tyr Val Lys Gln Glu Cys Asn Arg Thr His Asn Arg Val Cys
   70 75 80
   Glu Cys Lys Glu Gly Arg Tyr Leu Glu Ile Glu Phe Cys Leu Lys
   85 90 95
   His Arg Ser Cys Pro Pro Gly Phe Gly Val Val Gln Ala Gly Thr
   100 105 110
   Pro Glu Arg Asn Thr Val Cys Lys Arg Cys Pro Asp Gly Phe Phe
   115 120 125
   Ser Asn Glu Thr Ser Ser Lys Ala Pro Cys Arg Lys His Thr Asn
   130 135 140
   Cys Ser Val Phe Gly Leu Leu Leu Thr Gln Lys Gly Asn Ala Thr
   145 150 155
   His Asp Asn Ile Cys Ser Gly Asn Ser Glu Ser Thr Gln Lys Cys
   160 165 170
   Gly Ile Asp Val Thr Leu Cys Glu Glu Ala Phe Phe Arg Phe Ala
   175 180 185
   Val Pro Thr Lys Phe Thr Pro Asn Trp Leu Ser Val Leu Val Asp
   190 195 200
   Asn Leu Pro Gly Thr Lys Val Asn Ala Glu Ser Val Glu Arg Ile
   205 210 215
   Lys Arg Gln His Ser Ser Gln Glu Gln Thr Phe Gln Leu Leu Lys
   220 225 230
   Leu Trp Lys His Gln Asn Lys Asp Gln Asp Ile Val Lys Lys Ile
   235 240 245
   Ile Gln Asp Ile Asp Leu Cys Glu Asn Ser Val Gln Arg His Ile
   250 255 260
   Gly His Ala Asn Leu Thr Phe Glu Gln Leu Arg Ser Leu Met Glu
   265 270 275
   Ser Leu Pro Gly Lys Lys Val Gly Ala Glu Asp Ile Glu Lys Thr
   280 285 290
   Ile Lys Ala Cys Lys Pro Ser Asp Gln Ile Leu Lys Leu Leu Ser
   295 300 305
   Leu Trp Arg Ile Lys Asn Gly Asp Gln Asp Thr Leu Lys Gly Leu
   310 315 320
   Met His Ala Leu Lys His Ser Lys Thr Tyr His Phe Pro Lys Thr
   325 330 335
   Val Thr Gln Ser Leu Lys Lys Thr Ile Arg Phe Leu His Ser Phe
   340 345 350
   Thr Met Tyr Lys Leu Tyr Gln Lys Leu Phe Leu Glu Met Ile Gly
   355 360 365
   Asn Gln Val Gln Ser Val Lys Ile Ser Ser Leu
   370 375 380
(2) INFORMATION FOR SEQUENCE ID NO: 67:
   (i) SEQUENCE CHARACTERISTICS:
      (A) LENGTH : 360
      (B) TYPE : amino acid
      (C) STRANDEDNESS : single
      (D) TOPOLOGY : linear
   (ii) MOLECULE TYPE : Protein (OCIF-DCR1)
   (xi) SEQUENCE DESCRIPTION :SEQ ID NO: 67:
   Met Asn Asn Leu Leu Cys Cys Ala Leu Val Phe Leu Asp Ile Ser
   -20 -15 -10
   Ile Lys Trp Thr Thr Gln Glu Pro Cys Pro Asp His Tyr Tyr Thr
   -5 -1 1 5
   Asp Ser Trp His Thr Ser Asp Glu Cys Leu Tyr Cys Ser Pro Val
   10 15 20
   Cys Lys Glu Leu Gln Tyr Val Lys Gln Glu Cys Asn Arg Thr His
   25 30 35
   Asn Arg Val Cys Glu Cys Lys Glu Gly Arg Tyr Leu Glu Ile Glu
   40 45 50
   Phe Cys Leu Lys His Arg Ser Cys Pro Pro Gly Phe Gly Val Val
   55 60 65
   Gln Ala Gly Thr Pro Glu Arg Asn Thr Val Cys Lys Arg Cys Pro
   70 75 80
   Asp Gly Phe Phe Ser Asn Glu Thr Ser Ser Lys Ala Pro Cys Arg
   85 90 95
   Lys His Thr Asn Cys Ser Val Phe Gly Leu Leu Leu Thr Gln Lys
   100 105 110
   Gly Asn Ala Thr His Asp Asn Ile Cys Ser Gly Asn Ser Glu Ser
   115 120 125
   Thr Gln Lys Cys Gly Ile Asp Val Thr Leu Cys Glu Glu Ala Phe
   130 135 140
   Phe Arg Phe Ala Val Pro Thr Lys Phe Thr Pro Asn Trp Leu Ser
   145 150 155
   Val Leu Val Asp Asn Leu Pro Gly Thr Lys Val Asn Ala Glu Ser
   160 165 170
   Val Glu Arg Ile Lys Arg Gln His Ser Ser Gln Glu Gln Thr Phe
   175 180 185
   Gln Leu Leu Lys Leu Trp Lys His Gln Asn Lys Asp Gln Asp Ile
   190 195 200
   Val Lys Lys Ile Ile Gln Asp Ile Asp Leu Cys Glu Asn Ser Val
   205 210 215
   Gln Arg His Ile Gly His Ala Asn Leu Thr Phe Glu Gln Leu Arg
   220 225 230
   Ser Leu Met Glu Ser Leu Pro Gly Lys Lys Val Gly Ala Glu Asp
   235 240 245
   Ile Glu Lys Thr Ile Lys Ala Cys Lys Pro Ser Asp Gln Ile Leu
   250 255 260
   Lys Leu Leu Ser Leu Trp Arg Ile Lys Asn Gly Asp Gln Asp Thr
   265 270 275
   Leu Lys Gly Leu Met His Ala Leu Lys His Ser Lys Thr Tyr His
   280 285 290
   Phe Pro Lys Thr Val Thr Gln Ser Leu Lys Lys Thr Ile Arg Phe
   295 300 305
   Leu His Ser Phe Thr Met Tyr Lys Leu Tyr Gln Lys Leu Phe Leu
   310 315 320
   Glu Met Ile Gly Asn Gln Val Gln Ser Val Lys Ile Ser Cys Leu
   325 330 335
(2) INFORMATION FOR SEQUENCE ID NO: 68:
   (i) SEQUENCE CHARACTERISTICS:
      (A) LENGTH : 359
      (B) TYPE : amino acid
      (C) STRANDEDNESS : single
      (D) TOPOLOGY : linear
   (ii) MOLECULE TYPE : Protein (OCIF-DCR2)
   (xi) SEQUENCE DESCRIPTION :SEQ ID NO: 68:
   Met Asn Asn Leu Leu Cys Cys Ala Leu Val Phe Leu Asp Ile Ser
   -20 -15 -10
   Ile Lys Trp Thr Thr Gln Glu Thr Phe Pro Pro Lys Tyr Leu His
   -5 -1 1 5
   Tyr Asp Glu Glu Thr Ser His Gln Leu Leu Cys Asp Lys Cys Pro
   10 15 20
   Pro Gly Thr Tyr Leu Lys Gln His Cys Thr Ala Lys Trp Lys Thr
   25 30 35
   Val Cys Ala Glu Cys Lys Glu Gly Arg Tyr Leu Glu Ile Glu Phe
   40 45 50
   Cys Leu Lys His Arg Ser Cys Pro Pro Gly Phe Gly Val Val Gln
   55 60 65
   Ala Gly Thr Pro Glu Arg Asn Thr Val Cys Lys Arg Cys Pro Asp
   70 75 80
   Gly Phe Phe Ser Asn Glu Thr Ser Ser Lys Ala Pro Cys Arg Lys
   85 90 95
   His Thr Asn Cys Ser Val Phe Gly Leu Leu Leu Thr Gln Lys Gly
   100 105 110
   Asn Ala Thr His Asp Asn Ile Cys Ser Gly Asn Ser Glu Ser Thr
   115 120 125
   Gln Lys Cys Gly Ile Asp Val Thr Leu Cys Glu Glu Ala Phe Phe
   130 135 140
   Arg Phe Ala Val Pro Thr Lys Phe Thr Pro Asn Trp Leu Ser Val
   145 150 155
   Leu Val Asp Asn Leu Pro Gly Thr Lys Val Asn Ala Glu Ser Val
   160 165 170
   Glu Arg Ile Lys Arg Gln His Ser Ser Gln Glu Gln Thr Phe Gln
   175 180 185
   Leu Leu Lys Leu Trp Lys His Gln Asn Lys Asp Gln Asp Ile Val
   190 195 200
   Lys Lys Ile Ile Gln Asp Ile Asp Leu Cys Glu Asn Ser Val Gln
   205 210 215
   Arg His Ile Gly His Ala Asn Leu Thr Phe Glu Gln Leu Arg Ser
   220 225 230
   Leu Met Glu Ser Leu Pro Gly Lys Lys Val Gly Ala Glu Asp Ile
   235 240 245
   Glu Lys Thr Ile Lys Ala Cys Lys Pro Ser Asp Gln Ile Leu Lys
   250 255 260
   Leu Leu Ser Leu Trp Arg Ile Lys Asn Gly Asp Gln Asp Thr Leu
   265 270 275
   Lys Gly Leu Met His Ala Leu Lys His Ser Lys Thr Tyr His Phe
   280 285 290
   Pro Lys Thr Val Thr Gln Ser Leu Lys Lys Thr Ile Arg Phe Leu
   295 300 305
   His Ser Phe Thr Met Tyr Lys Leu Tyr Gln Lys Leu Phe Leu Glu
   310 315 320
   Met Ile Gly Asn Gln Val Gln Ser Val Lys Ile Ser Cys Leu
   325 330 335
(2) INFORMATION FOR SEQUENCE ID NO: 69:
   (i) SEQUENCE CHARACTERISTICS:
      (A) LENGTH : 363
      (B) TYPE : amino acid
      (C) STRANDEDNESS : single
      (D) TOPOLOGY : linear
   (ii) MOLECULE TYPE : protein (OCIF-DCR3)
   (xi) SEQUENCE DESCRIPTION :SEQ ID NO: 69:
   Met Asn Asn Leu Leu Cys Cys Ala Leu Val Phe Leu Asp Ile Ser
   -20 -15 -10
   Ile Lys Trp Thr Thr Gln Glu Thr Phe Pro Pro Lys Tyr Leu His
   -5 -1 1 5
   Tyr Asp Glu Glu Thr Ser His Gln Leu Leu Cys Asp Lys Cys Pro
   10 15 20
   Pro Gly Thr Tyr Leu Lys Gln His Cys Thr Ala Lys Trp Lys Thr
   25 30 35
   Val Cys Ala Pro Cys Pro Asp His Tyr Tyr Thr Asp Ser Trp His
   40 45 50
   Thr Ser Asp Glu Cys Leu Tyr Cys Ser Pro Val Cys Lys Glu Leu
   55 60 65
   Gln Tyr Val Lys Gln Glu Cys Asn Arg Thr His Asn Arg Val Cys
   70 75 80
   Arg Cys Pro Asp Gly Phe Phe Ser Asn Glu Thr Ser Ser Lys Ala
   85 90 95
   Pro Cys Arg Lys His Thr Asn Cys Ser Val Phe Gly Leu Leu Leu
   100 105 110
   Thr Gln Lys Gly Asn Ala Thr His Asp Asn Ile Cys Ser Gly Asn
   115 120 125
   Ser Glu Ser Thr Gln Lys Cys Gly Ile Asp Val Thr Leu Cys Glu
   130 135 140
   Glu Ala Phe Phe Arg Phe Ala Val Pro Thr Lys Phe Thr Pro Asn
   145 150 155
   Trp Leu Ser Val Leu Val Asp Asn Leu Pro Gly Thr Lys Val Asn
   160 165 170
   Ala Glu Ser Val Glu Arg Ile Lys Arg Gln His Ser Ser Gln Glu
   175 180 185
   Gln Thr Phe Gln Leu Leu Lys Leu Trp Lys His Gln Asn Lys Asp
   190 195 200
   Gln Asp Ile Val Lys Lys Ile Ile Gln Asp Ile Asp Leu Cys Glu
   205 210 215
   Asn Ser Val Gln Arg His Ile Gly His Ala Asn Leu Thr Phe Glu
   220 225 230
   Gln Leu Arg Ser Leu Met Glu Ser Leu Pro Gly Lys Lys Val Gly
   235 240 245
   Ala Glu Asp Ile Glu Lys Thr Ile Lys Ala Cys Lys Pro Ser Asp
   250 255 260
   Gln Ile Leu Lys Leu Leu Ser Leu Trp Arg Ile Lys Asn Gly Asp
   265 270 275
   Gln Asp Thr Leu Lys Gly Leu Met His Ala Leu Lys His Ser Lys
   280 285 290
   Thr Tyr His Phe Pro Lys Thr Val Thr Gln Ser Leu Lys Lys Thr
   295 300 305
   Ile Arg Phe Leu His Ser Phe Thr Met Tyr Lys Leu Tyr Gln Lys
   310 315 320
   Leu Phe Leu Glu Met Ile Gly Asn Gln Val Gln Ser Val Lys Ile
   325 330 335
   Ser Cys Leu
   340
(2) INFORMATION FOR SEQUENCE ID NO: 70:
   (i) SEQUENCE CHARACTERISTICS:
      (A) LENGTH : 359
      (B) TYPE : amino acid
      (C) STRANDEDNESS : single
      (D) TOPOLOGY : linear
   (ii) MOLECULE TYPE : protein (OCIF-DCR4)
   (xi) SEQUENCE DESCRIPTION :SEQ ID NO: 70:
   Met Asn Asn Leu Leu Cys Cys Ala Leu Val Phe Leu Asp Ile Ser
   -20 -15 -10
   Ile Lys Trp Thr Thr Gln Glu Thr Phe Pro Pro Lys Tyr Leu His
   -5 -1 1 5
   Tyr Asp Glu Glu Thr Ser His Gln Leu Leu Cys Asp Lys Cys Pro
   10 15 20
   Pro Gly Thr Tyr Leu Lys Gln His Cys Thr Ala Lys Trp Lys Thr
   25 30 35
   Val Cys Ala Pro Cys Pro Asp His Tyr Tyr Thr Asp Ser Trp His
   40 45 50
   Thr Ser Asp Glu Cys Leu Tyr Cys Ser Pro Val Cys Lys Glu Leu
   55 60 65
   Gln Tyr Val Lys Gln Glu Cys Asn Arg Thr His Asn Arg Val Cys
   70 75 80
   Glu Cys Lys Glu Gly Arg Tyr Leu Glu Ile Glu Phe Cys Leu Lys
   85 90 95
   His Arg Ser Cys Pro Pro Gly Phe Gly Val Val Gln Ala Gly Thr
   100 105 110
   Pro Glu Arg Asn Thr Val Cys Lys Ser Gly Asn Ser Glu Ser Thr
   115 120 125
   Gln Lys Cys Gly Ile Asp Val Thr Leu Cys Glu Glu Ala Phe Phe
   130 135 140
   Arg Phe Ala Val Pro Thr Lys Phe Thr Pro Asn Trp Leu Ser Val
   145 150 155
   Leu Val Asp Asn Leu Pro Gly Thr Lys Val Asn Ala Glu Ser Val
   160 165 170
   Glu Arg Ile Lys Arg Gln His Ser Ser Gln Glu Gln Thr Phe Gln
   175 180 185
   Leu Leu Lys Leu Trp Lys His Gln Asn Lys Asp Gln Asp Ile Val
   190 195 200
   Lys Lys Ile Ile Gln Asp Ile Asp Leu Cys Glu Asn Ser Val Gln
   205 210 215
   Arg His Ile Gly His Ala Asn Leu Thr Phe Glu Gln Leu Arg Ser
   220 225 230
   Leu Met Glu Ser Leu Pro Gly Lys Lys Val Gly Ala Glu Asp Ile
   235 240 245
   Glu Lys Thr Ile Lys Ala Cys Lys Pro Ser Asp Gln Ile Leu Lys
   250 255 260
   Leu Leu Ser Leu Trp Arg Ile Lys Asn Gly Asp Gln Asp Thr Leu
   265 270 275
   Lys Gly Leu Met His Ala Leu Lys His Ser Lys Thr Tyr His Phe
   280 285 290
   Pro Lys Thr Val Thr Gln Ser Leu Lys Lys Thr Ile Arg Phe Leu
   295 300 305
   His Ser Phe Thr Met Tyr Lys Leu Tyr Gln Lys Leu Phe Leu Glu
   310 315 320
   Met Ile Gly Asn Gln Val Gln Ser Val Lys Ile Ser Cys Leu
   325 330 335
(2) INFORMATION FOR SEQUENCE ID NO: 71:
   (i) SEQUENCE CHARACTERISTICS:
      (A) LENGTH : 326
      (B) TYPE : amino acid
      (C) STRANDEDNESS : single
      (D) TOPOLOGY : linear
   (ii) MOLECULE TYPE : protein (OCIF-DDD1)
   (xi) SEQUENCE DESCRIPTION :SEQ ID NO: 71:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5                  -1  1                5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys Arg Cys Pro Asp Gly Phe Phe
   115                 120                 125
   Ser Asn Glu Thr Ser Ser Lys Ala Pro Cys Arg Lys His Thr Asn
   130                 135                 140
   Cys Ser Val Phe Gly Leu Leu Leu Thr Gln Lys Gly Asn Ala Thr
   145                 150                 155
   His Asp Asn Ile Cys Ser Gly Asn Ser Glu Ser Thr Gln Lys Cys
   160                 165                 170
   Gly Ile Asp Ile Asp Leu Cys Glu Asn Ser Val Gln Arg His Ile
   175                 180                 185
   Gly His Ala Asn Leu Thr Phe Glu Gln Leu Arg Ser Leu Met Glu
   190 195 200
   Ser Leu Pro Gly Lys Lys Val Gly Ala Glu Asp Ile Glu Lys Thr
   205 210 215
   Ile Lys Ala Cys Lys Pro Ser Asp Gln Ile Leu Lys Leu Leu Ser
   220 225 230
   Leu Trp Arg Ile Lys Asn Gly Asp Gln Asp Thr Leu Lys Gly Leu
   235 240 245
   Met His Ala Leu Lys His Ser Lys Thr Tyr His Phe Pro Lys Thr
   250 255 260
   Val Thr Gln Ser Leu Lys Lys Thr Ile Arg Phe Leu His Ser Phe
   265 270 275
   Thr Met Tyr Lys Leu Tyr Gln Lys Leu Phe Leu Glu Met Ile Gly
   280 285 290
   Asn Gln Val Gln Ser Val Lys Ile Ser Cys Leu
   295 300 305
(2) INFORMATION FOR SEQUENCE ID NO: 72:
   (i) SEQUENCE CHARACTERISTICS:
      (A) LENGTH: 327
      (B) TYPE: amino acid
      (C) STRANDEDNESS : single
      (D) TOPOLOGY : linear
   (ii) MOLECULE TYPE : protein (OCIF-DDD2)
   (xi) SEQUENCE DESCRIPTION :SEQ ID NO: 72:
   Met Asn Asn Leu Leu Cys Cys Ala Leu Val Phe Leu Asp Ile Ser
   -20 -15 -10
   Ile Lys Trp Thr Thr Gln Glu Thr Phe Pro Pro Lys Tyr Leu His
   -5 -1 1 5
   Tyr Asp Glu Glu Thr Ser His Gln Leu Leu Cys Asp Lys Cys Pro
   10 15 20
   Pro Gly Thr Tyr Leu Lys Gln His Cys Thr Ala Lys Trp Lys Thr
   25 30 35
   Val Cys Ala Pro Cys Pro Asp His Tyr Tyr Thr Asp Ser Trp His
   40 45 50
   Thr Ser Asp Glu Cys Leu Tyr Cys Ser Pro Val Cys Lys Glu Leu
   55 60 65
   Gln Tyr Val Lys Gln Glu Cys Asn Arg Thr His Asn Arg Val Cys
   70 75 80
   Glu Cys Lys Glu Gly Arg Tyr Leu Glu Ile Glu Phe Cys Leu Lys
   85 90 95
   His Arg Ser Cys Pro Pro Gly Phe Gly Val Val Gln Ala Gly Thr
   100 105 110
   Pro Glu Arg Asn Thr Val Cys Lys Arg Cys Pro Asp Gly Phe Phe
   115 120 125
   Ser Asn Glu Thr Ser Ser Lys Ala Pro Cys Arg Lys His Thr Asn
   130 135 140
   Cys Ser Val Phe Gly Leu Leu Leu Thr Gln Lys Gly Asn Ala Thr
   145 150 155
   His Asp Asn Ile Cys Ser Gly Asn Ser Glu Ser Thr Gln Lys Cys
   160 165 170
   Gly Ile Asp Val Thr Leu Cys Glu Glu Ala Phe Phe Arg Phe Ala
   175 180 185
   Val Pro Thr Lys Phe Thr Pro Asn Trp Leu Ser Val Leu Val Asp
   190 195 200
   Asn Leu Pro Gly Thr Lys Val Asn Ala Glu Ser Val Glu Arg Ile
   205 210 215
   Lys Arg Gln His Ser Ser Gln Glu Gln Thr Phe Gln Leu Leu Lys
   220 225 230
   Leu Trp Lys His Gln Asn Lys Asp Gln Asp Ile Val Lys Lys Ile
   235 240 245
   Ile Gln Asp Ala Leu Lys His Ser Lys Thr Tyr His Phe Pro Lys
   250 255 260
   Thr Val Thr Gln Ser Leu Lys Lys Thr Ile Arg Phe Leu His Ser
   265 270 275
   Phe Thr Met Tyr Lys Leu Tyr Gln Lys Leu Phe Leu Glu Met Ile
   280 285 290
   Gly Asn Gln Val Gln Ser Val Lys Ile Ser Cys Leu
   295 300 305
(2) INFORMATION FOR SEQUENCE ID NO: 73:
   (i) SEQUENCE CHARACTERISTICS:
      (A) LENGTH : 399
      (B) TYPE : amino acid
      (C) STRANDEDNESS : single
      (D) TOPOLOGY : linear
   (ii) MOLECULE TYPE : protein (OCIF-CL)
   (xi) SEQUENCE DESCRIPTION :SEQ ID NO: 73:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5                  -1  1               5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys Arg Cys Pro Asp Gly Phe Phe
   115                 120                 125
   Ser Asn Glu Thr Ser Ser Lys Ala Pro Cys Arg Lys His Thr Asn
   130                 135                 140
   Cys Ser Val Phe Gly Leu Leu Leu Thr Gln Lys Gly Asn Ala Thr
   145                 150                 155
   His Asp Asn Ile Cys Ser Gly Asn Ser Glu Ser Thr Gln Lys Cys
   160                 165                 170
   Gly Ile Asp Val Thr Leu Cys Glu Glu Ala Phe Phe Arg Phe Ala
   175                 180                 185
   Val Pro Thr Lys Phe Thr Pro Asn Trp Leu Ser Val Leu Val Asp
   190                 195                 200
   Asn Leu Pro Gly Thr Lys Val Asn Ala Glu Ser Val Glu Arg Ile
   205 210 215
   Lys Arg Gln His Ser Ser Gln Glu Gln Thr Phe Gln Leu Leu Lys
   220 225 230
   Leu Trp Lys His Gln Asn Lys Asp Gln Asp Ile Val Lys Lys Ile
   235 240 245
   Ile Gln Asp Ile Asp Leu Cys Glu Asn Ser Val Gln Arg His Ile
   250 255 260
   Gly His Ala Asn Leu Thr Phe Glu Gln Leu Arg Ser Leu Met Glu
   265 270 275
   Ser Leu Pro Gly Lys Lys Val Gly Ala Glu Asp Ile Glu Lys Thr
   280 285 290
   Ile Lys Ala Cys Lys Pro Ser Asp Gln Ile Leu Lys Leu Leu Ser
   295 300 305
   Leu Trp Arg Ile Lys Asn Gly Asp Gln Asp Thr Leu Lys Gly Leu
   310 315 320
   Met His Ala Leu Lys His Ser Lys Thr Tyr His Phe Pro Lys Thr
   325 330 335
   Val Thr Gln Ser Leu Lys Lys Thr Ile Arg Phe Leu His Ser Phe
   340 345 350
   Thr Met Tyr Lys Leu Tyr Gln Lys Leu Phe Leu Glu Met Ile Gly
   355 360 365
   Asn Gln Val Gln Ser Val Lys Ile Ser
   370 375
(2) INFORMATION FOR SEQUENCE ID NO: 74:
   (i) SEQUENCE CHARACTERISTICS:
      (A) LENGTH : 351
      (B) TYPE : amino acid
      (C) STRANDEDNESS : single
      (D) TOPOLOGY : linear
   (ii) MOLECULE TYPE : protein (OCIF-CC)
   (xi) SEQUENCE DESCRIPTION :SEQ ID NO: 74:
   Met Asn Asn Leu Leu Cys Cys Ala Leu Val Phe Leu Asp Ile Ser
   -20 -15 -10
   Ile Lys Trp Thr Thr Gln Glu Thr Phe Pro Pro Lys Tyr Leu His
   -5 -1 1 5
   Tyr Asp Glu Glu Thr Ser His Gln Leu Leu Cys Asp Lys Cys Pro
   10 15 20
   Pro Gly Thr Tyr Leu Lys Gln His Cys Thr Ala Lys Trp Lys Thr
   25 30 35
   Val Cys Ala Pro Cys Pro Asp His Tyr Tyr Thr Asp Ser Trp His
   40 45 50
   Thr Ser Asp Glu Cys Leu Tyr Cys Ser Pro Val Cys Lys Glu Leu
   55 60 65
   Gln Tyr Val Lys Gln Glu Cys Asn Arg Thr His Asn Arg Val Cys
   70 75 80
   Glu Cys Lys Glu Gly Arg Tyr Leu Glu Ile Glu Phe Cys Leu Lys
   85 90 95
   His Arg Ser Cys Pro Pro Gly Phe Gly Val Val Gln Ala Gly Thr
   100 105 110
   Pro Glu Arg Asn Thr Val Cys Lys Arg Cys Pro Asp Gly Phe Phe
   115 120 125
   Ser Asn Glu Thr Ser Ser Lys Ala Pro Cys Arg Lys His Thr Asn
   130 135 140
   Cys Ser Val Phe Gly Leu Leu Leu Thr Gln Lys Gly Asn Ala Thr
   145 150 155
   His Asp Asn Ile Cys Ser Gly Asn Ser Glu Ser Thr Gln Lys Cys
   160 165 170
   Gly Ile Asp Val Thr Leu Cys Glu Glu Ala Phe Phe Arg Phe Ala
   175 180 185
   Val Pro Thr Lys Phe Thr Pro Asn Trp Leu Ser Val Leu Val Asp
   190 195 200
   Asn Leu Pro Gly Thr Lys Val Asn Ala Glu Ser Val Glu Arg Ile
   205 210 215
   Lys Arg Gln His Ser Ser Gln Glu Gln Thr Phe Gln Leu Leu Lys
   220 225 230
   Leu Trp Lys His Gln Asn Lys Asp Gln Asp Ile Val Lys Lys Ile
   235 240 245
   Ile Gln Asp Ile Asp Leu Cys Glu Asn Ser Val Gln Arg His Ile
   250 255 260
   Gly His Ala Asn Leu Thr Phe Glu Gln Leu Arg Ser Leu Met Glu
   265 270 275
   Ser Leu Pro Gly Lys Lys Val Gly Ala Glu Asp Ile Glu Lys Thr
   280 285 290
   Ile Lys Ala Cys Lys Pro Ser Asp Gln Ile Leu Lys Leu Leu Ser
   295 300 305
   Leu Trp Arg Ile Lys Asn Gly Asp Gln Asp Thr Leu Lys Gly Leu
   310 315 320
   Met His Ala Leu Lys His
   325 330
(2) INFORMATION FOR SEQUENCE ID NO: 75:
   (i) SEQUENCE CHARACTERISTICS:
      (A) LENGTH : 272
      (B) TYPE : amino acid
      (C) STRANDEDNESS : single
      (D) TOPOLOGY : linear
   (ii) MOLECULE TYPE : Protein (OCIF-CDD2)
   (xi) SEQUENCE DESCRIPTION :SEQ ID NO: 75:
   Met Asn Asn Leu Leu Cys Cys Ala Leu Val Phe Leu Asp Ile Ser
   -20 -15 -10
   Ile Lys Trp Thr Thr Gln Glu Thr Phe Pro Pro Lys Tyr Leu His
   -5 -1 1 5
   Tyr Asp Glu Glu Thr Ser His Gln Leu Leu Cys Asp Lys Cys Pro
   10 15 20
   Pro Gly Thr Tyr Leu Lys Gln His Cys Thr Ala Lys Trp Lys Thr
   25 30 35
   Val Cys Ala Pro Cys Pro Asp His Tyr Tyr Thr Asp Ser Trp His
   40 45 50
   Thr Ser Asp Glu Cys Leu Tyr Cys Ser Pro Val Cys Lys Glu Leu
   55 60 65
   Gln Tyr Val Lys Gln Glu Cys Asn Arg Thr His Asn Arg Val Cys
   70 75 80
   Glu Cys Lys Glu Gly Arg Tyr Leu Glu Ile Glu Phe Cys Leu Lys
   85 90 95
   His Arg Ser Cys Pro Pro Gly Phe Gly Val Val Gln Ala Gly Thr
   100 105 110
   Pro Glu Arg Asn Thr Val Cys Lys Arg Cys Pro Asp Gly Phe Phe
   115 120 125
   Ser Asn Glu Thr Ser Ser Lys Ala Pro Cys Arg Lys His Thr Asn
   130 135 140
   Cys Ser Val Phe Gly Leu Leu Leu Thr Gln Lys Gly Asn Ala Thr
   145 150 155
   His Asp Asn Ile Cys Ser Gly Asn Ser Glu Ser Thr Gln Lys Cys
   160 165 170
   Gly Ile Asp Val Thr Leu Cys Glu Glu Ala Phe Phe Arg Phe Ala
   175 180 185
   Val Pro Thr Lys Phe Thr Pro Asn Trp Leu Ser Val Leu Val Asp
   190 195 200
   Asn Leu Pro Gly Thr Lys Val Asn Ala Glu Ser Val Glu Arg Ile
   205 210 215
   Lys Arg Gln His Ser Ser Gln Glu Gln Thr Phe Gln Leu Leu Lys
   220 225 230
   Leu Trp Lys His Gln Asn Lys Asp Gln Asp Ile Val Lys Lys Ile
   235 240 245
   Ile Gln
   250
(2) INFORMATION FOR SEQUENCE ID NO: 76:
   (i) SEQUENCE CHARACTERISTICS:
      (A) LENGTH : 197
      (B) TYPE : amino acid
      (C) STRANDEDNESS : single
      (D) TOPOLOGY : linear
   (ii) MOLECULE TYPE : Protein (OCIF-CDD1)
   (xi) SEQUENCE DESCRIPTION :SEQ ID NO: 76:
   Met Asn Asn Leu Leu Cys Cys Ala Leu Val Phe Leu Asp Ile Ser
   -20 -15 -10
   Ile Lys Trp Thr Thr Gln Glu Thr Phe Pro Pro Lys Tyr Leu His
   -5 -1 1 5
   Tyr Asp Glu Glu Thr Ser His Gln Leu Leu Cys Asp Lys Cys Pro
   10 15 20
   Pro Gly Thr Tyr Leu Lys Gln His Cys Thr Ala Lys Trp Lys Thr
   25 30 35
   Val Cys Ala Pro Cys Pro Asp His Tyr Tyr Thr Asp Ser Trp His
   40 45 50
   Thr Ser Asp Glu Cys Leu Tyr Cys Ser Pro Val Cys Lys Glu Leu
   55 60 65
   Gln Tyr Val Lys Gln Glu Cys Asn Arg Thr His Asn Arg Val Cys
   70 75 80
   Glu Cys Lys Glu Gly Arg Tyr Leu Glu Ile Glu Phe Cys Leu Lys
   85 90 95
   His Arg Ser Cys Pro Pro Gly Phe Gly Val Val Gln Ala Gly Thr
   100 105 110
   Pro Glu Arg Asn Thr Val Cys Lys Arg Cys Pro Asp Gly Phe Phe
   115 120 125
   Ser Asn Glu Thr Ser Ser Lys Ala Pro Cys Arg Lys His Thr Asn
   130 135 140
   Cys Ser Val Phe Gly Leu Leu Leu Thr Gln Lys Gly Asn Ala Thr
   145 150 155
   His Asp Asn Ile Cys Ser Gly Asn Ser Glu Ser Thr Gln Lys Cys
   160 165 170
   Gly Ile
   175
(2) INFORMATION FOR SEQUENCE ID NO: 77:
   (i) SEQUENCE CHARACTERISTICS:
      (A) LENGTH : 143
      (B) TYPE : amino acid
      (C) STRANDEDNESS : single
      (D) TOPOLOGY : linear
   (ii) MOLECULE TYPE : Protein (OCIF-CCR4)
   (xi) SEQUENCE DESCRIPTION :SEQ ID NO: 77:
   Met Asn Asn Leu Leu Cys Cys Ala Leu Val Phe Leu Asp Ile Ser
   -20 -15 -10
   Ile Lys Trp Thr Thr Gln Glu Thr Phe Pro Pro Lys Tyr Leu His
   -5 -1 1 5
   Tyr Asp Glu Glu Thr Ser His Gln Leu Leu Cys Asp Lys Cys Pro
   10 15 20
   Pro Gly Thr Tyr Leu Lys Gln His Cys Thr Ala Lys Trp Lys Thr
   25 30 35
   Val Cys Ala Pro Cys Pro Asp His Tyr Tyr Thr Asp Ser Trp His
   40 45 50
   Thr Ser Asp Glu Cys Leu Tyr Cys Ser Pro Val Cys Lys Glu Leu
   55 60 65
   Gln Tyr Val Lys Gln Glu Cys Asn Arg Thr His Asn Arg Val Cys
   70 75 80
   Glu Cys Lys Glu Gly Arg Tyr Leu Glu Ile Glu Phe Cys Leu Lys
   85 90 95
   His Arg Ser Cys Pro Pro Gly Phe Gly Val Val Gln Ala Gly Thr
   100 105 110
   Pro Glu Arg Asn Thr Val Cys Lys
   115 120
(2) INFORMATION FOR SEQUENCE ID NO: 78:
   (i) SEQUENCE CHARACTERISTICS:
      (A) LENGTH : 106
      (B) TYPE : amino acid
      (C) STRANDEDNESS : single
      (D) TOPOLOGY : linear
   (ii) MOLECULE TYPE : Protein (OCIF-CCR3)
   (xi) SEQUENCE DESCRIPTION :SEQ ID NO: 78:
   Met Asn Asn Leu Leu Cys Cys Ala Leu Val Phe Leu Asp Ile Ser
   -20 -15 -10
   Ile Lys Trp Thr Thr Gln Glu Thr Phe Pro Pro Lys Tyr Leu His
   -5 -1 1 5
   Tyr Asp Glu Glu Thr Ser His Gln Leu Leu Cys Asp Lys Cys Pro
   10 15 20
   Pro Gly Thr Tyr Leu Lys Gln His Cys Thr Ala Lys Trp Lys Thr
   25 30 35
   Val Cys Ala Pro Cys Pro Asp His Tyr Tyr Thr Asp Ser Trp His
   40 45 50
   Thr Ser Asp Glu Cys Leu Tyr Cys Ser Pro Val Cys Lys Glu Leu
   55 60 65
   Gln Tyr Val Lys Gln Glu Cys Asn Arg Thr His Asn Arg Val Cys
   70 75 80
   Glu
   85
(2) INFORMATION FOR SEQUENCE ID NO: 79:
   (i) SEQUENCE CHARACTERISTICS:
      (A) LENGTH : 393
      (B) TYPE : amino acid
      (D) TOPOLOGY : linear
   (ii) MOLECULE TYPE : Protein (OCIF-CBst)
   (xi) SEQUENCE DESCRIPTION :SEQ ID NO: 79:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5                  -1   1                   5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys Arg Cys Pro Asp Gly Phe Phe
   115                 120                 125
   Ser Asn Glu Thr Ser Ser Lys Ala Pro Cys Arg Lys His Thr Asn
   130                 135                 140
   Cys Ser Val Phe Gly Leu Leu Leu Thr Gln Lys Gly Asn Ala Thr
   145                 150                 155
   His Asp Asn Ile Cys Ser Gly Asn Ser Glu Ser Thr Gln Lys Cys
   160                 165                 170
   Gly Ile Asp Val Thr Leu Cys Glu Glu Ala Phe Phe Arg Phe Ala
   175 180 185
   Val Pro Thr Lys Phe Thr Pro Asn Trp Leu Ser Val Leu Val Asp
   190 195 200
   Asn Leu Pro Gly Thr Lys Val Asn Ala Glu Ser Val Glu Arg Ile
   205 210 215
   Lys Arg Gln His Ser Ser Gln Glu Gln Thr Phe Gln Leu Leu Lys
   220 225 230
   Leu Trp Lys His Gln Asn Lys Asp Gln Asp Ile Val Lys Lys Ile
   235 240 245
   Ile Gln Asp Ile Asp Leu Cys Glu Asn Ser Val Gln Arg His Ile
   250 255 260
   Gly His Ala Asn Leu Thr Phe Glu Gln Leu Arg Ser Leu Met Glu
   265 270 275
   Ser Leu Pro Gly Lys Lys Val Gly Ala Glu Asp Ile Glu Lys Thr
   280 285 290
   Ile Lys Ala Cys Lys Pro Ser Asp Gln Ile Leu Lys Leu Leu Ser
   295 300 305
   Leu Trp Arg Ile Lys Asn Gly Asp Gln Asp Thr Leu Lys Gly Leu
   310 315 320
   Met His Ala Leu Lys His Ser Lys Thr Tyr His Phe Pro Lys Thr
   325 330 335
   Val Thr Gln Ser Leu Lys Lys Thr Ile Arg Phe Leu His Ser Phe
   340 345 350
   Thr Met Tyr Lys Leu Tyr Gln Lys Leu Phe Leu Glu Met Ile Gly
   355 360 365
   Asn Leu Val
   370
(2) INFORMATION FOR SEQUENCE ID NO: 80:
   (i) SEQUENCE CHARACTERISTICS:
      (A) LENGTH : 321
      (B) TYPE : amino acid
      (D) TOPOLOGY : linear
   (ii) MOLECULE TYPE : Protein (OCIF-CSph)
   (xi) SEQUENCE DESCRIPTION :SEQ ID NO: 80:
   Met Asn Asn Leu Leu Cys Cys Ala Leu Val Phe Leu Asp Ile Ser
   -20 -15 -10
   Ile Lys Trp Thr Thr Gln Glu Thr Phe Pro Pro Lys Tyr Leu His
   -5 -1 1 5
   Tyr Asp Glu Glu Thr Ser His Gln Leu Leu Cys Asp Lys Cys Pro
   10 15 20
   Pro Gly Thr Tyr Leu Lys Gln His Cys Thr Ala Lys Trp Lys Thr
   25 30 35
   Val Cys Ala Pro Cys Pro Asp His Tyr Tyr Thr Asp Ser Trp His
   40 45 50
   Thr Ser Asp Glu Cys Leu Tyr Cys Ser Pro Val Cys Lys Glu Leu
   55 60 65
   Gln Tyr Val Lys Gln Glu Cys Asn Arg Thr His Asn Arg Val Cys
   70 75 80
   Glu Cys Lys Glu Gly Arg Tyr Leu Glu Ile Glu Phe Cys Leu Lys
   85 90 95
   His Arg Ser Cys Pro Pro Gly Phe Gly Val Val Gln Ala Gly Thr
   100 105 110
   Pro Glu Arg Asn Thr Val Cys Lys Arg Cys Pro Asp Gly Phe Phe
   115 120 125
   Ser Asn Glu Thr Ser Ser Lys Ala Pro Cys Arg Lys His Thr Asn
   130 135 140
   Cys Ser Val Phe Gly Leu Leu Leu Thr Gln Lys Gly Asn Ala Thr
   145 150 155
   His Asp Asn Ile Cys Ser Gly Asn Ser Glu Ser Thr Gln Lys Cys
   160 165 170
   Gly Ile Asp Val Thr Leu Cys Glu Glu Ala Phe Phe Arg Phe Ala
   175 180 185
   Val Pro Thr Lys Phe Thr Pro Asn Trp Leu Ser Val Leu Val Asp
   190 195 200
   Asn Leu Pro Gly Thr Lys Val Asn Ala Glu Ser Val Glu Arg Ile
   205 210 215
   Lys Arg Gln His Ser Ser Gln Glu Gln Thr Phe Gln Leu Leu Lys
   220 225 230
   Leu Trp Lys His Gln Asn Lys Asp Gln Asp Ile Val Lys Lys Ile
   235 240 245
   Ile Gln Asp Ile Asp Leu Cys Glu Asn Ser Val Gln Arg His Ile
   250 255 260
   Gly His Ala Asn Leu Thr Phe Glu Gln Leu Arg Ser Leu Met Glu
   265 270 275
   Ser Leu Pro Gly Lys Lys Val Gly Ala Glu Asp Ile Glu Lys Thr
   280 285 290
   Ile Lys Ala Ser Leu Asp
   295 300
(2) INFORMATION FOR SEQUENCE ID NO: 81:
   (i) SEQUENCE CHARACTERISTICS:
      (A) LENGTH : 202
      (B) TYPE : amino acid
      (D) TOPOLOGY : linear
   (ii) MOLECULE TYPE : Protein (OCIF-CBsp)
   (xi) SEQUENCE DESCRIPTION :SEQ ID NO: 81:
(2) INFORMATION FOR SEQUENCE ID NO: 82:
   (i) SEQUENCE CHARACTERISTICS:
      (A) LENGTH : 84
      (B) TYPE : amino acid
      (D) TOPOLOGY : linear
   (ii) MOLECULE TYPE : Protein (OCIF-CPst)
   (xi) SEQUENCE DESCRIPTION :SEQ ID NO: 82:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5               -1   1                 5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Leu Val
   55                  60          63
(2) INFORMATION FOR SEQUENCE ID NO: 83:
   (i) SEQUENCE CHARACTERISTICS:
      (A) LENGTH : 1206
      (B) TYPE : nucleic acid
      (C) STRANDEDNESS : single
      (D) TOPOLOGY : linear
   (ii) MOLECULE TYPE : cDNA (OCIF-C19S)
   (xi) SEQUENCE DESCRIPTION :SEQ ID NO: 83:
   ATGAACAACT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCC CTTGCCCTGA CCACTACTAC ACAGACAGCT GGCACACCAG TGACGAGTGT  240
   CTATACTGCA GCCCCGTGTG CAAGGAGCTG CAGTACGTCA AGCAGGAGTG CAATCGCACC  300
   CACAACCGCG TGTGCGAATG CAAGGAAGGG CGCTACCTTG AGATAGAGTT CTGCTTGAAA  360
   CATAGGAGCT GCCCTCCTGG ATTTGGAGTG GTGCAAGCTG GAACCCCAGA GCGAAATACA  420
   GTTTGCAAAA GATGTCCAGA TGGGTTCTTC TCAAATGAGA CGTCATCTAA AGCACCCTGT  480
   AGAAAACACA CAAATTGCAG TGTCTTTGGT CTCCTGCTAA CTCAGAAAGG AAATGCAACA  540
   CACGACAACA TATGTTCCGG AAACAGTGAA TCAACTCAAA AAAGTGGAAT AGATGTTACC  600
   CTGTGTGAGG AGGCATTCTT CAGGTTTGCT GTTCCTACAA AGTTTACGCC TAACTGGCTT  660
   AGTGTCTTGG TAGACAATTT GCCTGGCACC AAAGTAAACG CAGAGAGTGT AGAGAGGATA  720
   AAACGGCAAC ACAGCTCACA AGAACAGACT TTCCAGCTGC TGAAGTTATG GAAACATCAA  780
   AACAAAGACC AAGATATAGT CAAGAAGATC ATCCAAGATA TTGACCTCTG TGAAAACAGC  840
   GTGCAGCGGC ACATTGGACA TGCTAACCTC ACCTTCGAGC AGCTTCGTAG CTTGATGGAA  900
   AGCTTACCGG GAAAGAAAGT GGGAGCAGAA GACATTGAAA AAACAATAAA GGCATGCAAA  960
   CCCAGTGACC AGATCCTGAA GCTGCTCAGT TTGTGGCGAA TAAAAAATGG CGACCAAGAC 1020
   ACCTTGAAGG GCCTAATGCA CGCACTAAAG CACTCAAAGA CGTACCACTT TCCCAAAACT 1080
   GTCACTCAGA GTCTAAAGAA GACCATCAGG TTCCTTCACA GCTTCACAAT GTACAAATTG 1140
   TATCAGAAGT TATTTTTAGA AATGATAGGT AACCAGGTCC AATCAGTAAA AATAAGCTGC 1200
   TTATAA                                                           1206
(2) INFORMATION FOR SEQUENCE ID NO: 84:
   (i) SEQUENCE CHARACTERISTICS:
      (A) LENGTH : 1206
      (B) TYPE : nucleic acid
      (C) STRANDEDNESS : single
      (D) TOPOLOGY : linear
   (ii) MOLECULE TYPE : cDNA (OCIF-C20S)
   (xi) SEQUENCE DESCRIPTION :SEQ ID NO: 84:
   ATGAACAACT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCC CTTGCCCTGA CCACTACTAC ACAGACAGCT GGCACACCAG TGACGAGTGT  240
   CTATACTGCA GCCCCGTGTG CAAGGAGCTG CAGTACGTCA AGCAGGAGTG CAATCGCACC  300
   CACAACCGCG TGTGCGAATG CAAGGAAGGG CGCTACCTTG AGATAGAGTT CTGCTTGAAA  360
   CATAGGAGCT GCCCTCCTGG ATTTGGAGTG GTGCAAGCTG GAACCCCAGA GCGAAATACA  420
   GTTTGCAAAA GATGTCCAGA TGGGTTCTTC TCAAATGAGA CGTCATCTAA AGCACCCTGT  480
   AGAAAACACA CAAATTGCAG TGTCTTTGGT CTCCTGCTAA CTCAGAAAGG AAATGCAACA  540
   CACGACAACA TATGTTCCGG AAACAGTGAA TCAACTCAAA AATGTGGAAT AGATGTTACC  600
   CTGAGTGAGG AGGCATTCTT CAGGTTTGCT GTTCCTACAA AGTTTACGCC TAACTGGCTT  660
   AGTGTCTTGG TAGACAATTT GCCTGGCACC AAAGTAAACG CAGAGAGTGT AGAGAGGATA  720
   AAACGGCAAC ACAGCTCACA AGAACAGACT TTCCAGCTGC TGAAGTTATG GAAACATCAA  780
   AACAAAGACC AAGATATAGT CAAGAAGATC ATCCAAGATA TTGACCTCTG TGAAAACAGC  840
   GTGCAGCGGC ACATTGGACA TGCTAACCTC ACCTTCGAGC AGCTTCGTAG CTTGATGGAA  900
   AGCTTACCGG GAAAGAAAGT GGGAGCAGAA GACATTGAAA AAACAATAAA GGCATGCAAA  960
   CCCAGTGACC AGATCCTGAA GCTGCTCAGT TTGTGGCGAA TAAAAAATGG CGACCAAGAC 1020
   ACCTTGAAGG GCCTAATGCA CGCACTAAAG CACTCAAAGA CGTACCACTT TCCCAAAACT 1080
   GTCACTCAGA GTCTAAAGAA GACCATCAGG TTCCTTCACA GCTTCACAAT GTACAAATTG 1140
   TATCAGAAGT TATTTTTAGA AATGATAGGT AACCAGGTCC AATCAGTAAA AATAAGCTGC 1200
   TTATAA                                                            1206
(2) INFORMATION FOR SEQUENCE ID NO: 85:
   (i) SEQUENCE CHARACTERISTICS:
      (A) LENGTH : 1206
      (B) TYPE : nucleic acid
      (C) STRANDEDNESS : single
      (D) TOPOLOGY : linear
   (ii) MOLECULE TYPE : cDNA (OCIF-C21S)
   (xi) SEQUENCE DESCRIPTION :SEQ ID NO: 85:
   ATGAACAACT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCC CTTGCCCTGA CCACTACTAC ACAGACAGCT GGCACACCAG TGACGAGTGT  240
   CTATACTGCA GCCCCGTGTG CAAGGAGCTG CAGTACGTCA AGCAGGAGTG CAATCGCACC  300
   CACAACCGCG TGTGCGAATG CAAGGAAGGG CGCTACCTTG AGATAGAGTT CTGCTTGAAA  360
   CATAGGAGCT GCCCTCCTGG ATTTGGAGTG GTGCAAGCTG GAACCCCAGA GCGAAATACA  420
   GTTTGCAAAA GATGTCCAGA TGGGTTCTTC TCAAATGAGA CGTCATCTAA AGCACCCTGT  480
   AGAAAACACA CAAATTGCAG TGTCTTTGGT CTCCTGCTAA CTCAGAAAGG AAATGCAACA  540
   CACGACAACA TATGTTCCGG AAACAGTGAA TCAACTCAAA AATGTGGAAT AGATGTTACC  600
   CTGTGTGAGG AGGCATTCTT CAGGTTTGCT GTTCCTACAA AGTTTACGCC TAACTGGCTT 660
   AGTGTCTTGG TAGACAATTT GCCTGGCACC AAAGTAAACG CAGAGAGTGT AGAGAGGATA 720
   AAACGGCAAC ACAGCTCACA AGAACAGACT TTCCAGCTGC TGAAGTTATG GAAACATCAA 780
   AACAAAGACC AAGATATAGT CAAGAAGATC ATCCAAGATA TTGACCTCAG TGAAAACAGC 840
   GTGCAGCGGC ACATTGGACA TGCTAACCTC ACCTTCGAGC AGCTTCGTAG CTTGATGGAA 900
   AGCTTACCGG GAAAGAAAGT GGGAGCAGAA GACATTGAAA AAACAATAAA GGCATGCAAA 960
   CCCAGTGACC AGATCCTGAA GCTGCTCAGT TTGTGGCGAA TAAAAAATGG CGACCAAGAC 1020
   ACCTTGAAGG GCCTAATGCA CGCACTAAAG CACTCAAAGA CGTACCACTT TCCCAAAACT 1080
   GTCACTCAGA GTCTAAAGAA GACCATCAGG TTCCTTCACA GCTTCACAAT GTACAAATTG 1140
   TATCAGAAGT TATTTTTAGA AATGATAGGT AACCAGGTCC AATCAGTAAA AATAAGCTGC 1200
   TTATAA 1206
(2) INFORMATION FOR SEQUENCE ID NO: 86:
   (i) SEQUENCE CHARACTERISTICS:
      (A) LENGTH : 1206
      (B) TYPE : nucleic acid
      (C) STRANDEDNESS : single
      (D) TOPOLOGY : linear
   (ii) MOLECULE TYPE : cDNA (OCIF-C22S)
   (xi) SEQUENCE DESCRIPTION :SEQ ID NO: 86:
   ATGAACAACT TGCTGTGCTG CGCGCTCGTG TTTCTGGACA TCTCCATTAA GTGGACCACC 60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG CAAGGAAGGG CGCTACCTTG AGATAGAGTT CTGCTTGAAA 360
   CATAGGAGCT GCCCTCCTGG ATTTGGAGTG GTGCAAGCTG GAACCCCAGA GCGAAATACA 420
   GTTTGCAAAA GATGTCCAGA TGGGTTCTTC TCAAATGAGA CGTCATCTAA AGCACCCTGT 480
   AGAAAACACA CAAATTGCAG TGTCTTTGGT CTCCTGCTAA CTCAGAAAGG AAATGCAACA 540
   CACGACAACA TATGTTCCGG AAACAGTGAA TCAACTCAAA AATGTGGAAT AGATGTTACC 600
   CTGTGTGAGG AGGCATTCTT CAGGTTTGCT GTTCCTACAA AGTTTACGCC TAACTGGCTT 660
   AGTGTCTTGG TAGACAATTT GCCTGGCACC AAAGTAAACG CAGAGAGTGT AGAGAGGATA 720
   AAACGGCAAC ACAGCTCACA AGAACAGACT TTCCAGCTGC TGAAGTTATG GAAACATCAA 780
   AACAAAGACC AAGATATAGT CAAGAAGATC ATCCAAGATA TTGACCTCTG TGAAAACAGC 840
   GTGCAGCGGC ACATTGGACA TGCTAACCTC ACCTTCGAGC AGCTTCGTAG CTTGATGGAA 900
   AGCTTACCGG GAAAGAAAGT GGGAGCAGAA GACATTGAAA AAACAATAAA GGCAAGCAAA 960
   CCCAGTGACC AGATCCTGAA GCTGCTCAGT TTGTGGCGAA TAAAAAATGG CGACCAAGAC 1020
   ACCTTGAAGG GCCTAATGCA CGCACTAAAG CACTCAAAGA CGTACCACTT TCCCAAAACT 1080
   GTCACTCAGA GTCTAAAGAA GACCATCAGG TTCCTTCACA GCTTCACAAT GTACAAATTG 1140
   TATCAGAAGT TATTTTTAGA AATGATAGGT AACCAGGTCC AATCAGTAAA AATAAGCTGC 1200
   TTATAA 1206
(2) INFORMATION FOR SEQUENCE ID NO: 87:
   (i) SEQUENCE CHARACTERISTICS:
      (A) LENGTH : 1206
      (B) TYPE : nucleic acid
      (C) STRANDEDNESS : single
      (D) TOPOLOGY : linear
   (ii) MOLECULE TYPE : cDNA (OCIF-C23S)
   (xi) SEQUENCE DESCRIPTION :SEQ ID NO: 87:
   ATGAACAACT TGCTGTGCTG CGCGCTCGTG TTTCTGGACA TCTCCATTAA GTGGACCACC 60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG CAAGGAAGGG CGCTACCTTG AGATAGAGTT CTGCTTGAAA 360
   CATAGGAGCT GCCCTCCTGG ATTTGGAGTG GTGCAAGCTG GAACCCCAGA GCGAAATACA 420
   GTTTGCAAAA GATGTCCAGA TGGGTTCTTC TCAAATGAGA CGTCATCTAA AGCACCCTGT 480
   AGAAAACACA CAAATTGCAG TGTCTTTGGT CTCCTGCTAA CTCAGAAAGG AAATGCAACA 540
   CACGACAACA TATGTTCCGG AAACAGTGAA TCAACTCAAA AATGTGGAAT AGATGTTACC 600
   CTGTGTGAGG AGGCATTCTT CAGGTTTGCT GTTCCTACAA AGTTTACGCC TAACTGGCTT 660
   AGTGTCTTGG TAGACAATTT GCCTGGCACC AAAGTAAACG CAGAGAGTGT AGAGAGGATA 720
   AAACGGCAAC ACAGCTCACA AGAACAGACT TTCCAGCTGC TGAAGTTATG GAAACATCAA 780
   AACAAAGACC AAGATATAGT CAAGAAGATC ATCCAAGATA TTGACCTCTG TGAAAACAGC 840
   GTGCAGCGGC ACATTGGACA TGCTAACCTC ACCTTCGAGC AGCTTCGTAG CTTGATGGAA 900
   AGCTTACCGG GAAAGAAAGT GGGAGCAGAA GACATTGAAA AAACAATAAA GGCATGCAAA 960
   CCCAGTGACC AGATCCTGAA GCTGCTCAGT TTGTGGCGAA TAAAAAATGG CGACCAAGAC 1020
   ACCTTGAAGG GCCTAATGCA CGCACTAAAG CACTCAAAGA CGTACCACTT TCCCAAAACT 1080
   GTCACTCAGA GTCTAAAGAA GACCATCAGG TTCCTTCACA GCTTCACAAT GTACAAATTG 1140
   TATCAGAAGT TATTTTTAGA AATGATAGGT AACCAGGTCC AATCAGTAAA AATAAGCAGC 1200
   TTATAA 1206
(2) INFORMATION FOR SEQUENCE ID NO: 88:
   (i) SEQUENCE CHARACTERISTICS:
      (A) LENGTH : 1083
      (B) TYPE : nucleic acid
      (C) STRANDEDNESS : single
      (D) TOPOLOGY : linear
   (ii) MOLECULE TYPE : cDNA (OCIF-DCR1)
   (xi) SEQUENCE DESCRIPTION :SEQ ID NO: 88:
   ATGAACAACT TGCTGTGCTG CGCGCTCGTG TTTCTGGACA TCTCCATTAA GTGGACCACC   60
   CAGGAACCTT GCCCTGACCA CTACTACACA GACAGCTGGC ACACCAGTGA CGAGTGTCTA  120
   TACTGCAGCC CCGTGTGCAA GGAGCTGCAG TACGTCAAGC AGGAGTGCAA TCGCACCCAC  180
   AACCGCGTGT GCGAATGCAA GGAAGGGCGC TACCTTGAGA TAGAGTTCTG CTTGAAACAT  240
   AGGAGCTGCC CTCCTGGATT TGGAGTGGTG CAAGCTGGAA CCCCAGAGCG AAATACAGTT  300
   TGCAAAAGAT GTCCAGATGG GTTCTTCTCA AATGAGACGT CATCTAAAGC ACCCTGTAGA  360
   AAACACACAA ATTGCAGTGT CTTTGGTCTC CTGCTAACTC AGAAAGGAAA TGCAACACAC  420
   GACAACATAT GTTCCGGAAA CAGTGAATCA ACTCAAAAAT GTGGAATAGA TGTTACCCTG  480
   TGTGAGGAGG CATTCTTCAG GTTTGCTGTT CCTACAAAGT TTACGCCTAA CTGGCTTAGT  540
   GTCTTGGTAG ACAATTTGCC TGGCACCAAA GTAAACGCAG AGAGTGTAGA GAGGATAAAA  600
   CGGCAACACA GCTCACAAGA ACAGACTTTC CAGCTGCTGA AGTTATGGAA ACATCAAAAC  660
   AAAGACCAAG ATATAGTCAA GAAGATCATC CAAGATATTG ACCTCTGTGA AAACAGCGTG  720
   CAGCGGCACA TTGGACATGC TAACCTCACC TTCGAGCAGC TTCGTAGCTT GATGGAAAGC  780
   TTACCGGGAA AGAAAGTGGG AGCAGAAGAC ATTGAAAAAA CAATAAAGGC ATGCAAACCC  840
   AGTGACCAGA TCCTGAAGCT GCTCAGTTTG TGGCGAATAA AAAATGGCGA CCAAGACACC  900
   TTGAAGGGCC TAATGCACGC ACTAAAGCAC TCAAAGACGT ACCACTTTCC CAAAACTGTC  960
   ACTCAGAGTC TAAAGAAGAC CATCAGGTTC CTTCACAGCT TCACAATGTA CAAATTGTAT 1020
   CAGAAGTTAT TTTTAGAAAT GATAGGTAAC CAGGTCCAAT CAGTAAAAAT AAGCTGCTTA 1080
   TAA                                                              1083
(2) INFORMATION FOR SEQUENCE ID NO: 89:
   (i) SEQUENCE CHARACTERISTICS:
      (A) LENGTH : 1080
      (B) TYPE : nucleic acid
      (C) STRANDEDNESS : single
      (D) TOPOLOGY : linear
   (ii) MOLECULE TYPE : cDNA (OCIF-DCR2)
   (xi) SEQUENCE DESCRIPTION :SEQ ID NO: 89:
   ATGAACAACT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCG AATGCAAGGA AGGGCGCTAC CTTGAGATAG AGTTCTGCTT GAAACATAGG  240
   AGCTGCCCTC CTGGATTTGG AGTGGTGCAA GCTGGAACCC CAGAGCGAAA TACAGTTTGC  300
   AAAAGATGTC CAGATGGGTT CTTCTCAAAT GAGACGTCAT CTAAAGCACC CTGTAGAAAA  360
   CACACAAATT GCAGTGTCTT TGGTCTCCTG CTAACTCAGA AAGGAAATGC AACACACGAC  420
   AACATATGTT CCGGAAACAG TGAATCAACT CAAAAATGTG GAATAGATGT TACCCTGTGT  480
   GAGGAGGCAT TCTTCAGGTT TGCTGTTCCT ACAAAGTTTA CGCCTAACTG GCTTAGTGTC  540
   TTGGTAGACA ATTTGCCTGG CACCAAAGTA AACGCAGAGA GTGTAGAGAG GATAAAACGG  600
   CAACACAGCT CACAAGAACA GACTTTCCAG CTGCTGAAGT TATGGAAACA TCAAAACAAA  660
   GACCAAGATA TAGTCAAGAA GATCATCCAA GATATTGACC TCTGTGAAAA CAGCGTGCAG  720
   CGGCACATTG GACATGCTAA CCTCACCTTC GAGCAGCTTC GTAGCTTGAT GGAAAGCTTA  780
   CCGGGAAAGA AAGTGGGAGC AGAAGACATT GAAAAAACAA TAAAGGCATG CAAACCCAGT  840
   GACCAGATCC TGAAGCTGCT CAGTTTGTGG CGAATAAAAA ATGGCGACCA AGACACCTTG  900
   AAGGGCCTAA TGCACGCACT AAAGCACTCA AAGACGTACC ACTTTCCCAA AACTGTCACT  960
   CAGAGTCTAA AGAAGACCAT CAGGTTCCTT CACAGCTTCA CAATGTACAA ATTGTATCAG 1020
   AAGTTATTTT TAGAAATGAT AGGTAACCAG GTCCAATCAG TAAAAATAAG CTGCTTATAA 1080
(2) INFORMATION FOR SEQUENCE ID NO: 90:
   (i) SEQUENCE CHARACTERISTICS:
      (A) LENGTH : 1092
      (B) TYPE : nucleic acid
      (C) STRANDEDNESS : single
      (D) TOPOLOGY : linear
   (ii) MOLECULE TYPE : cDNA (OCIF-DCR3)
   (xi) SEQUENCE DESCRIPTION :SEQ ID NO: 90:
   ATGAACAACT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCC CTTGCCCTGA CCACTACTAC ACAGACAGCT GGCACACCAG TGACGAGTGT  240
   CTATACTGCA GCCCCGTGTG CAAGGAGCTG CAGTACGTCA AGCAGGAGTG CAATCGCACC 300
   CACAACCGCG TGTGCAGATG TCCAGATGGG TTCTTCTCAA ATGAGACGTC ATCTAAAGCA 360
   CCCTGTAGAA AACACACAAA TTGCAGTGTC TTTGGTCTCC TGCTAACTCA GAAAGGAAAT 420
   GCAACACACG ACAACATATG TTCCGGAAAC AGTGAATCAA CTCAAAAATG TGGAATAGAT 480
   GTTACCCTGT GTGAGGAGGC ATTCTTCAGG TTTGCTGTTC CTACAAAGTT TACGCCTAAC 540
   TGGCTTAGTG TCTTGGTAGA CAATTTGCCT GGCACCAAAG TAAACGCAGA GAGTGTAGAG 600
   AGGATAAAAC GGCAACACAG CTCACAAGAA CAGACTTTCC AGCTGCTGAA GTTATGGAAA 660
   CATCAAAACA AAGACCAAGA TATAGTCAAG AAGATCATCC AAGATATTGA CCTCTGTGAA 720
   AACAGCGTGC AGCGGCACAT TGGACATGCT AACCTCACCT TCGAGCAGCT TCGTAGCTTG 780
   ATGGAAAGCT TACCGGGAAA GAAAGTGGGA GCAGAAGACA TTGAAAAAAC AATAAAGGCA 840
   TGCAAACCCA GTGACCAGAT CCTGAAGCTG CTCAGTTTGT GGCGAATAAA AAATGGCGAC 900
   CAAGACACCT TGAAGGGCCT AATGCACGCA CTAAAGCACT CAAAGACGTA CCACTTTCCC 960
   AAAACTGTCA CTCAGAGTCT AAAGAAGACC ATCAGGTTCC TTCACAGCTT CACAATGTAC 1020
   AAATTGTATC AGAAGTTATT TTTAGAAATG ATAGGTAACC AGGTCCAATC AGTAAAAATA 1080
   AGCTGCTTAT AA 1092
(2) INFORMATION FOR SEQUENCE ID NO: 91:
   (i) SEQUENCE CHARACTERISTICS:
      (A) LENGTH : 1080
      (B) TYPE : nucleic acid
      (C) STRANDEDNESS : single
      (D) TOPOLOGY : linear
   (ii) MOLECULE TYPE : cDNA (OCIF-DCR4)
   (xi) SEQUENCE DESCRIPTION :SEQ ID NO: 91:
   ATGAACAACT TGCTGTGCTG CGCGCTCGTG TTTCTGGACA TCTCCATTAA GTGGACCACC 60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG CAAGGAAGGG CGCTACCTTG AGATAGAGTT CTGCTTGAAA 360
   CATAGGAGCT GCCCTCCTGG ATTTGGAGTG GTGCAAGCTG GAACCCCAGA GCGAAATACA 420
   GTTTGCAAAT CCGGAAACAG TGAATCAACT CAAAAATGTG GAATAGATGT TACCCTGTGT 480
   GAGGAGGCAT TCTTCAGGTT TGCTGTTCCT ACAAAGTTTA CGCCTAACTG GCTTAGTGTC 540
   TTGGTAGACA ATTTGCCTGG CACCAAAGTA AACGCAGAGA GTGTAGAGAG GATAAAACGG 600
   CAACACAGCT CACAAGAACA GACTTTCCAG CTGCTGAAGT TATGGAAACA TCAAAACAAA 660
   GACCAAGATA TAGTCAAGAA GATCATCCAA GATATTGACC TCTGTGAAAA CAGCGTGCAG 720
   CGGCACATTG GACATGCTAA CCTCACCTTC GAGCAGCTTC GTAGCTTGAT GGAAAGCTTA 780
   CCGGGAAAGA AAGTGGGAGC AGAAGACATT GAAAAAACAA TAAAGGCATG CAAACCCAGT 840
   GACCAGATCC TGAAGCTGCT CAGTTTGTGG CGAATAAAAA ATGGCGACCA AGACACCTTG 900
   AAGGGCCTAA TGCACGCACT AAAGCACTCA AAGACGTACC ACTTTCCCAA AACTGTCACT 960
   CAGAGTCTAA AGAAGACCAT CAGGTTCCTT CACAGCTTCA CAATGTACAA ATTGTATCAG 1020
   AAGTTATTTT TAGAAATGAT AGGTAACCAG GTCCAATCAG TAAAAATAAG CTGCTTATAA 1080
(2) INFORMATION FOR SEQUENCE ID NO: 92:
   (i) SEQUENCE CHARACTERISTICS:
      (A) LENGTH : 981
      (B) TYPE : nucleic acid
      (C) STRANDEDNESS : single
      (D) TOPOLOGY : linear
   (ii) MOLECULE TYPE : cDNA (OCIF-DDD1)
   (xi) SEQUENCE DESCRIPTION :SEQ ID NO: 92:
   ATGAACAACT TGCTGTGCTG CGCGCTCGTG TTTCTGGACA TCTCCATTAA GTGGACCACC 60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG CAAGGAAGGG CGCTACCTTG AGATAGAGTT CTGCTTGAAA 360
   CATAGGAGCT GCCCTCCTGG ATTTGGAGTG GTGCAAGCTG GAACCCCAGA GCGAAATACA 420
   GTTTGCAAAA GATGTCCAGA TGGGTTCTTC TCAAATGAGA CGTCATCTAA AGCACCCTGT 480
   AGAAAACACA CAAATTGCAG TGTCTTTGGT CTCCTGCTAA CTCAGAAAGG AAATGCAACA 540
   CACGACAACA TATGTTCCGG AAACAGTGAA TCAACTCAAA AATGTGGAAT AGATATTGAC 600
   CTCTGTGAAA ACAGCGTGCA GCGGCACATT GGACATGCTA ACCTCACCTT CGAGCAGCTT 660
   CGTAGCTTGA TGGAAAGCTT ACCGGGAAAG AAAGTGGGAG CAGAAGACAT TGAAAAAACA 720
   ATAAAGGCAT GCAAACCCAG TGACCAGATC CTGAAGCTGC TCAGTTTGTG GCGAATAAAA 780
   AATGGCGACC AAGACACCTT GAAGGGCCTA ATGCACGCAC TAAAGCACTC AAAGACGTAC 840
   CACTTTCCCA AAACTGTCAC TCAGAGTCTA AAGAAGACCA TCAGGTTCCT TCACAGCTTC 900
   ACAATGTACA AATTGTATCA GAAGTTATTT TTAGAAATGA TAGGTAACCA GGTCCAATCA 960
   GTAAAAATAA GCTGCTTATA A 981
(2) INFORMATION FOR SEQUENCE ID NO: 93:
   (i) SEQUENCE CHARACTERISTICS:
      (A) LENGTH : 984
      (B) TYPE : nucleic acid
      (C) STRANDEDNESS : single
      (D) TOPOLOGY : linear
   (ii) MOLECULE TYPE : cDNA (OCIF-DDD2)
   (xi) SEQUENCE DESCRIPTION :SEQ ID NO: 93:
   ATGAACAACT TGCTGTGCTG CGCGCTCGTG TTTCTGGACA TCTCCATTAA GTGGACCACC  60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG CAAGGAAGGG CGCTACCTTG AGATAGAGTT CTGCTTGAAA 360
   CATAGGAGCT GCCCTCCTGG ATTTGGAGTG GTGCAAGCTG GAACCCCAGA GCGAAATACA 420
   GTTTGCAAAA GATGTCCAGA TGGGTTCTTC TCAAATGAGA CGTCATCTAA AGCACCCTGT 480
   AGAAAACACA CAAATTGCAG TGTCTTTGGT CTCCTGCTAA CTCAGAAAGG AAATGCAACA 540
   CACGACAACA TATGTTCCGG AAACAGTGAA TCAACTCAAA AATGTGGAAT AGATGTTACC 600
   CTGTGTGAGG AGGCATTCTT CAGGTTTGCT GTTCCTACAA AGTTTACGCC TAACTGGCTT 660
   AGTGTCTTGG TAGACAATTT GCCTGGCACC AAAGTAAACG CAGAGAGTGT AGAGAGGATA 720
   AAACGGCAAC ACAGCTCACA AGAACAGACT TTCCAGCTGC TGAAGTTATG GAAACATCAA 780
   AACAAAGACC AAGATATAGT CAAGAAGATC ATCCAAGACG CACTAAAGCA CTCAAAGACG 840
   TACCACTTTC CCAAAACTGT CACTCAGAGT CTAAAGAAGA CCATCAGGTT CCTTCACAGC 900
   TTCACAATGT ACAAATTGTA TCAGAAGTTA TTTTTAGAAA TGATAGGTAA CCAGGTCCAA 960
   TCAGTAAAAA TAAGCTGCTT ATAA                                       984
(2) INFORMATION FOR SEQUENCE ID NO: 94:
   (i) SEQUENCE CHARACTERISTICS:
      (A) LENGTH : 1200
      (B) TYPE : nucleic acid
      (C) STRANDEDNESS : single
      (D) TOPOLOGY : linear
   (ii) MOLECULE TYPE : cDNA (OCIF-CL)
   (xi) SEQUENCE DESCRIPTION :SEQ ID NO: 94:
   ATGAACAACT TGCTGTGCTG CGCGCTCGTG TTTCTGGACA TCTCCATTAA GTGGACCACC  60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG CAAGGAAGGG CGCTACCTTG AGATAGAGTT CTGCTTGAAA 360
   CATAGGAGCT GCCCTCCTGG ATTTGGAGTG GTGCAAGCTG GAACCCCAGA GCGAAATACA 420
   GTTTGCAAAA GATGTCCAGA TGGGTTCTTC TCAAATGAGA CGTCATCTAA AGCACCCTGT 480
   AGAAAACACA CAAATTGCAG TGTCTTTGGT CTCCTGCTAA CTCAGAAAGG AAATGCAACA 540
   CACGACAACA TATGTTCCGG AAACAGTGAA TCAACTCAAA AATGTGGAAT AGATGTTACC 600
   CTGTGTGAGG AGGCATTCTT CAGGTTTGCT GTTCCTACAA AGTTTACGCC TAACTGGCTT 660
   AGTGTCTTGG TAGACAATTT GCCTGGCACC AAAGTAAACG CAGAGAGTGT AGAGAGGATA 720
   AAACGGCAAC ACAGCTCACA AGAACAGACT TTCCAGCTGC TGAAGTTATG GAAACATCAA 780
   AACAAAGACC AAGATATAGT CAAGAAGATC ATCCAAGATA TTGACCTCTG TGAAAACAGC 840
   GTGCAGCGGC ACATTGGACA TGCTAACCTC ACCTTCGAGC AGCTTCGTAG CTTGATGGAA 900
   AGCTTACCGG GAAAGAAAGT GGGAGCAGAA GACATTGAAA AAACAATAAA GGCATGCAAA 960
   CCCAGTGACC AGATCCTGAA GCTGCTCAGT TTGTGGCGAA TAAAAAATGG CGACCAAGAC 1020
   ACCTTGAAGG GCCTAATGCA CGCACTAAAG CACTCAAAGA CGTACCACTT TCCCAAAACT 1080
   GTCACTCAGA GTCTAAAGAA GACCATCAGG TTCCTTCACA GCTTCACAAT GTACAAATTG 1140
   TATCAGAAGT TATTTTTAGA AATGATAGGT AACCAGGTCC AATCAGTAAA AATAAGCTAA 1200
(2) INFORMATION FOR SEQUENCE ID NO: 95:
   (i) SEQUENCE CHARACTERISTICS:
      (A) LENGTH : 1056
      (B) TYPE : nucleic acid
      (C) STRANDEDNESS : single
      (D) TOPOLOGY : linear
   (ii) MOLECULE TYPE : cDNA (OCIF-CC)
   (xi) SEQUENCE DESCRIPTION :SEQ ID NO: 95:
   ATGAACAACT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG CAAGGAAGGG CGCTACCTTG AGATAGAGTT CTGCTTGAAA 360
   CATAGGAGCT GCCCTCCTGG ATTTGGAGTG GTGCAAGCTG GAACCCCAGA GCGAAATACA 420
   GTTTGCAAAA GATGTCCAGA TGGGTTCTTC TCAAATGAGA CGTCATCTAA AGCACCCTGT 480
   AGAAAACACA CAAATTGCAG TGTCTTTGGT CTCCTGCTAA CTCAGAAAGG AAATGCAACA 540
   CACGACAACA TATGTTCCGG AAACAGTGAA TCAACTCAAA AATGTGGAAT AGATGTTACC 600
   CTGTGTGAGG AGGCATTCTT CAGGTTTGCT GTTCCTACAA AGTTTACGCC TAACTGGCTT 660
   AGTGTCTTGG TAGACAATTT GCCTGGCACC AAAGTAAACG CAGAGAGTGT AGAGAGGATA 720
   AAACGGCAAC ACAGCTCACA AGAACAGACT TTCCAGCTGC TGAAGTTATG GAAACATCAA 780
   AACAAAGACC AAGATATAGT CAAGAAGATC ATCCAAGATA TTGACCTCTG TGAAAACAGC 840
   GTGCAGCGGC ACATTGGACA TGCTAACCTC ACCTTCGAGC AGCTTCGTAG CTTGATGGAA 900
   AGCTTACCGG GAAAGAAAGT GGGAGCAGAA GACATTGAAA AAACAATAAA GGCATGCAAA 960
   CCCAGTGACC AGATCCTGAA GCTGCTCAGT TTGTGGCGAA TAAAAAATGG CGACCAAGAC 1020
   ACCTTGAAGG GCCTAATGCA CGCACTAAAG CACTGA 1056
(2) INFORMATION FOR SEQUENCE ID NO: 96:
   (i) SEQUENCE CHARACTERISTICS:
      (A) LENGTH : 819
      (B) TYPE : nucleic acid
      (C) STRANDEDNESS : single
      (D) TOPOLOGY : linear
   (ii) MOLECULE TYPE : cDNA (OCIF-CDD2)
   (xi) SEQUENCE DESCRIPTION :SEQ ID NO: 96:
   ATGAACAACT TGCTGTGCTG CGCGCTCGTG TTTCTGGACA TCTCCATTAA GTGGACCACC 60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG CAAGGAAGGG CGCTACCTTG AGATAGAGTT CTGCTTGAAA 360
   CATAGGAGCT GCCCTCCTGG ATTTGGAGTG GTGCAAGCTG GAACCCCAGA GCGAAATACA 420
   GTTTGCAAAA GATGTCCAGA TGGGTTCTTC TCAAATGAGA CGTCATCTAA AGCACCCTGT 480
   AGAAAACACA CAAATTGCAG TGTCTTTGGT CTCCTGCTAA CTCAGAAAGG AAATGCAACA 540
   CACGACAACA TATGTTCCGG AAACAGTGAA TCAACTCAAA AATGTGGAAT AGATGTTACC 600
   CTGTGTGAGG AGGCATTCTT CAGGTTTGCT GTTCCTACAA AGTTTACGCC TAACTGGCTT 660
   AGTGTCTTGG TAGACAATTT GCCTGGCACC AAAGTAAACG CAGAGAGTGT AGAGAGGATA 720
   AAACGGCAAC ACAGCTCACA AGAACAGACT TTCCAGCTGC TGAAGTTATG GAAACATCAA 780
   AACAAAGACC AAGATATAGT CAAGAAGATC ATCCAATGA 819
(2) INFORMATION FOR SEQUENCE ID NO: 97:
   (i) SEQUENCE CHARACTERISTICS:
      (A) LENGTH : 594
      (B) TYPE : nucleic acid
      (C) STRANDEDNESS : single
      (D) TOPOLOGY : linear
   (ii) MOLECULE TYPE : cDNA (OCIF-CDD1)
   (xi) SEQUENCE DESCRIPTION :SEQ ID NO: 97:
   ATGAACAACT TGCTGTGCTG CGCGCTCGTG TTTCTGGACA TCTCCATTAA GTGGACCACC  60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG CAAGGAAGGG CGCTACCTTG AGATAGAGTT CTGCTTGAAA 360
   CATAGGAGCT GCCCTCCTGG ATTTGGAGTG GTGCAAGCTG GAACCCCAGA GCGAAATACA 420
   GTTTGCAAAA GATGTCCAGA TGGGTTCTTC TCAAATGAGA CGTCATCTAA AGCACCCTGT 480
   AGAAAACACA CAAATTGCAG TGTCTTTGGT CTCCTGCTAA CTCAGAAAGG AAATGCAACA 540
   CACGACAACA TATGTTCCGG AAACAGTGAA TCAACTCAAA AATGTGGAAT ATGA        594
(2) INFORMATION FOR SEQUENCE ID NO: 98:
   (i) SEQUENCE CHARACTERISTICS:
      (A) LENGTH : 432
      (B) TYPE : nucleic acid
      (C) STRANDEDNESS : single
      (D) TOPOLOGY : linear
   (ii) MOLECULE TYPE : cDNA (OCIF-CCR4)
   (xi) SEQUENCE DESCRIPTION :SEQ ID NO: 98:
   ATGAACAACT TGCTGTGCTG CGCGCTCGTG TTTCTGGACA TCTCCATTAA GTGGACCACC  60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG CAAGGAAGGG CGCTACCTTG AGATAGAGTT CTGCTTGAAA 360
   CATAGGAGCT GCCCTCCTGG ATTTGGAGTG GTGCAAGCTG GAACCCCAGA GCGAAATACA 420
   GTTTGCAAAT GA                                                     432
(2) INFORMATION FOR SEQUENCE ID NO: 99:
   (i) SEQUENCE CHARACTERISTICS:
      (A) LENGTH : 321
      (B) TYPE : nucleic acid
      (C) STRANDEDNESS : single
      (D) TOPOLOGY : linear
   (ii) MOLECULE TYPE : cDNA (OCIF-CCR3)
   (xi) SEQUENCE DESCRIPTION :SEQ ID NO: 99:
   ATGAACAACT TGCTGTGCTG CGCGCTCGTG TTTCTGGACA TCTCCATTAA GTGGACCACC  60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG A                                          321
(2) INFORMATION FOR SEQUENCE ID NO: 100:
   (i) SEQUENCE CHARACTERISTICS:
      (A) LENGTH : 1182
      (B) TYPE : nucleic acid
      (C) STRANDEDNESS : single
      (D) TOPOLOGY : linear
   (ii) MOLECULE TYPE : cDNA (OCIF-CBst)
   (xi) SEQUENCE DESCRIPTION :SEQ ID NO: 100:
   ATGAACAACT TGCTGTGCTG CGCGCTCGTG TTTCTGGACA TCTCCATTAA GTGGACCACC  60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG CAAGGAAGGG CGCTACCTTG AGATAGAGTT CTGCTTGAAA 360
   CATAGGAGCT GCCCTCCTGG ATTTGGAGTG GTGCAAGCTG GAACCCCAGA GCGAAATACA 420
   GTTTGCAAAA GATGTCCAGA TGGGTTCTTC TCAAATGAGA CGTCATCTAA AGCACCCTGT 480
   AGAAAACACA CAAATTGCAG TGTCTTTGGT CTCCTGCTAA CTCAGAAAGG AAATGCAACA 540
   CACGACAACA TATGTTCCGG AAACAGTGAA TCAACTCAAA AATGTGGAAT AGATGTTACC 600
   CTGTGTGAGG AGGCATTCTT CAGGTTTGCT GTTCCTACAA AGTTTACGCC TAACTGGCTT 660
   AGTGTCTTGG TAGACAATTT GCCTGGCACC AAAGTAAACG CAGAGAGTGT AGAGAGGATA 720
   AAACGGCAAC ACAGCTCACA AGAACAGACT TTCCAGCTGC TGAAGTTATG GAAACATCAA 780
   AACAAAGACC AAGATATAGT CAAGAAGATC ATCCAAGATA TTGACCTCTG TGAAAACAGC 840
   GTGCAGCGGC ACATTGGACA TGCTAACCTC ACCTTCGAGC AGCTTCGTAG CTTGATGGAA 900
   AGCTTACCGG GAAAGAAAGT GGGAGCAGAA GACATTGAAA AAACAATAAA GGCATGCAAA 960
   CCCAGTGACC AGATCCTGAA GCTGCTCAGT TTGTGGCGAA TAAAAAATGG CGACCAAGAC 1020
   ACCTTGAAGG GCCTAATGCA CGCACTAAAG CACTCAAAGA CGTACCACTT TCCCAAAACT 1080
   GTCACTCAGA GTCTAAAGAA GACCATCAGG TTCCTTCACA GCTTCACAAT GTACAAATTG 1140
   TATCAGAAGT TATTTTTAGA AATGATAGGT AACCTAGTCT AG 1182
(2) INFORMATION FOR SEQUENCE ID NO: 101:
   (i) SEQUENCE CHARACTERISTICS:
      (A) LENGTH : 966
      (B) TYPE : nucleic acid
      (C) STRANDEDNESS : single
      (D) TOPOLOGY : linear
   (ii) MOLECULE TYPE : cDNA (OCIF-CSph)
   (xi) SEQUENCE DESCRIPTION :SEQ ID NO: 101:
   ATGAACAACT TGCTGTGCTG CGCGCTCGTG TTTCTGGACA TCTCCATTAA GTGGACCACC 60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG CAAGGAAGGG CGCTACCTTG AGATAGAGTT CTGCTTGAAA 360
   CATAGGAGCT GCCCTCCTGG ATTTGGAGTG GTGCAAGCTG GAACCCCAGA GCGAAATACA 420
   GTTTGCAAAA GATGTCCAGA TGGGTTCTTC TCAAATGAGA CGTCATCTAA AGCACCCTGT 480
   AGAAAACACA CAAATTGCAG TGTCTTTGGT CTCCTGCTAA CTCAGAAAGG AAATGCAACA 540
   CACGACAACA TATGTTCCGG AAACAGTGAA TCAACTCAAA AATGTGGAAT AGATGTTACC 600
   CTGTGTGAGG AGGCATTCTT CAGGTTTGCT GTTCCTACAA AGTTTACGCC TAACTGGCTT 660
   AGTGTCTTGG TAGACAATTT GCCTGGCACC AAAGTAAACG CAGAGAGTGT AGAGAGGATA 720
   AAACGGCAAC ACAGCTCACA AGAACAGACT TTCCAGCTGC TGAAGTTATG GAAACATCAA 780
   AACAAAGACC AAGATATAGT CAAGAAGATC ATCCAAGATA TTGACCTCTG TGAAAACAGC 840
   GTGCAGCGGC ACATTGGACA TGCTAACCTC ACCTTCGAGC AGCTTCGTAG CTTGATGGAA 900
   AGCTTACCGG GAAAGAAAGT GGGAGCAGAA GACATTGAAA AAACAATAAA GGCTAGTCTA 960
   GACTAG 966
(2) INFORMATION FOR SEQUENCE ID NO: 102:
   (i) SEQUENCE CHARACTERISTICS:
      (A) LENGTH : 564
      (B) TYPE : nucleic acid
      (C) STRANDEDNESS : single
      (D) TOPOLOGY : linear
   (ii) MOLECULE TYPE : cDNA (OCIF-CBsp)
   (xi) SEQUENCE DESCRIPTION :SEQ ID NO: 102:
   ATGAACAACT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCC CTTGCCCTGA CCACTACTAC ACAGACAGCT GGCACACCAG TGACGAGTGT  240
   CTATACTGCA GCCCCGTGTG CAAGGAGCTG CAGTACGTCA AGCAGGAGTG CAATCGCACC  300
   CACAACCGCG TGTGCGAATG CAAGGAAGGG CGCTACCTTG AGATAGAGTT CTGCTTGAAA  360
   CATAGGAGCT GCCCTCCTGG ATTTGGAGTG GTGCAAGCTG GAACCCCAGA GCGAAATACA  420
   GTTTGCAAAA GATGTCCAGA TGGGTTCTTC TCAAATGAGA CGTCATCTAA AGCACCCTGT  480
   AGAAAACACA CAAATTGCAG TGTCTTTGGT CTCCTGCTAA CTCAGAAAGG AAATGCAACA  540
   CACGACAACA TATGTTCCGG CTAG                                          564
(2) INFORMATION FOR SEQUENCE ID NO: 103:
   (i) SEQUENCE CHARACTERISTICS:
      (A) LENGTH : 255
      (B) TYPE : nucleic acid
      (C) STRANDEDNESS : single
      (D) TOPOLOGY : linear
   (ii) MOLECULE TYPE : cDNA (OCIF-Pst)
   (xi) SEQUENCE DESCRIPTION :SEQ ID NO: 103:
   ATGAACAACT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCC CTTGCCCTGA CCACTACTAC ACAGACAGCT GGCACACCAG TGACGAGTGT  240
   CTATACCTAG TCTAG                                                    255
(2) INFORMATION FOR SEQUENCE ID NO: 104:
   (i) SEQUENCE CHARACTERISTICS:
      (A) LENGTH : 1317
      (B) TYPE : nucleic acid
      (C) STRANDEDNESS : double
      (D) TOPOLOGY : linear
   (ii) MOLECULE TYPE : human OCIF genomic DNA-1
   (xi) SEQUENCE DESCRIPTION :SEQ ID NO: 104:
   CTGGAGACAT ATAACTTGAA CACTTGGCCC TGATGGGGAA GCAGCTCTGC AGGGACTTTT    60
   TCAGCCATCT GTAAACAATT TCAGTGGCAA CCCGCGAACT GTAATCCATG AATGGGACCA   120
   CACTTTACAA GTCATCAAGT CTAACTTCTA GACCAGGGAA TTAATGGGGG AGACAGCGAA   180
   CCCTAGAGCA AAGTGCCAAA CTTCTGTCGA TAGCTTGAGG CTAGTGGAAA GACCTCGAGG   240
   AGGCTACTCC AGAAGTTCAG CGCGTAGGAA GCTCCGATAC CAATAGCCCT TTGATGATGG   300
   TGGGGTTGGT GAAGGGAACA GTGCTCCGCA AGGTTATCCC TGCCCCAGGC AGTCCAATTT   360
   TCACTCTGCA GATTCTCTCT GGCTCTAACT ACCCCAGATA ACAAGGAGTG AATGCAGAAT   420
   AGCACGGGCT TTAGGGCCAA TCAGACATTA GTTAGAAAAA TTCCTACTAC ATGGTTTATG   480
   TAAACTTGAA GATGAATGAT TGCGAACTCC CCGAAAAGGG CTCAGACAAT GCCATGCATA   540
   AAGAGGGGCC CTGTAATTTG AGGTTTCAGA ACCCGAAGTG AAGGGGTCAG GCAGCCGGGT   600
   ACGGCGGAAA CTCACAGCTT TCGCCCAGCG AGAGGACAAA GGTCTGGGAC ACACTCCAAC   660
   TGCGTCCGGA TCTTGGCTGG ATCGGACTCT CAGGGTGGAG GAGACACAAG CACAGCAGCT   720
   GCCCAGCGTG TGCCCAGCCC TCCCACCGCT GGTCCCGGCT GCCAGGAGGC TGGCCGCTGG   780
   CGGGAAGGGG CCGGGAAACC TCAGAGCCCC GCGGAGACAG CAGCCGCCTT GTTCCTCAGC   840
   CCGGTGGCTT TTTTTTCCCC TGCTCTCCCA GGGGACAGAC ACCACCGCCC CACCCCTCAC   900
   GCCCCACCTC CCTGGGGGAT CCTTTCCGCC CCAGCCCTGA AAGCGTTAAT CCTGGAGCTT   960
   TCTGCACACC CCCCGACCGC TCCCGCCCAA GCTTCCTAAA AAAGAAAGGT GCAAAGTTTG   1020
   GTCCAGGATA GAAAAATGAC TGATCAAAGG CAGGCGATAC TTCCTGTTGC CGGGACGCTA  1080
   TATATAACGT GATGAGCGCA CGGGCTGCGG AGACGCACCG GAGCGCTCGC CCAGCCGCCG  1140
   CCTCCAAGCC CCTGAGGTTT CCGGGGACCA CA ATG AAC AAG TTG CTG TGC TGC    1193
                                       Met Asn Lys Leu Leu Cys Cys
                                           -20                 -15
   GCG CTC GTG GTAAGTCCCT GGGCCAGCCG ACGGGTGCCC GGCGCCTGGG            1242
   Ala Leu Val
   GAGGCTGCTG CCACCTGGTC TCCCAACCTC CCAGCGGACC GGCGGGGAAA AAGGCTCCAC  1302
   TCGCTCCCTC CCAAG                                                   1317
(2) INFORMATION FOR SEQUENCE ID NO: 105:
   (i) SEQUENCE CHARACTERISTICS:
      (A) LENGTH :
      (B) TYPE : nucleic acid
      (C) STRANDEDNESS : double
      (D) TOPOLOGY : linear
   (ii) MOLECULE TYPE : human OCIF genomic DNA-2
   (xi) SEQUENCE DESCRIPTION :SEQ ID NO: 105:

### SEQUENCE LISTING

<110> GOTO, Masaaki
   TSUDA, Eisuke
   MOCHIZUKI, Shin'ichi
   YANO, Kazuki
   KOBAYASHI, Fumie
   SHIMA, Nobuyuki
   YASUDA, Hisataka
   NAKAGAWA, Nobuaki
   MORINAGA, Tomonori
   UEDA, Masatsugu
   HIGASHIO, Kanji
<120> Novel Proteins and Methods for Producing the Proteins
<130> 16991.004
<150> PCT/JP96/00374
   <151> 1996-02-20
<150> 08/915,004
   <151> 1997-08-20
<160> 108
<170> PatentIn version 3.1
<210> 1
   <211> 6
   <212> PRT
   <213> Homo sapiens
<220>
   <221> MISC_FEATURE
   <222> (1)..(1)
   <223> X = unknown
<400> 1
   Xaa Tyr His Phe Pro Lys
   1               5
<210> 2
   <211> 14
   <212> PRT
   <213> Homo sapiens
<220>
   <221> MISC_FEATURE
   <222> (1)..(1)
   <223> X = unknown
<220>
   <221> MISC_FEATURE
   <222> (5)..(5)
   <223> x = unknown
<220>
   <221> MISC_FEATURE
   <222> (13)..(13)
   <223> x = unknown
<400> 2
   Xaa Gln His Ser Xaa Gln Glu Gln Thr Phe Gln Leu Xaa Lys
   1               5                   10
<210> 3
   <211> 12
   <212> PRT
   <213> Homo sapiens
<220>
   <221> MISC_FEATURE
   <222> (1)..(1)
   <223> x = unknown
<400> 3
   Xaa Ile Arg Phe Leu His Ser Phe Thr Met Tyr Lys
   1               5                   10
<210> 4
   <211> 380
   <212> PRT
   <213> Homo sapiens
<400> 4
   Glu Thr Phe Pro Pro Lys Tyr Leu His Tyr Asp Glu Glu Thr Ser His
   1               5                   10                  15
   Gln Leu Leu Cys Asp Lys Cys Pro Pro Gly Thr Tyr Leu Lys Gln His
               20                  25                  30
   Cys Thr Ala Lys Trp Lys Thr Val Cys Ala Pro Cys Pro Asp His Tyr
           35                  40                  45
   Tyr Thr Asp Ser Trp His Thr Ser Asp Glu Cys Leu Tyr Cys Ser Pro
       50                  55                  60
   Val Cys Lys Glu Leu Gln Tyr Val Lys Gln Glu Cys Asn Arg Thr His
   65                  70                  75                  80
   Asn Arg Val Cys Glu Cys Lys Glu Gly Arg Tyr Leu Glu Ile Glu Phe
                   85                  90                  95
   Cys Leu Lys His Arg Ser Cys Pro Pro Gly Phe Gly Val Val Gln Ala
               100                 105                 110
   Gly Thr Pro Glu Arg Asn Thr Val Cys Lys Arg Cys Pro Asp Gly Phe
           115                 120                 125
   Phe Ser Asn Glu Thr Ser Ser Lys Ala Pro Cys Arg Lys His Thr Asn
       130                 135                 140
   Cys Ser Val Phe Gly Leu Leu Leu Thr Gln Lys Gly Asn Ala Thr His
   145                 150                 155                 160
   Asp Asn Ile Cys Ser Gly Asn Ser Glu Ser Thr Gln Lys Cys Gly Ile
   165 170 175
   Asp Val Thr Leu Cys Glu Glu Ala Phe Phe Arg Phe Ala Val Pro Thr
   180 185 190
   Lys Phe Thr Pro Asn Trp Leu Ser Val Leu Val Asp Asn Leu Pro Gly
   195 200 205
   Thr Lys Val Asn Ala Glu Ser Val Glu Arg Ile Lys Arg Gln His Ser
   210 215 220
   Ser Gln Glu Gln Thr Phe Gln Leu Leu Lys Leu Trp Lys His Gln Asn
   225 230 235 240
   Lys Asp Gln Asp Ile Val Lys Lys Ile Ile Gln Asp Ile Asp Leu Cys
   245 250 255
   Glu Asn Ser Val Gln Arg His Ile Gly His Ala Asn Leu Thr Phe Glu
   260 265 270
   Gln Leu Arg Ser Leu Met Glu Ser Leu Pro Gly Lys Lys Val Gly Ala
   275 280 285
   Glu Asp Ile Glu Lys Thr Ile Lys Ala Cys Lys Pro Ser Asp Gln Ile
   290 295 300
   Leu Lys Leu Leu Ser Leu Trp Arg Ile Lys Asn Gly Asp Gln Asp Thr
   305 310 315 320
   Leu Lys Gly Leu Met His Ala Leu Lys His Ser Lys Thr Tyr His Phe
   325 330 335
   Pro Lys Thr Val Thr Gln Ser Leu Lys Lys Thr Ile Arg Phe Leu His
   340 345 350
   Ser Phe Thr Met Tyr Lys Leu Tyr Gln Lys Leu Phe Leu Glu Met Ile
   355 360 365
   Gly Asn Gln Val Gln Ser Val Lys Ile Ser Cys Leu
   370 375 380
<210> 5
   <211> 401
   <212> PRT
   <213> Homo sapiens
<400> 5
   Met Asn Asn Leu Leu Cys Cys Ala Leu Val Phe Leu Asp Ile Ser Ile
   1 5 10 15
   Lys Trp Thr Thr Gln Glu Thr Phe Pro Pro Lys Tyr Leu His Tyr Asp
               20                  25                  30
   Glu Glu Thr Ser His Gln Leu Leu Cys Asp Lys Cys Pro Pro Gly Thr
           35                  40                  45
   Tyr Leu Lys Gln His Cys Thr Ala Lys Trp Lys Thr Val Cys Ala Pro
       50                  55                  60
   Cys Pro Asp His Tyr Tyr Thr Asp Ser Trp His Thr Ser Asp Glu Cys
   65                  70                  75                  80
   Leu Tyr Cys Ser Pro Val Cys Lys Glu Leu Gln Tyr Val Lys Gln Glu
                   85                  90                  95
   Cys Asn Arg Thr His Asn Arg Val Cys Glu Cys Lys Glu Gly Arg Tyr
               100                 105                 110
   Leu Glu Ile Glu Phe Cys Leu Lys His Arg Ser Cys Pro Pro Gly Phe
           115                 120                 125
   Gly Val Val Gln Ala Gly Thr Pro Glu Arg Asn Thr Val Cys Lys Arg
       130                 135                 140
   Cys Pro Asp Gly Phe Phe Ser Asn Glu Thr Ser Ser Lys Ala Pro Cys
   145                 150                 155                 160
   Arg Lys His Thr Asn Cys Ser Val Phe Gly Leu Leu Leu Thr Gln Lys
                   165                 170                 175
   Gly Asn Ala Thr His Asp Asn Ile Cys Ser Gly Asn Ser Glu Ser Thr
               180                 185                 190
   Gln Lys Cys Gly Ile Asp Val Thr Leu Cys Glu Glu Ala Phe Phe Arg
           195                 200                 205
   Phe Ala Val Pro Thr Lys Phe Thr Pro Asn Trp Leu Ser Val Leu Val
       210                 215                 220
   Asp Asn Leu Pro Gly Thr Lys Val Asn Ala Glu Ser Val Glu Arg Ile
   225                 230                 235                 240
   Lys Arg Gln His Ser Ser Gln Glu Gln Thr Phe Gln Leu Leu Lys Leu
                   245                 250                 255
   Trp Lys His Gln Asn Lys Asp Gln Asp Ile Val Lys Lys Ile Ile Gln
               260                 265                 270
   Asp Ile Asp Leu Cys Glu Asn Ser Val Gln Arg His Ile Gly His Ala
           275                 280                 285
   Asn Leu Thr Phe Glu Gln Leu Arg Ser Leu Met Glu Ser Leu Pro Gly
   290 295 300
   Lys Lys Val Gly Ala Glu Asp Ile Glu Lys Thr Ile Lys Ala Cys Lys
   305 310 315 320
   Pro Ser Asp Gln Ile Leu Lys Leu Leu Ser Leu Trp Arg Ile Lys Asn
   325 330 335
   Gly Asp Gln Asp Thr Leu Lys Gly Leu Met His Ala Leu Lys His Ser
   340 345 350
   Lys Thr Tyr His Phe Pro Lys Thr Val Thr Gln Ser Leu Lys Lys Thr
   355 360 365
   Ile Arg Phe Leu His Ser Phe Thr Met Tyr Lys Leu Tyr Gln Lys Leu
   370 375 380
   Phe Leu Glu Met Ile Gly Asn Gln Val Gln Ser Val Lys Ile Ser Cys
   385 390 395 400
   Leu
<210> 6
   <211> 1206
   <212> DNA
   <213> Homo sapiens
<400> 6
   atgaacaact tgctgtgctg cgcgctcgtg tttctggaca tctccattaa gtggaccacc 60
   caggaaacgt ttcctccaaa gtaccttcat tatgacgaag aaacctctca tcagctgttg 120
   tgtgacaaat gtcctcctgg tacctaccta aaacaacact gtacagcaaa gtggaagacc 180
   gtgtgcgccc cttgccctga ccactactac acagacagct ggcacaccag tgacgagtgt 240
   ctatactgca gccccgtgtg caaggagctg cagtacgtca agcaggagtg caatcgcacc 300
   cacaaccgcg tgtgcgaatg caaggaaggg cgctaccttg agatagagtt ctgcttgaaa 360
   cataggagct gccctcctgg atttggagtg gtgcaagctg gaaccccaga gcgaaataca 420
   gtttgcaaaa gatgtccaga tgggttcttc tcaaatgaga cgtcatctaa agcaccctgt 480
   agaaaacaca caaattgcag tgtctttggt ctcctgctaa ctcagaaagg aaatgcaaca 540
   cacgacaaca tatgttccgg aaacagtgaa tcaactcaaa aatgtggaat agatgttacc 600
   ctgtgtgagg aggcattctt caggtttgct gttcctacaa agtttacgcc taactggctt 660
   agtgtcttgg tagacaattt gcctggcacc aaagtaaacg cagagagtgt agagaggata 720
   aaacggcaac acagctcaca agaacagact ttccagctgc tgaagttatg gaaacatcaa 780
   aacaaagacc aagatatagt caagaagatc atccaagata ttgacctctg tgaaaacagc 840
   gtgcagcggc acattggaca tgctaacctc accttcgagc agcttcgtag cttgatggaa 900
   agcttaccgg gaaagaaagt gggagcagaa gacattgaaa aaacaataaa ggcatgcaaa 960
   cccagtgacc agatcctgaa gctgctcagt ttgtggcgaa taaaaaatgg cgaccaagac 1020
   accttgaagg gcctaatgca cgcactaaag cactcaaaga cgtaccactt tcccaaaact 1080
   gtcactcaga gtctaaagaa gaccatcagg ttccttcaca gcttcacaat gtacaaattg 1140
   tatcagaagt tatttttaga aatgataggt aaccaggtcc aatcagtaaa aataagctgc 1200
   ttataa 1206
<210> 7
   <211> 15
   <212> PRT
   <213> Homo sapiens
<400> 7
   Glu Thr Phe Pro Pro Lys Tyr Leu His Tyr Asp Glu Glu Thr Ser
   1               5                   10                  15
<210> 8
   <211> 1185
   <212> DNA
   <213> Homo sapiens
<400> 8
   atgaacaact tgctgtgctg cgcgctcgtg tttctggaca tctccattaa gtggaccacc      60
   caggaaacgt ttcctccaaa gtaccttcat tatgacgaag aaacctctca tcagctgttg     120
   tgtgacaaat gtcctcctgg tacctaccta aaacaacact gtacagcaaa gtggaagacc     180
   gtgtgcgccc cttgccctga ccactactac acagacagct ggcacaccag tgacgagtgt     240
   ctatactgca gccccgtgtg caaggagtgc aatcgcaccc acaaccgcgt gtgcgaatgc     300
   aaggaagggc gctaccttga gatagagttc tgcttgaaac ataggagctg ccctcctgga     360
   tttggagtgg tgcaagctgg aaccccagag cgaaatacag tttgcaaaag atgtccagat     420
   gggttcttct caaatgagac gtcatctaaa gcaccctgta gaaaacacac aaattgcagt     480
   gtctttggtc tcctgctaac tcagaaagga aatgcaacac acgacaacat atgttccgga     540
   aacagtgaat caactcaaaa atgtggaata gatgttaccc tgtgtgagga ggcattcttc     600
   aggtttgctg ttcctacaaa gtttacgcct aactggctta gtgtcttggt agacaatttg     660
   cctggcacca aagtaaacgc agagagtgta gagaggataa aacggcaaca cagctcacaa     720
   gaacagactt tccagctgct gaagttatgg aaacatcaaa acaaagacca agatatagtc     780
   aagaagatca tccaagatat tgacctctgt gaaaacagcg tgcagcggca cattggacat     840
   gctaacctca ccttcgagca gcttcgtagc ttgatggaaa gcttaccggg aaagaaagtg     900
   ggagcagaag acattgaaaa aacaataaag gcatgcaaac ccagtgacca gatcctgaag     960
   ctgctcagtt tgtggcgaat aaaaaatggc gaccaagaca ccttgaaggg cctaatgcac    1020
   gcactaaagc actcaaagac gtaccacttt cccaaaactg tcactcagag tctaaagaag    1080
   accatcaggt tccttcacag cttcacaatg tacaaattgt atcagaagtt atttttagaa    1140
   atgataggta accaggtcca atcagtaaaa ataagctgct tataa                     1185
<210> 9
   <211> 394
   <212> PRT
   <213> Homo sapiens
<400> 9
   Met Asn Asn Leu Leu Cys Cys Ala Leu Val Phe Leu Asp Ile Ser Ile
   1 5 10 15
   Lys Trp Thr Thr Gln Glu Thr Phe Pro Pro Lys Tyr Leu His Tyr Asp
   20 25 30
   Glu Glu Thr Ser His Gln Leu Leu Cys Asp Lys Cys Pro Pro Gly Thr
   35 40 45
   Tyr Leu Lys Gln His Cys Thr Ala Lys Trp Lys Thr Val Cys Ala Pro
   50 55 60
   Cys Pro Asp His Tyr Tyr Thr Asp Ser Trp His Thr Ser Asp Glu Cys
   65 70 75 80
   Leu Tyr Cys Ser Pro Val Cys Lys Glu Cys Asn Arg Thr His Asn Arg
   85 90 95
   Val Cys Glu Cys Lys Glu Gly Arg Tyr Leu Glu Ile Glu Phe Cys Leu
   100 105 110
   Lys His Arg Ser Cys Pro Pro Gly Phe Gly Val Val Gln Ala Gly Thr
   115 120 125
   Pro Glu Arg Asn Thr Val Cys Lys Arg Cys Pro Asp Gly Phe Phe Ser
   130 135 140
   Asn Glu Thr Ser Ser Lys Ala Pro Cys Arg Lys His Thr Asn Cys Ser
   145 150 155 160
   Val Phe Gly Leu Leu Leu Thr Gln Lys Gly Asn Ala Thr His Asp Asn
   165 170 175
   Ile Cys Ser Gly Asn Ser Glu Ser Thr Gln Lys Cys Gly Ile Asp Val
   180 185 190
   Thr Leu Cys Glu Glu Ala Phe Phe Arg Phe Ala Val Pro Thr Lys Phe
   195 200 205
   Thr Pro Asn Trp Leu Ser Val Leu Val Asp Asn Leu Pro Gly Thr Lys
   210 215 220
   Val Asn Ala Glu Ser Val Glu Arg Ile Lys Arg Gln His Ser Ser Gln
   225 230 235 240
   Glu Gln Thr Phe Gln Leu Leu Lys Leu Trp Lys His Gln Asn Lys Asp
   245 250 255
   Gln Asp Ile Val Lys Lys Ile Ile Gln Asp Ile Asp Leu Cys Glu Asn
   260 265 270
   Ser Val Gln Arg His Ile Gly His Ala Asn Leu Thr Phe Glu Gln Leu
   275 280 285
   Arg Ser Leu Met Glu Ser Leu Pro Gly Lys Lys Val Gly Ala Glu Asp
   290 295 300
   Ile Glu Lys Thr Ile Lys Ala Cys Lys Pro Ser Asp Gln Ile Leu Lys
   305 310 315 320
   Leu Leu Ser Leu Trp Arg Ile Lys Asn Gly Asp Gln Asp Thr Leu Lys
   325 330 335
   Gly Leu Met His Ala Leu Lys His Ser Lys Thr Tyr His Phe Pro Lys
   340 345 350
   Thr Val Thr Gln Ser Leu Lys Lys Thr Ile Arg Phe Leu His Ser Phe
   355 360 365
   Thr Met Tyr Lys Leu Tyr Gln Lys Leu Phe Leu Glu Met Ile Gly Asn
   370 375 380
   Gln Val Gln Ser Val Lys Ile Ser Cys Leu
   385 390
<210> 10
   <211> 1089
   <212> DNA
   <213> Homo sapiens
<400> 10
   atgaacaagt tgctgtgctg cgcgctcgtg tttctggaca tctccattaa gtggaccacc      60
   caggaaacgt ttcctccaaa gtaccttcat tatgacgaag aaacctctca tcagctgttg     120
   tgtgacaaat gtcctcctgg tacctaccta aaacaacact gtacagcaaa gtggaagacc     180
   gtgtgcgccc cttgccctga ccactactac acagacagct ggcacaccag tgacgagtgt     240
   ctatactgca gccccgtgtg caaggagctg cagtacgtca agcaggagtg caatcgcacc     300
   cacaaccgcg tgtgcgaatg caaggaaggg cgctaccttg agatagagtt ctgcttgaaa     360
   cataggagct gccctcctgg atttggagtg gtgcaagctg gaaccccaga gcgaaataca     420
   gtttgcaaaa gatgtccaga tgggttcttc tcaaatgaga cgtcatctaa agcaccctgt     480
   agaaaacaca caaattgcag tgtctttggt ctcctgctaa ctcagaaagg aaatgcaaca     540
   cacgacaaca tatgttccgg aaacagtgaa tcaactcaaa aatgtggaat agatgttacc     600
   ctgtgtgagg aggcattctt caggtttgct gttcctacaa agtttacgcc taactggctt     660
   agtgtcttgg tagacaattt gcctggcacc aaagtaaacg cagagagtgt agagaggata     720
   aaacggcaac acagctcaca agaacagact ttccagctgc tgaagttatg gaaacatcaa     780
   aacaaagacc aagatatagt caagaagatc atccaagata ttgacctctg tgaaaacagc     840
   gtgcagcggc acattggaca tgctaacctc agtttgtggc gaataaaaaa tggcgaccaa     900
   gacaccttga agggcctaat gcacgcacta aagcactcaa agacgtacca ctttcccaaa     960
   actgtcactc agagtctaaa gaagaccatc aggttccttc acagcttcac aatgtacaaa    1020
   ttgtatcaga agttattttt agaaatgata ggtaaccagg tccaatcagt aaaaataagc    1080
   tgcttataa                                                            1089
<210> 11
   <211> 362
   <212> PRT
   <213> Homo sapiens
<400> 11
   Met Asn Lys Leu Leu Cys Cys Ala Leu Val Phe Leu Asp Ile Ser Ile
   1               5                   10                  15
   Lys Trp Thr Thr Gln Glu Thr Phe Pro Pro Lys Tyr Leu His Tyr Asp
               20                  25                  30
   Glu Glu Thr Ser His Gln Leu Leu Cys Asp Lys Cys Pro Pro Gly Thr
           35                  40                  45
   Tyr Leu Lys Gln His Cys Thr Ala Lys Trp Lys Thr Val Cys Ala Pro
       50                  55                  60
   Cys Pro Asp His Tyr Tyr Thr Asp Ser Trp His Thr Ser Asp Glu Cys
   65                  70                  75                  80
   Leu Tyr Cys Ser Pro Val Cys Lys Glu Leu Gln Tyr Val Lys Gln Glu
                   85                  90                  95
   Cys Asn Arg Thr His Asn Arg Val Cys Glu Cys Lys Glu Gly Arg Tyr
               100                 105                 110
   Leu Glu Ile Glu Phe Cys Leu Lys His Arg Ser Cys Pro Pro Gly Phe
           115                 120                 125
   Gly Val Val Gln Ala Gly Thr Pro Glu Arg Asn Thr Val Cys Lys Arg
       130                 135                 140
   Cys Pro Asp Gly Phe Phe Ser Asn Glu Thr Ser Ser Lys Ala Pro Cys
   145                 150                 155                 160
   Arg Lys His Thr Asn Cys Ser Val Phe Gly Leu Leu Leu Thr Gln Lys
   165 170 175
   Gly Asn Ala Thr His Asp Asn Ile Cys Ser Gly Asn Ser Glu Ser Thr
   180 185 190
   Gln Lys Cys Gly Ile Asp Val Thr Leu Cys Glu Glu Ala Phe Phe Arg
   195 200 205
   Phe Ala Val Pro Thr Lys Phe Thr Pro Asn Trp Leu Ser Val Leu Val
   210 215 220
   Asp Asn Leu Pro Gly Thr Lys Val Asn Ala Glu Ser Val Glu Arg Ile
   225 230 235 240
   Lys Arg Gln His Ser Ser Gln Glu Gln Thr Phe Gln Leu Leu Lys Leu
   245 250 255
   Trp Lys His Gln Asn Lys Asp Gln Asp Ile Val Lys Lys Ile Ile Gln
   260 265 270
   Asp Ile Asp Leu Cys Glu Asn Ser Val Gln Arg His Ile Gly His Ala
   275 280 285
   Asn Leu Ser Leu Trp Arg Ile Lys Asn Gly Asp Gln Asp Thr Leu Lys
   290 295 300
   Gly Leu Met His Ala Leu Lys His Ser Lys Thr Tyr His Phe Pro Lys
   305 310 315 320
   Thr Val Thr Gln Ser Leu Lys Lys Thr Ile Arg Phe Leu His Ser Phe
   325 330 335
   Thr Met Tyr Lys Leu Tyr Gln Lys Leu Phe Leu Glu Met Ile Gly Asn
   340 345 350
   Gln Val Gln Ser Val Lys Ile Ser Cys Leu
   355 360
<210> 12
   <211> 465
   <212> DNA
   <213> Homo sapiens
<400> 12
   atgaacaagt tgctgtgctg ctcgctcgtg tttctggaca tctccattaa gtggaccacc 60
   caggaaacgt ttcctccaaa gtaccttcat tatgacgaag aaacctctca tcagctgttg 120
   tgtgacaaat gtcctcctgg tacctaccta aaacaacact gtacagcaaa gtggaagacc 180
   gtgtgcgccc cttgccctga ccactactac acagacagct ggcacaccag tgacgagtgt 240
   ctatactgca gccccgtgtg caaggagctg cagtacgtca agcaggagtg caatcgcacc 300
   cacaaccgcg tgtgcgaatg caaggaaggg cgctaccttg agatagagtt ctgcttgaaa 360
   cataggagct gccctcctgg atttggagtg gtgcaagctg gtacgtgtca atgtgcagca 420
   aaattaatta ggatcatgca aagtcagata gttgtgacag tttag 465
<210> 13
   <211> 154
   <212> PRT
   <213> Homo sapiens
<400> 13
   Met Asn Lys Leu Leu Cys Cys Ser Leu Val Phe Leu Asp Ile Ser Ile
   1               5                   10                  15
   Lys Trp Thr Thr Gln Glu Thr Phe Pro Pro Lys Tyr Leu His Tyr Asp
               20                  25                  30
   Glu Glu Thr Ser His Gln Leu Leu Cys Asp Lys Cys Pro Pro Gly Thr
           35                  40                  45
   Tyr Leu Lys Gln His Cys Thr Ala Lys Trp Lys Thr Val Cys Ala Pro
       50                  55                  60
   Cys Pro Asp His Tyr Tyr Thr Asp Ser Trp His Thr Ser Asp Glu Cys
   65                  70                  75                  80
   Leu Tyr Cys Ser Pro Val Cys Lys Glu Leu Gln Tyr Val Lys Gln Glu
                   85                  90                  95
   Cys Asn Arg Thr His Asn Arg Val Cys Glu Cys Lys Glu Gly Arg Tyr
               100                 105                 110
   Leu Glu Ile Glu Phe Cys Leu Lys His Arg Ser Cys Pro Pro Gly Phe
           115                 120                 125
   Gly Val Val Gln Ala Gly Thr Cys Gln Cys Ala Ala Lys Leu Ile Arg
       130                 135                 140
   Ile Met Gln Ser Gln Ile Val Val Thr Val
   145                 150
<210> 14
   <211> 438
   <212> DNA
   <213> Homo sapiens
<400> 14
   atgaacaagt tgctgtgctg cgcgctcgtg tttctggaca tctccattaa gtggaccacc 60
   caggaaacgt ttcctccaaa gtaccttcat tatgacgaag aaacctctca tcagctgttg 120
   tgtgacaaat gtcctcctgg tacctaccta aaacaacact gtacagcaaa gtggaagacc 180
   gtgtgcgccc cttgccctga ccactactac acagacagct ggcacaccag tgacgagtgt 240
   ctatactgca gccccgtgtg caaggagctg cagtacgtca agcaggagtg caatcgcacc 300
   cacaaccgcg tgtgcgaatg caaggaaggg cgctaccttg agatagagtt ctgcttgaaa 360
   cataggagct gccctcctgg atttggagtg gtgcaagctg gatgcaggag aagacccaag 420
   ccacagatat gtatctga 438
<210> 15
   <211> 145
   <212> PRT
   <213> Homo sapiens
<400> 15
<210> 16
   <211> 20
   <212> DNA
   <213> Artificial sequence
<220>
   <223> Synthetic sequence
<400> 16
   aattaaccct cactaaaggg 20
<210> 17
   <211> 22
   <212> DNA
   <213> Artificial Sequence
<220>
   <223> Synthetic Sequence
<400> 17
   gtaatacgac tcactatagg gc 22
<210> 18
   <211> 20
   <212> DNA
   <213> Artificial Sequence
<220>
   <223> Synthetic Sequence
<400> 18
   acatcaaaac aaagaccaag 20
<210> 19
   <211> 20
   <212> DNA
   <213> Artificial Sequence
<220>
   <223> synthetic sequence
<400> 19
   tcttggtctt tgttttgatg 20
<210> 20
   <211> 20
   <212> DNA
   <213> Artificial Sequence
<220>
   <223> Synthetic Sequence
<400> 20
   ttattcgcca caaactgagc 20
<210> 21
   <211> 20
   <212> DNA
   <213> Artificial Sequence
<220>
   <223> synthetic sequence
<400> 21
   ttgtgaagct gtgaaggaac 20
<210> 22
   <211> 20
   <212> DNA
   <213> Artificial Sequence
<220>
   <223> Synthetic sequence
<400> 22
   gctcagtttg tggcgaataa 20
<210> 23
   <211> 20
   <212> DNA
   <213> Artificial sequence
<220>
   <223> synthetic Sequence
<400> 23
   gtgggagcag aagacattga 20
<210> 24
   <211> 20
   <212> DNA
   <213> Artificial sequence
<220>
   <223> synthetic sequence
<400> 24
   aatgaacaac ttgctgtgct 20
<210> 25
   <211> 20
   <212> DNA
   <213> Artificial sequence
<220>
   <223> synthetic Sequence
<400> 25
   tgacaaatgt cctcctggta 20
<210> 26
   <211> 20
   <212> DNA
   <213> Artificial Sequence
<220>
   <223> Synthetic Sequence
<400> 26
   aggtaggtac caggaggaca 20
<210> 27
   <211> 20
   <212> DNA
   <213> Artificial sequence
<220>
   <223> Synthetic sequence
<400> 27
   gagctgccct cctggatttg 20
<210> 28
   <211> 20
   <212> DNA
   <213> Artificial sequence
<220>
   <223> Synthetic Sequence
<400> 28
   caaactgtat ttcgctctgg 20
<210> 29
   <211> 20
   <212> DNA
   <213> Artificial sequence
<220>
   <223> Synthetic Sequence
<400> 29
   gtgtgaggag gcattcttca 20
<210> 30
   <211> 32
   <212> DNA
   <213> Artificial sequence
<220>
   <223> synthetic Sequence
<400> 30
   gaatcaactc aaaaaagtgg aatagatgtt ac 32
<210> 31
   <211> 32
   <212> DNA
   <213> Artificial sequence
<220>
   <223> Synthetic sequence
<400> 31
   gtaacatcta ttccactttt ttgagttgat tc 32
<210> 32
   <211> 30
   <212> DNA
   <213> Artificial Sequence
<220>
   <223> Synthetic Sequence
<400> 32
   atagatgtta ccctgagtga ggaggcattc 30
<210> 33
   <211> 30
   <212> DNA
   <213> Artificial Sequence
<220>
   <223> Synthetic Sequence
<400> 33
   gaatgcctcc tcactcaggg taacatctat 30
<210> 34
   <211> 31
   <212> DNA
   <213> Artificial Sequence
<220>
   <223> synthetic Sequence
<400> 34
   caagatattg acctcagtga aaacagcgtg c 31
<210> 35
   <211> 31
   <212> DNA
   <213> Artificial Sequence
<220>
   <223> Synthetic Sequence
<400> 35
   gcacgctgtt ttcactgagg gcaatatctt g 31
<210> 36
   <211> 31
   <212> DNA
   <213> Artificial Sequence
<220>
   <223> Synthetic Sequence
<400> 36
   aaaacaataa aggcaagcaa acccagtgac c 31
<210> 37
   <211> 31
   <212> DNA
   <213> Artificial sequence
<220>
   <223> Synthetic Sequence
<400> 37
   ggtcactggg tttgcttgcc tttattgttt t 31
<210> 38
   <211> 31
   <212> DNA
   <213> Artificial Sequence
<220>
   <223> Synthetic Sequence
<400> 38
   tcagtaaaaa taagcagctt ataactggcc a 31
<210> 39
   <211> 31
   <212> DNA
   <213> Artificial Sequence
<220>
   <223> Synthetic Sequence
<400> 39
   tggccagtta taagctgctt atttttactg a 31
<210> 40
   <211> 22
   <212> DNA
   <213> Artificial sequence
<220>
   <223> Synthetic sequence
<400> 40
   ttggggttta ttggaggaga tg 22
<210> 41
   <211> 36
   <212> DNA
   <213> Artificial Sequence
<220>
   <223> Synthetic Sequence
<400> 41
   accacccagg aaccttgccc tgaccactac tacaca 36
<210> 42
   <211> 36
   <212> DNA
   <213> Artificial Sequence
<220>
   <223> Synthetic sequence
<400> 42
   gtcagggcaa ggttcctggg tggtccactt aatgga 36
<210> 43
   <211> 36
   <212> DNA
   <213> Artificial Sequence
<220>
   <223> Synthetic sequence
<400> 43
   accgtgtgcg ccgaatgcaa ggaagggcgc tacctt 36
<210> 44
   <211> 36
   <212> DNA
   <213> Artificial Sequence
<220>
   <223> Synthetic Sequence
<400> 44
   ttccttgcat tcggcgcaca cggtcttcca ctttgc 36
<210> 45
   <211> 36
   <212> DNA
   <213> Artificial Sequence
<220>
   <223> Synthetic sequence
<400> 45
   aaccgcgtgt gcagatgtcc agatgggttc ttctca 36
<210> 46
   <211> 36
   <212> DNA
   <213> Artificial Sequence
<220>
   <223> Synthetic Sequence
<400> 46
   atctggacat ctgcacacgc ggttgtgggt gcgatt 36
<210> 47
   <211> 36
   <212> DNA
   <213> Artificial Sequence
<220>
   <223> synthetic sequence
<400> 47
   acagtttgca aatccggaaa cagtgaatca actcaa 36
<210> 48
   <211> 36
   <212> DNA
   <213> Artificial Sequence
<220>
   <223> Synthetic Sequence
<400> 48
   actgtttccg gatttgcaaa ctgtatttcg ctctgg 36
<210> 49
   <211> 36
   <212> DNA
   <213> Artificial Sequence
<220>
   <223> Synthetic Sequence
<400> 49
   aatgtggaat agatattgac ctctgtgaaa acagcg 36
<210> 50
   <211> 36
   <212> DNA
   <213> Artificial Sequence
<220>
   <223> Synthetic Sequence
<400> 50
   agaggtcaat atctattcca catttttgag ttgatt 36
<210> 51
   <211> 36
   <212> DNA
   <213> Artificial sequence
<220>
   <223> synthetic Sequence
<400> 51
   agatcatcca agacgcacta aagcactcaa agacgt 36
<210> 52
   <211> 36
   <212> DNA
   <213> Artificial sequence
<220>
   <223> Synthetic Sequence
<400> 52
   gctttagtgc gtcttggatg atcttcttga ctatat 36
<210> 53
   <211> 29
   <212> DNA
   <213> Artificial Sequence
<220>
   <223> synthetic sequence
<400> 53
   ggctcgagcg cccagccgcc gcctccaag 29
<210> 54
   <211> 20
   <212> DNA
   <213> Artificial sequence
<220>
   <223> Synthetic Sequence
<400> 54
   tttgagtgct ttagtgcgtg 20
<210> 55
   <211> 30
   <212> DNA
   <213> Artificial Sequence
<220>
   <223> synthetic sequence
<400> 55
   tcagtaaaaa taagctaact ggaaatggcc 30
<210> 56
   <211> 30
   <212> DNA
   <213> Artificial Sequence
<220>
   <223> Synthetic sequence
<400> 56
   ggccatttcc agttagctta tttttactga 30
<210> 57
   <211> 29
   <212> DNA
   <213> Artificial Sequence
<220>
   <223> Synthetic Sequence
<400> 57
   ccggatcctc agtgctttag tgcgtgcat 29
<210> 58
   <211> 29
   <212> DNA
   <213> Artificial Sequence
<220>
   <223> Synthetic Sequence
<400> 58
   ccggatcctc attggatgat cttcttgac 29
<210> 59
   <211> 29
   <212> DNA
   <213> Artificial sequence
<220>
   <223> Synthetic Sequence
<400> 59
   ccggatcctc atattccaca tttttgagt 29
<210> 60
   <211> 29
   <212> DNA
   <213> Artificial Sequence
<220>
   <223> Synthetic sequence
<400> 60
   ccggatcctc atttgcaaac tgtatttcg 29
<210> 61
   <211> 29
   <212> DNA
   <213> Artificial Sequence
<220>
   <223> Synthetic sequence
<400> 61
   ccggatcctc attcgcacac gcggttgtg 29
<210> 62
   <211> 401
   <212> PRT
   <213> Homo sapiens
<400> 62
   Met Asn Asn Leu Leu Cys Cys Ala Leu Val Phe Leu Asp Ile Ser Ile
   1 5 10 15
   Lys Trp Thr Thr Gln Glu Thr Phe Pro Pro Lys Tyr Leu His Tyr Asp
   20 25 30
   Glu Glu Thr Ser His Gln Leu Leu Cys Asp Lys Cys Pro Pro Gly Thr
   35 40 45
   Tyr Leu Lys Gln His Cys Thr Ala Lys Trp Lys Thr Val Cys Ala Pro
   50 55 60
   Cys Pro Asp His Tyr Tyr Thr Asp Ser Trp His Thr Ser Asp Glu Cys
   65 70 75 80
   Leu Tyr Cys Ser Pro Val Cys Lys Glu Leu Gln Tyr Val Lys Gln Glu
   85 90 95
   Cys Asn Arg Thr His Asn Arg Val Cys Glu Cys Lys Glu Gly Arg Tyr
   100 105 110
   Leu Glu Ile Glu Phe Cys Leu Lys His Arg Ser Cys Pro Pro Gly Phe
   115 120 125
   Gly Val Val Gln Ala Gly Thr Pro Glu Arg Asn Thr Val Cys Lys Arg
   130 135 140
   Cys Pro Asp Gly Phe Phe Ser Asn Glu Thr Ser Ser Lys Ala Pro Cys
   145 150 155 160
   Arg Lys His Thr Asn Cys Ser Val Phe Gly Leu Leu Leu Thr Gln Lys
   165 170 175
   Gly Asn Ala Thr His Asp Asn Ile Cys Ser Gly Asn Ser Glu Ser Thr
   180 185 190
   Gln Lys Ser Gly Ile Asp Val Thr Leu Cys Glu Glu Ala Phe Phe Arg
   195 200 205
   Phe Ala Val Pro Thr Lys Phe Thr Pro Asn Trp Leu Ser Val Leu Val
   210 215 220
   Asp Asn Leu Pro Gly Thr Lys Val Asn Ala Glu Ser Val Glu Arg Ile
   225 230 235 240
   Lys Arg Gln His Ser Ser Gln Glu Gln Thr Phe Gln Leu Leu Lys Leu
   245 250 255
   Trp Lys His Gln Asn Lys Asp Gln Asp Ile Val Lys Lys Ile Ile Gln
   260 265 270
   Asp Ile Asp Leu Cys Glu Asn Ser Val Gln Arg His Ile Gly His Ala
   275 280 285
   Asn Leu Thr Phe Glu Gln Leu Arg Ser Leu Met Glu Ser Leu Pro Gly
   290 295 300
   Lys Lys Val Gly Ala Glu Asp Ile Glu Lys Thr Ile Lys Ala Cys Lys
   305 310 315 320
   Pro Ser Asp Gln Ile Leu Lys Leu Leu Ser Leu Trp Arg Ile Lys Asn
   325 330 335
   Gly Asp Gln Asp Thr Leu Lys Gly Leu Met His Ala Leu Lys His Ser
   340 345 350
   Lys Thr Tyr His Phe Pro Lys Thr Val Thr Gln Ser Leu Lys Lys Thr
   355 360 365
   Ile Arg Phe Leu His Ser Phe Thr Met Tyr Lys Leu Tyr Gln Lys Leu
   370 375 380
   Phe Leu Glu Met Ile Gly Asn Gln Val Gln Ser Val Lys Ile Ser Cys
   385 390 395 400
   Leu
<210> 63
   <211> 401
   <212> PRT
   <213> Homo sapiens
<400> 63
   Met Asn Asn Leu Leu Cys Cys Ala Leu Val Phe Leu Asp Ile Ser Ile
   1               5                   10                  15
   Lys Trp Thr Thr Gln Glu Thr Phe Pro Pro Lys Tyr Leu His Tyr Asp
               20                  25                  30
   Glu Glu Thr Ser His Gln Leu Leu Cys Asp Lys Cys Pro Pro Gly Thr
           35                  40                  45
   Tyr Leu Lys Gln His Cys Thr Ala Lys Trp Lys Thr Val Cys Ala Pro
       50                  55                  60
   Cys Pro Asp His Tyr Tyr Thr Asp Ser Trp His Thr Ser Asp Glu Cys
   65                  70                  75                  80
   Leu Tyr Cys Ser Pro Val Cys Lys Glu Leu Gln Tyr Val Lys Gln Glu
                   85                  90                  95
   Cys Asn Arg Thr His Asn Arg Val Cys Glu Cys Lys Glu Gly Arg Tyr
   100 105 110
   Leu Glu Ile Glu Phe Cys Leu Lys His Arg Ser Cys Pro Pro Gly Phe
   115 120 125
   Gly Val Val Gln Ala Gly Thr Pro Glu Arg Asn Thr Val Cys Lys Arg
   130 135 140
   Cys Pro Asp Gly Phe Phe Ser Asn Glu Thr Ser Ser Lys Ala Pro Cys
   145 150 155 160
   Arg Lys His Thr Asn Cys Ser Val Phe Gly Leu Leu Leu Thr Gln Lys
   165 170 175
   Gly Asn Ala Thr His Asp Asn Ile Cys Ser Gly Asn Ser Glu Ser Thr
   180 185 190
   Gln Lys Cys Gly Ile Asp Val Thr Leu Ser Glu Glu Ala Phe Phe Arg
   195 200 205
   Phe Ala Val Pro Thr Lys Phe Thr Pro Asn Trp Leu Ser Val Leu Val
   210 215 220
   Asp Asn Leu Pro Gly Thr Lys Val Asn Ala Glu Ser Val Glu Arg Ile
   225 230 235 240
   Lys Arg Gln His Ser Ser Gln Glu Gln Thr Phe Gln Leu Leu Lys Leu
   245 250 255
   Trp Lys His Gln Asn Lys Asp Gln Asp Ile Val Lys Lys Ile Ile Gln
   260 265 270
   Asp Ile Asp Leu Cys Glu Asn Ser Val Gln Arg His Ile Gly His Ala
   275 280 285
   Asn Leu Thr Phe Glu Gln Leu Arg Ser Leu Met Glu Ser Leu Pro Gly
   290 295 300
   Lys Lys Val Gly Ala Glu Asp Ile Glu Lys Thr Ile Lys Ala Cys Lys
   305 310 315 320
   Pro Ser Asp Gln Ile Leu Lys Leu Leu Ser Leu Trp Arg Ile Lys Asn
   325 330 335
   Gly Asp Gln Asp Thr Leu Lys Gly Leu Met His Ala Leu Lys His Ser
   340 345 350
   Lys Thr Tyr His Phe Pro Lys Thr Val Thr Gln Ser Leu Lys Lys Thr
   355 360 365
   Ile Arg Phe Leu His Ser Phe Thr Met Tyr Lys Leu Tyr Gln Lys Leu
   370 375 380
   Phe Leu Glu Met Ile Gly Asn Gln Val Gln Ser Val Lys Ile Ser Cys
   385 390 395 400
   Leu
<210> 64
   <211> 401
   <212> PRT
   <213> Homo sapiens
<400> 64
   Met Asn Asn Leu Leu Cys Cys Ala Leu Val Phe Leu Asp Ile Ser Ile
   1 5 10 15
   Lys Trp Thr Thr Gln Glu Thr Phe Pro Pro Lys Tyr Leu His Tyr Asp
   20 25 30
   Glu Glu Thr Ser His Gln Leu Leu Cys Asp Lys Cys Pro Pro Gly Thr
   35 40 45
   Tyr Leu Lys Gln His Cys Thr Ala Lys Trp Lys Thr Val Cys Ala Pro
   50 55 60
   Cys Pro Asp His Tyr Tyr Thr Asp Ser Trp His Thr Ser Asp Glu Cys
   65 70 75 80
   Leu Tyr Cys Ser Pro Val Cys Lys Glu Leu Gln Tyr Val Lys Gln Glu
   85 90 95
   Cys Asn Arg Thr His Asn Arg Val Cys Glu Cys Lys Glu Gly Arg Tyr
   100 105 110
   Leu Glu Ile Glu Phe Cys Leu Lys His Arg Ser Cys Pro Pro Gly Phe
   115 120 125
   Gly Val Val Gln Ala Gly Thr Pro Glu Arg Asn Thr Val Cys Lys Arg
   130 135 140
   Cys Pro Asp Gly Phe Phe Ser Asn Glu Thr Ser Ser Lys Ala Pro Cys
   145 150 155 160
   Arg Lys His Thr Asn Cys Ser Val Phe Gly Leu Leu Leu Thr Gln Lys
   165 170 175
   Gly Asn Ala Thr His Asp Asn Ile Cys Ser Gly Asn Ser Glu Ser Thr
   180 185 190
   Gln Lys Cys Gly Ile Asp Val Thr Leu Cys Glu Glu Ala Phe Phe Arg
   195 200 205
   Phe Ala Val Pro Thr Lys Phe Thr Pro Asn Trp Leu Ser Val Leu Val
   210 215 220
   Asp Asn Leu Pro Gly Thr Lys Val Asn Ala Glu Ser Val Glu Arg Ile
   225 230 235 240
   Lys Arg Gln His Ser Ser Gln Glu Gln Thr Phe Gln Leu Leu Lys Leu
   245 250 255
   Trp Lys His Gln Asn Lys Asp Gln Asp Ile Val Lys Lys Ile Ile Gln
   260 265 270
   Asp Ile Asp Leu Ser Glu Asn Ser Val Gln Arg His Ile Gly His Ala
   275 280 285
   Asn Leu Thr Phe Glu Gln Leu Arg Ser Leu Met Glu Ser Leu Pro Gly
   290 295 300
   Lys Lys Val Gly Ala Glu Asp Ile Glu Lys Thr Ile Lys Ala Cys Lys
   305 310 315 320
   Pro Ser Asp Gln Ile Leu Lys Leu Leu Ser Leu Trp Arg Ile Lys Asn
   325 330 335
   Gly Asp Gln Asp Thr Leu Lys Gly Leu Met His Ala Leu Lys His Ser
   340 345 350
   Lys Thr Tyr His Phe Pro Lys Thr Val Thr Gln Ser Leu Lys Lys Thr
   355 360 365
   Ile Arg Phe Leu His Ser Phe Thr Met Tyr Lys Leu Tyr Gln Lys Leu
   370 375 380
   Phe Leu Glu Met Ile Gly Asn Gln Val Gln Ser Val Lys Ile Ser Cys
   385 390 395 400
   Leu
<210> 65
   <211> 401
   <212> PRT
   <213> Homo sapiens
<400> 65
   Met Asn Asn Leu Leu Cys Cys Ala Leu Val Phe Leu Asp Ile Ser Ile
   1               5                   10                  15
   Lys Trp Thr Thr Gln Glu Thr Phe Pro Pro Lys Tyr Leu His Tyr Asp
   20 25 30
   Glu Glu Thr Ser His Gln Leu Leu Cys Asp Lys Cys Pro Pro Gly Thr
   35 40 45
   Tyr Leu Lys Gln His Cys Thr Ala Lys Trp Lys Thr Val Cys Ala Pro
   50 55 60
   Cys Pro Asp His Tyr Tyr Thr Asp Ser Trp His Thr Ser Asp Glu Cys
   65 70 75 80
   Leu Tyr Cys Ser Pro Val Cys Lys Glu Leu Gln Tyr Val Lys Gln Glu
   85 90 95
   Cys Asn Arg Thr His Asn Arg Val Cys Glu Cys Lys Glu Gly Arg Tyr
   100 105 110
   Leu Glu Ile Glu Phe Cys Leu Lys His Arg Ser Cys Pro Pro Gly Phe
   115 120 125
   Gly Val Val Gln Ala Gly Thr Pro Glu Arg Asn Thr Val Cys Lys Arg
   130 135 140
   Cys Pro Asp Gly Phe Phe Ser Asn Glu Thr Ser Ser Lys Ala Pro Cys
   145 150 155 160
   Arg Lys His Thr Asn Cys Ser Val Phe Gly Leu Leu Leu Thr Gln Lys
   165 170 175
   Gly Asn Ala Thr His Asp Asn Ile Cys Ser Gly Asn Ser Glu Ser Thr
   180 185 190
   Gln Lys Cys Gly Ile Asp Val Thr Leu Cys Glu Glu Ala Phe Phe Arg
   195 200 205
   Phe Ala Val Pro Thr Lys Phe Thr Pro Asn Trp Leu Ser Val Leu Val
   210 215 220
   Asp Asn Leu Pro Gly Thr Lys Val Asn Ala Glu Ser Val Glu Arg Ile
   225 230 235 240
   Lys Arg Gln His Ser Ser Gln Glu Gln Thr Phe Gln Leu Leu Lys Leu
   245 250 255
   Trp Lys His Gln Asn Lys Asp Gln Asp Ile Val Lys Lys Ile Ile Gln
   260 265 270
   Asp Ile Asp Leu Cys Glu Asn Ser Val Gln Arg His Ile Gly His Ala
   275 280 285
   Asn Leu Thr Phe Glu Gln Leu Arg Ser Leu Met Glu Ser Leu Pro Gly
   290 295 300
   Lys Lys Val Gly Ala Glu Asp Ile Glu Lys Thr Ile Lys Ala Ser Lys
   305 310 315 320
   Pro Ser Asp Gln Ile Leu Lys Leu Leu Ser Leu Trp Arg Ile Lys Asn
   325 330 335
   Gly Asp Gln Asp Thr Leu Lys Gly Leu Met His Ala Leu Lys His Ser
   340 345 350
   Lys Thr Tyr His Phe Pro Lys Thr Val Thr Gln Ser Leu Lys Lys Thr
   355 360 365
   Ile Arg Phe Leu His Ser Phe Thr Met Tyr Lys Leu Tyr Gln Lys Leu
   370 375 380
   Phe Leu Glu Met Ile Gly Asn Gln Val Gln Ser Val Lys Ile Ser Cys
   385 390 395 400
   Leu
<210> 66
   <211> 401
   <212> PRT
   <213> Homo sapiens
<400> 66
   Met Asn Asn Leu Leu Cys Cys Ala Leu Val Phe Leu Asp Ile Ser Ile
   1               5                   10                  15
   Lys Trp Thr Thr Gln Glu Thr Phe Pro Pro Lys Tyr Leu His Tyr Asp
               20                  25                  30
   Glu Glu Thr Ser His Gln Leu Leu Cys Asp Lys Cys Pro Pro Gly Thr
           35                  40                  45
   Tyr Leu Lys Gln His Cys Thr Ala Lys Trp Lys Thr Val Cys Ala Pro
       50                  55                  60
   Cys Pro Asp His Tyr Tyr Thr Asp Ser Trp His Thr Ser Asp Glu Cys
   65                  70                  75                  80
   Leu Tyr Cys Ser Pro Val Cys Lys Glu Leu Gln Tyr Val Lys Gln Glu
                   85                  90                  95
   Cys Asn Arg Thr His Asn Arg Val Cys Glu Cys Lys Glu Gly Arg Tyr
               100                 105                 110
   Leu Glu Ile Glu Phe Cys Leu Lys His Arg Ser Cys Pro Pro Gly Phe
           115                 120                 125
   Gly Val Val Gln Ala Gly Thr Pro Glu Arg Asn Thr Val Cys Lys Arg
       130                 135                 140
   Cys Pro Asp Gly Phe Phe Ser Asn Glu Thr Ser Ser Lys Ala Pro Cys
   145                 150                 155                 160
   Arg Lys His Thr Asn Cys Ser Val Phe Gly Leu Leu Leu Thr Gln Lys
                   165                 170                 175
   Gly Asn Ala Thr His Asp Asn Ile Cys Ser Gly Asn Ser Glu Ser Thr
               180                 185                 190
   Gln Lys Cys Gly Ile Asp Val Thr Leu Cys Glu Glu Ala Phe Phe Arg
           195                 200                 205
   Phe Ala Val Pro Thr Lys Phe Thr Pro Asn Trp Leu Ser Val Leu Val
       210                 215                 220
   Asp Asn Leu Pro Gly Thr Lys Val Asn Ala Glu Ser Val Glu Arg Ile
   225                 230                 235                 240
   Lys Arg Gln His Ser Ser Gln Glu Gln Thr Phe Gln Leu Leu Lys Leu
                   245                 250                 255
   Trp Lys His Gln Asn Lys Asp Gln Asp Ile Val Lys Lys Ile Ile Gln
               260                 265                 270
   Asp Ile Asp Leu Cys Glu Asn Ser Val Gln Arg His Ile Gly His Ala
           275                 280                 285
   Asn Leu Thr Phe Glu Gln Leu Arg Ser Leu Met Glu Ser Leu Pro Gly
       290                 295                 300
   Lys Lys Val Gly Ala Glu Asp Ile Glu Lys Thr Ile Lys Ala Cys Lys
   305                 310                 315                 320
   Pro Ser Asp Gln Ile Leu Lys Leu Leu Ser Leu Trp Arg Ile Lys Asn
                   325                 330                 335
   Gly Asp Gln Asp Thr Leu Lys Gly Leu Met His Ala Leu Lys His Ser
               340                 345                 350
   Lys Thr Tyr His Phe Pro Lys Thr Val Thr Gln Ser Leu Lys Lys Thr
           355                 360                 365
   Ile Arg Phe Leu His Ser Phe Thr Met Tyr Lys Leu Tyr Gln Lys Leu
       370                 375                 380
   Phe Leu Glu Met Ile Gly Asn Gln Val Gln Ser Val Lys Ile Ser Ser
   385                 390                 395                 400
   Leu
<210> 67
   <211> 360
   <212> PRT
   <213> Homo sapiens
<400> 67
   Met Asn Asn Leu Leu Cys Cys Ala Leu Val Phe Leu Asp Ile Ser Ile
   1               5                   10                  15
   Lys Trp Thr Thr Gln Glu Pro Cys Pro Asp His Tyr Tyr Thr Asp Ser
               20                  25                  30
   Trp His Thr Ser Asp Glu Cys Leu Tyr Cys Ser Pro Val Cys Lys Glu
           35                  40                  45
   Leu Gln Tyr Val Lys Gln Glu Cys Asn Arg Thr His Asn Arg Val Cys
       50                  55                  60
   Glu Cys Lys Glu Gly Arg Tyr Leu Glu Ile Glu Phe Cys Leu Lys His
   65                  70                  75                  80
   Arg Ser Cys Pro Pro Gly Phe Gly Val Val Gln Ala Gly Thr Pro Glu
                   85                  90                  95
   Arg Asn Thr Val Cys Lys Arg Cys Pro Asp Gly Phe Phe Ser Asn Glu
               100                 105                 110
   Thr Ser Ser Lys Ala Pro Cys Arg Lys His Thr Asn Cys Ser Val Phe
           115                 120                 125
   Gly Leu Leu Leu Thr Gln Lys Gly Asn Ala Thr His Asp Asn Ile Cys
       130                 135                 140
   Ser Gly Asn Ser Glu Ser Thr Gln Lys Cys Gly Ile Asp Val Thr Leu
   145                 150                 155                 160
   Cys Glu Glu Ala Phe Phe Arg Phe Ala Val Pro Thr Lys Phe Thr Pro
                   165                 170                 175
   Asn Trp Leu Ser Val Leu Val Asp Asn Leu Pro Gly Thr Lys Val Asn
               180                 185                 190
   Ala Glu Ser Val Glu Arg Ile Lys Arg Gln His Ser Ser Gln Glu Gln
           195                 200                 205
   Thr Phe Gln Leu Leu Lys Leu Trp Lys His Gln Asn Lys Asp Gln Asp
       210                 215                 220
   Ile Val Lys Lys Ile Ile Gln Asp Ile Asp Leu Cys Glu Asn Ser Val
   225 230 235 240
   Gln Arg His Ile Gly His Ala Asn Leu Thr Phe Glu Gln Leu Arg Ser
   245 250 255
   Leu Met Glu Ser Leu Pro Gly Lys Lys Val Gly Ala Glu Asp Ile Glu
   260 265 270
   Lys Thr Ile Lys Ala Cys Lys Pro Ser Asp Gln Ile Leu Lys Leu Leu
   275 280 285
   Ser Leu Trp Arg Ile Lys Asn Gly Asp Gln Asp Thr Leu Lys Gly Leu
   290 295 300
   Met His Ala Leu Lys His Ser Lys Thr Tyr His Phe Pro Lys Thr Val
   305 310 315 320
   Thr Gln Ser Leu Lys Lys Thr Ile Arg Phe Leu His Ser Phe Thr Met
   325 330 335
   Tyr Lys Leu Tyr Gln Lys Leu Phe Leu Glu Met Ile Gly Asn Gln Val
   340 345 350
   Gln Ser Val Lys Ile Ser Cys Leu
   355 360
<210> 68
   <211> 359
   <212> PRT
   <213> Homo sapiens
<400> 68
   Met Asn Asn Leu Leu Cys Cys Ala Leu Val Phe Leu Asp Ile Ser Ile
   1               5                   10                  15
   Lys Trp Thr Thr Gln Glu Thr Phe Pro Pro Lys Tyr Leu His Tyr Asp
               20                  25                  30
   Glu Glu Thr Ser His Gln Leu Leu Cys Asp Lys Cys Pro Pro Gly Thr
           35                  40                  45
   Tyr Leu Lys Gln His Cys Thr Ala Lys Trp Lys Thr Val Cys Ala Glu
       50                  55                  60
   Cys Lys Glu Gly Arg Tyr Leu Glu Ile Glu Phe Cys Leu Lys His Arg
   65                  70                  75                  80
   Ser Cys Pro Pro Gly Phe Gly Val Val Gln Ala Gly Thr Pro Glu Arg
                   85                  90                  95
   Asn Thr Val Cys Lys Arg Cys Pro Asp Gly Phe Phe Ser Asn Glu Thr
   100 105 110
   Ser Ser Lys Ala Pro Cys Arg Lys His Thr Asn Cys Ser Val Phe Gly
   115 120 125
   Leu Leu Leu Thr Gln Lys Gly Asn Ala Thr His Asp Asn Ile Cys Ser
   130 135 140
   Gly Asn Ser Glu Ser Thr Gln Lys Cys Gly Ile Asp Val Thr Leu Cys
   145 150 155 160
   Glu Glu Ala Phe Phe Arg Phe Ala Val Pro Thr Lys Phe Thr Pro Asn
   165 170 175
   Trp Leu Ser Val Leu Val Asp Asn Leu Pro Gly Thr Lys Val Asn Ala
   180 185 190
   Glu Ser Val Glu Arg Ile Lys Arg Gln His Ser Ser Gln Glu Gln Thr
   195 200 205
   Phe Gln Leu Leu Lys Leu Trp Lys His Gln Asn Lys Asp Gln Asp Ile
   210 215 220
   Val Lys Lys Ile Ile Gln Asp Ile Asp Leu Cys Glu Asn Ser Val Gln
   225 230 235 240
   Arg His Ile Gly His Ala Asn Leu Thr Phe Glu Gln Leu Arg Ser Leu
   245 250 255
   Met Glu Ser Leu Pro Gly Lys Lys Val Gly Ala Glu Asp Ile Glu Lys
   260 265 270
   Thr Ile Lys Ala Cys Lys Pro Ser Asp Gln Ile Leu Lys Leu Leu Ser
   275 280 285
   Leu Trp Arg Ile Lys Asn Gly Asp Gln Asp Thr Leu Lys Gly Leu Met
   290 295 300
   His Ala Leu Lys His Ser Lys Thr Tyr His Phe Pro Lys Thr Val Thr
   305 310 315 320
   Gln Ser Leu Lys Lys Thr Ile Arg Phe Leu His Ser Phe Thr Met Tyr
   325 330 335
   Lys Leu Tyr Gln Lys Leu Phe Leu Glu Met Ile Gly Asn Gln Val Gln
   340 345 350
   Ser Val Lys Ile Ser Cys Leu
   355
<210> 69
   <211> 363
   <212> PRT
   <213> Homo sapiens
<400> 69
   Met Asn Asn Leu Leu Cys Cys Ala Leu Val Phe Leu Asp Ile Ser Ile
   1               5                   10                  15
   Lys Trp Thr Thr Gln Glu Thr Phe Pro Pro Lys Tyr Leu His Tyr Asp
               20                  25                  30
   Glu Glu Thr Ser His Gln Leu Leu Cys Asp Lys Cys Pro Pro Gly Thr
           35                  40                  45
   Tyr Leu Lys Gln His Cys Thr Ala Lys Trp Lys Thr Val Cys Ala Pro
       50                  55                  60
   Cys Pro Asp His Tyr Tyr Thr Asp Ser Trp His Thr Ser Asp Glu Cys
   65                  70                  75                  80
   Leu Tyr Cys Ser Pro Val Cys Lys Glu Leu Gln Tyr Val Lys Gln Glu
                   85                  90                  95
   Cys Asn Arg Thr His Asn Arg Val Cys Arg Cys Pro Asp Gly Phe Phe
               100                 105                 110
   Ser Asn Glu Thr Ser Ser Lys Ala Pro Cys Arg Lys His Thr Asn Cys
           115                 120                 125
   Ser Val Phe Gly Leu Leu Leu Thr Gln Lys Gly Asn Ala Thr His Asp
       130                 135                 140
   Asn Ile Cys Ser Gly Asn Ser Glu Ser Thr Gln Lys Cys Gly Ile Asp
   145                 150                 155                 160
   Val Thr Leu Cys Glu Glu Ala Phe Phe Arg Phe Ala Val Pro Thr Lys
                   165                 170                 175
   Phe Thr Pro Asn Trp Leu Ser Val Leu Val Asp Asn Leu Pro Gly Thr
               180                 185                 190
   Lys Val Asn Ala Glu Ser Val Glu Arg Ile Lys Arg Gln His Ser Ser
           195                 200                 205
   Gln Glu Gln Thr Phe Gln Leu Leu Lys Leu Trp Lys His Gln Asn Lys
       210                 215                 220
   Asp Gln Asp Ile Val Lys Lys Ile Ile Gln Asp Ile Asp Leu Cys Glu
   225                 230                 235                 240
   Asn Ser Val Gln Arg His Ile Gly His Ala Asn Leu Thr Phe Glu Gln
   245 250 255
   Leu Arg Ser Leu Met Glu Ser Leu Pro Gly Lys Lys Val Gly Ala Glu
   260 265 270
   Asp Ile Glu Lys Thr Ile Lys Ala Cys Lys Pro Ser Asp Gln Ile Leu
   275 280 285
   Lys Leu Leu Ser Leu Trp Arg Ile Lys Asn Gly Asp Gln Asp Thr Leu
   290 295 300
   Lys Gly Leu Met His Ala Leu Lys His Ser Lys Thr Tyr His Phe Pro
   305 310 315 320
   Lys Thr Val Thr Gln Ser Leu Lys Lys Thr Ile Arg Phe Leu His Ser
   325 330 335
   Phe Thr Met Tyr Lys Leu Tyr Gln Lys Leu Phe Leu Glu Met Ile Gly
   340 345 350
   Asn Gln Val Gln Ser Val Lys Ile Ser Cys Leu
   355 360
<210> 70
   <211> 359
   <212> PRT
   <213> Homo sapiens
<400> 70
   Met Asn Asn Leu Leu Cys Cys Ala Leu Val Phe Leu Asp Ile Ser Ile
   1 5 10 15
   Lys Trp Thr Thr Gln Glu Thr Phe Pro Pro Lys Tyr Leu His Tyr Asp
   20 25 30
   Glu Glu Thr Ser His Gln Leu Leu Cys Asp Lys Cys Pro Pro Gly Thr
   35 40 45
   Tyr Leu Lys Gln His Cys Thr Ala Lys Trp Lys Thr Val Cys Ala Pro
   50 55 60
   Cys Pro Asp His Tyr Tyr Thr Asp Ser Trp His Thr Ser Asp Glu Cys
   65 70 75 80
   Leu Tyr Cys Ser Pro Val Cys Lys Glu Leu Gln Tyr Val Lys Gln Glu
   85 90 95
   Cys Asn Arg Thr His Asn Arg Val Cys Glu Cys Lys Glu Gly Arg Tyr
   100 105 110
   Leu Glu Ile Glu Phe Cys Leu Lys His Arg Ser Cys Pro Pro Gly Phe
   115 120 125
   Gly Val Val Gln Ala Gly Thr Pro Glu Arg Asn Thr Val Cys Lys Ser
   130 135 140
   Gly Asn Ser Glu Ser Thr Gln Lys Cys Gly Ile Asp Val Thr Leu Cys
   145 150 155 160
   Glu Glu Ala Phe Phe Arg Phe Ala Val Pro Thr Lys Phe Thr Pro Asn
   165 170 175
   Trp Leu Ser Val Leu Val Asp Asn Leu Pro Gly Thr Lys Val Asn Ala
   180 185 190
   Glu Ser Val Glu Arg Ile Lys Arg Gln His Ser Ser Gln Glu Gln Thr
   195 200 205
   Phe Gln Leu Leu Lys Leu Trp Lys His Gln Asn Lys Asp Gln Asp Ile
   210 215 220
   Val Lys Lys Ile Ile Gln Asp Ile Asp Leu Cys Glu Asn Ser Val Gln
   225 230 235 240
   Arg His Ile Gly His Ala Asn Leu Thr Phe Glu Gln Leu Arg Ser Leu
   245 250 255
   Met Glu Ser Leu Pro Gly Lys Lys Val Gly Ala Glu Asp Ile Glu Lys
   260 265 270
   Thr Ile Lys Ala Cys Lys Pro Ser Asp Gln Ile Leu Lys Leu Leu Ser
   275 280 285
   Leu Trp Arg Ile Lys Asn Gly Asp Gln Asp Thr Leu Lys Gly Leu Met
   290 295 300
   His Ala Leu Lys His Ser Lys Thr Tyr His Phe Pro Lys Thr Val Thr
   305 310 315 320
   Gln Ser Leu Lys Lys Thr Ile Arg Phe Leu His Ser Phe Thr Met Tyr
   325 330 335
   Lys Leu Tyr Gln Lys Leu Phe Leu Glu Met Ile Gly Asn Gln Val Gln
   340 345 350
   Ser Val Lys Ile Ser Cys Leu
   355
<210> 71
   <211> 326
   <212> PRT
   <213> Homo sapiens
<400> 71
   Met Asn Asn Leu Leu Cys Cys Ala Leu Val Phe Leu Asp Ile Ser Ile
   1               5                   10                  15
   Lys Trp Thr Thr Gln Glu Thr Phe Pro Pro Lys Tyr Leu His Tyr Asp
               20                  25                  30
   Glu Glu Thr Ser His Gln Leu Leu Cys Asp Lys Cys Pro Pro Gly Thr
           35                  40                  45
   Tyr Leu Lys Gln His Cys Thr Ala Lys Trp Lys Thr Val Cys Ala Pro
       50                  55                  60
   Cys Pro Asp His Tyr Tyr Thr Asp Ser Trp His Thr Ser Asp Glu Cys
   65                  70                  75                  80
   Leu Tyr Cys Ser Pro Val Cys Lys Glu Leu Gln Tyr Val Lys Gln Glu
                   85                  90                  95
   Cys Asn Arg Thr His Asn Arg Val Cys Glu Cys Lys Glu Gly Arg Tyr
               100                 105                 110
   Leu Glu Ile Glu Phe Cys Leu Lys His Arg Ser Cys Pro Pro Gly Phe
           115                 120                 125
   Gly Val Val Gln Ala Gly Thr Pro Glu Arg Asn Thr Val Cys Lys Arg
       130                 135                 140
   Cys Pro Asp Gly Phe Phe Ser Asn Glu Thr Ser Ser Lys Ala Pro Cys
   145                 150                 155                 160
   Arg Lys His Thr Asn Cys Ser Val Phe Gly Leu Leu Leu Thr Gln Lys
                   165                 170                 175
   Gly Asn Ala Thr His Asp Asn Ile Cys Ser Gly Asn Ser Glu Ser Thr
               180                 185                 190
   Gln Lys Cys Gly Ile Asp Ile Asp Leu Cys Glu Asn Ser Val Gln Arg
           195                 200                 205
   His Ile Gly His Ala Asn Leu Thr Phe Glu Gln Leu Arg Ser Leu Met
       210                 215                 220
   Glu Ser Leu Pro Gly Lys Lys Val Gly Ala Glu Asp Ile Glu Lys Thr
   225                 230                 235                 240
   Ile Lys Ala Cys Lys Pro Ser Asp Gln Ile Leu Lys Leu Leu Ser Leu
                   245                 250                 255
   Trp Arg Ile Lys Asn Gly Asp Gln Asp Thr Leu Lys Gly Leu Met His
               260                 265                 270
   Ala Leu Lys His Ser Lys Thr Tyr His Phe Pro Lys Thr Val Thr Gln
   275 280 285
   Ser Leu Lys Lys Thr Ile Arg Phe Leu His Ser Phe Thr Met Tyr Lys
   290 295 300
   Leu Tyr Gln Lys Leu Phe Leu Glu Met Ile Gly Asn Gln Val Gln Ser
   305 310 315 320
   Val Lys Ile Ser Cys Leu
   325
<210> 72
   <211> 327
   <212> PRT
   <213> Homo sapiens
<400> 72
   Met Asn Asn Leu Leu Cys Cys Ala Leu Val Phe Leu Asp Ile Ser Ile
   1               5                   10                  15
   Lys Trp Thr Thr Gln Glu Thr Phe Pro Pro Lys Tyr Leu His Tyr Asp
               20                  25                  30
   Glu Glu Thr Ser His Gln Leu Leu Cys Asp Lys Cys Pro Pro Gly Thr
           35                  40                  45
   Tyr Leu Lys Gln His Cys Thr Ala Lys Trp Lys Thr Val Cys Ala Pro
       50                  55                  60
   Cys Pro Asp His Tyr Tyr Thr Asp Ser Trp His Thr Ser Asp Glu Cys
   65                  70                  75                  80
   Leu Tyr Cys Ser Pro Val Cys Lys Glu Leu Gln Tyr Val Lys Gln Glu
                   85                  90                  95
   Cys Asn Arg Thr His Asn Arg Val Cys Glu Cys Lys Glu Gly Arg Tyr
               100                 105                 110
   Leu Glu Ile Glu Phe Cys Leu Lys His Arg Ser Cys Pro Pro Gly Phe
           115                 120                 125
   Gly Val Val Gln Ala Gly Thr Pro Glu Arg Asn Thr Val Cys Lys Arg
       130                 135                 140
   Cys Pro Asp Gly Phe Phe Ser Asn Glu Thr Ser Ser Lys Ala Pro Cys
   145                 150                 155                 160
   Arg Lys His Thr Asn Cys Ser Val Phe Gly Leu Leu Leu Thr Gln Lys
                   165                 170                 175
   Gly Asn Ala Thr His Asp Asn Ile Cys Ser Gly Asn Ser Glu Ser Thr
   180 185 190
   Gln Lys Cys Gly Ile Asp Val Thr Leu Cys Glu Glu Ala Phe Phe Arg
   195 200 205
   Phe Ala Val Pro Thr Lys Phe Thr Pro Asn Trp Leu Ser Val Leu Val
   210 215 220
   Asp Asn Leu Pro Gly Thr Lys Val Asn Ala Glu Ser Val Glu Arg Ile
   225 230 235 240
   Lys Arg Gln His Ser Ser Gln Glu Gln Thr Phe Gln Leu Leu Lys Leu
   245 250 255
   Trp Lys His Gln Asn Lys Asp Gln Asp Ile Val Lys Lys Ile Ile Gln
   260 265 270
   Asp Ala Leu Lys His Ser Lys Thr Tyr His Phe Pro Lys Thr Val Thr
   275 280 285
   Gln Ser Leu Lys Lys Thr Ile Arg Phe Leu His Ser Phe Thr Met Tyr
   290 295 300
   Lys Leu Tyr Gln Lys Leu Phe Leu Glu Met Ile Gly Asn Gln Val Gln
   305 310 315 320
   Ser Val Lys Ile Ser Cys Leu
   325
<210> 73
   <211> 399
   <212> PRT
   <213> Homo sapiens
<400> 73
   Met Asn Asn Leu Leu Cys Cys Ala Leu Val Phe Leu Asp Ile Ser Ile
   1               5                   10                  15
   Lys Trp Thr Thr Gln Glu Thr Phe Pro Pro Lys Tyr Leu His Tyr Asp
               20                  25                  30
   Glu Glu Thr Ser His Gln Leu Leu Cys Asp Lys Cys Pro Pro Gly Thr
           35                  40                  45
   Tyr Leu Lys Gln His Cys Thr Ala Lys Trp Lys Thr Val Cys Ala Pro
       50                  55                  60
   Cys Pro Asp His Tyr Tyr Thr Asp Ser Trp His Thr Ser Asp Glu Cys
   65                  70                  75                  80
   Leu Tyr Cys Ser Pro Val Cys Lys Glu Leu Gln Tyr Val Lys Gln Glu
   85 90 95
   Cys Asn Arg Thr His Asn Arg Val Cys Glu Cys Lys Glu Gly Arg Tyr
   100 105 110
   Leu Glu Ile Glu Phe Cys Leu Lys His Arg Ser Cys Pro Pro Gly Phe
   115 120 125
   Gly Val Val Gln Ala Gly Thr Pro Glu Arg Asn Thr Val Cys Lys Arg
   130 135 140
   Cys Pro Asp Gly Phe Phe Ser Asn Glu Thr Ser Ser Lys Ala Pro Cys
   145 150 155 160
   Arg Lys His Thr Asn Cys Ser Val Phe Gly Leu Leu Leu Thr Gln Lys
   165 170 175
   Gly Asn Ala Thr His Asp Asn Ile Cys Ser Gly Asn Ser Glu Ser Thr
   180 185 190
   Gln Lys Cys Gly Ile Asp Val Thr Leu Cys Glu Glu Ala Phe Phe Arg
   195 200 205
   Phe Ala Val Pro Thr Lys Phe Thr Pro Asn Trp Leu Ser Val Leu Val
   210 215 220
   Asp Asn Leu Pro Gly Thr Lys Val Asn Ala Glu Ser Val Glu Arg Ile
   225 230 235 240
   Lys Arg Gln His Ser Ser Gln Glu Gln Thr Phe Gln Leu Leu Lys Leu
   245 250 255
   Trp Lys His Gln Asn Lys Asp Gln Asp Ile Val Lys Lys Ile Ile Gln
   260 265 270
   Asp Ile Asp Leu Cys Glu Asn Ser Val Gln Arg His Ile Gly His Ala
   275 280 285
   Asn Leu Thr Phe Glu Gln Leu Arg Ser Leu Met Glu Ser Leu Pro Gly
   290 295 300
   Lys Lys Val Gly Ala Glu Asp Ile Glu Lys Thr Ile Lys Ala Cys Lys
   305 310 315 320
   Pro Ser Asp Gln Ile Leu Lys Leu Leu Ser Leu Trp Arg Ile Lys Asn
   325 330 335
   Gly Asp Gln Asp Thr Leu Lys Gly Leu Met His Ala Leu Lys His Ser
   340 345 350
   Lys Thr Tyr His Phe Pro Lys Thr Val Thr Gln Ser Leu Lys Lys Thr
   355 360 365
   Ile Arg Phe Leu His Ser Phe Thr Met Tyr Lys Leu Tyr Gln Lys Leu
   370 375 380
   Phe Leu Glu Met Ile Gly Asn Gln Val Gln Ser Val Lys Ile Ser
   385 390 395
<210> 74
   <211> 351
   <212> PRT
   <213> Homo sapiens
<400> 74
   Met Asn Asn Leu Leu Cys Cys Ala Leu Val Phe Leu Asp Ile Ser Ile
   1               5                   10                  15
   Lys Trp Thr Thr Gln Glu Thr Phe Pro Pro Lys Tyr Leu His Tyr Asp
               20                  25                  30
   Glu Glu Thr Ser His Gln Leu Leu Cys Asp Lys Cys Pro Pro Gly Thr
           35                  40                  45
   Tyr Leu Lys Gln His Cys Thr Ala Lys Trp Lys Thr Val Cys Ala Pro
       50                  55                  60
   Cys Pro Asp His Tyr Tyr Thr Asp Ser Trp His Thr Ser Asp Glu Cys
   65                  70                  75                  80
   Leu Tyr Cys Ser Pro Val Cys Lys Glu Leu Gln Tyr Val Lys Gln Glu
                   85                  90                  95
   Cys Asn Arg Thr His Asn Arg Val Cys Glu Cys Lys Glu Gly Arg Tyr
               100                 105                 110
   Leu Glu Ile Glu Phe Cys Leu Lys His Arg Ser Cys Pro Pro Gly Phe
           115                 120                 125
   Gly Val Val Gln Ala Gly Thr Pro Glu Arg Asn Thr Val Cys Lys Arg
       130                 135                 140
   Cys Pro Asp Gly Phe Phe Ser Asn Glu Thr Ser Ser Lys Ala Pro Cys
   145                 150                 155                 160
   Arg Lys His Thr Asn Cys Ser Val Phe Gly Leu Leu Leu Thr Gln Lys
                   165                 170                 175
   Gly Asn Ala Thr His Asp Asn Ile Cys Ser Gly Asn Ser Glu Ser Thr
               180                 185                 190
   Gln Lys Cys Gly Ile Asp Val Thr Leu Cys Glu Glu Ala Phe Phe Arg
           195                 200                 205
   Phe Ala Val Pro Thr Lys Phe Thr Pro Asn Trp Leu Ser Val Leu Val
   210 215 220
   Asp Asn Leu Pro Gly Thr Lys Val Asn Ala Glu Ser Val Glu Arg Ile
   225 230 235 240
   Lys Arg Gln His Ser Ser Gln Glu Gln Thr Phe Gln Leu Leu Lys Leu
   245 250 255
   Trp Lys His Gln Asn Lys Asp Gln Asp Ile Val Lys Lys Ile Ile Gln
   260 265 270
   Asp Ile Asp Leu Cys Glu Asn Ser Val Gln Arg His Ile Gly His Ala
   275 280 285
   Asn Leu Thr Phe Glu Gln Leu Arg Ser Leu Met Glu Ser Leu Pro Gly
   290 295 300
   Lys Lys Val Gly Ala Glu Asp Ile Glu Lys Thr Ile Lys Ala Cys Lys
   305 310 315 320
   Pro Ser Asp Gln Ile Leu Lys Leu Leu Ser Leu Trp Arg Ile Lys Asn
   325 330 335
   Gly Asp Gln Asp Thr Leu Lys Gly Leu Met His Ala Leu Lys His
   340 345 350
<210> 75
   <211> 272
   <212> PRT
   <213> Homo sapiens
<400> 75
   Met Asn Asn Leu Leu Cys Cys Ala Leu Val Phe Leu Asp Ile Ser Ile
   1               5                   10                  15
   Lys Trp Thr Thr Gln Glu Thr Phe Pro Pro Lys Tyr Leu His Tyr Asp
               20                  25                  30
   Glu Glu Thr Ser His Gln Leu Leu Cys Asp Lys Cys Pro Pro Gly Thr
           35                  40                  45
   Tyr Leu Lys Gln His Cys Thr Ala Lys Trp Lys Thr Val Cys Ala Pro
       50                  55                  60
   Cys Pro Asp His Tyr Tyr Thr Asp Ser Trp His Thr Ser Asp Glu Cys
   65                  70                  75                  80
   Leu Tyr Cys Ser Pro Val Cys Lys Glu Leu Gln Tyr Val Lys Gln Glu
                   85                  90                  95
   Cys Asn Arg Thr His Asn Arg Val Cys Glu Cys Lys Glu Gly Arg Tyr
   100 105 110
   Leu Glu Ile Glu Phe Cys Leu Lys His Arg Ser Cys Pro Pro Gly Phe
   115 120 125
   Gly Val Val Gln Ala Gly Thr Pro Glu Arg Asn Thr Val Cys Lys Arg
   130 135 140
   Cys Pro Asp Gly Phe Phe Ser Asn Glu Thr Ser Ser Lys Ala Pro Cys
   145 150 155 160
   Arg Lys His Thr Asn Cys Ser Val Phe Gly Leu Leu Leu Thr Gln Lys
   165 170 175
   Gly Asn Ala Thr His Asp Asn Ile Cys Ser Gly Asn Ser Glu Ser Thr
   180 185 190
   Gln Lys Cys Gly Ile Asp Val Thr Leu Cys Glu Glu Ala Phe Phe Arg
   195 200 205
   Phe Ala Val Pro Thr Lys Phe Thr Pro Asn Trp Leu Ser Val Leu Val
   210 215 220
   Asp Asn Leu Pro Gly Thr Lys Val Asn Ala Glu Ser Val Glu Arg Ile
   225 230 235 240
   Lys Arg Gln His Ser Ser Gln Glu Gln Thr Phe Gln Leu Leu Lys Leu
   245 250 255
   Trp Lys His Gln Asn Lys Asp Gln Asp Ile Val Lys Lys Ile Ile Gln
   260 265 270
<210> 76
   <211> 197
   <212> PRT
   <213> Homo sapiens
<400> 76
   Met Asn Asn Leu Leu Cys Cys Ala Leu Val Phe Leu Asp Ile Ser Ile
   1               5                   10                  15
   Lys Trp Thr Thr Gln Glu Thr Phe Pro Pro Lys Tyr Leu His Tyr Asp
               20                  25                  30
   Glu Glu Thr Ser His Gln Leu Leu Cys Asp Lys Cys Pro Pro Gly Thr
           35                  40                  45
   Tyr Leu Lys Gln His Cys Thr Ala Lys Trp Lys Thr Val Cys Ala Pro
       50                  55                  60
   Cys Pro Asp His Tyr Tyr Thr Asp Ser Trp His Thr Ser Asp Glu Cys
   65                  70                  75                  80
   Leu Tyr Cys Ser Pro Val Cys Lys Glu Leu Gln Tyr Val Lys Gln Glu
                   85                  90                  95
   Cys Asn Arg Thr His Asn Arg Val Cys Glu Cys Lys Glu Gly Arg Tyr
               100                 105                 110
   Leu Glu Ile Glu Phe Cys Leu Lys His Arg Ser Cys Pro Pro Gly Phe
           115                 120                 125
   Gly Val Val Gln Ala Gly Thr Pro Glu Arg Asn Thr Val Cys Lys Arg
       130                 135                 140
   Cys Pro Asp Gly Phe Phe Ser Asn Glu Thr Ser Ser Lys Ala Pro Cys
   145                 150                 155                 160
   Arg Lys His Thr Asn Cys Ser Val Phe Gly Leu Leu Leu Thr Gln Lys
                   165                 170                 175
   Gly Asn Ala Thr His Asp Asn Ile Cys Ser Gly Asn Ser Glu Ser Thr
               180                 185                 190
   Gln Lys Cys Gly Ile
           195
<210> 77
   <211> 143
   <212> PRT
   <213> Homo sapiens
<400> 77
   Met Asn Asn Leu Leu Cys Cys Ala Leu Val Phe Leu Asp Ile Ser Ile
   1               5                   10                  15
   Lys Trp Thr Thr Gln Glu Thr Phe Pro Pro Lys Tyr Leu His Tyr Asp
               20                  25                  30
   Glu Glu Thr Ser His Gln Leu Leu Cys Asp Lys Cys Pro Pro Gly Thr
           35                  40                  45
   Tyr Leu Lys Gln His Cys Thr Ala Lys Trp Lys Thr Val Cys Ala Pro
       50                  55                  60
   Cys Pro Asp His Tyr Tyr Thr Asp Ser Trp His Thr Ser Asp Glu Cys
   65                  70                  75                  80
   Leu Tyr Cys Ser Pro Val Cys Lys Glu Leu Gln Tyr Val Lys Gln Glu
                   85                  90                  95
   Cys Asn Arg Thr His Asn Arg Val Cys Glu Cys Lys Glu Gly Arg Tyr
               100                 105                 110
   Leu Glu Ile Glu Phe Cys Leu Lys His Arg Ser Cys Pro Pro Gly Phe
           115                 120                 125
   Gly Val Val Gln Ala Gly Thr Pro Glu Arg Asn Thr Val Cys Lys
       130                 135                 140
<210> 78
   <211> 106
   <212> PRT
   <213> Homo sapiens
<400> 78
   Met Asn Asn Leu Leu Cys Cys Ala Leu Val Phe Leu Asp Ile Ser Ile
   1 5 10 15
   Lys Trp Thr Thr Gln Glu Thr Phe Pro Pro Lys Tyr Leu His Tyr Asp
   20 25 30
   Glu Glu Thr Ser His Gln Leu Leu Cys Asp Lys Cys Pro Pro Gly Thr
   35 40 45
   Tyr Leu Lys Gln His Cys Thr Ala Lys Trp Lys Thr Val Cys Ala Pro
   50 55 60
   Cys Pro Asp His Tyr Tyr Thr Asp Ser Trp His Thr Ser Asp Glu Cys
   65 70 75 80
   Leu Tyr Cys Ser Pro Val Cys Lys Glu Leu Gln Tyr Val Lys Gln Glu
   85 90 95
   Cys Asn Arg Thr His Asn Arg Val Cys Glu
   100 105
<210> 79
   <211> 393
   <212> PRT
   <213> Homo sapiens
<400> 79
   Met Asn Asn Leu Leu Cys Cys Ala Leu Val Phe Leu Asp Ile Ser Ile
   1 5 10 15
   Lys Trp Thr Thr Gln Glu Thr Phe Pro Pro Lys Tyr Leu His Tyr Asp
   20 25 30
   Glu Glu Thr Ser His Gln Leu Leu Cys Asp Lys Cys Pro Pro Gly Thr
   35 40 45
   Tyr Leu Lys Gln His Cys Thr Ala Lys Trp Lys Thr Val Cys Ala Pro
   50 55 60
   Cys Pro Asp His Tyr Tyr Thr Asp Ser Trp His Thr Ser Asp Glu Cys
   65                  70                  75                  80
   Leu Tyr Cys Ser Pro Val Cys Lys Glu Leu Gln Tyr Val Lys Gln Glu
                   85                  90                  95
   Cys Asn Arg Thr His Asn Arg Val Cys Glu Cys Lys Glu Gly Arg Tyr
               100                 105                 110
   Leu Glu Ile Glu Phe Cys Leu Lys His Arg Ser Cys Pro Pro Gly Phe
           115                 120                 125
   Gly Val Val Gln Ala Gly Thr Pro Glu Arg Asn Thr Val Cys Lys Arg
       130                 135                 140
   Cys Pro Asp Gly Phe Phe Ser Asn Glu Thr Ser Ser Lys Ala Pro Cys
   145                 150                 155                 160
   Arg Lys His Thr Asn Cys Ser Val Phe Gly Leu Leu Leu Thr Gln Lys
                   165                 170                 175
   Gly Asn Ala Thr His Asp Asn Ile Cys Ser Gly Asn Ser Glu Ser Thr
               180                 185                 190
   Gln Lys Cys Gly Ile Asp Val Thr Leu Cys Glu Glu Ala Phe Phe Arg
           195                 200                 205
   Phe Ala Val Pro Thr Lys Phe Thr Pro Asn Trp Leu Ser Val Leu Val
       210                 215                 220
   Asp Asn Leu Pro Gly Thr Lys Val Asn Ala Glu Ser Val Glu Arg Ile
   225                 230                 235                 240
   Lys Arg Gln His Ser Ser Gln Glu Gln Thr Phe Gln Leu Leu Lys Leu
                   245                 250                 255
   Trp Lys His Gln Asn Lys Asp Gln Asp Ile Val Lys Lys Ile Ile Gln
               260                 265                 270
   Asp Ile Asp Leu Cys Glu Asn Ser Val Gln Arg His Ile Gly His Ala
           275                 280                 285
   Asn Leu Thr Phe Glu Gln Leu Arg Ser Leu Met Glu Ser Leu Pro Gly
       290                 295                 300
   Lys Lys Val Gly Ala Glu Asp Ile Glu Lys Thr Ile Lys Ala Cys Lys
   305                 310                 315                 320
   Pro Ser Asp Gln Ile Leu Lys Leu Leu Ser Leu Trp Arg Ile Lys Asn
                   325                 330                 335
   Gly Asp Gln Asp Thr Leu Lys Gly Leu Met His Ala Leu Lys His Ser
   340 345 350
   Lys Thr Tyr His Phe Pro Lys Thr Val Thr Gln Ser Leu Lys Lys Thr
   355 360 365
   Ile Arg Phe Leu His Ser Phe Thr Met Tyr Lys Leu Tyr Gln Lys Leu
   370 375 380
   Phe Leu Glu Met Ile Gly Asn Leu Val
   385 390
<210> 80
   <211> 321
   <212> PRT
   <213> Homo sapiens
<400> 80
   Met Asn Asn Leu Leu Cys Cys Ala Leu Val Phe Leu Asp Ile Ser Ile
   1 5 10 15
   Lys Trp Thr Thr Gln Glu Thr Phe Pro Pro Lys Tyr Leu His Tyr Asp
   20 25 30
   Glu Glu Thr Ser His Gln Leu Leu Cys Asp Lys Cys Pro Pro Gly Thr
   35 40 45
   Tyr Leu Lys Gln His Cys Thr Ala Lys Trp Lys Thr Val Cys Ala Pro
   50 55 60
   Cys Pro Asp His Tyr Tyr Thr Asp Ser Trp His Thr Ser Asp Glu Cys
   65 70 75 80
   Leu Tyr Cys Ser Pro Val Cys Lys Glu Leu Gln Tyr Val Lys Gln Glu
   85 90 95
   Cys Asn Arg Thr His Asn Arg Val Cys Glu Cys Lys Glu Gly Arg Tyr
   100 105 110
   Leu Glu Ile Glu Phe Cys Leu Lys His Arg Ser Cys Pro Pro Gly Phe
   115 120 125
   Gly Val Val Gln Ala Gly Thr Pro Glu Arg Asn Thr Val Cys Lys Arg
   130 135 140
   Cys Pro Asp Gly Phe Phe Ser Asn Glu Thr Ser Ser Lys Ala Pro Cys
   145 150 155 160
   Arg Lys His Thr Asn Cys Ser Val Phe Gly Leu Leu Leu Thr Gln Lys
   165 170 175
   Gly Asn Ala Thr His Asp Asn Ile Cys Ser Gly Asn Ser Glu Ser Thr
   180 185 190
   Gln Lys Cys Gly Ile Asp Val Thr Leu Cys Glu Glu Ala Phe Phe Arg
   195 200 205
   Phe Ala Val Pro Thr Lys Phe Thr Pro Asn Trp Leu Ser Val Leu Val
   210 215 220
   Asp Asn Leu Pro Gly Thr Lys Val Asn Ala Glu Ser Val Glu Arg Ile
   225 230 235 240
   Lys Arg Gln His Ser Ser Gln Glu Gln Thr Phe Gln Leu Leu Lys Leu
   245 250 255
   Trp Lys His Gln Asn Lys Asp Gln Asp Ile Val Lys Lys Ile Ile Gln
   260 265 270
   Asp Ile Asp Leu Cys Glu Asn Ser Val Gln Arg His Ile Gly His Ala
   275 280 285
   Asn Leu Thr Phe Glu Gln Leu Arg Ser Leu Met Glu Ser Leu Pro Gly
   290 295 300
   Lys Lys Val Gly Ala Glu Asp Ile Glu Lys Thr Ile Lys Ala Ser Leu
   305 310 315 320
   Asp
<210> 81
   <211> 187
   <212> PRT
   <213> Homo sapiens
<400> 81
   Met Asn Asn Leu Leu Cys Cys Ala Leu Val Phe Leu Asp Ile Ser Ile
   1               5                   10                  15
   Lys Trp Thr Thr Gln Glu Thr Phe Pro Pro Lys Tyr Leu His Tyr Asp
               20                  25                  30
   Glu Glu Thr Ser His Gln Leu Leu Cys Asp Lys Cys Pro Pro Gly Thr
           35                  40                  45
   Tyr Leu Lys Gln His Cys Thr Ala Lys Trp Lys Thr Val Cys Ala Pro
       50                  55                  60
   Cys Pro Asp His Tyr Tyr Thr Asp Ser Trp His Thr Ser Asp Glu Cys
   65                  70                  75                  80
   Leu Tyr Cys Ser Pro Val Cys Lys Glu Leu Gln Tyr Val Lys Gln Glu
                   85                  90                  95
   Cys Asn Arg Thr His Asn Arg Val Cys Glu Cys Lys Glu Gly Arg Tyr
               100                 105                 110
   Leu Glu Ile Glu Phe Cys Leu Lys His Arg Ser Cys Pro Pro Gly Phe
           115                 120                 125
   Gly Val Val Gln Ala Gly Thr Pro Glu Arg Asn Thr Val Cys Lys Arg
       130                 135                 140
   Cys Pro Asp Gly Phe Phe Ser Asn Glu Thr Ser Ser Lys Ala Pro Cys
   145                 150                 155                 160
   Arg Lys His Thr Asn Cys Ser Val Phe Gly Leu Leu Leu Thr Gln Lys
                   165                 170                 175
   Gly Asn Ala Thr His Asp Asn Ile Cys Ser Gly
               180                 185
<210> 82
   <211> 84
   <212> PRT
   <213> Homo sapiens
<400> 82
   Met Asn Asn Leu Leu Cys Cys Ala Leu Val Phe Leu Asp Ile Ser Ile
   1               5                   10                  15
   Lys Trp Thr Thr Gln Glu Thr Phe Pro Pro Lys Tyr Leu His Tyr Asp
               20                  25                  30
   Glu Glu Thr Ser His Gln Leu Leu Cys Asp Lys Cys Pro Pro Gly Thr
           35                  40                  45
   Tyr Leu Lys Gln His Cys Thr Ala Lys Trp Lys Thr Val Cys Ala Pro
       50                  55                  60
   Cys Pro Asp His Tyr Tyr Thr Asp Ser Trp His Thr Ser Asp Glu Cys
   65                  70                  75                  80
   Leu Tyr Leu Val
<210> 83
   <211> 1206
   <212> DNA
   <213> Homo sapiens
<400> 83
   atgaacaact tgctgtgctg cgcgctcgtg tttctggaca tctccattaa gtggaccacc     60
   caggaaacgt ttcctccaaa gtaccttcat tatgacgaag aaacctctca tcagctgttg    120
   tgtgacaaat gtcctcctgg tacctaccta aaacaacact gtacagcaaa gtggaagacc     180
   gtgtgcgccc cttgccctga ccactactac acagacagct ggcacaccag tgacgagtgt     240
   ctatactgca gccccgtgtg caaggagctg cagtacgtca agcaggagtg caatcgcacc     300
   cacaaccgcg tgtgcgaatg caaggaaggg cgctaccttg agatagagtt ctgcttgaaa     360
   cataggagct gccctcctgg atttggagtg gtgcaagctg gaaccccaga gcgaaataca     420
   gtttgcaaaa gatgtccaga tgggttcttc tcaaatgaga cgtcatctaa agcaccctgt     480
   agaaaacaca caaattgcag tgtctttggt ctcctgctaa ctcagaaagg aaatgcaaca     540
   cacgacaaca tatgttccgg aaacagtgaa tcaactcaaa aaagtggaat agatgttacc     600
   ctgtgtgagg aggcattctt caggtttgct gttcctacaa agtttacgcc taactggctt     660
   agtgtcttgg tagacaattt gcctggcacc aaagtaaacg cagagagtgt agagaggata     720
   aaacggcaac acagctcaca agaacagact ttccagctgc tgaagttatg gaaacatcaa     780
   aacaaagacc aagatatagt caagaagatc atccaagata ttgacctctg tgaaaacagc     840
   gtgcagcggc acattggaca tgctaacctc accttcgagc agcttcgtag cttgatggaa     900
   agcttaccgg gaaagaaagt gggagcagaa gacattgaaa aaacaataaa ggcatgcaaa     960
   cccagtgacc agatcctgaa gctgctcagt ttgtggcgaa taaaaaatgg cgaccaagac    1020
   accttgaagg gcctaatgca cgcactaaag cactcaaaga cgtaccactt tcccaaaact    1080
   gtcactcaga gtctaaagaa gaccatcagg ttccttcaca gcttcacaat gtacaaattg    1140
   tatcagaagt tatttttaga aatgataggt aaccaggtcc aatcagtaaa aataagctgc    1200
   ttataa                                                               1206
<210> 84
   <211> 1206
   <212> DNA
   <213> Homo sapiens
<400> 84
   atgaacaact tgctgtgctg cgcgctcgtg tttctggaca tctccattaa gtggaccacc    60
   caggaaacgt ttcctccaaa gtaccttcat tatgacgaag aaacctctca tcagctgttg   120
   tgtgacaaat gtcctcctgg tacctaccta aaacaacact gtacagcaaa gtggaagacc   180
   gtgtgcgccc cttgccctga ccactactac acagacagct ggcacaccag tgacgagtgt   240
   ctatactgca gccccgtgtg caaggagctg cagtacgtca agcaggagtg caatcgcacc   300
   cacaaccgcg tgtgcgaatg caaggaaggg cgctaccttg agatagagtt ctgcttgaaa   360
   cataggagct gccctcctgg atttggagtg gtgcaagctg gaaccccaga gcgaaataca   420
   gtttgcaaaa gatgtccaga tgggttcttc tcaaatgaga cgtcatctaa agcaccctgt   480
   agaaaacaca caaattgcag tgtctttggt ctcctgctaa ctcagaaagg aaatgcaaca   540
   cacgacaaca tatgttccgg aaacagtgaa tcaactcaaa aatgtggaat agatgttacc   600
   ctgagtgagg aggcattctt caggtttgct gttcctacaa agtttacgcc taactggctt   660
   agtgtcttgg tagacaattt gcctggcacc aaagtaaacg cagagagtgt agagaggata   720
   aaacggcaac acagctcaca agaacagact ttccagctgc tgaagttatg gaaacatcaa   780
   aacaaagacc aagatatagt caagaagatc atccaagata ttgacctctg tgaaaacagc   840
   gtgcagcggc acattggaca tgctaacctc accttcgagc agcttcgtag cttgatggaa   900
   agcttaccgg gaaagaaagt gggagcagaa gacattgaaa aaacaataaa ggcatgcaaa   960
   cccagtgacc agatcctgaa gctgctcagt ttgtggcgaa taaaaaatgg cgaccaagac  1020
   accttgaagg gcctaatgca cgcactaaag cactcaaaga cgtaccactt tcccaaaact  1080
   gtcactcaga gtctaaagaa gaccatcagg ttccttcaca gcttcacaat gtacaaattg  1140
   tatcagaagt tatttttaga aatgataggt aaccaggtcc aatcagtaaa aataagctgc  1200
   ttataa                                                             1206
<210> 85
   <211> 1206
   <212> DNA
   <213> Homo sapiens
<400> 85
   atgaacaact tgctgtgctg cgcgctcgtg tttctggaca tctccattaa gtggaccacc 60
   caggaaacgt ttcctccaaa gtaccttcat tatgacgaag aaacctctca tcagctgttg 120
   tgtgacaaat gtcctcctgg tacctaccta aaacaacact gtacagcaaa gtggaagacc 180
   gtgtgcgccc cttgccctga ccactactac acagacagct ggcacaccag tgacgagtgt 240
   ctatactgca gccccgtgtg caaggagctg cagtacgtca agcaggagtg caatcgcacc 300
   cacaaccgcg tgtgcgaatg caaggaaggg cgctaccttg agatagagtt ctgcttgaaa 360
   cataggagct gccctcctgg atttggagtg gtgcaagctg gaaccccaga gcgaaataca 420
   gtttgcaaaa gatgtccaga tgggttcttc tcaaatgaga cgtcatctaa agcaccctgt 480
   agaaaacaca caaattgcag tgtctttggt ctcctgctaa ctcagaaagg aaatgcaaca 540
   cacgacaaca tatgttccgg aaacagtgaa tcaactcaaa aatgtggaat agatgttacc 600
   ctgtgtgagg aggcattctt caggtttgct gttcctacaa agtttacgcc taactggctt 660
   agtgtcttgg tagacaattt gcctggcacc aaagtaaacg cagagagtgt agagaggata 720
   aaacggcaac acagctcaca agaacagact ttccagctgc tgaagttatg gaaacatcaa 780
   aacaaagacc aagatatagt caagaagatc atccaagata ttgacctcag tgaaaacagc 840
   gtgcagcggc acattggaca tgctaacctc accttcgagc agcttcgtag cttgatggaa 900
   agcttaccgg gaaagaaagt gggagcagaa gacattgaaa aaacaataaa ggcatgcaaa 960
   cccagtgacc agatcctgaa gctgctcagt ttgtggcgaa taaaaaatgg cgaccaagac 1020
   accttgaagg gcctaatgca cgcactaaag cactcaaaga cgtaccactt tcccaaaact 1080
   gtcactcaga gtctaaagaa gaccatcagg ttccttcaca gcttcacaat gtacaaattg 1140
   tatcagaagt tatttttaga aatgataggt aaccaggtcc aatcagtaaa aataagctgc 1200
   ttataa 1206
<210> 86
   <211> 1206
   <212> DNA
   <213> Homo sapiens
<400> 86
   atgaacaact tgctgtgctg cgcgctcgtg tttctggaca tctccattaa gtggaccacc 60
   caggaaacgt ttcctccaaa gtaccttcat tatgacgaag aaacctctca tcagctgttg 120
   tgtgacaaat gtcctcctgg tacctaccta aaacaacact gtacagcaaa gtggaagacc 180
   gtgtgcgccc cttgccctga ccactactac acagacagct ggcacaccag tgacgagtgt 240
   ctatactgca gccccgtgtg caaggagctg cagtacgtca agcaggagtg caatcgcacc 300
   cacaaccgcg tgtgcgaatg caaggaaggg cgctaccttg agatagagtt ctgcttgaaa 360
   cataggagct gccctcctgg atttggagtg gtgcaagctg gaaccccaga gcgaaataca 420
   gtttgcaaaa gatgtccaga tgggttcttc tcaaatgaga cgtcatctaa agcaccctgt 480
   agaaaacaca caaattgcag tgtctttggt ctcctgctaa ctcagaaagg aaatgcaaca 540
   cacgacaaca tatgttccgg aaacagtgaa tcaactcaaa aatgtggaat agatgttacc 600
   ctgtgtgagg aggcattctt caggtttgct gttcctacaa agtttacgcc taactggctt 660
   agtgtcttgg tagacaattt gcctggcacc aaagtaaacg cagagagtgt agagaggata 720
   aaacggcaac acagctcaca agaacagact ttccagctgc tgaagttatg gaaacatcaa 780
   aacaaagacc aagatatagt caagaagatc atccaagata ttgacctctg tgaaaacagc 840
   gtgcagcggc acattggaca tgctaacctc accttcgagc agcttcgtag cttgatggaa 900
   agcttaccgg gaaagaaagt gggagcagaa gacattgaaa aaacaataaa ggcaagcaaa 960
   cccagtgacc agatcctgaa gctgctcagt ttgtggcgaa taaaaaatgg cgaccaagac 1020
   accttgaagg gcctaatgca cgcactaaag cactcaaaga cgtaccactt tcccaaaact 1080
   gtcactcaga gtctaaagaa gaccatcagg ttccttcaca gcttcacaat gtacaaattg 1140
   tatcagaagt tatttttaga aatgataggt aaccaggtcc aatcagtaaa aataagctgc 1200
   ttataa 1206
<210> 87
   <211> 1206
   <212> DNA
   <213> Homo sapiens
<400> 87
   atgaacaact tgctgtgctg cgcgctcgtg tttctggaca tctccattaa gtggaccacc 60
   caggaaacgt ttcctccaaa gtaccttcat tatgacgaag aaacctctca tcagctgttg 120
   tgtgacaaat gtcctcctgg tacctaccta aaacaacact gtacagcaaa gtggaagacc 180
   gtgtgcgccc cttgccctga ccactactac acagacagct ggcacaccag tgacgagtgt 240
   ctatactgca gccccgtgtg caaggagctg cagtacgtca agcaggagtg caatcgcacc 300
   cacaaccgcg tgtgcgaatg caaggaaggg cgctaccttg agatagagtt ctgcttgaaa 360
   cataggagct gccctcctgg atttggagtg gtgcaagctg gaaccccaga gcgaaataca     420
   gtttgcaaaa gatgtccaga tgggttcttc tcaaatgaga cgtcatctaa agcaccctgt     480
   agaaaacaca caaattgcag tgtctttggt ctcctgctaa ctcagaaagg aaatgcaaca     540
   cacgacaaca tatgttccgg aaacagtgaa tcaactcaaa aatgtggaat agatgttacc     600
   ctgtgtgagg aggcattctt caggtttgct gttcctacaa agtttacgcc taactggctt     660
   agtgtcttgg tagacaattt gcctggcacc aaagtaaacg cagagagtgt agagaggata     720
   aaacggcaac acagctcaca agaacagact ttccagctgc tgaagttatg gaaacatcaa     780
   aacaaagacc aagatatagt caagaagatc atccaagata ttgacctctg tgaaaacagc     840
   gtgcagcggc acattggaca tgctaacctc accttcgagc agcttcgtag cttgatggaa     900
   agcttaccgg gaaagaaagt gggagcagaa gacattgaaa aaacaataaa ggcatgcaaa     960
   cccagtgacc agatcctgaa gctgctcagt ttgtggcgaa taaaaaatgg cgaccaagac    1020
   accttgaagg gcctaatgca cgcactaaag cactcaaaga cgtaccactt tcccaaaact    1080
   gtcactcaga gtctaaagaa gaccatcagg ttccttcaca gcttcacaat gtacaaattg    1140
   tatcagaagt tatttttaga aatgataggt aaccaggtcc aatcagtaaa aataagcagc    1200
   ttataa                                                               1206
<210> 88
   <211> 1083
   <212> DNA
   <213> Homo sapiens
<400> 88
   atgaacaact tgctgtgctg cgcgctcgtg tttctggaca tctccattaa gtggaccacc     60
   caggaacctt gccctgacca ctactacaca gacagctggc acaccagtga cgagtgtcta    120
   tactgcagcc ccgtgtgcaa ggagctgcag tacgtcaagc aggagtgcaa tcgcacccac    180
   aaccgcgtgt gcgaatgcaa ggaagggcgc taccttgaga tagagttctg cttgaaacat    240
   aggagctgcc ctcctggatt tggagtggtg caagctggaa ccccagagcg aaatacagtt    300
   tgcaaaagat gtccagatgg gttcttctca aatgagacgt catctaaagc accctgtaga    360
   aaacacacaa attgcagtgt ctttggtctc ctgctaactc agaaaggaaa tgcaacacac    420
   gacaacatat gttccggaaa cagtgaatca actcaaaaat gtggaataga tgttaccctg    480
   tgtgaggagg cattcttcag gtttgctgtt cctacaaagt ttacgcctaa ctggcttagt    540
   gtcttggtag acaatttgcc tggcaccaaa gtaaacgcag agagtgtaga gaggataaaa    600
   cggcaacaca gctcacaaga acagactttc cagctgctga agttatggaa acatcaaaac    660
   aaagaccaag atatagtcaa gaagatcatc caagatattg acctctgtga aaacagcgtg    720
   cagcggcaca ttggacatgc taacctcacc ttcgagcagc ttcgtagctt gatggaaagc    780
   ttaccgggaa agaaagtggg agcagaagac attgaaaaaa caataaaggc atgcaaaccc    840
   agtgaccaga tcctgaagct gctcagtttg tggcgaataa aaaatggcga ccaagacacc    900
   ttgaagggcc taatgcacgc actaaagcac tcaaagacgt accactttcc caaaactgtc    960
   actcagagtc taaagaagac catcaggttc cttcacagct tcacaatgta caaattgtat   1020
   cagaagttat ttttagaaat gataggtaac caggtccaat cagtaaaaat aagctgctta   1080
   taa                                                                 1083
<210> 89
   <211> 1080
   <212> DNA
   <213> Homo sapiens
<400> 89
   atgaacaact tgctgtgctg cgcgctcgtg tttctggaca tctccattaa gtggaccacc 60
   caggaaacgt ttcctccaaa gtaccttcat tatgacgaag aaacctctca tcagctgttg 120
   tgtgacaaat gtcctcctgg tacctaccta aaacaacact gtacagcaaa gtggaagacc 180
   gtgtgcgccg aatgcaagga agggcgctac cttgagatag agttctgctt gaaacatagg 240
   agctgccctc ctggatttgg agtggtgcaa gctggaaccc cagagcgaaa tacagtttgc 300
   aaaagatgtc cagatgggtt cttctcaaat gagacgtcat ctaaagcacc ctgtagaaaa 360
   cacacaaatt gcagtgtctt tggtctcctg ctaactcaga aaggaaatgc aacacacgac 420
   aacatatgtt ccggaaacag tgaatcaact caaaaatgtg gaatagatgt taccctgtgt 480
   gaggaggcat tcttcaggtt tgctgttcct acaaagttta cgcctaactg gcttagtgtc 540
   ttggtagaca atttgcctgg caccaaagta aacgcagaga gtgtagagag gataaaacgg 600
   caacacagct cacaagaaca gactttccag ctgctgaagt tatggaaaca tcaaaacaaa 660
   gaccaagata tagtcaagaa gatcatccaa gatattgacc tctgtgaaaa cagcgtgcag 720
   cggcacattg gacatgctaa cctcaccttc gagcagcttc gtagcttgat ggaaagctta 780
   ccgggaaaga aagtgggagc agaagacatt gaaaaaacaa taaaggcatg caaacccagt 840
   gaccagatcc tgaagctgct cagtttgtgg cgaataaaaa atggcgacca agacaccttg 900
   aagggcctaa tgcacgcact aaagcactca aagacgtacc actttcccaa aactgtcact 960
   cagagtctaa agaagaccat caggttcctt cacagcttca caatgtacaa attgtatcag 1020
   aagttatttt tagaaatgat aggtaaccag gtccaatcag taaaaataag ctgcttataa 1080
<210> 90
   <211> 1092
   <212> DNA
   <213> Homo sapiens
<400> 90
   atgaacaact tgctgtgctg cgcgctcgtg tttctggaca tctccattaa gtggaccacc 60
   caggaaacgt ttcctccaaa gtaccttcat tatgacgaag aaacctctca tcagctgttg 120
   tgtgacaaat gtcctcctgg tacctaccta aaacaacact gtacagcaaa gtggaagacc 180
   gtgtgcgccc cttgccctga ccactactac acagacagct ggcacaccag tgacgagtgt 240
   ctatactgca gccccgtgtg caaggagctg cagtacgtca agcaggagtg caatcgcacc 300
   cacaaccgcg tgtgcagatg tccagatggg ttcttctcaa atgagacgtc atctaaagca 360
   ccctgtagaa aacacacaaa ttgcagtgtc tttggtctcc tgctaactca gaaaggaaat     420
   gcaacacacg acaacatatg ttccggaaac agtgaatcaa ctcaaaaatg tggaatagat     480
   gttaccctgt gtgaggaggc attcttcagg tttgctgttc ctacaaagtt tacgcctaac     540
   tggcttagtg tcttggtaga caatttgcct ggcaccaaag taaacgcaga gagtgtagag     600
   aggataaaac ggcaacacag ctcacaagaa cagactttcc agctgctgaa gttatggaaa     660
   catcaaaaca aagaccaaga tatagtcaag aagatcatcc aagatattga cctctgtgaa     720
   aacagcgtgc agcggcacat tggacatgct aacctcacct tcgagcagct tcgtagcttg     780
   atggaaagct taccgggaaa gaaagtggga gcagaagaca ttgaaaaaac aataaaggca     840
   tgcaaaccca gtgaccagat cctgaagctg ctcagtttgt ggcgaataaa aaatggcgac     900
   caagacacct tgaagggcct aatgcacgca ctaaagcact caaagacgta ccactttccc     960
   aaaactgtca ctcagagtct aaagaagacc atcaggttcc ttcacagctt cacaatgtac    1020
   aaattgtatc agaagttatt tttagaaatg ataggtaacc aggtccaatc agtaaaaata    1080
   agctgcttat aa                                                        1092
<210> 91
   <211> 1080
   <212> DNA
   <213> Homo sapiens
<400> 91
   atgaacaact tgctgtgctg cgcgctcgtg tttctggaca tctccattaa gtggaccacc      60
   caggaaacgt ttcctccaaa gtaccttcat tatgacgaag aaacctctca tcagctgttg     120
   tgtgacaaat gtcctcctgg tacctaccta aaacaacact gtacagcaaa gtggaagacc     180
   gtgtgcgccc cttgccctga ccactactac acagacagct ggcacaccag tgacgagtgt     240
   ctatactgca gccccgtgtg caaggagctg cagtacgtca agcaggagtg caatcgcacc     300
   cacaaccgcg tgtgcgaatg caaggaaggg cgctaccttg agatagagtt ctgcttgaaa     360
   cataggagct gccctcctgg atttggagtg gtgcaagctg gaaccccaga gcgaaataca     420
   gtttgcaaat ccggaaacag tgaatcaact caaaaatgtg gaatagatgt taccctgtgt     480
   gaggaggcat tcttcaggtt tgctgttcct acaaagttta cgcctaactg gcttagtgtc     540
   ttggtagaca atttgcctgg caccaaagta aacgcagaga gtgtagagag gataaaacgg     600
   caacacagct cacaagaaca gactttccag ctgctgaagt tatggaaaca tcaaaacaaa     660
   gaccaagata tagtcaagaa gatcatccaa gatattgacc tctgtgaaaa cagcgtgcag     720
   cggcacattg gacatgctaa cctcaccttc gagcagcttc gtagcttgat ggaaagctta     780
   ccgggaaaga aagtgggagc agaagacatt gaaaaaacaa taaaggcatg caaacccagt     840
   gaccagatcc tgaagctgct cagtttgtgg cgaataaaaa atggcgacca agacaccttg     900
   aagggcctaa tgcacgcact aaagcactca aagacgtacc actttcccaa aactgtcact     960
   cagagtctaa agaagaccat caggttcctt cacagcttca caatgtacaa attgtatcag    1020
   aagttatttt tagaaatgat aggtaaccag gtccaatcag taaaaataag ctgcttataa    1080
<210> 92
   <211> 981
   <212> DNA
   <213> Homo sapiens
<400> 92
   atgaacaact tgctgtgctg cgcgctcgtg tttctggaca tctccattaa gtggaccacc      60
   caggaaacgt ttcctccaaa gtaccttcat tatgacgaag aaacctctca tcagctgttg     120
   tgtgacaaat gtcctcctgg tacctaccta aaacaacact gtacagcaaa gtggaagacc     180
   gtgtgcgccc cttgccctga ccactactac acagacagct ggcacaccag tgacgagtgt     240
   ctatactgca gccccgtgtg caaggagctg cagtacgtca agcaggagtg caatcgcacc     300
   cacaaccgcg tgtgcgaatg caaggaaggg cgctaccttg agatagagtt ctgcttgaaa     360
   cataggagct gccctcctgg atttggagtg gtgcaagctg gaaccccaga gcgaaataca     420
   gtttgcaaaa gatgtccaga tgggttcttc tcaaatgaga cgtcatctaa agcaccctgt     480
   agaaaacaca caaattgcag tgtctttggt ctcctgctaa ctcagaaagg aaatgcaaca     540
   cacgacaaca tatgttccgg aaacagtgaa tcaactcaaa aatgtggaat agatattgac     600
   ctctgtgaaa acagcgtgca gcggcacatt ggacatgcta acctcacctt cgagcagctt     660
   cgtagcttga tggaaagctt accgggaaag aaagtgggag cagaagacat tgaaaaaaca     720
   ataaaggcat gcaaacccag tgaccagatc ctgaagctgc tcagtttgtg gcgaataaaa     780
   aatggcgacc aagacacctt gaagggccta atgcacgcac taaagcactc aaagacgtac     840
   cactttccca aaactgtcac tcagagtcta aagaagacca tcaggttcct tcacagcttc     900
   acaatgtaca aattgtatca gaagttattt ttagaaatga taggtaacca ggtccaatca     960
   gtaaaaataa gctgcttata a                                               981
<210> 93
   <211> 984
   <212> DNA
   <213> Homo sapiens
<400> 93
   atgaacaact tgctgtgctg cgcgctcgtg tttctggaca tctccattaa gtggaccacc      60
   caggaaacgt ttcctccaaa gtaccttcat tatgacgaag aaacctctca tcagctgttg     120
   tgtgacaaat gtcctcctgg tacctaccta aaacaacact gtacagcaaa gtggaagacc     180
   gtgtgcgccc cttgccctga ccactactac acagacagct ggcacaccag tgacgagtgt     240
   ctatactgca gccccgtgtg caaggagctg cagtacgtca agcaggagtg caatcgcacc     300
   cacaaccgcg tgtgcgaatg caaggaaggg cgctaccttg agatagagtt ctgcttgaaa     360
   cataggagct gccctcctgg atttggagtg gtgcaagctg gaaccccaga gcgaaataca     420
   gtttgcaaaa gatgtccaga tgggttcttc tcaaatgaga cgtcatctaa agcaccctgt     480
   agaaaacaca caaattgcag tgtctttggt ctcctgctaa ctcagaaagg aaatgcaaca     540
   cacgacaaca tatgttccgg aaacagtgaa tcaactcaaa aatgtggaat agatgttacc 600
   ctgtgtgagg aggcattctt caggtttgct gttcctacaa agtttacgcc taactggctt 660
   agtgtcttgg tagacaattt gcctggcacc aaagtaaacg cagagagtgt agagaggata 720
   aaacggcaac acagctcaca agaacagact ttccagctgc tgaagttatg gaaacatcaa 780
   aacaaagacc aagatatagt caagaagatc atccaagacg cactaaagca ctcaaagacg 840
   taccactttc ccaaaactgt cactcagagt ctaaagaaga ccatcaggtt ccttcacagc 900
   ttcacaatgt acaaattgta tcagaagtta tttttagaaa tgataggtaa ccaggtccaa 960
   tcagtaaaaa taagctgctt ataa 984
<210> 94
   <211> 1200
   <212> DNA
   <213> Homo sapiens
<400> 94
   atgaacaact tgctgtgctg cgcgctcgtg tttctggaca tctccattaa gtggaccacc 60
   caggaaacgt ttcctccaaa gtaccttcat tatgacgaag aaacctctca tcagctgttg 120
   tgtgacaaat gtcctcctgg tacctaccta aaacaacact gtacagcaaa gtggaagacc 180
   gtgtgcgccc cttgccctga ccactactac acagacagct ggcacaccag tgacgagtgt 240
   ctatactgca gccccgtgtg caaggagctg cagtacgtca agcaggagtg caatcgcacc 300
   cacaaccgcg tgtgcgaatg caaggaaggg cgctaccttg agatagagtt ctgcttgaaa 360
   cataggagct gccctcctgg atttggagtg gtgcaagctg gaaccccaga gcgaaataca 420
   gtttgcaaaa gatgtccaga tgggttcttc tcaaatgaga cgtcatctaa agcaccctgt 480
   agaaaacaca caaattgcag tgtctttggt ctcctgctaa ctcagaaagg aaatgcaaca 540
   cacgacaaca tatgttccgg aaacagtgaa tcaactcaaa aatgtggaat agatgttacc 600
   ctgtgtgagg aggcattctt caggtttgct gttcctacaa agtttacgcc taactggctt 660
   agtgtcttgg tagacaattt gcctggcacc aaagtaaacg cagagagtgt agagaggata 720
   aaacggcaac acagctcaca agaacagact ttccagctgc tgaagttatg gaaacatcaa 780
   aacaaagacc aagatatagt caagaagatc atccaagata ttgacctctg tgaaaacagc 840
   gtgcagcggc acattggaca tgctaacctc accttcgagc agcttcgtag cttgatggaa 900
   agcttaccgg gaaagaaagt gggagcagaa gacattgaaa aaacaataaa ggcatgcaaa 960
   cccagtgacc agatcctgaa gctgctcagt ttgtggcgaa taaaaaatgg cgaccaagac 1020
   accttgaagg gcctaatgca cgcactaaag cactcaaaga cgtaccactt tcccaaaact 1080
   gtcactcaga gtctaaagaa gaccatcagg ttccttcaca gcttcacaat gtacaaattg 1140
   tatcagaagt tatttttaga aatgataggt aaccaggtcc aatcagtaaa aataagctaa 1200
<210> 95
   <211> 1056
   <212> DNA
   <213> Homo sapiens
<400> 95
   atgaacaact tgctgtgctg cgcgctcgtg tttctggaca tctccattaa gtggaccacc      60
   caggaaacgt ttcctccaaa gtaccttcat tatgacgaag aaacctctca tcagctgttg     120
   tgtgacaaat gtcctcctgg tacctaccta aaacaacact gtacagcaaa gtggaagacc     180
   gtgtgcgccc cttgccctga ccactactac acagacagct ggcacaccag tgacgagtgt     240
   ctatactgca gccccgtgtg caaggagctg cagtacgtca agcaggagtg caatcgcacc     300
   cacaaccgcg tgtgcgaatg caaggaaggg cgctaccttg agatagagtt ctgcttgaaa     360
   cataggagct gccctcctgg atttggagtg gtgcaagctg gaaccccaga gcgaaataca     420
   gtttgcaaaa gatgtccaga tgggttcttc tcaaatgaga cgtcatctaa agcaccctgt     480
   agaaaacaca caaattgcag tgtctttggt ctcctgctaa ctcagaaagg aaatgcaaca     540
   cacgacaaca tatgttccgg aaacagtgaa tcaactcaaa aatgtggaat agatgttacc     600
   ctgtgtgagg aggcattctt caggtttgct gttcctacaa agtttacgcc taactggctt     660
   agtgtcttgg tagacaattt gcctggcacc aaagtaaacg cagagagtgt agagaggata     720
   aaacggcaac acagctcaca agaacagact ttccagctgc tgaagttatg gaaacatcaa     780
   aacaaagacc aagatatagt caagaagatc atccaagata ttgacctctg tgaaaacagc     840
   gtgcagcggc acattggaca tgctaacctc accttcgagc agcttcgtag cttgatggaa     900
   agcttaccgg gaaagaaagt gggagcagaa gacattgaaa aaacaataaa ggcatgcaaa     960
   cccagtgacc agatcctgaa gctgctcagt ttgtggcgaa taaaaaatgg cgaccaagac    1020
   accttgaagg gcctaatgca cgcactaaag cactga                              1056
<210> 96
   <211> 819
   <212> DNA
   <213> Homo sapiens
<400> 96
   atgaacaact tgctgtgctg cgcgctcgtg tttctggaca tctccattaa gtggaccacc 60
   caggaaacgt ttcctccaaa gtaccttcat tatgacgaag aaacctctca tcagctgttg 120
   tgtgacaaat gtcctcctgg tacctaccta aaacaacact gtacagcaaa gtggaagacc 180
   gtgtgcgccc cttgccctga ccactactac acagacagct ggcacaccag tgacgagtgt 240
   ctatactgca gccccgtgtg caaggagctg cagtacgtca agcaggagtg caatcgcacc 300
   cacaaccgcg tgtgcgaatg caaggaaggg cgctaccttg agatagagtt ctgcttgaaa 360
   cataggagct gccctcctgg atttggagtg gtgcaagctg gaaccccaga gcgaaataca 420
   gtttgcaaaa gatgtccaga tgggttcttc tcaaatgaga cgtcatctaa agcaccctgt 480
   agaaaacaca caaattgcag tgtctttggt ctcctgctaa ctcagaaagg aaatgcaaca 540
   cacgacaaca tatgttccgg aaacagtgaa tcaactcaaa aatgtggaat agatgttacc 600
   ctgtgtgagg aggcattctt caggtttgct gttcctacaa agtttacgcc taactggctt 660
   agtgtcttgg tagacaattt gcctggcacc aaagtaaacg cagagagtgt agagaggata 720
   aaacggcaac acagctcaca agaacagact ttccagctgc tgaagttatg gaaacatcaa 780
   aacaaagacc aagatatagt caagaagatc atccaatga 819
<210> 97
   <211> 594
   <212> DNA
   <213> Homo sapiens
<400> 97
   atgaacaact tgctgtgctg cgcgctcgtg tttctggaca tctccattaa gtggaccacc 60
   caggaaacgt ttcctccaaa gtaccttcat tatgacgaag aaacctctca tcagctgttg 120
   tgtgacaaat gtcctcctgg tacctaccta aaacaacact gtacagcaaa gtggaagacc 180
   gtgtgcgccc cttgccctga ccactactac acagacagct ggcacaccag tgacgagtgt 240
   ctatactgca gccccgtgtg caaggagctg cagtacgtca agcaggagtg caatcgcacc 300
   cacaaccgcg tgtgcgaatg caaggaaggg cgctaccttg agatagagtt ctgcttgaaa 360
   cataggagct gccctcctgg atttggagtg gtgcaagctg gaaccccaga gcgaaataca 420
   gtttgcaaaa gatgtccaga tgggttcttc tcaaatgaga cgtcatctaa agcaccctgt 480
   agaaaacaca caaattgcag tgtctttggt ctcctgctaa ctcagaaagg aaatgcaaca 540
   cacgacaaca tatgttccgg aaacagtgaa tcaactcaaa aatgtggaat atga 594
<210> 98
   <211> 432
   <212> DNA
   <213> Homo sapiens
<400> 98
   atgaacaact tgctgtgctg cgcgctcgtg tttctggaca tctccattaa gtggaccacc     60
   caggaaacgt ttcctccaaa gtaccttcat tatgacgaag aaacctctca tcagctgttg    120
   tgtgacaaat gtcctcctgg tacctaccta aaacaacact gtacagcaaa gtggaagacc    180
   gtgtgcgccc cttgccctga ccactactac acagacagct ggcacaccag tgacgagtgt    240
   ctatactgca gccccgtgtg caaggagctg cagtacgtca agcaggagtg caatcgcacc    300
   cacaaccgcg tgtgcgaatg caaggaaggg cgctaccttg agatagagtt ctgcttgaaa    360
   cataggagct gccctcctgg atttggagtg gtgcaagctg gaaccccaga gcgaaataca    420
   gtttgcaaat ga                                                         432
<210> 99
   <211> 321
   <212> DNA
   <213> Homo sapiens
<400> 99
   atgaacaact tgctgtgctg cgcgctcgtg tttctggaca tctccattaa gtggaccacc     60
   caggaaacgt ttcctccaaa gtaccttcat tatgacgaag aaacctctca tcagctgttg    120
   tgtgacaaat gtcctcctgg tacctaccta aaacaacact gtacagcaaa gtggaagacc    180
   gtgtgcgccc cttgccctga ccactactac acagacagct ggcacaccag tgacgagtgt    240
   ctatactgca gccccgtgtg caaggagctg cagtacgtca agcaggagtg caatcgcacc    300
   cacaaccgcg tgtgcgaatg a                                              321
<210> 100
   <211> 1182
   <212> DNA
   <213> Homo sapiens
<400> 100
   atgaacaact tgctgtgctg cgcgctcgtg tttctggaca tctccattaa gtggaccacc      60
   caggaaacgt ttcctccaaa gtaccttcat tatgacgaag aaacctctca tcagctgttg     120
   tgtgacaaat gtcctcctgg tacctaccta aaacaacact gtacagcaaa gtggaagacc     180
   gtgtgcgccc cttgccctga ccactactac acagacagct ggcacaccag tgacgagtgt     240
   ctatactgca gccccgtgtg caaggagctg cagtacgtca agcaggagtg caatcgcacc     300
   cacaaccgcg tgtgcgaatg caaggaaggg cgctaccttg agatagagtt ctgcttgaaa     360
   cataggagct gccctcctgg atttggagtg gtgcaagctg gaaccccaga gcgaaataca     420
   gtttgcaaaa gatgtccaga tgggttcttc tcaaatgaga cgtcatctaa agcaccctgt     480
   agaaaacaca caaattgcag tgtctttggt ctcctgctaa ctcagaaagg aaatgcaaca     540
   cacgacaaca tatgttccgg aaacagtgaa tcaactcaaa aatgtggaat agatgttacc     600
   ctgtgtgagg aggcattctt caggtttgct gttcctacaa agtttacgcc taactggctt     660
   agtgtcttgg tagacaattt gcctggcacc aaagtaaacg cagagagtgt agagaggata     720
   aaacggcaac acagctcaca agaacagact ttccagctgc tgaagttatg gaaacatcaa     780
   aacaaagacc aagatatagt caagaagatc atccaagata ttgacctctg tgaaaacagc     840
   gtgcagcggc acattggaca tgctaacctc accttcgagc agcttcgtag cttgatggaa     900
   agcttaccgg gaaagaaagt gggagcagaa gacattgaaa aaacaataaa ggcatgcaaa     960
   cccagtgacc agatcctgaa gctgctcagt ttgtggcgaa taaaaaatgg cgaccaagac    1020
   accttgaagg gcctaatgca cgcactaaag cactcaaaga cgtaccactt tcccaaaact    1080
   gtcactcaga gtctaaagaa gaccatcagg ttccttcaca gcttcacaat gtacaaattg    1140
   tatcagaagt tatttttaga aatgataggt aacctagtct ag                       1182
<210> 101
   <211> 966
   <212> DNA
   <213> Homo sapiens
<400> 101
   atgaacaact tgctgtgctg cgcgctcgtg tttctggaca tctccattaa gtggaccacc      60
   caggaaacgt ttcctccaaa gtaccttcat tatgacgaag aaacctctca tcagctgttg     120
   tgtgacaaat gtcctcctgg tacctaccta aaacaacact gtacagcaaa gtggaagacc     180
   gtgtgcgccc cttgccctga ccactactac acagacagct ggcacaccag tgacgagtgt     240
   ctatactgca gccccgtgtg caaggagctg cagtacgtca agcaggagtg caatcgcacc 300
   cacaaccgcg tgtgcgaatg caaggaaggg cgctaccttg agatagagtt ctgcttgaaa 360
   cataggagct gccctcctgg atttggagtg gtgcaagctg gaaccccaga gcgaaataca 420
   gtttgcaaaa gatgtccaga tgggttcttc tcaaatgaga cgtcatctaa agcaccctgt 480
   agaaaacaca caaattgcag tgtctttggt ctcctgctaa ctcagaaagg aaatgcaaca 540
   cacgacaaca tatgttccgg aaacagtgaa tcaactcaaa aatgtggaat agatgttacc 600
   ctgtgtgagg aggcattctt caggtttgct gttcctacaa agtttacgcc taactggctt 660
   agtgtcttgg tagacaattt gcctggcacc aaagtaaacg cagagagtgt agagaggata 720
   aaacggcaac acagctcaca agaacagact ttccagctgc tgaagttatg gaaacatcaa 780
   aacaaagacc aagatatagt caagaagatc atccaagata ttgacctctg tgaaaacagc 840
   gtgcagcggc acattggaca tgctaacctc accttcgagc agcttcgtag cttgatggaa 900
   agcttaccgg gaaagaaagt gggagcagaa gacattgaaa aaacaataaa ggctagtcta 960
   gactag 966
<210> 102
   <211> 564
   <212> DNA
   <213> Homo sapiens
<400> 102
   atgaacaact tgctgtgctg cgcgctcgtg tttctggaca tctccattaa gtggaccacc 60
   caggaaacgt ttcctccaaa gtaccttcat tatgacgaag aaacctctca tcagctgttg 120
   tgtgacaaat gtcctcctgg tacctaccta aaacaacact gtacagcaaa gtggaagacc 180
   gtgtgcgccc cttgccctga ccactactac acagacagct ggcacaccag tgacgagtgt 240
   ctatactgca gccccgtgtg caaggagctg cagtacgtca agcaggagtg caatcgcacc 300
   cacaaccgcg tgtgcgaatg caaggaaggg cgctaccttg agatagagtt ctgcttgaaa 360
   cataggagct gccctcctgg atttggagtg gtgcaagctg gaaccccaga gcgaaataca 420
   gtttgcaaaa gatgtccaga tgggttcttc tcaaatgaga cgtcatctaa agcaccctgt 480
   agaaaacaca caaattgcag tgtctttggt ctcctgctaa ctcagaaagg aaatgcaaca 540
   cacgacaaca tatgttccgg ctag 564
<210> 103
   <211> 255
   <212> DNA
   <213> Homo sapiens
<400> 103
   atgaacaact tgctgtgctg cgcgctcgtg tttctggaca tctccattaa gtggaccacc 60
   caggaaacgt ttcctccaaa gtaccttcat tatgacgaag aaacctctca tcagctgttg 120
   tgtgacaaat gtcctcctgg tacctaccta aaacaacact gtacagcaaa gtggaagacc 180
   gtgtgcgccc cttgccctga ccactactac acagacagct ggcacaccag tgacgagtgt 240
   ctatacctag tctag 255
<210> 104
   <211> 1317
   <212> DNA
   <213> Homo sapiens
<400> 104
   ctggagacat ataacttgaa cacttggccc tgatggggaa gcagctctgc agggactttt 60
   tcagccatct gtaaacaatt tcagtggcaa cccgcgaact gtaatccatg aatgggacca 120
   cactttacaa gtcatcaagt ctaacttcta gaccagggaa ttaatggggg agacagcgaa 180
   ccctagagca aagtgccaaa cttctgtcga tagcttgagg ctagtggaaa gacctcgagg 240
   aggctactcc agaagttcag cgcgtaggaa gctccgatac caatagccct ttgatgatgg 300
   tggggttggt gaagggaaca gtgctccgca aggttatccc tgccccaggc agtccaattt 360
   tcactctgca gattctctct ggctctaact accccagata acaaggagtg aatgcagaat 420
   agcacgggct ttagggccaa tcagacatta gttagaaaaa ttcctactac atggtttatg 480
   taaacttgaa gatgaatgat tgcgaactcc ccgaaaaggg ctcagacaat gccatgcata 540
   aagaggggcc ctgtaatttg aggtttcaga acccgaagtg aaggggtcag gcagccgggt 600
   acggcggaaa ctcacagctt tcgcccagcg agaggacaaa ggtctgggac acactccaac 660
   tgcgtccgga tcttggctgg atcggactct cagggtggag gagacacaag cacagcagct 720
   gcccagcgtg tgcccagccc tcccaccgct ggtcccggct gccaggaggc tggccgctgg 780
   cgggaagggg ccgggaaacc tcagagcccc gcggagacag cagccgcctt gttcctcagc 840
   ccggtggctt ttttttcccc tgctctccca ggggacagac accaccgccc cacccctcac 900
   gccccacctc cctgggggat cctttccgcc ccagccctga aagcgttaat cctggagctt 960
   tctgcacacc ccccgaccgc tcccgcccaa gcttcctaaa aaagaaaggt gcaaagtttg 1020
   gtccaggata gaaaaatgac tgatcaaagg caggcgatac ttcctgttgc cgggacgcta 1080
   tatataacgt gatgagcgca cgggctgcgg agacgcaccg gagcgctcgc ccagccgccg 1140
   cctccaagcc cctgaggttt ccggggacca caatgaacaa gttgctgtgc tgcgcgctcg 1200
   tggtaagtcc ctgggccagc cgacgggtgc ccggcgcctg gggaggctgc tgccacctgg 1260
   tctcccaacc tcccagcgga ccggcgggga aaaaggctcc actcgctccc tcccaag 1317
<210> 105
   <211> 10190
   <212> DNA
   <213> Homo sapiens
<400> 105
   gcttactttg tgccaaatct cattaggctt aaggtaatac aggactttga gtcaaatgat      60
   actgttgcac ataagaacaa acctattttc atgctaagat gatgccactg tgttcctttc     120
   tccttctagt ttctggacat ctccattaag tggaccaccc aggaaacgtt tcctccaaag     180
   taccttcatt atgacgaaga aacctctcat cagctgttgt gtgacaaatg tcctcctggt     240
   acctacctaa aacaacactg tacagcaaag tggaagaccg tgtgcgcccc ttgccctgac 300
   cactactaca cagacagctg gcacaccagt gacgagtgtc tatactgcag ccccgtgtgc 360
   aaggagctgc agtacgtcaa gcaggagtgc aatcgcaccc acaaccgcgt gtgcgaatgc 420
   aaggaagggc gctaccttga gatagagttc tgcttgaaac ataggagctg ccctcctgga 480
   tttggagtgg tgcaagctgg tacgtgtcaa tgtgcagcaa aattaattag gatcatgcaa 540
   agtcagatag ttgtgacagt ttaggagaac acttttgttc tgatgacatt ataggatagc 600
   aaattgcaaa ggtaatgaaa cctgccaggt aggtactatg tgtctggagt gcttccaaag 660
   gaccattgct cagaggaata ctttgccact acagggcaat ttaatgacaa atctcaaatg 720
   cagcaaatta ttctctcatg agatgcatga tggttttttt tttttttttt aaagaaacaa 780
   actcaagttg cactattgat agttgatcta tacctctata tttcacttca gcatggacac 840
   cttcaaactg cagcactttt tgacaaacat cagaaatgtt aatttatacc aagagagtaa 900
   ttatgctcat attaatgaga ctctggagtg ctaacaataa gcagttataa ttaattatgt 960
   aaaaaatgag aatggtgagg ggaattgcat ttcattatta aaaacaaggc tagttcttcc 1020
   tttagcatgg gagctgagtg tttgggaggg taaggactat agcagaatct cttcaatgag 1080
   cttattcttt atcttagaca aaacagattg tcaagccaag agcaagcact tgcctataaa 1140
   ccaagtgctt tctcttttgc attttgaaca gcattggtca gggctcatgt gtattgaatc 1200
   ttttaaacca gtaacccacg ttttttttct gccacatttg cgaagcttca gtgcagccta 1260
   taacttttca tagcttgaga aaattaagag tatccactta cttagatgga agaagtaatc 1320
   agtatagatt ctgatgactc agtttgaagc agtgtttctc aactgaagcc ctgctgatat 1380
   tttaagaaat atctggattc ctaggctgga ctcctttttg tgggcagctg tcctgcgcat 1440
   tgtagaattt tggcagcacc cctggactct agccactaga taccaatagc agtccttccc 1500
   ccatgtgaca gccaaaaatg tcttcagaca ctgtcaaatg tcgccaggtg gcaaaatcac 1560
   tcctggttga gaacagggtc atcaatgcta agtatctgta actattttaa ctctcaaaac 1620
   ttgtgatata caaagtctaa attattagac gaccaatact ttaggtttaa aggcatacaa 1680
   atgaaacatt caaaaatcaa aatctattct gtttctcaaa tagtgaatct tataaaatta 1740
   atcacagaag atgcaaattg catcagagtc ccttaaaatt cctcttcgta tgagtatttg 1800
   agggaggaat tggtgatagt tcctactttc tattggatgg tactttgaga ctcaaaagct 1860
   aagctaagtt gtgtgtgtgt cagggtgcgg ggtgtggaat cccatcagat aaaagcaaat 1920
   ccatgtaatt cattcagtaa gttgtatatg tagaaaaatg aaaagtgggc tatgcagctt 1980
   ggaaactaga gaattttgaa aaataatgga aatcacaagg atctttctta aataagtaag 2040
   aaaatctgtt tgtagaatga agcaagcagg cagccagaag actcagaaca aaagtacaca 2100
   ttttactctg tgtacactgg cagcacagtg ggatttattt acctctccct ccctaaaaac 2160
   ccacacagcg gttcctcttg ggaaataaga ggtttccagc ccaaagagaa ggaaagacta 2220
   tgtggtgtta ctctaaaaag tatttaataa ccgttttgtt gttgctgttg ctgttttgaa 2280
   atcagattgt ctcctctcca tattttattt acttcattct gttaattcct gtggaattac 2340
   ttagagcaag catggtgaat tctcaactgt aaagccaaat ttctccatca ttataatttc 2400
   acattttgcc tggcaggtta taatttttat atttccactg atagtaataa ggtaaaatca 2460
   ttacttagat ggatagatct ttttcataaa aagtaccatc agttatagag ggaagtcatg 2520
   ttcatgttca ggaaggtcat tagataaagc ttctgaatat attatgaaac attagttctg 2580
   tcattcttag attctttttg ttaaataact ttaaaagcta acttacctaa aagaaatatc 2640
   tgacacatat gaacttctca ttaggatgca ggagaagacc caagccacag atatgtatct 2700
   gaagaatgaa caagattctt aggcccggca cggtggctca catctgtaat ctcaagagtt 2760
   tgagaggtca aggcgggcag atcacctgag gtcaggagtt caagaccagc ctggccaaca 2820
   tgatgaaacc ctgcctctac taaaaataca aaaattagca gggcatggtg gtgcatgcct 2880
   gcaaccctag ctactcagga ggctgagaca ggagaatctc ttgaaccctc gaggcggagg 2940
   ttgtggtgag ctgagatccc tctactgcac tccagcctgg gtgacagaga tgagactccg 3000
   tccctgccgc cgcccccgcc ttccccccca aaaagattct tcttcatgca gaacatacgg 3060
   cagtcaacaa agggagacct gggtccaggt gtccaagtca cttatttcga gtaaattagc 3120
   aatgaaagaa tgccatggaa tccctgccca aatacctctg cttatgatat tgtagaattt 3180
   gatatagagt tgtatcccat ttaaggagta ggatgtagta ggaaagtact aaaaacaaac 3240
   acacaaacag aaaaccctct ttgctttgta aggtggttcc taagataatg tcagtgcaat 3300
   gctggaaata atatttaata tgtgaaggtt ttaggctgtg ttttcccctc ctgttctttt 3360
   tttctgccag ccctttgtca tttttgcagg tcaatgaatc atgtagaaag agacaggaga 3420
   tgaaactaga accagtccat tttgcccctt tttttatttt ctggttttgg taaaagatac 3480
   aatgaggtag gaggttgaga tttataaatg aagtttaata agtttctgta gctttgattt 3540
   ttctctttca tatttgttat cttgcataag ccagaattgg cctgtaaaat ctacatatgg 3600
   atattgaagt ctaaatctgt tcaactagct tacactagat ggagatattt tcatattcag 3660
   atacactgga atgtatgatc tagccatgcg taatatagtc aagtgtttga aggtatttat 3720
   ttttaatagc gtctttagtt gtggactggt tcaagttttt ctgccaatga tttcttcaaa 3780
   tttatcaaat atttttccat catgaagtaa aatgcccttg cagtcaccct tcctgaagtt 3840
   tgaacgactc tgctgtttta aacagtttaa gcaaatggta tatcatcttc cgtttactat 3900
   gtagcttaac tgcaggctta cgcttttgag tcagcggcca actttattgc caccttcaaa 3960
   agtttattat aatgttgtaa atttttactt ctcaaggtta gcatacttag gagttgcttc 4020
   acaattagga ttcaggaaag aaagaacttc agtaggaact gattggaatt taatgatgca 4080
   gcattcaatg ggtactaatt tcaaagaatg atattacagc agacacacag cagttatctt 4140
   gattttctag gaataattgt atgaagaata tggctgacaa cacggcctta ctgccactca 4200
   gcggaggctg gactaatgaa caccctaccc ttctttcctt tcctctcaca tttcatgagc 4260
   gttttgtagg taacgagaaa attgacttgc atttgcatta caaggaggag aaactggcaa 4320
   aggggatgat ggtggaagtt ttgttctgtc taatgaagtg aaaaatgaaa atgctagagt 4380
   tttgtgcaac ataatagtag cagtaaaaac caagtgaaaa gtctttccaa aactgtgtta 4440
   agagggcatc tgctgggaaa cgatttgagg agaaggtact aaattgcttg gtattttccg 4500
   taggaacccc agagcgaaat acagtttgca aaagatgtcc agatgggttc ttctcaaatg 4560
   agacgtcatc taaagcaccc tgtagaaaac acacaaattg cagtgtcttt ggtctcctgc 4620
   taactcagaa aggaaatgca acacacgaca acatatgttc cggaaacagt gaatcaactc 4680
   aaaaatgtgg aataggtaat tacattccaa aatacgtctt tgtacgattt tgtagtatca 4740
   tctctctctc tgagttgaac acaaggcctc cagccacatt cttggtcaaa cttacatttt 4800
   ccctttcttg aatcttaacc agctaaggct actctcgatg cattactgct aaagctacca 4860
   ctcagaatct ctcaaaaact catcttctca cagataacac ctcaaagctt gattttctct 4920
   cctttcacac tgaaatcaaa tcttgcccat aggcaaaggg cagtgtcaag tttgccactg 4980
   agatgaaatt aggagagtcc aaactgtaga attcacgttg tgtgttatta ctttcacgaa 5040
   tgtctgtatt attaactaaa gtatatattg gcaactaaga agcaaagtga tataaacatg 5100
   atgacaaatt aggccaggca tggtggctta ctcctataat cccaacattt tggggggcca 5160
   aggtaggcag atcacttgag gtcaggattt caagaccagc ctgaccaaca tggtgaaacc 5220
   ttgtctctac taaaaataca aaaattagct gggcatggta gcaggcactt ctagtaccag 5280
   ctactcaggg ctgaggcagg agaatcgctt gaacccagga gatggaggtt gcagtgagct 5340
   gagattgtac cactgcactc cagtctgggc aacagagcaa gatttcatca cacacacaca 5400
   cacacacaca cacacacaca ttagaaatgt gtacttggct ttgttaccta tggtattagt 5460
   gcatctattg catggaactt ccaagctact ctggttgtgt taagctcttc attgggtaca 5520
   ggtcactagt attaagttca ggttattcgg atgcattcca cggtagtgat gacaattcat 5580
   caggctagtg tgtgtgttca ccttgtcact cccaccacta gactaatctc agaccttcac 5640
   tcaaagacac attacactaa agatgatttg cttttttgtg tttaatcaag caatggtata 5700
   aaccagcttg actctcccca aacagttttt cgtactacaa agaagtttat gaagcagaga 5760
   aatgtgaatt gatatatata tgagattcta acccagttcc agcattgttt cattgtgtaa 5820
   ttgaaatcat agacaagcca ttttagcctt tgctttctta tctaaaaaaa aaaaaaaaaa 5880
   aatgaaggaa ggggtattaa aaggagtgat caaattttaa cattctcttt aattaattca 5940
   tttttaattt tacttttttt catttattgt gcacttacta tgtggtactg tgctatagag 6000
   gctttaacat ttataaaaac actgtgaaag ttgcttcaga tgaatatagg tagtagaacg 6060
   gcagaactag tattcaaagc caggtctgat gaatccaaaa acaaacaccc attactccca 6120
   ttttctggga catacttact ctacccagat gctctgggct ttgtaatgcc tatgtaaata 6180
   acatagtttt atgtttggtt attttcctat gtaatgtcta cttatatatc tgtatctatc 6240
   tcttgctttg tttccaaagg taaactatgt gtctaaatgt gggcaaaaaa taacacacta 6300
   ttccaaatta ctgttcaaat tcctttaagt cagtgataat tatttgtttt gacattaatc 6360
   atgaagttcc ctgtgggtac taggtaaacc tttaatagaa tgttaatgtt tgtattcatt 6420
   ataagaattt ttggctgtta cttatttaca acaatatttc actctaatta gacatttact 6480
   aaactttctc ttgaaaacaa tgcccaaaaa agaacattag aagacacgta agctcagttg 6540
   gtctctgcca ctaagaccag ccaacagaag cttgatttta ttcaaacttt gcattttagc 6600
   atattttatc ttggaaaatt caattgtgtt ggttttttgt ttttgtttgt attgaataga 6660
   ctctcagaaa tccaattgtt gagtaaatct tctgggtttt ctaacctttc tttagatgtt 6720
   accctgtgtg aggaggcatt cttcaggttt gctgttccta caaagtttac gcctaactgg 6780
   cttagtgtct tggtagacaa tttgcctggc accaaagtaa acgcagagag tgtagagagg 6840
   ataaaacggc aacacagctc acaagaacag actttccagc tgctgaagtt atggaaacat 6900
   caaaacaaag accaagatat agtcaagaag atcatccaag gtaattacat tccaaaatac 6960
   gtctttgtac gattttgtag tatcatctct ctctctgagt tgaacacaag gcctccagcc 7020
   acattcttgg tcaaacttac attttccctt tcttgaatct taaccagcta aggctactct 7080
   cgatgcatta ctgctaaagc taccactcag aatctctcaa aaactcatct tctcacagat 7140
   aacacctcaa agcttgattt tctctccttt cacactgaaa tcaaatcttg cccataggca 7200
   aagggcagtg tcaagtttgc cactgagatg aaattaggag agtccaaact gtagaattca 7260
   cgttgtgtgt tattactttc acgaatgtct gtattattaa ctaaagtata tattggcaac 7320
   taagaagcaa agtgatataa acatgatgac aaattaggcc aggcatggtg gcttactcct 7380
   ataatcccaa cattttgggg ggccaaggta ggcagatcac ttgaggtcag gatttcaaga 7440
   ccagcctgac caacatggtg aaaccttgtc tctactaaaa atacaaaaat tagctgggca 7500
   tggtagcagg cacttctagt accagctact cagggctgag gcaggagaat cgcttgaacc 7560
   caggagatgg aggttgcagt gagctgagat tgtaccactg cactccagtc tgggcaacag 7620
   agcaagattt catcacacac acacacacac acacacacac acacattaga aatgtgtact 7680
   tggctttgtt acctatggta ttagtgcatc tattgcatgg aacttccaag ctactctggt 7740
   tgtgttaagc tcttcattgg gtacaggtca ctagtattaa gttcaggtta ttcggatgca 7800
   ttccacggta gtgatgacaa ttcatcaggc tagtgtgtgt gttcaccttg tcactcccac 7860
   cactagacta atctcagacc ttcactcaaa gacacattac actaaagatg atttgctttt 7920
   ttgtgtttaa tcaagcaatg gtataaacca gcttgactct ccccaaacag tttttcgtac 7980
   tacaaagaag tttatgaagc agagaaatgt gaattgatat atatatgaga ttctaaccca 8040
   gttccagcat tgtttcattg tgtaattgaa atcatagaca agccatttta gcctttgctt 8100
   tcttatctaa aaaaaaaaaa aaaaaaatga aggaaggggt attaaaagga gtgatcaaat 8160
   tttaacattc tctttaatta attcattttt aattttactt tttttcattt attgtgcact 8220
   tactatgtgg tactgtgcta tagaggcttt aacatttata aaaacactgt gaaagttgct 8280
   tcagatgaat ataggtagta gaacggcaga actagtattc aaagccaggt ctgatgaatc 8340
   caaaaacaaa cacccattac tcccattttc tgggacatac ttactctacc cagatgctct 8400
   gggctttgta atgcctatgt aaataacata gttttatgtt tggttatttt cctatgtaat 8460
   gtctacttat atatctgtat ctatctcttg ctttgtttcc aaaggtaaac tatgtgtcta 8520
   aatgtgggca aaaaataaca cactattcca aattactgtt caaattcctt taagtcagtg 8580
   ataattattt gttttgacat taatcatgaa gttccctgtg ggtactaggt aaacctttaa 8640
   tagaatgtta atgtttgtat tcattataag aatttttggc tgttacttat ttacaacaat 8700
   atttcactct aattagacat ttactaaact ttctcttgaa aacaatgccc aaaaaagaac 8760
   attagaagac acgtaagctc agttggtctc tgccactaag accagccaac agaagcttga 8820
   ttttattcaa actttgcatt ttagcatatt ttatcttgga aaattcaatt gtgttggttt 8880
   tttgtttttg tttgtattga atagactctc agaaatccaa ttgttgagta aatcttctgg 8940
   gttttctaac ctttctttag atattgacct ctgtgaaaac agcgtgcagc ggcacattgg 9000
   acatgctaac ctcaccttcg agcagcttcg tagcttgatg gaaagcttac cgggaaagaa 9060
   agtgggagca gaagacattg aaaaaacaat aaaggcatgc aaacccagtg accagatcct 9120
   gaagctgctc agtttgtggc gaataaaaaa tggcgaccaa gacaccttga agggcctaat 9180
   gcacgcacta aagcactcaa agacgtacca ctttcccaaa actgtcactc agagtctaaa 9240
   gaagaccatc aggttccttc acagcttcac aatgtacaaa ttgtatcaga agttattttt 9300
   agaaatgata ggtaaccagg tccaatcagt aaaaataagc tgcttataac tggaaatggc 9360
   cattgagctg tttcctcaca attggcgaga tcccatggat gagtaaactg tttctcaggc 9420
   acttgaggct ttcagtgata tctttctcat taccagtgac taattttgcc acagggtact 9480
   aaaagaaact atgatgtgga gaaaggacta acatctcctc caataaaccc caaatggtta 9540
   atccaactgt cagatctgga tcgttatcta ctgactatat tttcccttat tactgcttgc 9600
   agtaattcaa ctggaaatta aaaaaaaaaa actagactcc actgggcctt actaaatatg 9660
   ggaatgtcta acttaaatag ctttgggatt ccagctatgc tagaggcttt tattagaaag 9720
   ccatattttt ttctgtaaaa gttactaata tatctgtaac actattacag tattgctatt 9780
   tatattcatt cagatataag atttggacat attatcatcc tataaagaaa cggtatgact 9840
   taattttaga aagaaaatta tattctgttt attatgacaa atgaaagaga aaatatatat 9900
   ttttaatgga aagtttgtag catttttcta ataggtactg ccatattttt ctgtgtggag 9960
   tatttttata attttatctg tataagctgt aatatcattt tatagaaaat gcattattta 10020
   gtcaattgtt taatgttgga aaacatatga aatataaatt atctgaatat tagatgctct 10080
   gagaaattga atgtacctta tttaaaagat tttatggttt tataactata taaatgacat 10140
   tattaaagtt ttcaaattat tttttattgc tttctctgtt gcttttattt 10190